(19) Europäisches Patentamt
European Patent Office
Office européen des brevets

(11) EP 4 101 852 A1

(12) EUROPEAN PATENT APPLICATION
published in accordance with Art. 153(4) EPC

(43) Date of publication:
**14.12.2022 Bulletin 2022/50**

(21) Application number: **20917977.9**

(22) Date of filing: **16.12.2020**

(51) International Patent Classification (IPC):
***C07D 471/04*** *(2006.01)* ***A61K 31/519*** *(2006.01)*
***A61P 35/00*** *(2006.01)*

(86) International application number:
**PCT/CN2020/136848**

(87) International publication number:
**WO 2021/155716 (12.08.2021 Gazette 2021/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.02.2020 CN 202010080029**
**29.10.2020 CN 202011182716**

(71) Applicant: **Bebetter Med Inc.**
**Guangzhou, Guangdong 510660 (CN)**

(72) Inventors:
- **CAI, Xiong**
  **Guangzhou, Guangdong 510660 (CN)**
- **WENG, Yunwo**
  **Guangzhou, Guangdong 510660 (CN)**
- **QING, Yuanhui**
  **Guangzhou, Guangdong 510660 (CN)**
- **LIN, Mingsheng**
  **Guangzhou, Guangdong 510660 (CN)**
- **LIU, Bin**
  **Guangzhou, Guangdong 510660 (CN)**
- **HE, Qijie**
  **Guangzhou, Guangdong 510660 (CN)**
- **LIU, Yiting**
  **Guangzhou, Guangdong 510660 (CN)**
- **FENG, Qiao**
  **Guangzhou, Guangdong 510660 (CN)**
- **FAN, Fushun**
  **Guangzhou, Guangdong 510660 (CN)**
- **QIAN, Changgeng**
  **Guangzhou, Guangdong 510660 (CN)**

(74) Representative: **Haseltine Lake Kempner LLP**
**One Portwall Square**
**Portwall Lane**
**Bristol BS1 6BH (GB)**

# (54) PYRIDOPYRIMIDINONE COMPOUND AND APPLICATION THEREOF

(57) The present disclosure provides the Pyridopyrimidinone compounds having the structure represented by the general Formula (II) and applications thereof. Studies have shown that the compounds provided by the present disclosure can effectively inhibit the KRAS G12C mutation. KRAS mutation accounts for a large proportion of tumors, and currently there is no approved drug for its treatment. The compounds provided by the present disclosure have the potential to become a therapeutic medicine for malignant tumors (especially non-small cell lung cancer (NSCLC) and colorectal cancer) haboring KRAS G12C mutation, and have great application value.

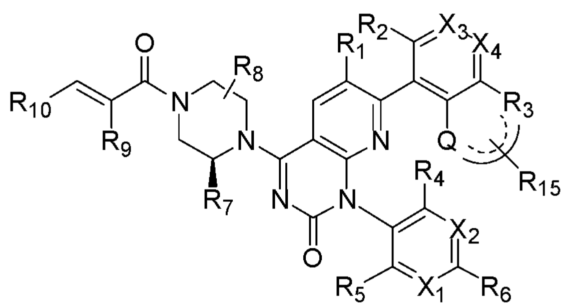

(II)

EP 4 101 852 A1

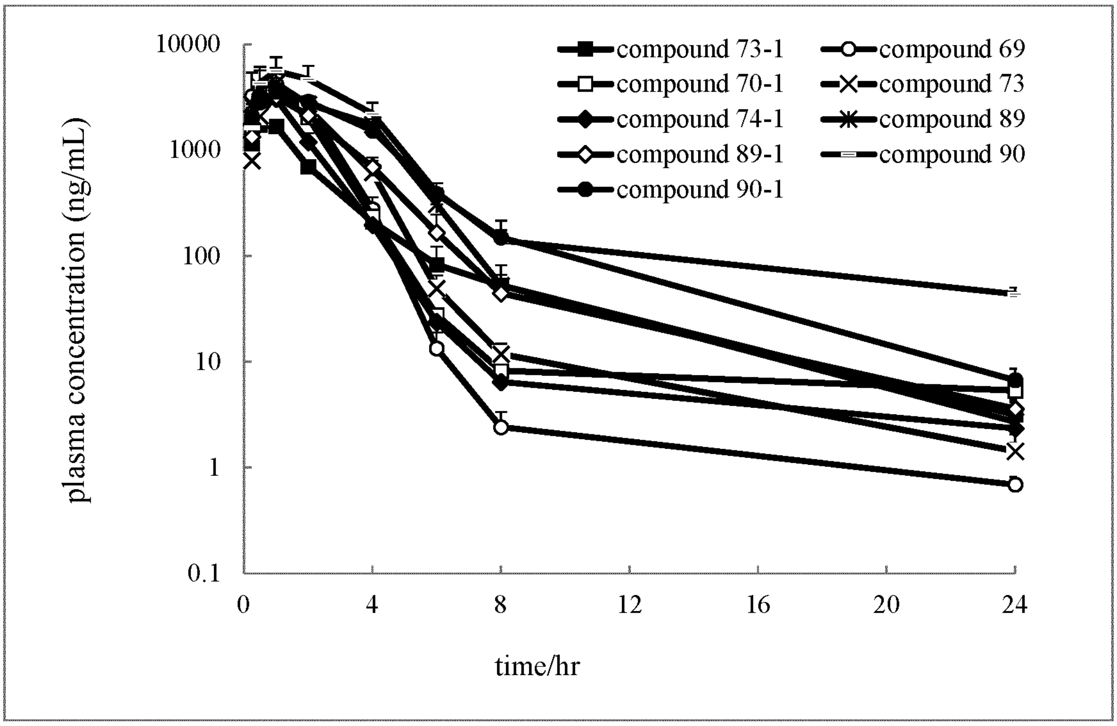
compound 73-1
compound 69
compound 70-1
compound 73
compound 74-1
compound 89
compound 89-1
compound 90
compound 90-1
plasma concentration (ng/mL)
10000
1000
100
10
1
0.1
0
4
8
12
16
20
24
time/hr

Fig. 1

## Description

## Technical Field

The present disclosure relates to the chemical and pharmaceutical technology, in particular to a kind of Pyridopyrimidinone compounds and the applications thereof.

## Background Art

RAS protein belongs to the small GTPase protein family, which plays a crucial role in regulating cell growth, proliferation and differentiation. In cells, RAS protein transits between inactivated and activated states. When RAS is bound to guanosine diphosphate (GDP), it is in an inactivated state. When it is bound to guanosine triphosphate (GTP), it is in an activated state (Cell 170:17-33, 2017), and it can activate multiple downstream signaling pathways, including RAF-MEK-ERK (RAF-MAPK/ERK kinase-extracellular signal-regulated kinase) (Cell 74:205-214, 1993), PI3K-AKT-mTOR (phosphoinositide 3-kinase-AKT-mechanistic target of rapamycin) (Nature 370:527-532, 1994) and RALGDS-RAL (RAL guanine nucleotide-dissociation stimulator-RAL) (EMBO J 15: 5256 -5267, 1996). The transition of RAS between inactivated and activated states is regulated by two types of factors. One is the guanine nucleotide exchange factor (GEF), which catalyzes the binding of RAS to GTP, thereby promoting the activation of RAS. Another type is GTPase activating protein (GAP), which can promote the hydrolysis of GTP bound to RAS to GDP, thereby inhibiting the activity of RAS (Nature 575: 217-223, 2019).

The RAS protein family consists of three members, KRAS, HRAS and NRAS. About 25% of human malignancies are associated with RAS gene mutations, which kill millions of people worldwide every year. Among the RAS protein family, KRAS is the most common mutant, accounting for about 85% of total mutations, especially in solid tumors, followed by NRAS (12%) and HRAS (3%) (Cell 170:17-33, 2017).

In human cancers, mutations in the KRAS gene are found in nearly 95% of pancreatic cancers, 45% of colon cancers, and 35% of lung cancers. It also occurs in cancer types such as cholangiocarcinoma, cervical cancer, bladder cancer, liver cancer, and breast cancer (C&EN 94.23:28-33, 2016). Most KRAS gene mutations occur at codon 12, 13, and 61. Structural studies have shown that most of these genetic mutations interfere with the ability of KRAS hydrolyzing GTP. For example, the most common KRAS G12C mutation, results in a glycine-to-cysteine conversion at position 12 of the protein, affecting the binding of GAP proteins and KRAS, thereby inhibiting GAP-stimulated GTP hydrolysis. Due to the decreased hydrolysis capacity of GTPase, more KRAS protein is bond to GTP, resulting in the gradual accumulation of activated KRAS, which is associated with poor prognosis of tumor and treatment resistance (Nat. Rev. Drug Discov 13:828-851, 2014).

Specific mutations in KRAS G12C account for approximately 13% of non-small cell lung cancers (approximately 14,000 new cases per year in the U.S.), 3% to 5% of colorectal cancers (approximately 5,000 new cases per year in the U.S.), and 1% to 2% of numerous others solid tumors.

In the United States, approximately 30,000 patients are diagnosed with cancers caused by KRAS G12C mutation each year. KRAS G12C mutation constitutes a patient population with an annual incidence of more than 100,000 people worldwide.

In view of these, the development of drugs targeting KRAS has a significant meaning in the treatment of cancer.

There are five following strategies for drugs targeting KRAS (Nat Rev Drug Discov 15: 771-785, 2016): The first one is to directly bind to KRAS molecules and disrupt its interaction with guanine nucleotide exchange factors (such as compounds SML-8-73-1 and ARS-853, etc.), or its interaction with RAF serine threonine kinase effector molecules (such as compound ARS-1620, etc.); the second is to regulate the localization of KRAS proteins in the cell membrane (farnesyltransferas inhibitors and PDEd, etc.); the third is to target KRAS effector molecules (RAF and PI3K, etc.); the fourth is to identify synthetic lethal targets (TBK1, WT1 and CDK4) etc.); the fifth is targeting the metabolic and immune processes regulated by KRAS in cancer cells.

There are at least five KRAS modulators currently in clinical development, among which the three candidates, AMG 510 (ClinicalTrials.gov Identifier: NCT03600883), MRTX84 (ClinicalTrials.gov Identifier: NCT03785249) and ARS-324 (ClinicalTrials.gov Identifier: NCT04006301), only target the KRAS G12C mutant; while the inhibitor BI1701963 (ClinicalTrials.gov Identifier: NCT04111458) is the first pan-KRAS inhibitor. BI1701963 targets the SOS1 protein, which promotes KRAS activation. mRNA-5671 (ClinicalTrials.gov Identifier: NCT03948763) is an mRNA cancer vaccine that manufactures antigens in vivo, promoting T cells to seek and destroy cells expressing four key KRAS mutants (G12C, G12D, G12V and G13C).

Clinical trial results of AMG 510 in phase I showed that out of 23 evaluable non-small cell lung cancer (NSCLC) patients, 11 patients (48%) achieved partial response (PR). 11 patients (48%) had stable disease (SD). 1 patients (4%) had progressive disease (PD), which means the objective response rate (ORR) was 48%, and the disease control rate (DCR) was 96%. In addition, out of the 13 evaluable patients who received the highest dose of 960 mg, 7 patients (54%)

achieved PR and 6 patients (46%) achieved SD, which meant the ORR was 54%, and the DCR was 100%. In terms of safety, AMG 510 was well tolerated, and the main adverse reactions were decreased appetite, diarrhea, fatigue, headache, cough, hot flashes, and nausea. No dose-limiting toxicity (DLT) and drug-related adverse reactions above grade IV were found (WCLC 2019 Congress).

In the phase I of clinical study on MRTX849, there were 12 patients with KRAS G12C-mutated advanced solid tumors (6 NSCLC, 4 colorectal cancer, and 2 appendix cancer) who had received prior systemic therapy. Among the NSCLC patients, the ORR was 50%, and the DCR was 100%. Among the patients with colorectal cancer, the ORR was 25%, and the DCR was 75%. Both patients with appendix cancer achieved SD. From the safety data, the most common adverse reactions of receiving MRTX849 are diarrhea, nausea, vomiting, increased AST, etc. Most of the adverse reactions are mild and controllable (AACR-NCI-EORTC 2019 Congress).

In addition to monotherapy, KRAS G12C inhibitors can also be used in combination with other drugs (Nature 575: 217-223, 2019). According to reports, in mouse experiments using AMG 510 or PD-1 antibody pembrolizumab alone, only 10% of the mice could make the tumor completely disappear and survive for a long time; while AMG 510 combined with pembrolizumab could make 90% of the mouse tumors disappear completely and the curative effect maintained for a long time. In addition, mice cured with AMG 510 combined with pembrolizumab also possessed the ability to reject KRAS G12D tumors, suggesting that the combination treatment may drive an adaptive immune response. In addition to PD-1 antibody, clinical studies of the combination therapy of AMG 510 and SHP2 phosphatase inhibitor (related to the activation-inactivation mechanism of KRAS) will be also carried out. Pre-clinical studies have also confirmed that KRAS G12C inhibitor combined with MAPK, AKT signaling pathway inhibitors, and chemotherapeutic drug carboplatin can enhance tumor killing and overcome drug resistance in vitro.

## Summary of the Disclosure

The present disclosure provides a new class of Pyridopyrimidinone compounds, which can effectively inhibit KRAS G12C mutation and have the potential to be used as a therapeutic drug for malignant tumors harboring KRAS G12C mutation.

The specific technical solutions are as follows:

The Pyridopyrimidinone compounds with the structure shown in Formula (I) or their pharmaceutically acceptable salts or stereoisomers or prodrug molecules:

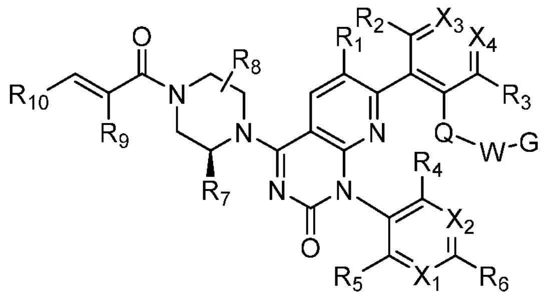

(I)

Wherein:

Q is selected from: O, S, S(O), $S(O)_2$, $N(R_{12})$, C(O), $(CR_{13}R_{14})n$, C3-C8 cycloalkyl, vinyl, ethynyl;
W is selected from: O, S, S(O), $S(O)_2$, $N(R_{12})$, or nonexistence;
G is selected from: H, halogen, C1-C6 alkyl, C2-C6 alkenyl, C2-C6 alkynyl, C3-C8 cycloalkyl, C3-C8 cycloalkyl-substituted C1-C6 alkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino-substituted C1-C6 Alkyl, C6-C10 aryl, 3-8 membered heterocyclyl, $-C(O)N(R)_2$, -C(O)R;

And, when W is nonexistence and Q is O or $N(R_{12})$, G is not H;

Q and W cannot be heteroatoms at the same time and directly connected to each other; n is selected from: 1, 2 or 3;
$X_1$ and $X_2$ are independently selected from: N or CH;
$X_3$ and $X_4$ are independently selected from: N or $CR_{11}$;

$R_1$ is selected from: halogen;
$R_2$ is selected from: H, halogen, C1-C6 alkyl, C1-C6 alkoxy;
$R_3$, each $R_{11}$ is independently selected from: H, halogen, -OR, -SR, $-N(R)_2$, nitro, cyano, C1-C6 alkyl, C2-C6 alkenyl, C2-C6 alkynyl, C3-C8 cycloalkyl, C3-C8 cycloalkyl substituted-C1-C6 alkyl, halogen-substituted C1-C6 alkyl, hydroxy-substituted C1-C6 alkyl, C1-C6 alkoxy-substituted C1-C6 Alkyl, amino-substituted C1-C6 alkyl, C1-C6 alkylamino-substituted C1-C6 alkyl; or, when Q is selected from: O, S, $N(R_{12})$, $R_3$ with-QWG and the attached C atoms together form a 5-7 membered heterocyclic group or a heteroaryl group;
$R_4$, $R_5$ are independently selected from: H, C1-C6 alkyl group, C2-C6 alkenyl group, C2-C6 alkynyl group, C3-C8 cycloalkyl group, cyano group, hydroxy-substituted C1-C6 alkyl group;
$R_6$ is selected from: H, halogen, -OR, nitro, cyano, $-N(R)_2$, C1-C6 alkyl, $-C(O)N(R)_2$, - C(O)R;
$R_7$, $R_8$ are independently selected from: H, C1-C6 alkyl, cyano-substituted C1-C6 alkyl;
$R_9$ is selected from: H, halogen;
$R_{10}$ is selected from: H, halogen, C1-C4 alkyl, C1-C3 alkoxy-substituted C1-C4 alkyl, amino-substituted C1-C4 alkyl, C1-C3 alkylamino-substituted C1-C4 alkyl, C1-C4 alkyl substituted by 3-8 membered heterocyclyl; or, $R_9$ and $R_{10}$ are connected to form a carbon-carbon bond;
each $R_{12}$ is independently selected from: H, C1-C6 alkyl;
$R_{13}$, $R_{14}$ are independently selected from: H, halogen, C1-C6 alkyl;
each R is independently selected from: H, C1-C6 alkyl, C3-C8 cycloalkyl, C3-C8 cycloalkyl-substituted C1-C6 alkyl, halogen-substituted C1-C6 alkyl, hydroxy-substituted C1- C6 alkyl, C1-C6 alkoxy-substituted C1-C6 alkyl, amino-substituted C1-C6 alkyl, C1-C6 alkylamino-substituted C1-C6 alkyl.

In some embodiments, Q was selected from: S, S(O), $S(O)_2$,

W is selected from: nonexistence;
G is selected from: H, C1-C6 alkyl, C2-C6 alkenyl, C2-C6 alkynyl, C3-C8 cycloalkyl, C3-C8 cycloalkyl-substituted C1-C6 alkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino-substituted C1-C6 alkyl, C6-C10 aryl, 3-8 membered heterocyclyl, $-C(O)N(R)_2$, -C(O)R.

In some embodiments, Q is selected from: S, S(O), $S(O)_2$,

W is selected from: nonexistence;
G is selected from: C1-C6 alkyl, C3-C8 cycloalkyl, C3-C8 cycloalkyl-substituted C1-C6 alkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino- substituted C1-C6 alkyl, C6-C10 aryl, 3-8 membered heterocyclyl, $-C(O)N(R)_2$, -C(O)R.

In some embodiments, Q is selected from: S, S(O), $S(O)_2$,

W is selected from: nonexistence;
G is selected from: C1-C6 alkyl, cyclopropyl, cyclopropyl-substituted C1-C3 alkyl, 3-6 membered heterocyclyl, C6-C10 aryl, -C(O)R; wherein, R is selected from C1-C6 alkyl. In some embodiments, Q is selected from: C(O), $(CR_{13}R_{14})n$, C3-C8 cycloalkyl, vinyl, ethynyl;
n is selected from: 1, 2 or 3;
W is selected from: O, S, S(O), $S(O)_2$, $N(R_{12})$, or nonexistence;
G is selected from: H, halogen, C1-C6 alkyl, C2-C6 alkenyl, C2-C6 alkynyl, C3-C8 cycloalkyl, C3-C8 cycloalkyl-substituted C1-C6 alkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino-substituted C1-C6 Alkyl, C6-C10 aryl, 3-8 membered heterocyclyl, $-C(O)N(R)_2$, -C(O)R.

In some embodiments, Q is selected from: C(O), $(CR_{13}R_{14})n$, C3-C8 cycloalkyl, vinyl, ethynyl;

n is selected from: 1 or 2;
W is selected from: O, S, S(O), $S(O)_2$, $N(R_{12})$, or nonexistence;
G is selected from: H, halogen, C1-C6 alkyl, C3-C6 cycloalkyl, C3-C6 cycloalkyl-substituted C1-C3 alkyl, halogen-substituted C1-C3 alkyl, hydroxy-substituted C1-C3 Alkyl, C1-C3 alkoxy-substituted C1-C3 alkyl, amino-substituted C1-C3 alkyl, C1-C3 alkylamino-substituted C1-C3 alkyl, C6-C10 aryl, 3-6 A membered heterocyclic group, -$C(O)N(R)_2$, -C(O)R; wherein, R is selected from: H, C1-C6 alkyl.

In some embodiments, Q is selected from: O, $N(R_{12})$;

W is selected from: nonexistence;
G is selected from: C1-C6 alkyl, C2-C6 alkenyl, C2-C6 alkynyl, C3-C8 cycloalkyl, C3-C8 cycloalkyl-substituted C1-C6 alkyl, halogen-substituted C1-C6 alkane group, hydroxyl-substituted C1-C6 alkyl, C1-C6 alkoxy-substituted C1-C6 alkyl, amino-substituted C1-C6 alkyl, C1-C6 alkylamino-substituted C1-C6 alkyl, C6-C10 aryl, 3-8 membered heterocyclyl, $-C(O)N(R)_2$, -C(O)R.

In some embodiments, Q is selected from: O, $N(R_{12})$;

W is selected from: nonexistence;
G is selected from: C1-C6 alkyl, C3-C6 cycloalkyl, C3-C6 cycloalkyl-substituted C1-C3 alkyl, halogen-substituted C1-C3 alkyl, hydroxy-substituted C1-C3 alkyl, C1-C3 alkoxy-substituted C1-C3 alkyl, amino-substituted C1-C3 alkyl, C6-C10 aryl, 3-6 membered heterocyclyl, $-C(O)N(R)_2$, -C(O) R; wherein, R is selected from: H, C1-C6 alkyl.

Pyridopyrimidinone compounds with the structure shown in Formula (II), or their pharmaceutically acceptable salts, or stereoisomers or prodrug molecules:

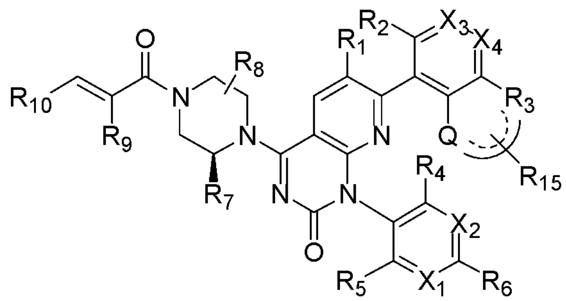

(II)

Wherein:

the dotted line between Q and $R_3$ indicates that the chemical bond between Q and $R_3$ can be a single bond or a double bond;
Q is selected from: O, S.
$R_3$ is selected from: N, $CR_{15}$, O, S, $NR_{15}$, $CHR_{15}$, $C(R_{15})_2$;
Q, $R_3$ and the attached C atoms together form a 5-7 membered heterocyclic group or a heteroaryl group;
$X_1$ and $X_2$ are independently selected from: N or CH;
$X_3$ and $X_4$ are independently selected from: N or $CR_{11}$;
$R_1$ is selected from: halogen;
$R_2$ is selected from: H, halogen, C1-C6 alkyl, C1-C6 alkoxy;
$R_4$ and $R_5$ are independently selected from: H, C1-C6 alkyl group, C2-C6 alkenyl group, C2-C6 alkynyl group, C3-C8 cycloalkyl group, cyano group, hydroxy substituted C1-C6 alkyl group;
$R_6$ is selected from: H, halogen, -OR, nitro, cyano, $-N(R)_2$, C1-C6 alkyl, $-C(O)N(R)_2$, - C(O)R;
$R_7$, $R_8$ are independently selected from: H, C1-C6 alkyl, cyano-substituted C1-C6 alkyl;
$R_9$ is selected from: H, halogen;
$R_{10}$ is selected from: H, halogen, C1-C4 alkyl, C1-C4 alkyl substituted by C1-C3 alkoxy, amino-substituted C1-C4 alky, C1-C3 alkylamino-substituted C1-C4 alkyl, C1-C4 alkyl substituted by 3-8 membered heterocyclyl; or, $R_9$ and $R_{10}$ were connected to form a carbon-carbon bond;
each $R_{11}$ is independently selected from: H, halogen, -OR, -SR, $-N(R)_2$, nitro, cyano, C1-C6 alkyl, C2-C6 alkenyl, C2-C6 alkynyl, C3- C8 cycloalkyl, C1-C6 alkyl substituted by C3-C8 cycloalkyl, halogen-substituted C1-C6 alkyl, hydroxy-substituted C1-C6 alkyl, C1-C6 alkyl substituted by C1-C6 alkoxy, C1-C6 alkyl substituted by amino group, C1-C6 alkyl substituted by C1-C6 alkylamino group;
each $R_{15}$ is independently selected from: H, halogen, OR, -SR, $-N(R)_2$, C1-C6 alkyl, C3-C8 cycloalkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino- substituted C1-C6 alkyl;
each R is independently selected from: H, C1-C6 alkyl, C3-C8 cycloalkyl, C3-C8 cycloalkyl-substituted C1-C6 alkyl,

halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino-substituted C1-C6 alkyl.

In some embodiments, the Pyridopyrimidinone compounds have the structure shown in Formula (III):

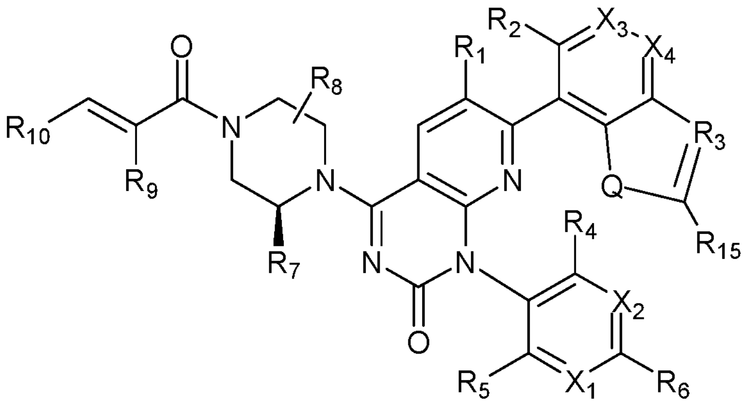

(III)

Wherein:

Q is selected from: O, S;
$R_3$ is selected from: N, $CR_{15}$.

In some embodiments, $R_3$ is selected from: CH.

In some embodiments, the Pyridopyrimidinone compounds have the structure shown in Formula (IV):

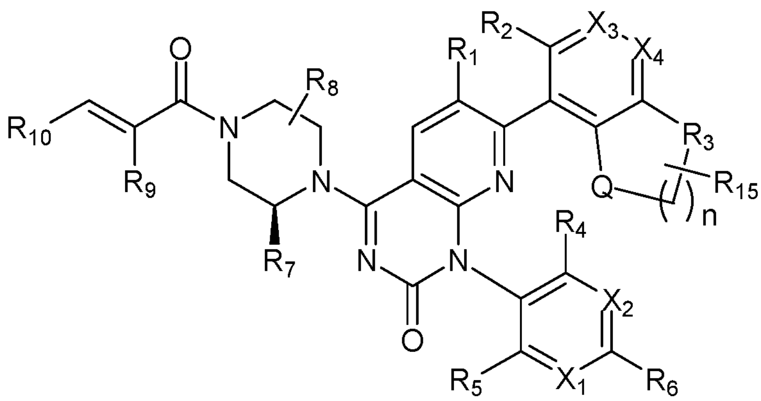

(IV)

wherein, Q is selected from: O, S;
$R_3$ is selected from: O, S, $NR_{15}$, $CHR_{15}$, $C(R_{15})_2$;
n is selected from: 1 or 2.

In some embodiments, $R_3$ is selected from: O, S, $CH_2$.

In some embodiments, Q, $R_3$ and the C atom connected with them together form a 5-6 membered heterocyclic group or a heteroaryl group; the 5-6 membered heterocyclic group or heteroaryl group combined with a six-membered aryl group to form the following structures:

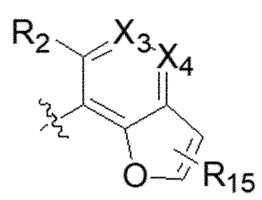
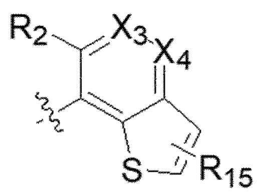
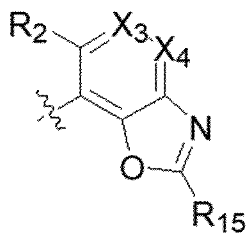
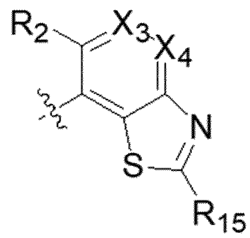

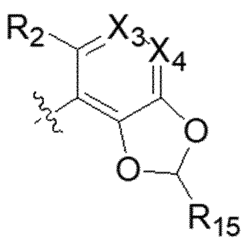
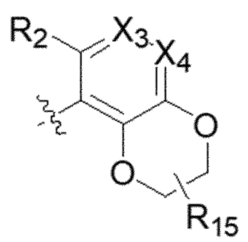
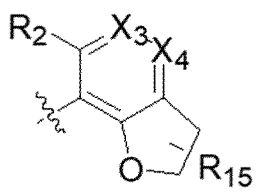
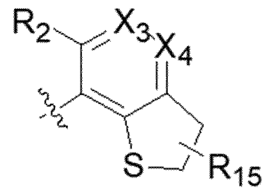

.

In some embodiments, the 5-6 membered heterocyclic group or a heteroaryl group combined with the six-membered aryl group to form the following structures:

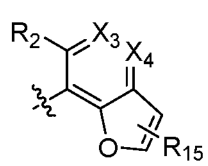
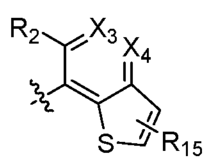
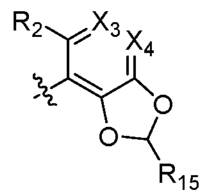
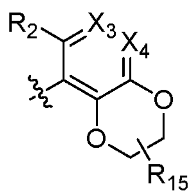

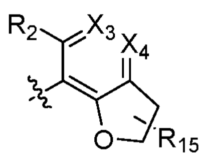
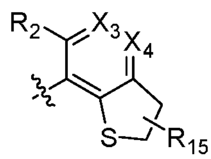

In some embodiments, $X_1$ is N, $X_2$ is CH.

In some embodiments, $X_1$ is CH, $X_2$ is CH, $R_4$ is cyano, and $R_5$ is C1-C6 alkyl.

In some embodiments, $X_1$ is N, $X_2$ is N, $R_4$ is isopropyl, and $R_5$ is C1-C6 alkyl.

In some embodiments, both $X_3$ and $X_4$ are $CR_{11}$; wherein, $R_{11}$ is selected from: H, halogen, OR, and R is selected from: H, C1-C3 alkyl.

In some embodiments, $X_3$ is selected from: CH, COH, CF; $X_4$ is selected from: CH, CF.

In some embodiments, $R_1$ is selected from: F, Cl.

In some embodiments, $R_2$ is selected from: F, Cl.

In some embodiments, $R_4$ and $R_5$ are independently selected from: H, C1-C3 alkyl, C3-C6 cycloalkyl, cyano, and hydroxy-substituted C1-C3 alkyl.

In some embodiments, $R_4$ is selected from: methyl, isopropyl, cyano; $R_5$ is selected from: isopropyl.

In some embodiments, $R_6$ is selected from: H, C1-C3 alkyl.

In some embodiments, $R_7$ and $R_8$ are independently selected from: H, C1-C3 alkyl, and cyano-substituted C1-C3 alkyl.

In some embodiments, $R_7$ is selected from: methyl; $R_8$ is selected from: H, methyl, cyanomethyl.

In some embodiments, $R_9$ and $R_{10}$ are independently selected from: H, halogen.

In some embodiments, $R_{15}$ is selected from: H.

In some embodiments, $X_1$ and $X_2$ are independently selected from: N or CH;

$X_3$ and $X_4$ are independently selected from: N or $CR_{11}$;

$R_1$, $R_2$ are independently selected from: F, Cl;

$R_4$, $R_5$ are independently selected from: C1-C6 alkyl, C2-C6 alkenyl, C3-C8 cycloalkyl, cyano;

$R_6$ is selected from: H, halogen, OR, C1-C6 alkyl;
$R_7$ is selected from: C1-C6 alkyl;
$R_8$ is selected from: H, C1-C6 alkyl, cyano-substituted C1-C6 alkyl;
$R_9$ is selected from: H;
$R_{10}$ is selected from: H;
each $R_{11}$ is independently selected from: H, halogen, OR, -SR, $-N(R)_2$, C1-C6 alkyl, C3-C8 cycloalkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino- substituted C1-C6 alkyl;
each R is independently selected from: H, C1-C6 alkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkyl substituted by C1-C6 alkylamino.

In some embodiments, the Pyridopyrimidinone compounds were selected from following compounds:

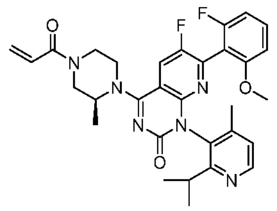

compound 1

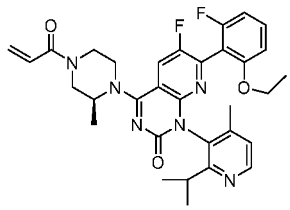

compound 2

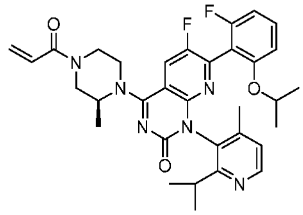

compound 3

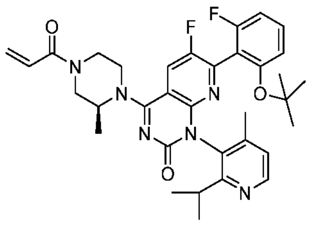

compound 4

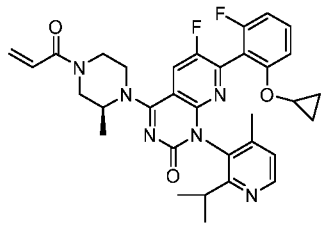

compound 5

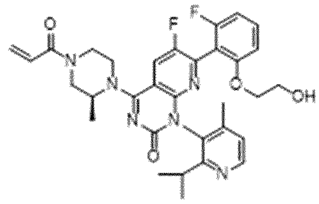

compound 6

compound 7

compound 8

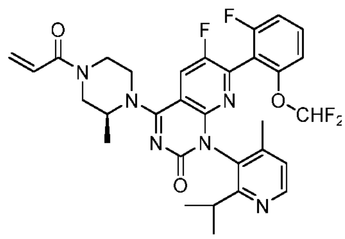

compound 9

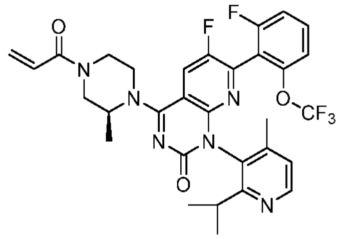

compound 10

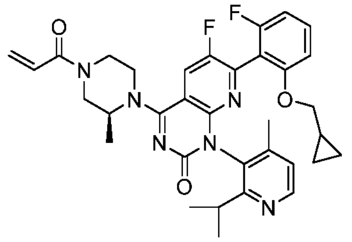

compound 11

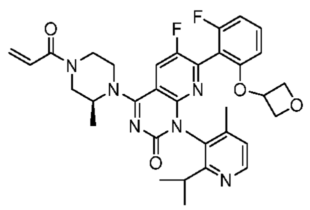

compound 12

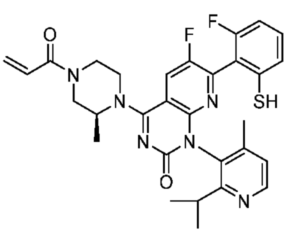

compound 13

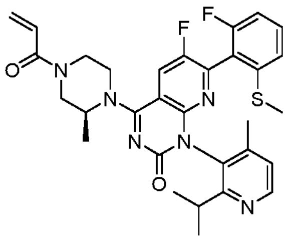

compound 14

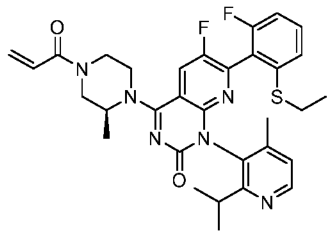

compound 15

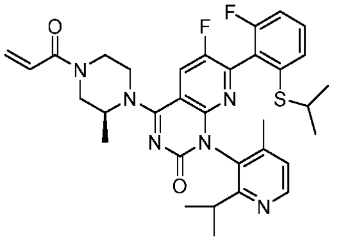

compound 16

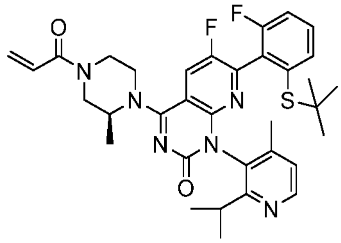

compound 17

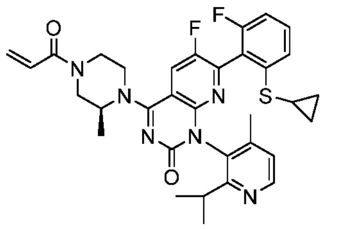

compound 18

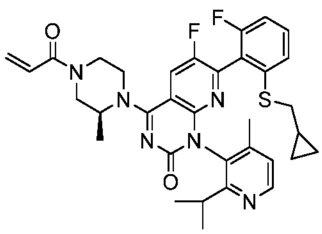

compound 19

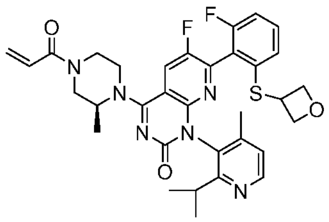

compound 20

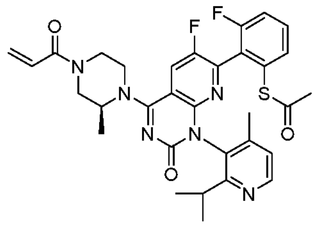

compound 21

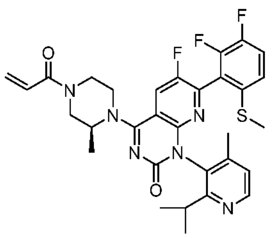

compound 22

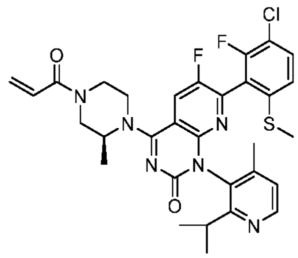

compound 23

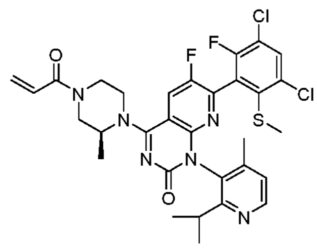

compound 24

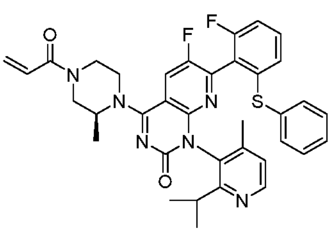

compound 25

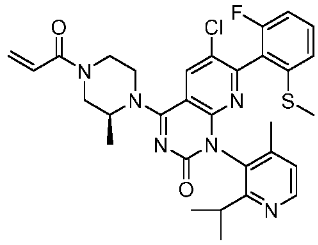

compound 26

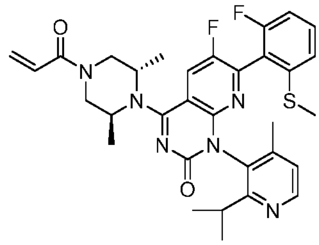

compound 27

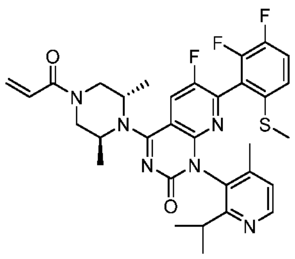

compound 28

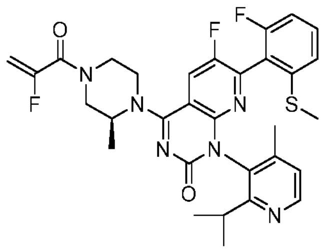

compound 29

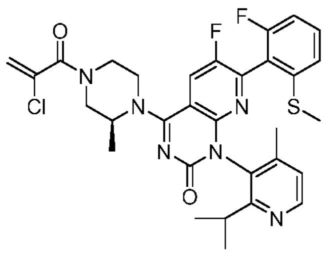

compound 30

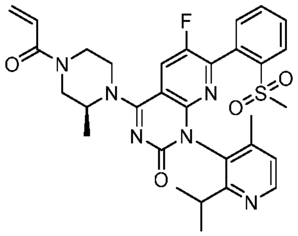

compound 31

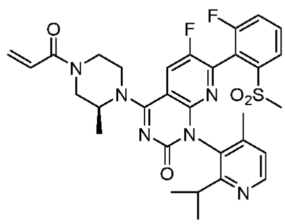

compound 32

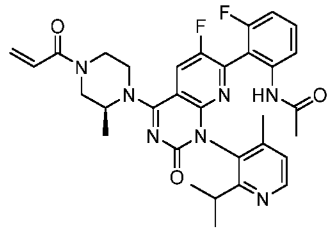

compound 33

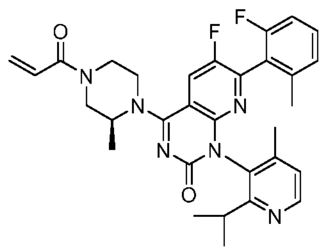

compound 34

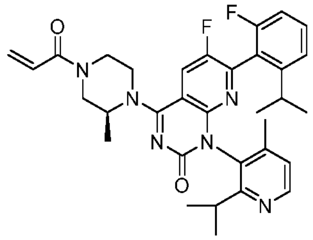

compound 35

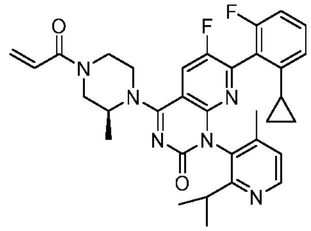

compound 36

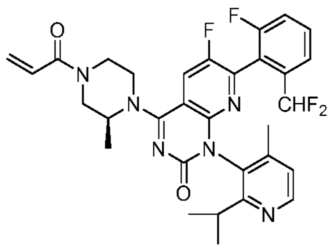

compound 37

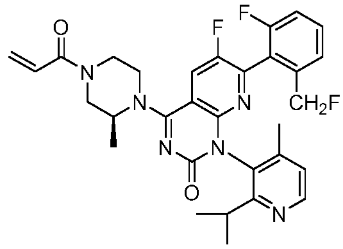

compound 38

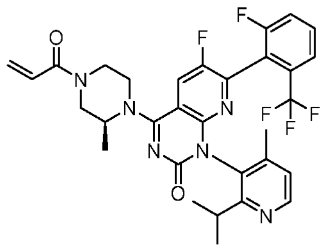

compound 39

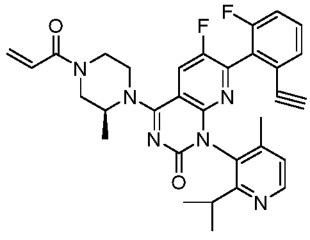

compound 40

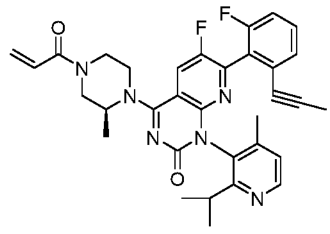

compound 41

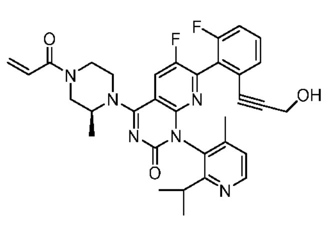

compound 42

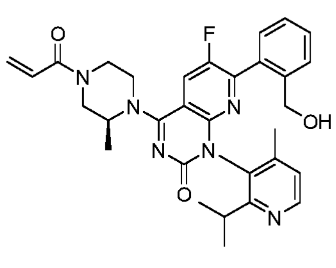

compound 43

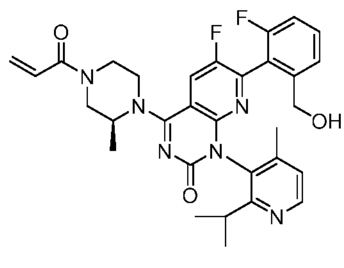

compound 44

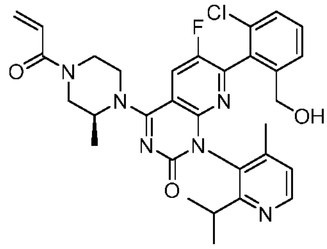

compound 45

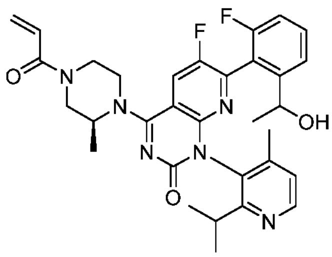

compound 46

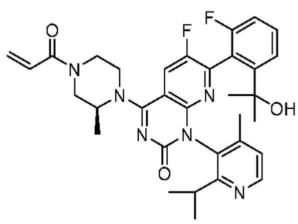

compound 47

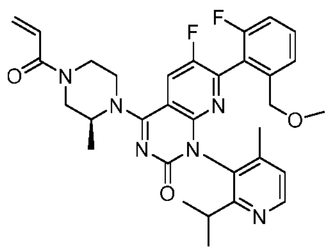

compound 48

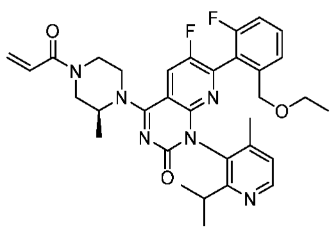

compound 49

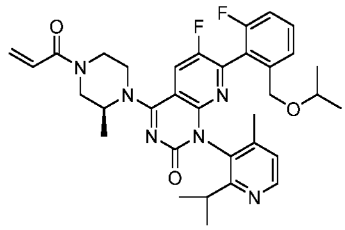

compound 50

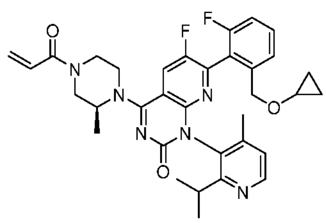

compound 51

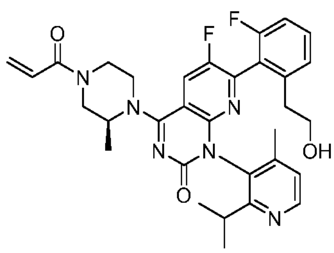

compound 52

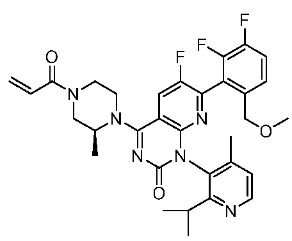

compound 53

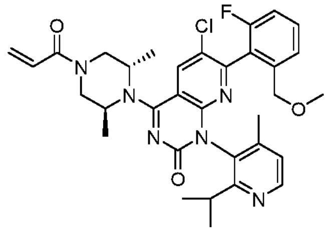

compound 54

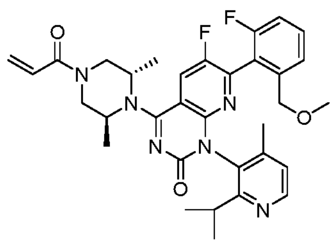

compound 55

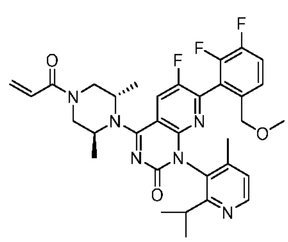

compound 56

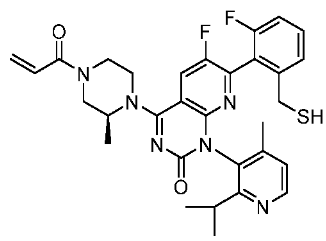

compound 57

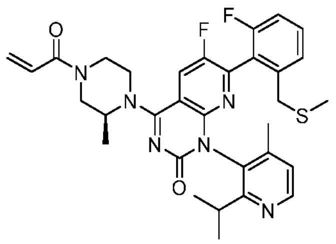

compound 58

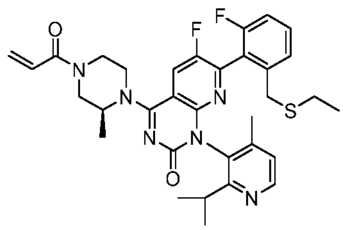

compound 59

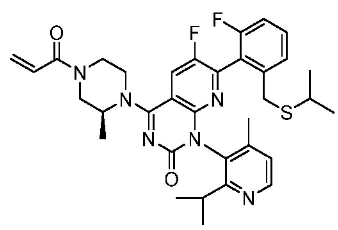

compound 60

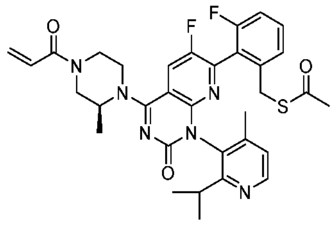

compound 61

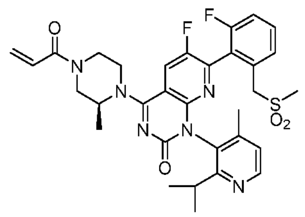

compound 62

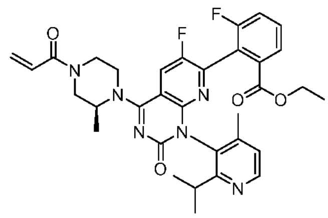

compound 63

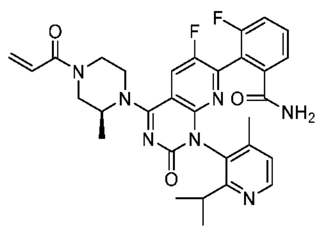

compound 64

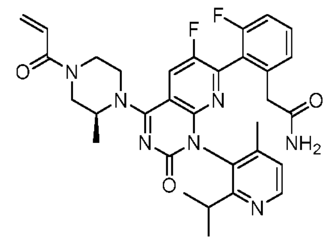

compound 65

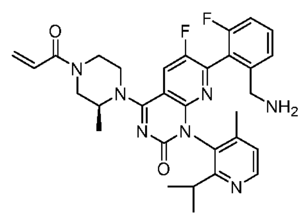

compound 66

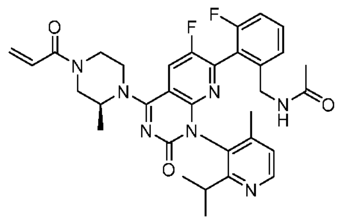

compound 67

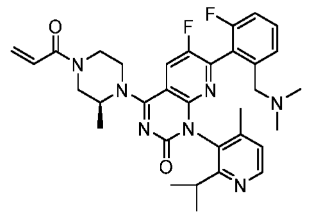

compound 68

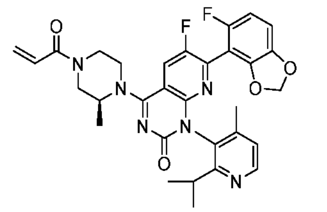

compound 69

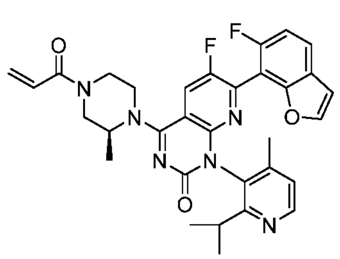

compound 70

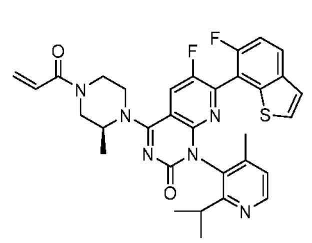

compound 71

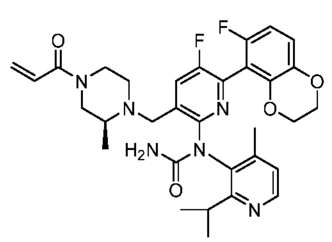

compound 72

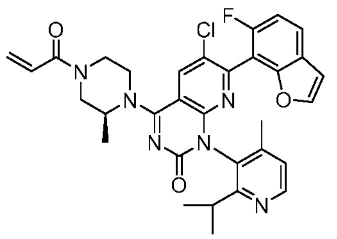

compound 73

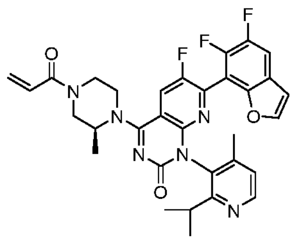

compound 74

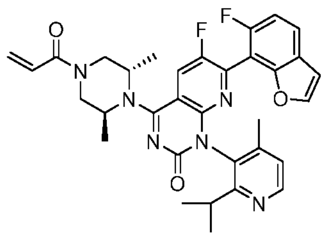

compound 75

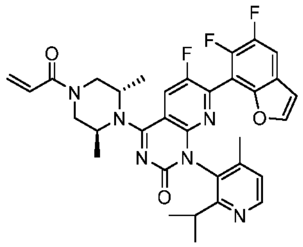

compound 76

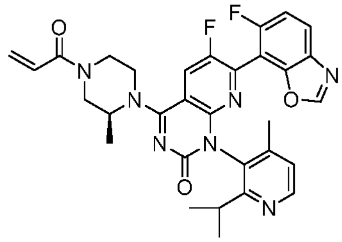

compound 77

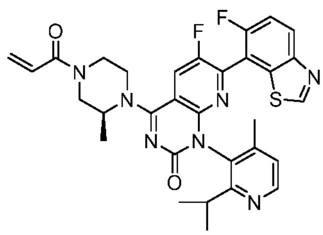

compound 78

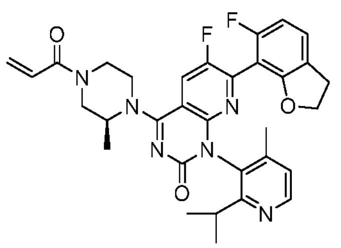

compound 79

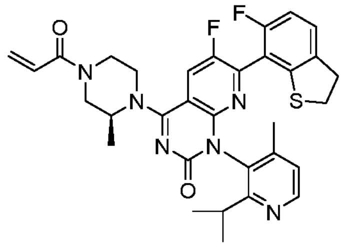

compound 80

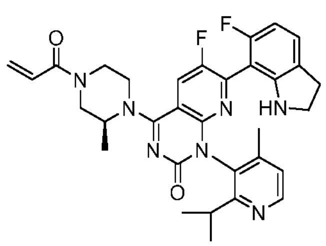

compound 81

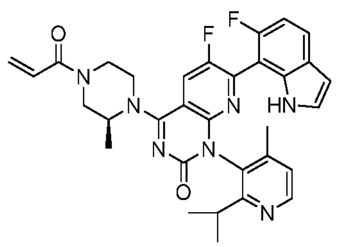

compound 82

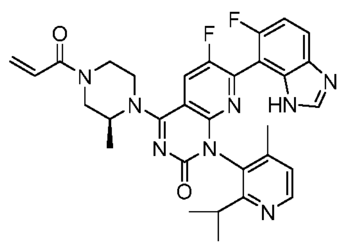

compound 83

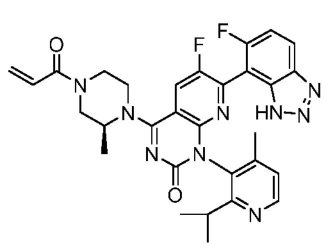

compound 84

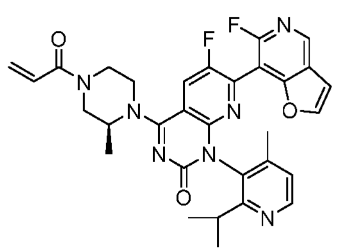

compound 85

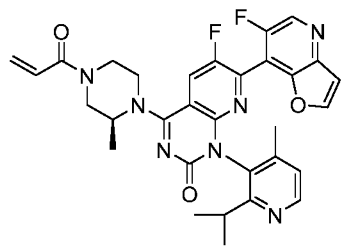

compound 86

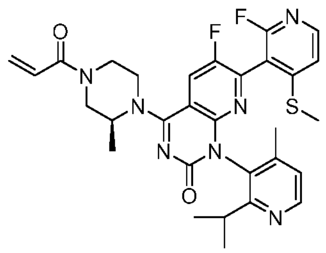

compound 87

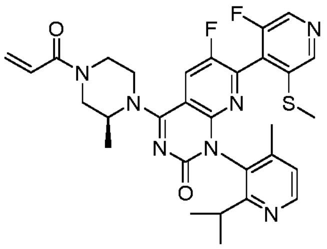
compound 88

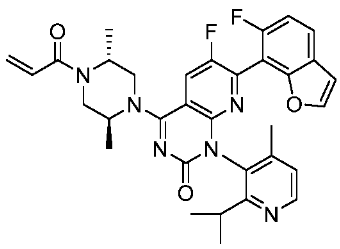
compound 89

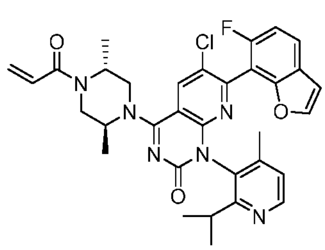
compound 90

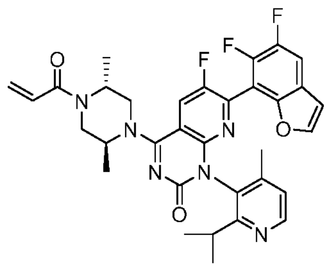
compound 91

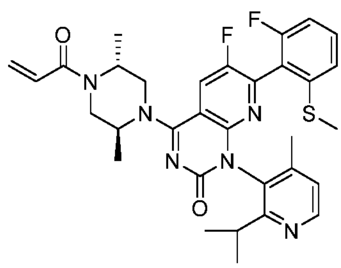
compound 92

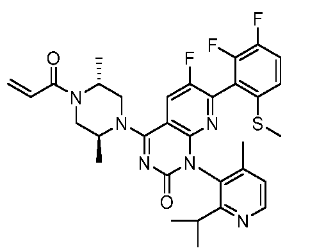
compound 93

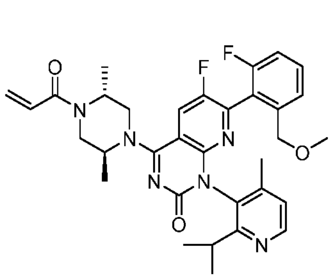
compound 94

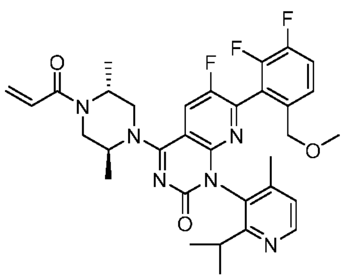
compound 95

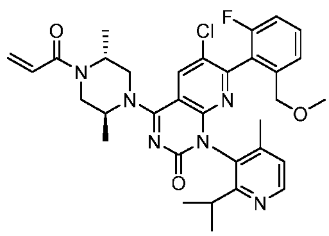
compound 96

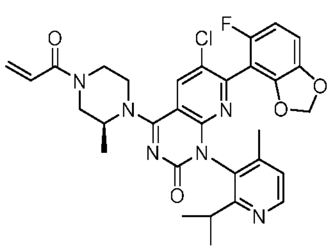
compound 97

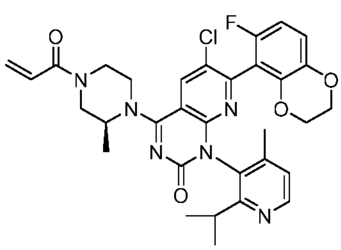
compound 98

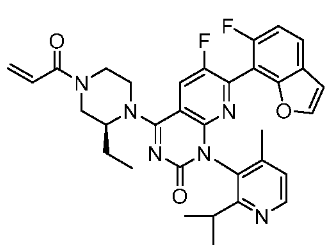
compound 99

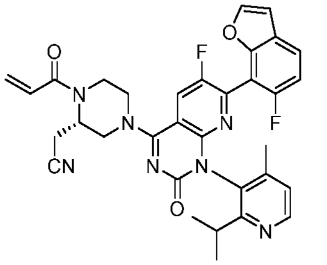
compound 100

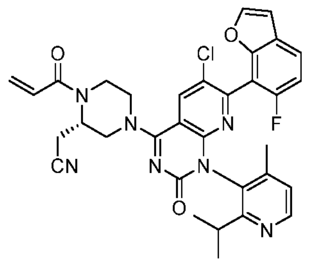
compound 101

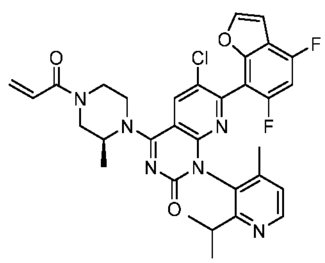
compound 102

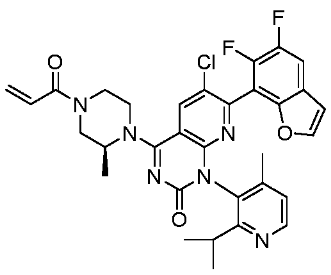

compound 103

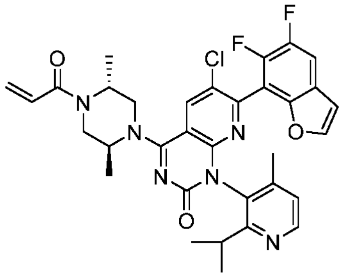

compound 104

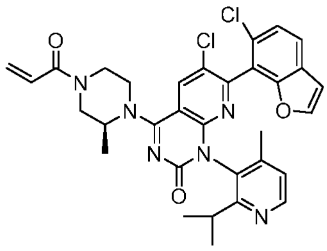

compound 105

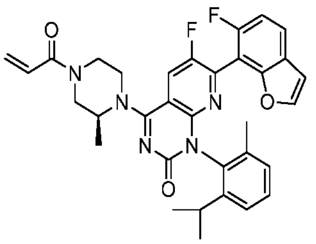

compound 106

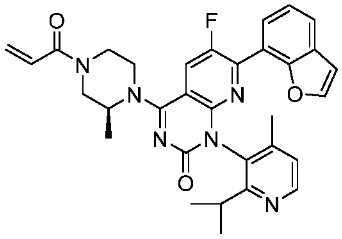

compound 107

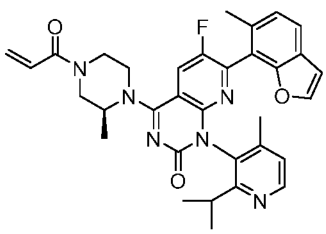

compound 108

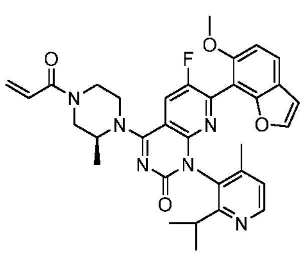

compound 109

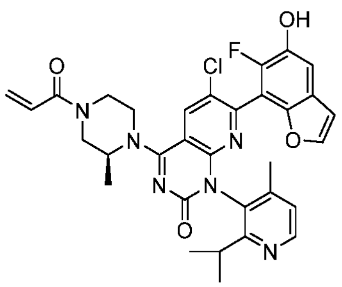

compound 110

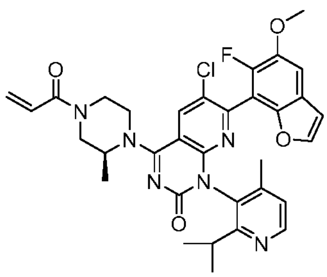

compound 111

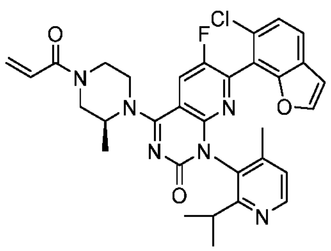

compound 112

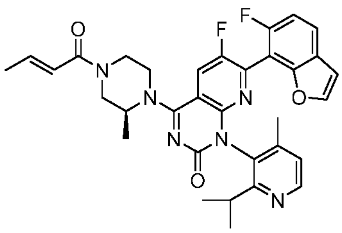

compound 113

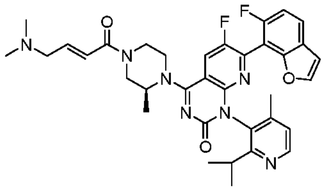

compound 114

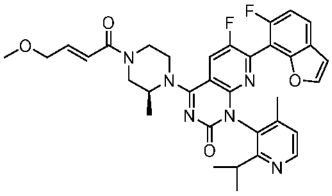

compound 115

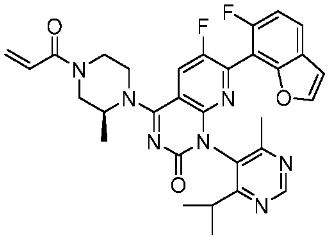

compound 116

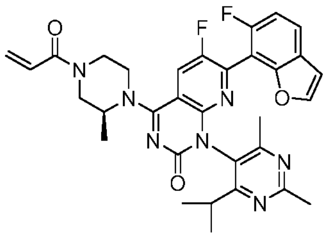

compound 117

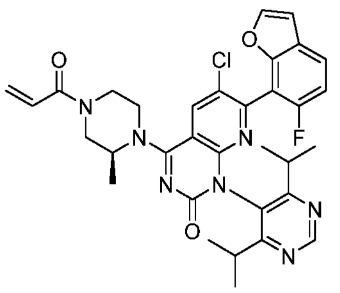

compound 118

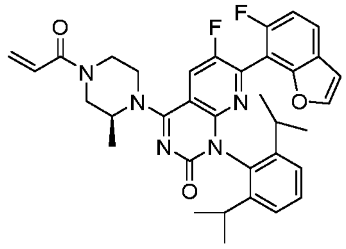

compound 119

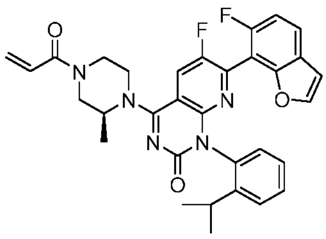

compound 120

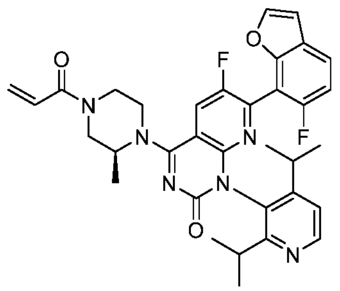

compound 121

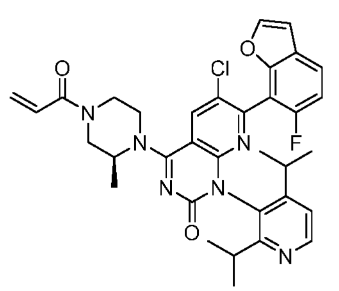

compound 122

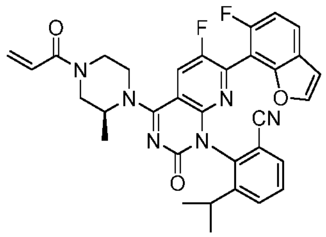

compound 123

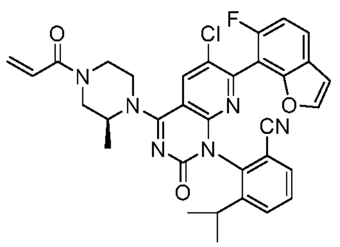

compound 124

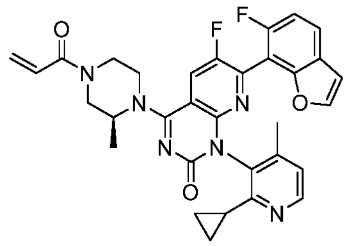

compound 125

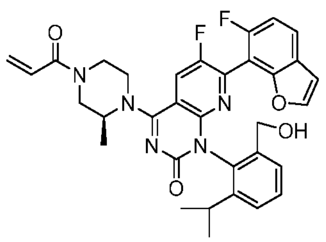

compound 126

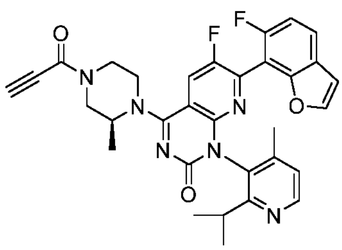

compound 127

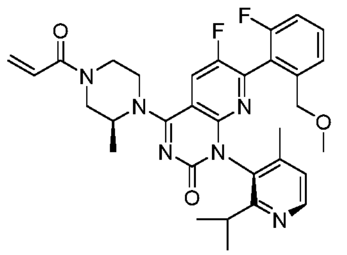

compound 48-1

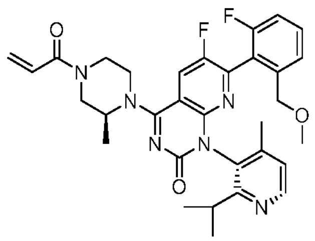

compound 48-2

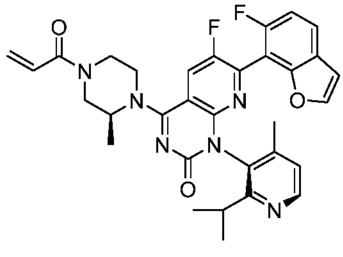

compound 70-1

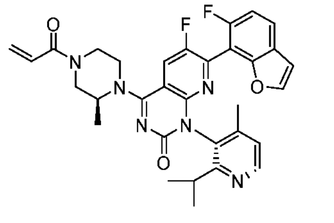

compound 70-2

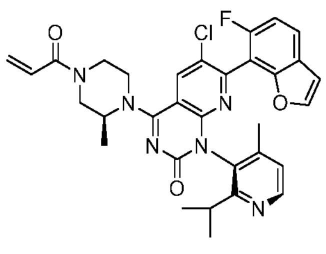

compound 73-1

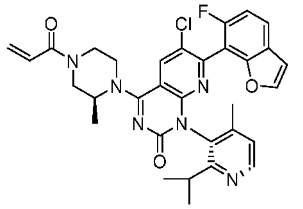
compound 73-2

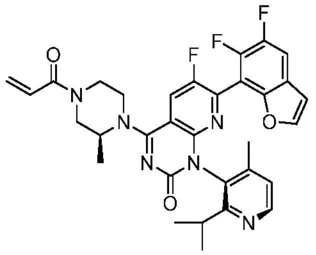
compound 74-1

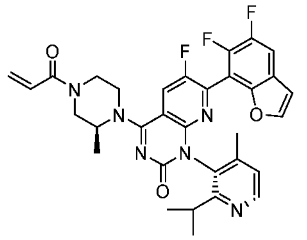
compound 74-2

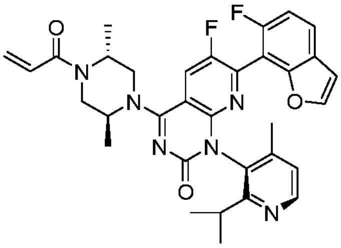
compound 89-1

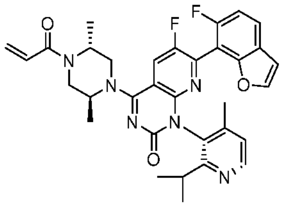
compound 89-2

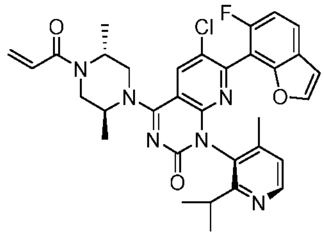
compound 90-1

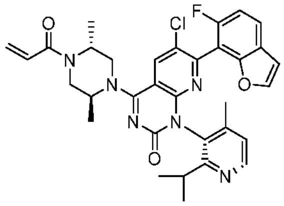
compound 90-2

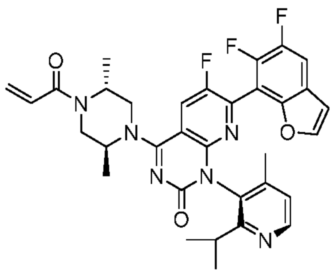
compound 91-1

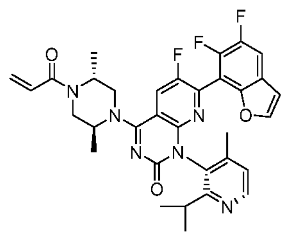
compound 91-2

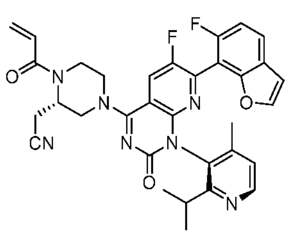
compound 100-1

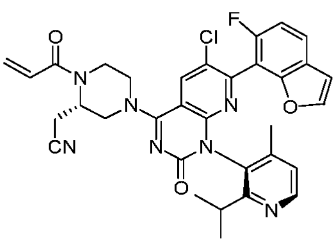
compound 101-1

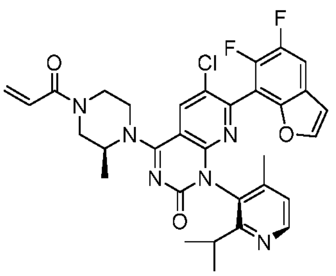
compound 103-1

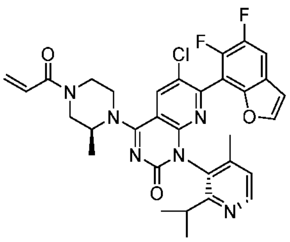
compound 103-2

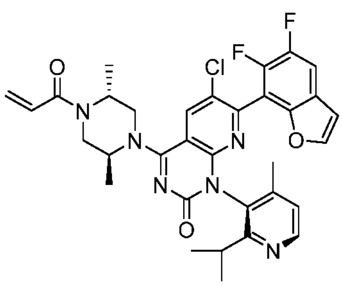
compound 104-1

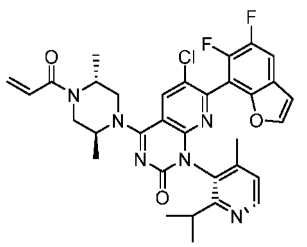
compound 104-2.

The present disclosure also provides the use of the above-mentioned Pyridopyrimidinone compounds or their pharmaceutically acceptable salts or stereoisomers or prodrug molecules.

The specific technical solutions are as follows:

Applications of the above-mentioned Pyridopyrimidinone compounds or their pharmaceutically acceptable salts or stereoisomers or prodrug molecules in the preparation of mutant KRAS inhibitors.

Applications of the above-mentioned Pyridopyrimidinone compounds or their pharmaceutically acceptable salts or stereoisomers or prodrug molecules in the preparation of KRAS G12C inhibitors.

Applications of the above-mentioned Pyridopyrimidinone compounds or their pharmaceutically acceptable salts or stereoisomers or prodrug molecules in the preparation of medicaments for preventing and/or treating tumors.

In some embodiments, the tumor is a hematological tumor or a solid tumor haboring KRAS G12C gene mutation.

In some embodiments, the tumor is: non-small cell lung cancer, small cell lung cancer, lymphoma, esophageal cancer, ovarian cancer, melanoma, cervical cancer, urothelial cancer, pancreatic cancer, breast cancer, liver cancer, stomach cancer, bile duct cancer, leukemia, melanoma, colon cancer, rectal cancer, endometrial cancer or glioma.

The present disclosure also provides an inhibitor against KRAS mutant.

The specific technical solutions are as follows:

An inhibitor against KRAS mutant, wherein its active ingredient comprises the Pyridopyrimidinone compounds or their pharmaceutically acceptable salts or stereoisomers or prodrug molecules.

The present disclosure also provides a pharmaceutical composition for preventing and/or treating tumors.

The specific technical solutions are as follows:

A pharmaceutical composition for preventing and/or treating tumors, comprising an active ingredient and a pharmaceutically acceptable excipient and/or carrier; The active ingredient comprises the Pyridopyrimidinone compounds or their pharmaceutically acceptable salts or stereoisomers or prodrug molecules.

The Pyridopyrimidinone compounds provided by the present disclosure can effectively inhibit KRAS G12C mutation, have strong anti-cell proliferation activity against KRAS G12C mutation tumors, and have the potential to be used for preventing or treating malignant tumors haboring KRAS G12C mutation, especially on non-small cell lung cancer (NSCLC) and colorectal cancer, which has great application value.

## Brief Description of Drawings

Fig. 1 is a graph of the mean drug time curve of compounds 69, 70-1, 73, 73-1, 74-1, 89, 89-1, 90 and 90-1 administered to rats by gavage (20 mg/kg).

Fig. 2 is a graph of the mean drug time curve of compounds AMG510, 91-1, 97, 102, 103, 103-1, 104, 104-1 and 122 administered to rats by gavage (20 mg/kg).

Fig. 3 shows the antitumor activity of compound 73-1 and compound 90-1 in H358 xenograft tumor model.

Fig. 4 shows the dose-response activity of compound 73-1 in H358 xenograft tumor model.

Fig. 5 shows the antitumor activity of compound 73-1 in the MIA Paca2 T2 xenograft tumor model.

## Description of Embodiments

In the compounds of the present disclosure, when any variable (eg. R, etc.) occurs more than once in any component, its definition at each occurrence is independent of the definition at each other occurrences. Similarly, combinations of substituents and variables are permissible if only the compound with such combinations are stabilized. A line from a substituent to a ring system indicates that the indicated bond may be attached to any substitutable ring atom. If the ring system is polycyclic, it means that such bonds are only attached to any suitable carbon atoms adjacent to the ring. It should be appreciated that an ordinary skilled in the art can select substituents and substitution patterns for the compounds of the present disclosure to provide compounds that are chemically stable and readily synthesized from the available starting materials by the methods described below. If a substituent itself is substituted by more than one group, it should be understood that these groups may be on the same carbon atom or on different carbon atoms, so long as the structure is stabilized.

The term "alkyl" in the present disclosure is meant to include branched and straight chain saturated aliphatic hydrocarbon groups having the specified number of carbon atoms. For example, the definition of "C1-C6" in "C1-C6 alkyl" includes groups having 1, 2, 3, 4, 5 or 6 carbon atoms arranged in a straight or branched chain. For example, "C1-C6 alkyl" specifically includes methyl, ethyl, n-propyl, isopropyl, n-butyl, tert-butyl, isobutyl, pentyl, hexyl.

The term "cycloalkyl" refers to a monocyclic saturated aliphatic hydrocarbon group having the specified number

of carbon atoms. For example, "cycloalkyl" includes cyclopropyl, cyclobutyl, cyclopentyl or cyclohexyl, and the like.

The term "alkoxy" refers to a group in which an alkyl group is directly attached to oxygen, a group with an -O-alkyl structure, such as -OCH3, $-OCH_2CH_3$, $-OCH_2CH_2CH_3$, $-O-CH_2CH(CH_3)_2$, $-OCH_2CH_2CH_2CH_3$, $-O-CH(CH_3)_2$, etc.

The term "heterocyclyl" is a saturated or partially unsaturated monocyclic or polycyclic cyclic substituent wherein one or more ring atoms are selected from N, O or $S(O)_m$ (wherein m is an integer from 0 to 2 ), and the remaining ring atoms are carbon, such as: morpholinyl, piperidinyl, tetrahydropyrrolyl, pyrrolidinyl, dihydroimidazolyl, dihydroisoxazolyl, dihydroisothiazolyl, dihydro oxadiazolyl, dihydrooxazolyl, dihydropyrazinyl, dihydropyrazolyl, dihydropyridyl, dihydropyrimidinyl, dihydropyrrolyl, dihydrotetrazolyl, dihydrothiadiazolyl , dihydrothiazolyl, dihydrothienyl, dihydrotriazolyl, dihydroazetidine, tetrahydrofuranyl, tetrahydrothienyl, etc., and their N-oxides. The connection of heterocyclic substituents can be carbon atoms or through heteroatoms.

The term "heteroaryl" refers to an aromatic ring containing one or more heteroatoms selected from O, N or S. Heteroaryl groups within the scope of the present disclosure include, but not limited to: quinolinyl, pyrazolyl, pyrrolyl, thienyl, furanyl, pyridyl, pyrimidinyl, pyrazinyl, triazolyl, imidazolyl, oxazolyl, isoxazolyl, pyridazinyl; "heteroaryl" can also be understood to include any nitrogen-containing N-oxide derivatives of heteroaryl groups.

The term "substituted" refers to the replacement of a hydrogen group in a particular structure with a group of the designated substituent.

As understood by the skilled in the art, "halogen" or "halo" as used herein means chlorine, fluorine, bromine and iodine. Unless otherwise defined, alkyl, cycloalkyl, aryl, heteroaryl and heterocycloalkyl substituents can be unsubstituted or substituted. For example, a C1-C6 alkyl group can be substituted by one, two, or three substituents selected from OH, halogen, alkoxy, dialkylamino, or heterocyclyl groups such as morpholinyl, piperidinyl, etc.

The present disclosure comprises free forms of compounds of Formula (I), Formula (II), Formula (III) or Formula (IV), as well as their pharmaceutically acceptable salts and stereoisomers. Some of the specific exemplary compounds herein are protonated salts of amine compounds. The term "free form" refers to the amine compound in non-salt form. The pharmaceutically acceptable salts" include not only exemplary salts of the particular compounds described herein, but also typical pharmaceutically acceptable salts of all compounds of Formula (I), Formula (II), Formula (III) or Formula (IV) in free form acceptable salt. The free forms of specific salts of the compounds can be isolated using techniques known in the art. For example, the free form can be regenerated by treating the salt with an appropriate dilute aqueous base such as dilute aqueous NaOH, dilute aqueous potassium carbonate, dilute aqueous ammonia, and dilute aqueous sodium bicarbonate. The free forms differ somewhat from their respective salt forms in certain physical properties such as solubility in polar solvents, but for the purposes of the invention, such salts of acid or base are otherwise pharmaceutically equivalent to their respective free forms.

The pharmaceutically acceptable salts of the present disclosure can be synthesized from the compounds containing a basic or acidic moiety in the present disclosure by conventional chemical methods. Generally, salts of basic compounds can be prepared by ion exchanged chromatography or by reacting the free base with a stoichiometric or excess amount of inorganic or organic acid in the desired salt form in a suitable solvent or combination of solvents. Similarly, salts of acidic compounds can be formed by reaction with a suitable inorganic or organic base.

Accordingly, pharmaceutically acceptable salts of the compounds in the present disclosure include conventional non-toxic salts of the compounds in the present disclosure formed by reacting a basic compound of the present disclosure with inorganic or organic acid. For example, conventional non-toxic salts include those derived from inorganic acids such as hydrochloric acid, hydrobromic acid, sulfuric acid, sulfamic acid, phosphoric acid, nitric acid. They also include those derived from organic acids such as acetic acid, propionic acid, succinic acid, glycolic acid, hard Fatty acid, lactic acid, malic acid, tartaric acid, citric acid, ascorbic acid, pamoic acid, maleic acid, hydroxymaleic acid, phenylacetic acid, glutamic acid, benzoic acid, salicylic acid, p-aminobenzenesulfonic acid, 2 - acetoxy - benzoic acid, fumaric acid, toluenesulfonic acid, methanesulfonic acid, ethanedisulfonic acid, oxalic acid, isethionic acid, and trifluoroacetic acid, etc.

If the compounds of the present disclosure are acidic, the appropriate "pharmaceutically acceptable salts" refers to the salts prepared by pharmaceutically acceptable non-toxic bases including inorganic bases and organic bases. The salts derived from inorganic bases include aluminum, ammonium, calcium, copper, iron, ferrous, lithium, magnesium, manganese, manganous, potassium, sodium, zinc, etc. Particularly preferably, ammonium salts, calcium salts, magnesium salts, potassium salts, and sodium salts. The salts derived from pharmaceutically acceptable organic non-toxic bases include salts of primary, secondary and tertiary amines. Substituted amines include naturally occurring substituted amines, cyclic amines and basic ion exchange resins such as Amino acid, betaine, caffeine, choline, N, N'-dibenzylethylenediamine, diethylamine, 2-diethylaminoethanol, 2-dimethylaminoethanol, aminoethanol, ethanolamine, ethyl Diamine, N-ethylmorpholine, N-ethylpiperidine, Glucosamine, Glucosamine, Histidine, Hydroxocobalamin, Isopropylamine, Lysine, Methylglucamine, Morpholine, Piperazine, Piperidine, quack, polyamine resin, procaine, purine, theobromine, triethylamine, trimethylamine, tripropylamine, tromethamine, etc.

The preparation of the pharmaceutically acceptable salts described above and other typical pharmaceutically acceptable salts was described in more detail by Berg et al., in "Pharmaceutical Salts," J. Pharm. Sci. 1977:66:1-19.

Since under physiological conditions, the deprotonated acidic moieties (such as carboxyl groups) in compounds

can be anions, which carry electric charges and can be neutralized by internally cationic protonated or alkylated basic moieties (such as tetravalent nitrogen atoms), so it should be noted that the compounds of the present disclosure are potential inner salts or zwitterions.

In one embodiment, the present disclosure provides compounds with the structure of Formula (I), Formula (II), Formula (III) or Formula (IV) and their pharmaceutically acceptable salts for the treatment of transitional proliferative diseases or symptoms such as human or other mammalian tumors.

In one embodiment, the compounds of the present disclosure and their pharmaceutically acceptable salts can be used for the treatment or control of non-small cell lung cancer, small cell lung cancer, lymphoma, esophageal cancer, ovarian cancer, melanoma, cervical cancer, urinary tract cancer, skin cancer, pancreatic cancer, breast cancer, liver cancer, stomach cancer, bile duct cancer, leukemia, melanoma, colon cancer, rectal cancer, endometrial cancer or glioma.

Drug metabolites and prodrugs: the metabolites of the compounds in the present disclosure and their pharmaceutically acceptable salts, and prodrugs that can be converted into the structures of the compounds in the present disclosure or their pharmaceutically acceptable salts in vivo, are also protected in the claims of this application.

Drug combinations: Compounds of Formula (I), Formula (II), Formula (III) or Formula (IV) may be used in combination with other drugs known to treat or improve similar conditions. When combined administration, the administration mode and dosage of the original drug remain unchanged, while the compounds of Formula (I), Formula (II), Formula (III) or Formula (IV) are administered simultaneously or subsequently. When the compounds of Formula (I), Formula (II), Formula (III) or Formula (IV) are administered concomitantly with one or more other drugs, it is preferable to use a pharmaceutical composition containing one or more known drugs and compounds of Formula (I), Formula (II), Formula (III) or Formula (IV). Drug combinations also include administration of compounds of Formula (I) with one or more other known drugs at an overlapping time. When the compounds of Formula (I), Formula (II), Formula (III) or Formula (IV) is used in combination with one or more other drugs, compounds of Formula (I), Formula (II), Formula (III) or Formula (IV) or known drugs may be administered at lower doses than they would be when administered alone.

Drugs or active ingredients that can be used in combination with compounds of Formula (I), Formula (II), Formula (III) or Formula (IV) include, but not limited to: Estrogen receptor modulators, androgen receptor modulators, retinal-like receptor modulators, cytotoxic/cytostatics, antiproliferative agents, protein transferase inhibitors, HMG-CoA reductase inhibitors, HIV protein kinase inhibitors agents, reverse transcriptase inhibitors, angiogenesis inhibitors, cell proliferation and survival signaling inhibitors, drugs that interfere with cell cycle checkpoints and apoptosis inducers, cytotoxic drugs, tyrosine protein inhibitors, EGFR inhibitors, VEGFR inhibitors, serine/threonine protein inhibitors, Bcr-Abl inhibitors, c-Kit inhibitors, Met inhibitors, Raf inhibitors, MEK inhibitors, MMP inhibitors, topoisomerase inhibitors, histidine Acid deacetylase inhibitors, proteasome inhibitors, CDK inhibitors, Bcl-2 family protein inhibitors, MDM2 family protein inhibitors, IAP family protein inhibitors, STAT family protein inhibitors, PI3K inhibitors, AKT inhibitors , integrin blocker, interferon-a, interleukin-12, COX-2 inhibitor, p53, p53 activator, VEGF antibody, EGF antibody, cytotoxic T lymphocyte-associated antigen 4 (CTLA4) antibody, programmed Cell death 1 (PD-1) antibody, programmed cell death-ligand 1 (PD-L1) antibody, etc.

In one embodiment, drugs or active ingredients that can be used in combination with compounds of Formula (I), Formula (II), Formula (III) or Formula (IV) include, but are not limited to: Aldesleukin, Alendronic Acid, Interferon, Altranoin, Allopurinol, Sodium Allopurinol, Palonosetron Hydrochloride, Hexamelamine, Aminoglumitide, Amifostine, Ammonium rubicin, ampicillin, anastrozole, dolasetron, aranesp, arglabin, arsenic trioxide, anoxin, 5-azacytidine, azathioprine, bacille Calmette-Guerin or tic BCG, betadine, beta-acetate Metasone, betamethasone sodium phosphate preparation, bexarotene, bleomycin sulfate, bromouridine, bortezomib, busulfan, calcitonin, alezolizumab injection, capecitabine, carboplatin, kangshi cefesone, cymoleukin, daunorubicin, chlorambucil, cisplatin, cladribine, cladribine, clodronate, cyclophosphamide, cytarabine, dacarbazine, actinobacteria D, Daunorubicin Liposome, Dexamethasone, Dexamethasone Phosphate, Estradiol Valerate, Denisole 2, Dipomet, Delorelin, Delazoxan, Diethylstilbestrol, Dafucon, Docetaxel, Deoxyfluridine, Doxorubicin, Dronabinol, Chin-166-chitosan complex, eligard, rasburicase, epirubicin hydrochloride, aprepitant, epirubicin, epoetin alfa, erythropoietin, eplatin, Levamisole tablets, estradiol preparations, 17-beta-estradiol, estramustine sodium phosphate, ethinyl estradiol, amifostine, hydroxyphosphate, fenbifu, etoposide, fadrozole, tamoxib fenestrate, filgrastim, finasteride, filgrastim, floxuridine, fluconazole, fludarabine, 5-fluorodeoxyuridine monophosphate, 5-fluorouracil, fluoxymesterone, Flutamide, Formestan, 1-β-D-D-arabinofuranocytothidine-5'-stearoyl phosphate, Formustine, Fulvestrant, Gammaglobulin, Gemcitabine, Gemtox Monoclonal antibody, imatinib mesylate, carbazide, wax paper capsules, goserelin, granisilone hydrochloride, histrelin, and methoxine, hydrocortisone, erythro-hydroxynonyl adrenal gland Purine, hydroxyurea, titan isibemumab, idarubicin, ifosfamide, interferon alpha, interferon-alpha2, interferon alpha-2A, interferon alpha-2B, interferon alpha-nl, Interferon alpha-n3, interferon beta, interferon gamma-la, interleukin-2, intron A, Iressa, irinotecan, keteri, lentinan sulfate, letrozole, tetrahydroform Folic acid, leuprolide, leuprolide acetate, levothyroxine, levothyroxine calcium salt, levothyroxine sodium, levothyroxine sodium preparations, lomustine, lonidamine, dronabinol, Nitrogen mustard, mecobalamin, medroxyprogesterone acetate, megestrol acetate, melphalan, esterified estrogen, 6-mercaptopurine, mesna, methotrexate, methyl aminolevulinate , mitifoxine,

minocycline, mitomycin C, mitotane, mitosodium quinone, trolosteine, doxorubicin citrate liposome, nedaplatin, pegylated filgras kiosk, opryleukin, neupogen, nilutamide, tamoxifen, NSC-631570, Recombinant Human Interleukin 1-β, Octreotide, Ondansetron Hydrochloride, Dehydrocortisone Oral Solution, Oxaliplatin, Paclitaxel, Prednisone Sodium Phosphate, Pegaspargase, Pegasin, Pentostatin, Streptolyticum, Pilocarpine Hydrochloride, Pirarubicin, Pukamycin, Porfimer Sodium, Prednimustine, Steprednisolone, Prednisone, Premax Li, Procarb, Recombinant Human Erythropoietin, Raltitrexed, Ribi, Etidronate, Rhenium-186, Rituxan, Strength-A, Romotide, Pilocarpine Hydrochloride Tablets, Octreotide, Samostim, Semustine, Sizoran, Sobuzoxan, Methylprednisolone, Paphos Acid, Stem Cell Therapy, Streptozocin, Strontium Chloride-89, Levothyroxine Sodium, Tamoxifen, Tansu Loxin, Tasonamin, Tastolactone, Taxotere, Tecethiazine, Temozolomide, Teniposide, Testosterone Propionate, Methyltestosterone, Thioguanine, Thiatepa, Thyroid Stimulating Hormone, Tiludronic Acid, Topotecan, Toremifene, Tosilimumab, Trastuzumab, Triosulfan, Tretinoin, Methotrexate Tablets, Trimethylmelamine, Trimethrexate, Tripro acetate Relin, triptorelin pamoate, eufradine, uridine, valrubicin, veslinone, vinblastine, vincristine, vincristine, vinorelbine, velulizine, dextran Propionimine, net statin, zofenin, paclitaxel protein stabilizer, acolbifene, interferon r-lb, affinitak, aminopterin, azoxifene, asoprisnil, atamestane, atrasentan, BAY 43-9006, Avastin, CCI-779, CDC-501, Celebrex, Cetuximab, Crinator, Cyproterone Acetate, Decitabine, DN-101, Doxorubicin-MTC, dSLIM, dutasteride, edotecarin, eflunomine, ixitecan, fenretinide, histamine dihydrochloride, histidine hydrogel implant, holmium-166 DOTMP, ibandronic acid, interferon gamma, intron-PEG, ixabepilone, keyhole limpet hemocyanin, L-651582, lanreotide, Lasoxifene, libra, lonafamib, imiprexifene, minoxifene, MS-209, liposomal MTP-PE, MX-6, nafarelin, nemorubicin, novarestat , nolatrexide, olimerson, onco-TCS, osidem, paclitaxel polyglutamate, sodium paclitaxel, PN-401, QS-21, quasiyang, R-1549, raloxifene, leopard Ranazyme, 13-cis-retinoic acid, satraplatin, siocalcidol, T-138067, tarceva, docosahexaenoate paclitaxel, thymosin alphal, gazofurin, tipifarnib, tirapazamine, TLK-286, toremifene, trans MID-lo7R, valspoda, vapretide, vatalanib, verteporfin, vinflunine, Z-100 and zolelinic acid or their combination.

Synthetic methods: In addition to standard methods known in the literature or exemplified in experimental procedures, the compounds of the present disclosure can be prepared using the methods in the following synthetic schemes (Schemes 1-10). Combined with the following synthetic schemes, the compounds and synthetic methods described in the present disclosure can be better understood. The synthetic schemes describe the methods that can be used to prepare the compounds in the present disclosure, and the methods are merely illustrative scheme descriptions for illustrative purposes, but do not limit the scope of the present disclosure.

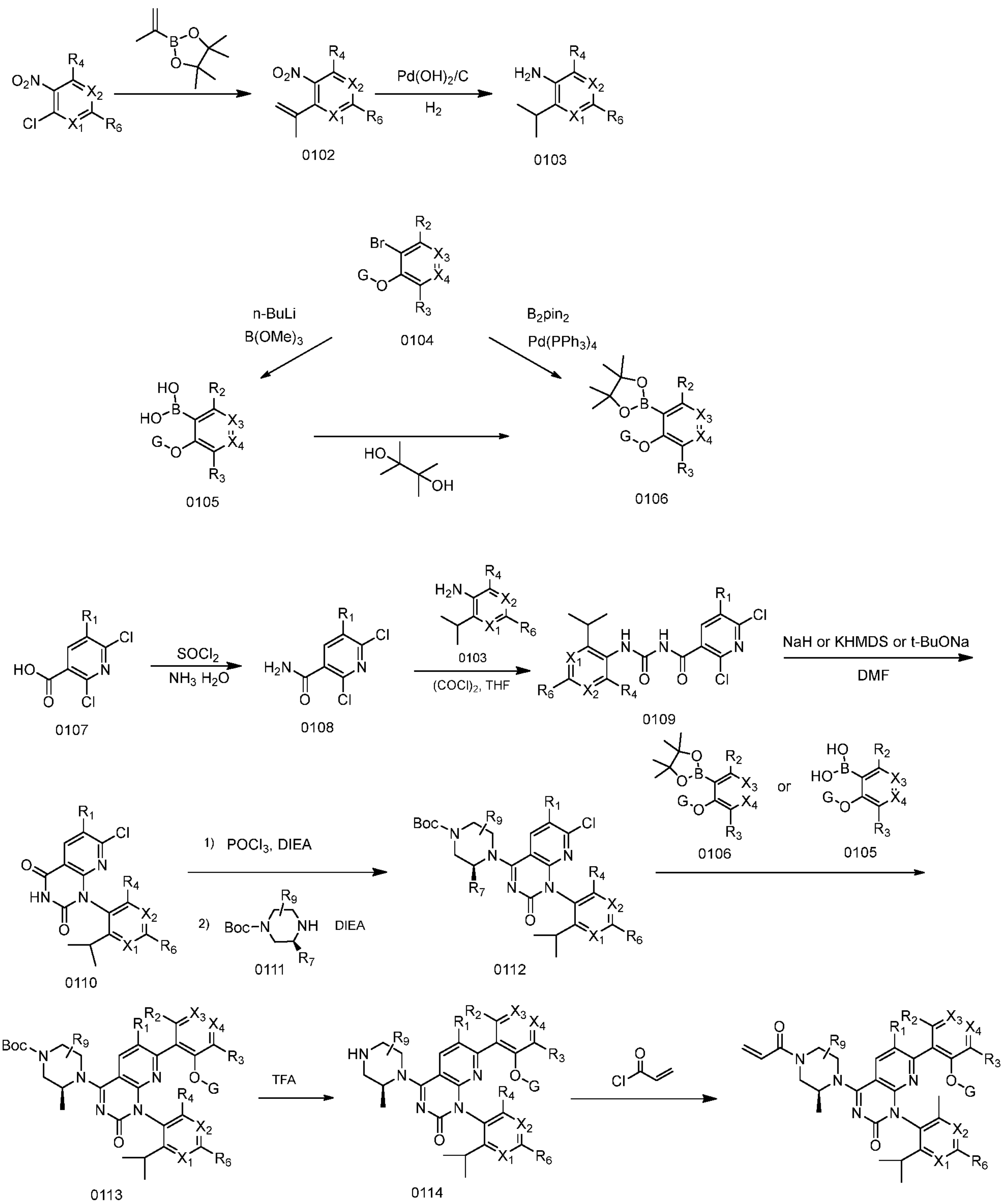


0102 0103 0104 0105 0106 0107 0108 0103 0109 0110 0111 0112 0106 or 0105 0113 0114

Scheme 1

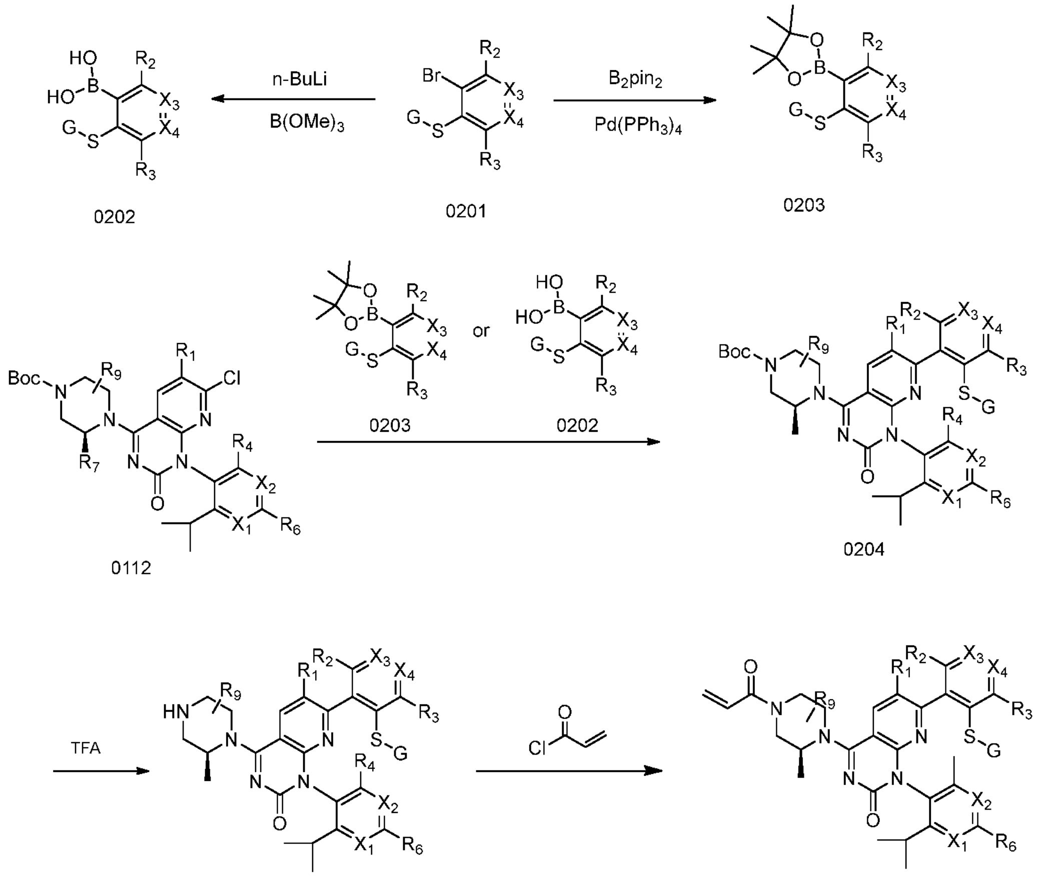


0202 0201 0203

0203 0202

0112 0204

0205

Scheme 2

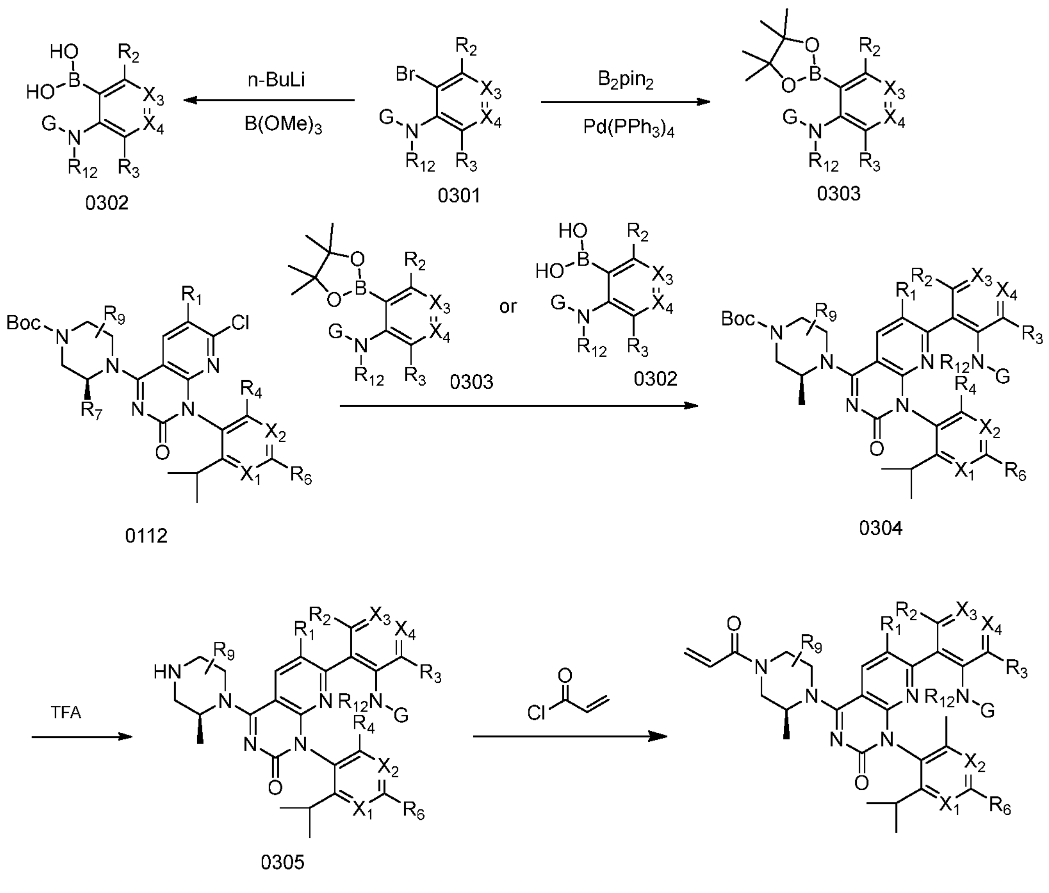
n-BuLi
B(OMe)3
B2pin2
Pd(PPh3)4
0302
0301
0303
or
0303
0302
0112
0304
TFA
0305

Scheme 3

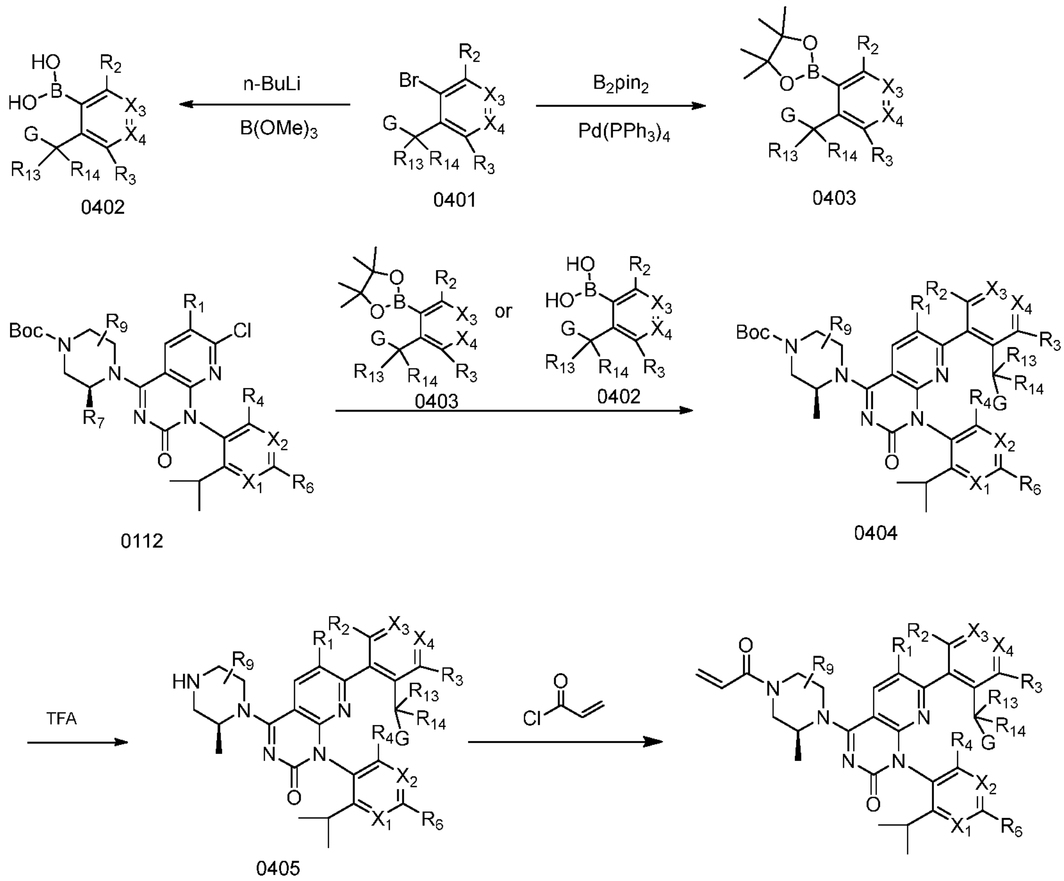


0402 0401 0403

0112 0403 0402 0404

0405

Scheme 4

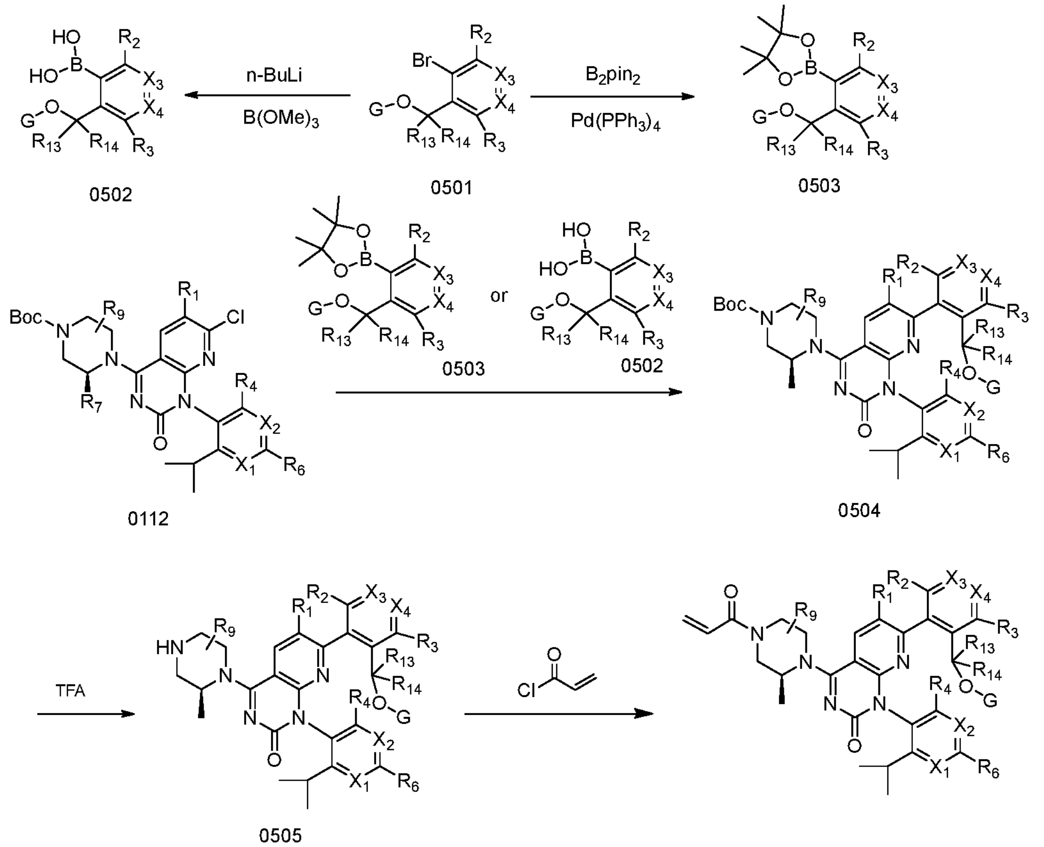


0502 0501 0503

0503 or 0502

0112 0504

0505

Scheme 5

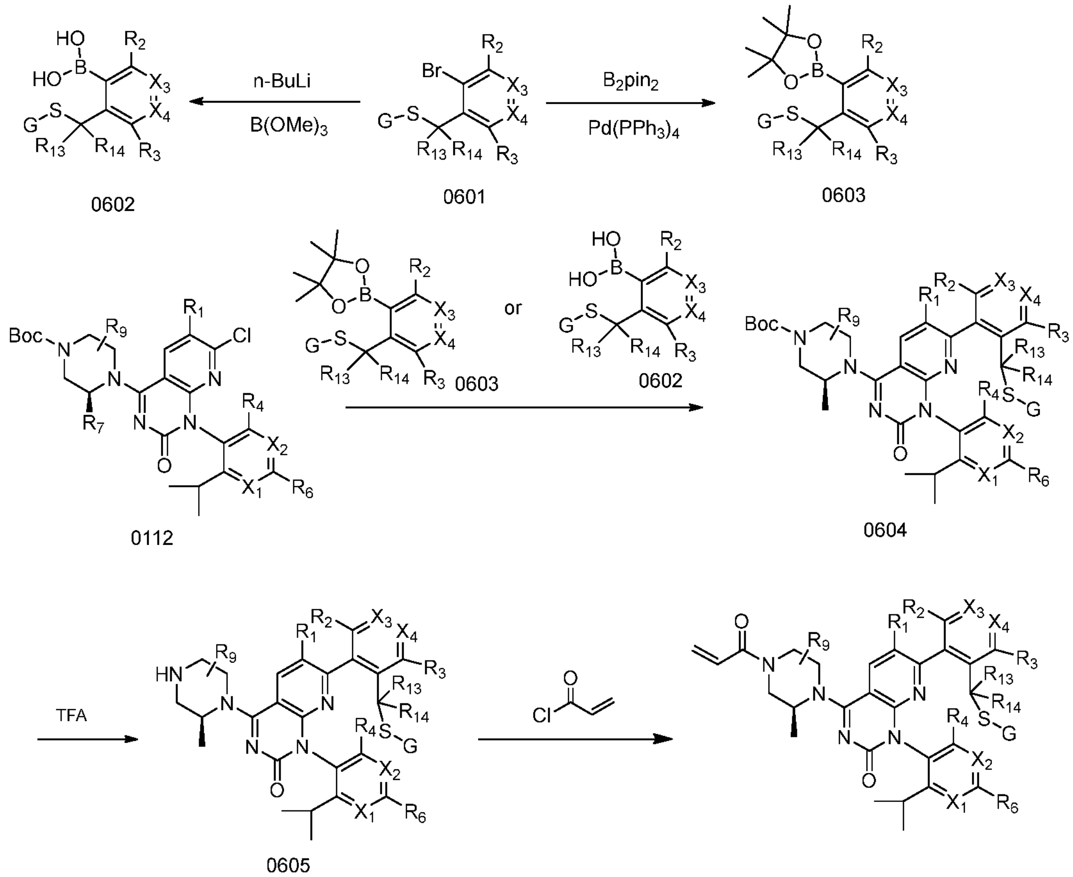


0602 0601 0603

0112 0603 0602 0604

0605

Scheme 6

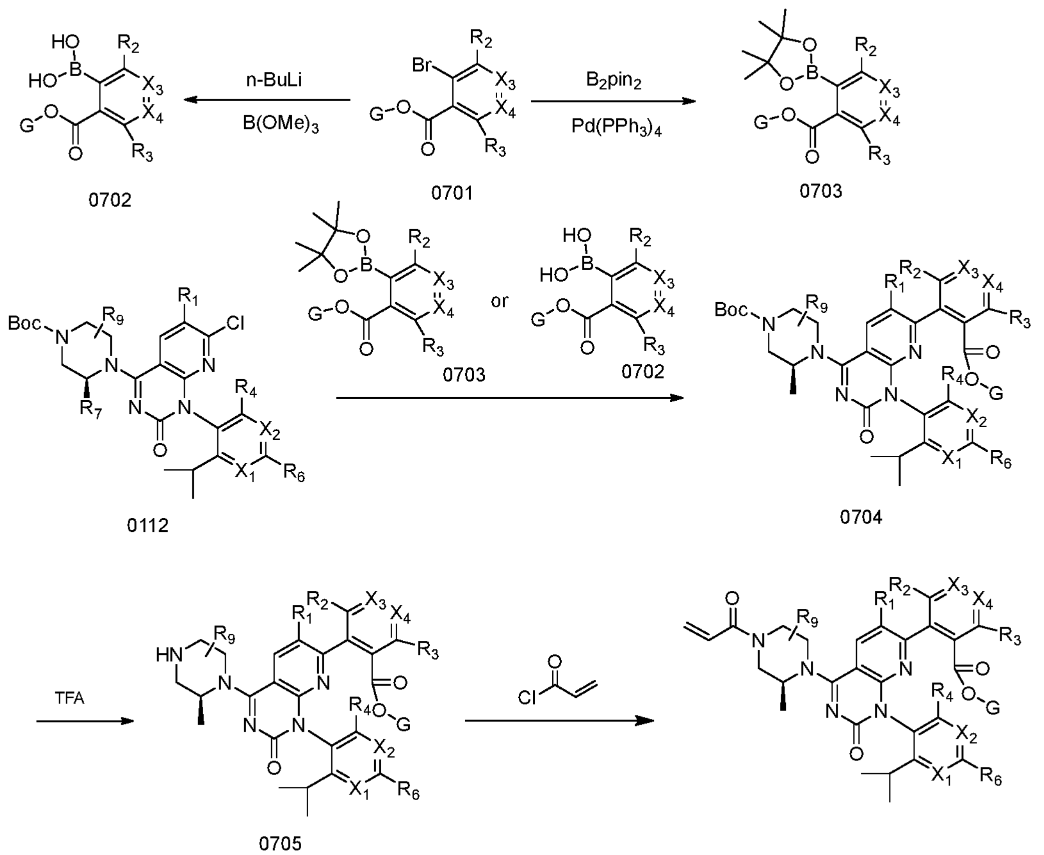
n-BuLi
B(OMe)3
B2pin2
Pd(PPh3)4
0702
0701
0703
or
0703
0702
0112
0704
TFA
0705

Scheme 7

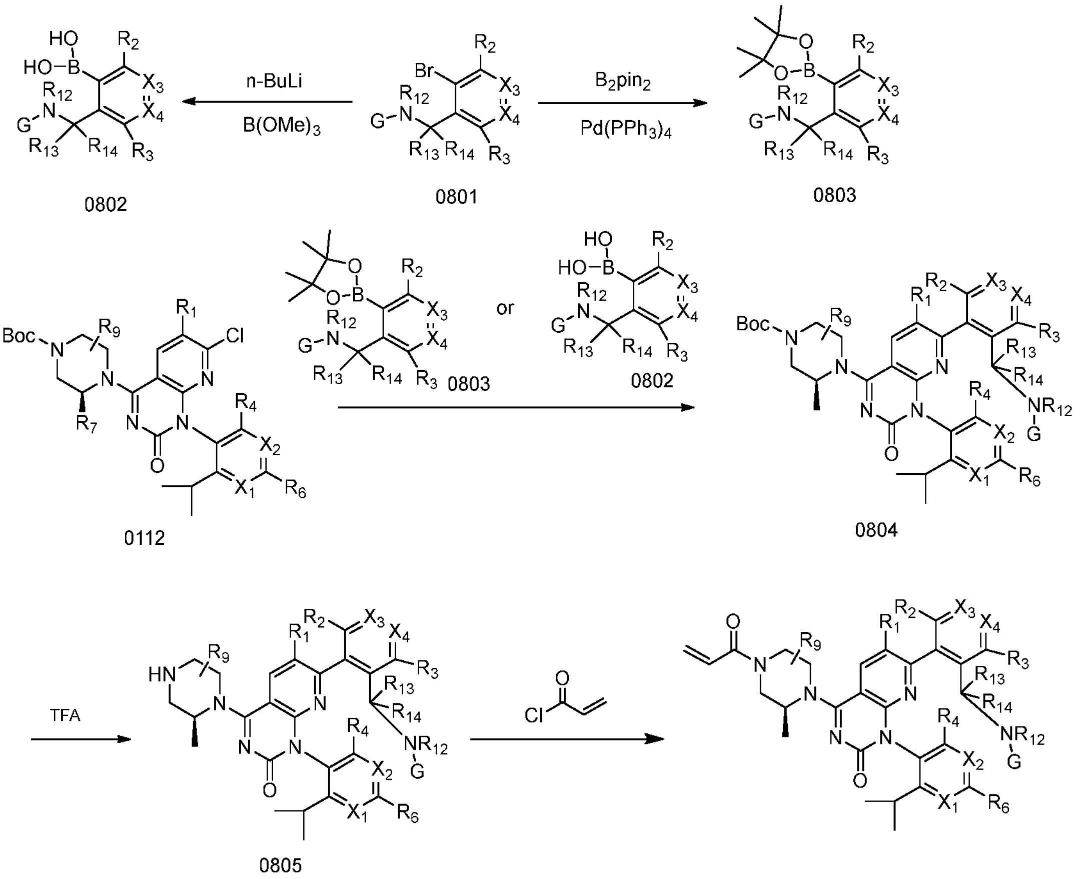


0802 0801 0803

0112 0803 0802 0804

0805

Scheme 8

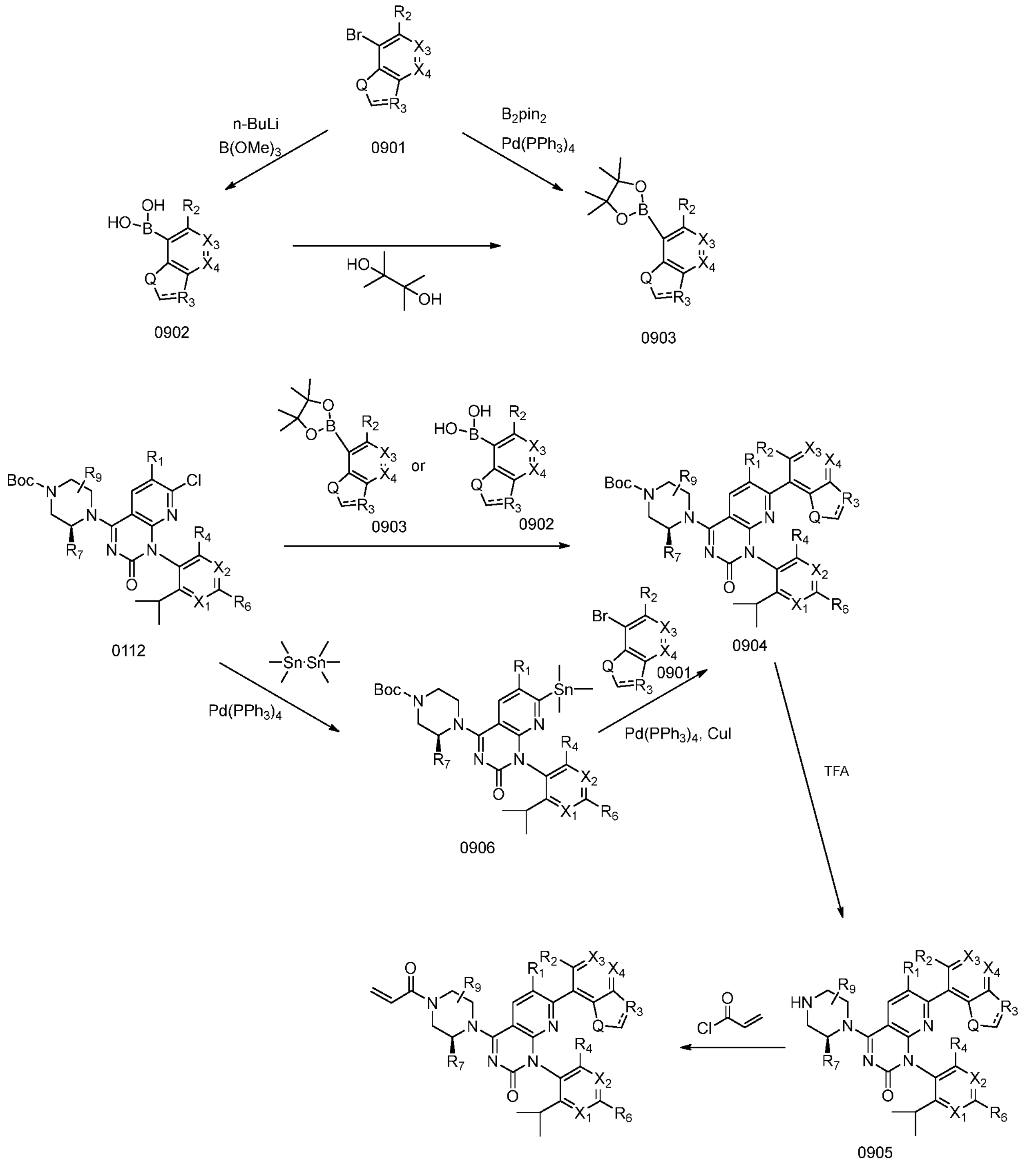
n-BuLi
B(OMe)$_3$
0901
B$_2$pin$_2$
Pd(PPh$_3$)$_4$
0902
0903
0903
or
0902
0112
0904
0901
Pd(PPh$_3$)$_4$
Pd(PPh$_3$)$_4$, CuI
0906
TFA
0905

Scheme 9

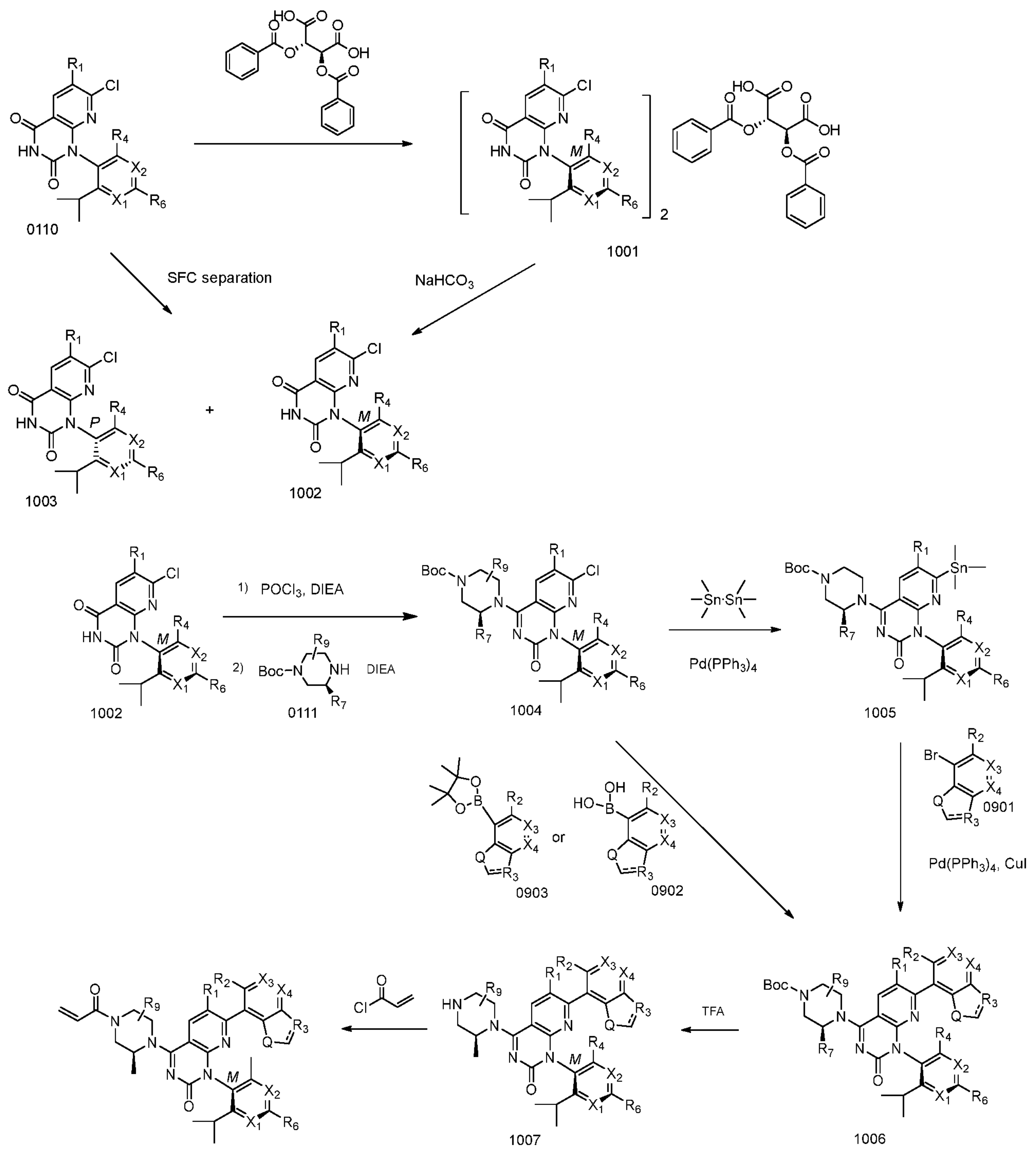
0110
1001
SFC separation
NaHCO$_3$
1003
+
1002
1) POCl$_3$, DIEA
2) DIEA
0111
1004
Pd(PPh$_3$)$_4$
1005
0901
Pd(PPh$_3$)$_4$, CuI
0903
or
0902
TFA
1006
1007

Scheme 10-1

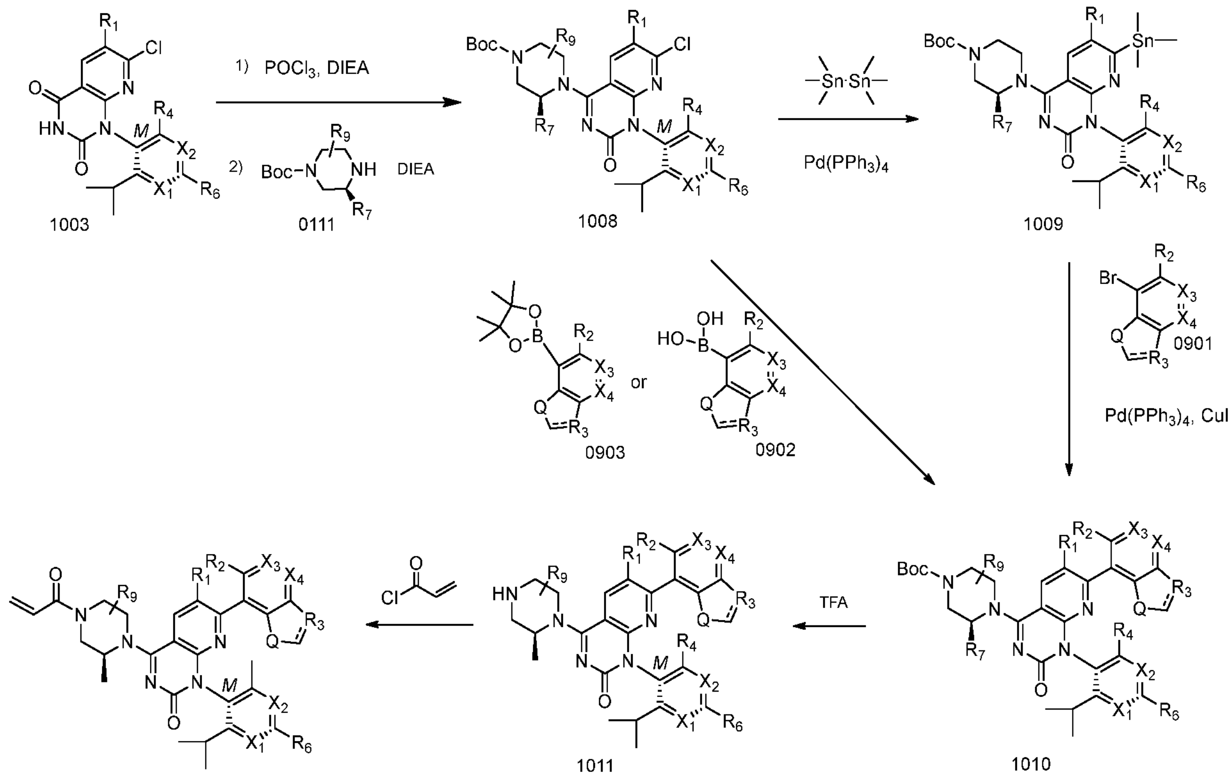


Scheme 10-2

The following are specific examples:

**Example 1: Preparation of 4-((S)-4-acryloyl-2-methylpiperazin-1-yl)-6-fluoro-7-(2-fluoro-6-methoxyphenyl)-1-(2-isopropyl-4-methylpyridin-3-yl)pyrido[2,3-d]pyrimidin-2(1H)-one (compound 1) (prepared according to Scheme 1)**

Step 1a: Preparation of 4-methyl-3-nitro-2-(prop-1-en-2-yl)pyridine (compound 0102-1): A mixture of 2-chloro-4-methyl-3-nitropyridine (10.0 g, 58.0 mmol, 1.0 eq.), 4,4,5,5-tetramethyl-2-(prop-1-en-2-yl)-1,3,2-dioxaborolane (10.7 g, 64.0 mmol, 1.1 eq.), potassium carbonate (16.0 g, 116.0 mmol, 2.0 eq.) and bis(triphenylphosphine)palladium(II) chloride (1.0 g, 1.45 mmol, 0.025 eq.) in DMF/water=10/1 (150 mL) was stirred at 92°C overnight under the protection of nitrogen. After cooling to room temperature, the mixture was poured into water and extracted with ethyl acetate. The organic layer was washed with 2M HCl, and the aqueous layer was added sodium hydroxide to adjust PH>8 and then extracted with ethyl acetate. The organic layer was dried over anhydrous sodium sulfate and concentrated in vacuo to give 4-methyl-3-nitro-2-(prop-1-en-2-yl)pyridine (9.0 g, yield: 90.0%) as a yellow oil. MS ($ES^+$): m/z=179 $(M+H)^+$.

Step 1b: Preparation of 2-isopropyl-4-methylpyridin-3-amine (compound 0103-1): A mixture of 4-methyl-3-nitro-2-(prop-1-en-2-yl)pyridine (0102-1) (9.0 g, 50.6 mmol, 1.0 eq.) and $Pd(OH)_2/C$ (900 mg, 10% w/w) in methanol (80 mL) was stirred at room temperature under hydrogen atmosphere overnight. The reaction mixture was filtered and the filtrate was concentrated under reduced pressure to give 2-isopropyl-4-methylpyridin-3-amine (8.5 g, yield: 94.4%) as a yellow oil. MS ($ES^+$): m/z=151 $(M+H)^+$.

Step 1c: Preparation of 2,6-dichloro-5-fluoronicotinamide (compound 0108-1): A solution of 2,6-dichloro-5-fluoronicotinic acid (0107-1) (10.0 g, 48.0 mmol, 1.0 eq.) in thionyl chloride (30 mL) was refluxed for 3 h protected form moisture. After cooling to room temperature, the solvent was removed in vacuo. The residue was dissolved in tetrahydrofuran (50 mL), and the solution was added dropwise to an ammonium hydroxide solution (30 mL) in an ice-water bath. After addition, the mixture was stirred for 30 min and water was then added. The mixture was filtered and the filter cake was washed with water and dried in vacuo to give 2,6-dichloro-5-fluoronicotinamide (6.1 g, yield: 61.0%) as a white solid. MS ($ES^+$): m/z=209 $(M+H)^+$.

Step 1d: Preparation of 2,6-dichloro-5-fluoro-N-((2-isopropyl-4-methylpyridin-3-yl)carbamoyl)nicotinamide (compound 0109-1): A mixture of 2,6-dichloro-5-fluoronicotinamide (0108-1) (2.0 g, 10.0 mmol, 1.0 eq.) and oxalyl chloride (2.5 g, 20.0 mmol, 2.0 eq.) in tetrahydrofuran (40 mL) was stirred at 75°C for 2 h under the protection of nitrogen. After cooling to room temperature, the solvent was removed in vacuo. The residue was dissolved in tetrahydrofuran (20

mL), and a solution of 2-isopropyl-4-methylpyridin-3-amine (0103-1) (1.8 g, 12.0 mmol, 1.2 eq.) in tetrahydrofuran (15 mL) was added dropwsie in an ice-water bath. After addition, the mixture was stirred for 30 min and ethyl acetate was added. The mixture was filtered and the filtrate was washed with aqueous sodium carbonate solution, water and brine, dried over anhydrous sodium sulfate and concentrated in vacuo to give the crude product 2,6-dichloro-5-fluoro-N-((2-isopropyl-4-methylpyridin-3-yl)carbamoyl)nicotinamide which was used directly in the next step without further purification. MS ($ES^+$): m/z=385 $(M+H)^+$.

Step 1e: Preparation of 7-chloro-6-fluoro-1-(2-isopropyl-4-methylpyridin-3-yl)pyrido[2,3-d]pyrimidine-2,4(1H,3H)-dione (compound 0110-1): Under the protection of nitrogen, to a solution of 2,6-dichloro-5-fluoro-N-((2-isopropyl-4-methylpyridin-3-yl)carbamoyl)nicotinamide (compound 0109-1) (3.2 g, 8.3 mmol, 1.0 eq.) in DMF (50 mL) was added 60% sodium hydride (1.0 g, 24.9 mmol, 3.0 eq.) portionwise in an ice-water bath, and the mixture was stirred for 1 h. The reaction solution was quenched with saturated aqueous ammonium chloride solution and stirred for 10 min, and then extracted with ethyl acetate. The organic layer was washed with brine and concentrated in vacuo. The residue was purified by column chromatography on silica gel (eluent: petroleum ether/ethyl acetate=10/1 to 4/1) to give 7-chloro-6-fluoro-1-(2-isopropyl-4-methylpyridin-3-yl)pyrido[2,3-d]pyrimidine-2,4(1H,3H)-dione (2.0 g, yield: 69.0%) as a white solid. MS ($ES^+$): m/z=349 $(M+H)^+$.

Step 1f: Preparation of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0112-1): Under the protection of nitrogen, a mixture of 7-chloro-6-fluoro-1-(2-isopropyl-4-methylpyridin-3-yl)pyrido[2,3-d]pyrimidine-2,4(1H,3H)-dione (0110-1) (1.6 g, 4.6 mmol, 1.0 eq.), DIPEA (3.0 g, 23.0 mmol, 5.0 eq.) and phosphorus oxychloride (1.0 g, 6.9 mmol, 1.5 eq.) in acetonitrile (30 mL) was stirred at 85°C for 2 h. After cooling to room temperature, the solvent was removed in vacuo. The residue was dissolved in tetrahydrofuran (20 mL), and DIPEA (1.8 g, 13.8 mmol, 3.0 eq.) and tert-butyl (S)-3-methylpiperazine-1-carboxylate (0111-1) (1.1 g, 5.5 mmol, 1.2 eq.) were added in an ice-water bath, and the mixture was stirred for 1 h. Ethyl acetate was added and the mixture was washed with water and brine. The organic layer was concentrated in vacuo. The residue was purified by column chromatography on silica gel (eluent: dichloromethane/methanol=200/1 to 80/1) to give tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (1.2 g, yield: 50.0%) as a yellow solid. MS ($ES^+$): m/z=531 $(M+H)^+$.

Step 1g: Preparation of tert-butyl (3*S*)-4-(6-fluoro-7-(2-fluoro-6-methoxyphenyl)-1-(2-isopropyl-4-methylpyridin-3-yl)-2-oxo-1,2-dihydropyrido[2,3-*d*]pyrimidin-4-yl)-3-methylpiperazine-1-carboxylate (compound 0113-1): Under the protection of nitrogen, (2-fluoro-6-methoxyphenyl)boronic acid (0105-1) (80 mg, 0.47 mmol, 1.7 eq.), tert-butyl (S)-4-(7-chloro-6-fluoro-1-(2-isopropyl-4-methylpyridin-3-yl)-2-oxo-1,2-dihydropyrido[2,3-*d*]pyrimidin-4-yl)-3-methylpiperazine-1-carboxylate (0112-1) (150 mg, 0.28 mmol, 1.0 eq.), tetrakis(triphenylphosphine)palladium (33 mg, 0.028 mmol, 0.1 eq.), and sodium carbonate (90 mg, 0.85 mmol, 3.0 eq.) were added into a mixed solvent of 25 mL acetonitrile and 5 mL water. The mixture was heated and stirred at 80°C for 6.0 h. The mixture was cooled to room temperature, and extracted with ethyl acetate and water. The organic phase was washed with brine, and concentrated under vacuum. The residue was purified by column chromatography on silica gel (eluent: dichloromethane/methanol=30/1 to 15/1) to give tert-butyl (3*S*)-4-(6-fluoro-7-(2-fluoro-6-methoxyphenyl)-1-(2-isopropyl-4-methylpyridin-3-yl)-2-oxo-1,2-dihydropyrido[2,3-*d*]pyrimidin-4-yl)-3-methylpiperazine-1-carboxylate (170 mg, yield: 96%) as a yellow solid. MS ($ES^+$): m/z=621 $(M+H)^+$.

Step 1h: Preparation of 6-fluoro-7-(2-fluoro-6-methoxyphenyl)-1-(2-isopropyl-4-methylpyridin-3-yl)-4-((*S*)-2-methylpiperazin-1-yl)pyrido[2,3-*d*]pyrimidin-2(1*H*)-one (compound 0114-1): tert-butyl (3*S*)-4-(6-fluoro-7-(2-fluoro-6-methoxyphenyl)-1-(2-isopropyl 4-methylpyridin-3-yl)-2-oxo-1,2-dihydropyrido[2,3-*d*]pyrimidin-4-yl)-3-methylpiperazine-1-carboxylate (0113-1) (170 mg, 0.27 mmol, 1.0 eq.) was dissolved in 50 mL dichloromethane. TFA (5 mL) was added dropwise, and the mixture was stirred at room temperature for 1 h. The mixture was concentrated under vacuum and the residue was used directly in the next step without further purification. MS (ES+): m/z=521 (M+H)+.

Step 1i: Preparation of 4-((*S*)-4-acryloyl-2-methylpiperazin-1-yl)-6-fluoro-7-(2-fluoro-6-methoxyphenyl)-1-(2-isopropyl-4-methylpyridin-3-yl)pyrido[2,3-*d*]pyrimidin-2(1*H*)-one (compound 1): The crude product of 6-fluoro-7-(2-fluoro-6-methoxyphenyl)-1-(2-isopropyl-4-methylpyridin-3-yl)-4-((*S*)-2-methylpiperazin-1-yl)pyrido[2,3-*d*]pyrimidin-2(1*H*)-one (0114-1) was dissolved in 20 mL tetrahydrofuran, and then 4.0 mL DIPEA was added. The mixture was cooled in an ice-water bath and acryloyl chloride (37 mg, 0.41 mmol, 1.5 eq.), pre-dissolved in 0.5 mL tetrahydrofuran, was added dropwise. The mixture was stirred for 30 min and water was added and extracted with ethyl acetate. The organic phase was washed with brine and concentrated under vacuum to give a residue which was purified by prep-TLC (silica gel, eluent: dichloromethane/methanol=30/1) to give 4-((*S*)-4-acryloyl-2-methylpiperazin-1-yl)-6-fluoro-7-(2-fluoro-6-methoxyphenyl)-1-(2-isopropyl-4-methylpyridin-3-yl)pyrido[2,3-*d*]pyrimidin-2(1*H*)-one (50 mg, yield: 32%) as a yellow solid. MS ($ES^+$): m/z=575 $(M+H)^+$. Melting point: 158~160°C. $^1H$ NMR (500 MHz, DMSO) $\delta$ 8.38 (d, *J*= 4.8 Hz, 1H), 8.32 (m, 1H), 7.46 (dd, *J* = 15.6, 8.0 Hz, 1H), 7.18 (d, *J* = 4.8 Hz, 1H), 6.97 (d, *J* = 8.5 Hz, 1H), 6.86 (m, 2H), 6.21 (d, *J* = 16.3 Hz, 1H), 5.76 (d, *J* = 10.9 Hz, 1H), 4.93 (d, *J* = 29.1 Hz, 1H), 4.34 (dd, *J* = 34.0, 18.7 Hz, 2H), 4.08 (dd, *J* = 58.7, 11.0 Hz, 1H), 3.65 (m, 5H), 3.13 (m, 1H), 2.72 (m, 1H), 1.90 (s, 3H), 1.34 (dd, *J* = 15.3, 6.7 Hz, 3H), 1.08 (d, *J* = 6.6 Hz, 3H),

0.92 (d, *J* = 5.7 Hz, 3H).

**Example 2: 4-((*S*)-4-acryloyl-2-methylpiperazin-1-yl)-6-fluoro-7-(2-fluoro-6-isopropoxyphenyl)-1-(2-isopropyl-4-methylpyridin-3-yl)pyrido[2,3-*d*]pyrimidin-2(1*H*)-one (compound 3) (prepared according to Scheme 1)**

Step 2a: Preparation of (2-fluoro-6-isopropoxyphenyl)boronic acid (compound 0105-3):1-fluoro-2-bromo-3-isopropoxybenzene (0104-3) (300 mg, 1.29 mmol, 1.0 eq.) was dissolved in 20 mL anhydrous tetrahydrofuran. The solution was cooled to -70°C. n-BuLi (1.6M in hexane, 1.45 mL, 2.32 mmol, 1.8 eq.) was added dropwise under the protection of nitrogen. The mixture was stirred at -70°C for 30 min and trimethyl borate (201 mg, 1.93 mmol, 1.5 eq.), pre-dissolved in 1 mL anhydrous tetrahydrofuran, was added dropwise into the mixture above. The mixture was stirred at -70°C for 1 h and quenched with aqueous ammonium chloride solution. ethyl acetate and water was added to extract. The organic phase was washed with brine and dried over anhydrous sodium sulfate, concentrated under vacuum. The residue was used directly in the next step without further purification.

Step 2b: Preparation of tert-butyl (3*S*)-4-(6-fluoro-7-(2-fluoro-6-isopropoxyphenyl)-1-(2-isopropyl-4-methylpyridin-3-yl)-2-oxo-1,2-dihydropyrido[2,3-*d*]pyrimidin-4-yl)-3-methylpiperazine-1-carboxylate (compound 0113-3): The synthetic method was similar to Step 1g of EXAMPLE 1, except that compound 0105-1 therein was replaced by (2-fluoro-6-isopropoxyphenyl)boronic acid (0105-3), tert-butyl (3*S*)-4-(6-fluoro-7-(2-fluoro-6-isopropoxyphenyl)-1-(2-isopropyl-4-methylpyridin-3-yl)-2-oxo-1,2-dihydropyrido[2,3-*d*]pyrimidin-4-yl)-3-methylpiperazine-1-carboxylate was obtained (125 mg, yield: 83%) as a yellow solid. MS ($ES^+$): m/z=649 $(M+H)^+$.

Step 2c: Preparation of 6-fluoro-7-(2-fluoro-6-isopropoxyphenyl)-1-(2-isopropyl-4-methylpyridin-3-yl)-4-((*S*)-2-methylpiperazin-1-yl)pyrido[2,3-*d*]pyrimidin-2(1*H*)-one (compound 0114-3): The synthetic method was similar to Step 1h of EXAMPLE 1, except that compound 0113-1 therein was replaced by tert-butyl (3*S*)-4-(6-fluoro-7-(2-fluoro-6-isopropoxyphenyl)-1-(2-isopropyl-4-methylpyridin-3-yl)-2-oxo-1,2-dihydropyrido[2,3-*d*]pyrimidin-4-yl)-3-methylpiperazine-1-carboxylate (0113-3) (125 mg, 0.19 mmol, 1.0 eq.), 6-fluoro-7-(2-fluoro-6-isopropoxyphenyl)-1-(2-isopropyl-4-methylpyridin-3-yl)-4-((*S*)-2-methylpiperazin-1-yl)pyrido[2,3-*d*]pyrimidin-2(1*H*)-one was obtained which was used directly in the next step without further purification. MS ($ES^+$): m/z=549 $(M+H)^+$.

Step 2d: Preparation of 4-((*S*)-4-acryloyl-2-methylpiperazin-1-yl)-6-fluoro-7-(2-fluoro-6-isopropoxyphenyl)-1-(2-isopropyl-4-methylpyridin-3-yl)pyrido[2,3-*d*]pyrimidin-2(1*H*)-one (compound 3): The synthetic method was similar to Step 1i of EXAMPLE 1, except that compound 0114-1 therein was replaced by 6-fluoro-7-(2-fluoro-6-isopropoxyphenyl)-1-(2-isopropyl-4-methylpyridin-3-yl)-4-((*S*)-2-methylpiperazin-1-yl)pyrido[2,3-*d*]pyrimidin-2(1*H*)-one (0114-3), 4-((*S*)-4-acryloyl-2-methylpiperazin-1-yl)-6-fluoro-7-(2-fluoro-6-isopropoxyphenyl)-1-(2-isopropyl-4-methylpyridin-3-yl)pyrido[2,3-*d*]pyrimidin-2(1*H*)-one was obtained (58 mg, yield: 50%) as a yellow solid. MS ($ES^+$): m/z=603 $(M+H)^+$. Melting point: 138 °C to 140 °C. $^1H$ NMR (500 MHz, DMSO) δ 8.35 (t, *J* = 14.2 Hz, 2H), 7.41 (dd, *J* = 15.6, 8.1 Hz, 1H), 7.17 (m, 1H), 6.96 (d, *J*= 8.4 Hz, 1H), 6.84 (dd, *J*= 17.2, 9.2 Hz, 2H), 6.20 (d, *J* = 16.2 Hz, 1H), 5.76 (m, 1H), 4.90 (s, 1H), 4.59 (s, 1H), 4.34 (m, 2H), 4.08 (dd, *J*= 58.0, 11.7 Hz, 1H), 3.64 (m, 2H), 3.16 (s, 1H), 2.66 (m, 1H), 1.90 (m, 3H), 1.34 (m, 3H), 1.12 (dd, *J*= 21.0, 5.4 Hz, 3H), 1.06 (d, *J*= 6.2 Hz, 3H), 0.99 (dd, *J*= 28.3, 5.2 Hz, 3H), 0.87 (dd, *J*= 33.6, 5.8 Hz, 3H).

**Example 3: 4-((S)-4-acryloyl-2-methylpiperazin-1-yl)-6-fluoro-7-(2-fluoro-6-(2-hydroxyethoxy)phenyl)-1-(2-isopropyl-4-methylpyridin-3-yl)pyrido[2,3-d]pyrimidin-2(1H)-one (compound 6) (prepared according to Scheme 1)**

Step 3a: Preparation of 2-(2-fluoro-6-(2-(methoxymethoxy)ethoxy)phenyl)-4,4,5,5-tetramethyl-1,3,2-dioxaborolane (compound 0106-6): A mixture of 2-(2-bromo-3-fluorophenoxy)ethan-1-ol (700 mg, 3.0 mmol, 1.0 eq.), MOMBr (563 mg, 4.5 mmol, 1.5 eq.) and DIPEA (1.2 g, 9.0 mmol, 3.0 eq.) in dichloromethane (20 mL) was stirred at room temperature overnight. The reaction solution was washed with brine and concentrated in vacuo. The residue was purified by column chromatography on silica gel (eluent: petroleum ether/ethyl acetate=20/1 to 10/1) to get 2-bromo-1-fluoro-3-(2-(methoxymethoxy)ethoxy)benzene (460 mg, yield: 55.4%) as a colorless oil. Under the protection of nitrogen, a mixture of 2-bromo-1-fluoro-3-(2-(methoxymethoxy)ethoxy)benzene (280 mg, 1.0 mmol, 1.0 eq.) obtained above, bis(pinacolato)diboron (381 mg, 1.5 mmol, 1.5 eq.), potassium acetate(294 mg, 3.0 mmol, 3.0 eq.) and tetrakis(triphenylphosphine)palladium (58 mg, 0.05 mmol, 0.05 eq.) in toluene (10 mL) was refluxed overnight. After cooling to room temperature, the reaction was filtered, and the filtrate was concentrated in vacuo to give the crude product 2-(2-fluoro-6-(2-(methoxymethoxy)ethoxy)phenyl)-4,4,5,5-tetramethyl-1,3,2-dioxaborolane which was used directly in the next step without further purification.

Step 3b: Preparation of tert-butyl (3S)-4-(6-fluoro-7-(2-fluoro-6-(2-(methoxymethoxy)ethoxy)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0113-6): Under the protection of nitrogen, a mixture of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (81 mg, 0.15 mmol, 1.0 eq.), 2-(2-fluoro-6-(2-(methoxymethoxy)ethoxy)phenyl)-4,4,5,5-tetramethyl-1,3,2-dioxaborolane (147 mg,

0.45 mmol, 3.0 eq.), sodium carbonate(48 mg, 0.45 mmol, 3.0 eq.) and tetrakis(triphenylphosphine)palladium (17 mg, 0.015 mmol, 0.1 eq.) in acetonitrile/water=10/1 (11 mL) was stirred at 82°C overnight. After cooling to room temperature, the mixture was poured into water, and ethyl acetate was added to extracted. The organic layer was concentrated in vacuo and the residue was purified by prep-TLC (silica gel, eluent: ethyl acetate/methanol=100/1) to give tert-butyl (3S)-4-(6-fluoro-7-(2-fluoro-6-(2-(methoxymethoxy)ethoxy)phenyl)-1-(2-isopropyl-4-methylpyridin-3-yl)-2-oxo-1,2-dihydropyrido[2,3-d]pyrimidin-4-yl)-3-methylpiperazine-1-carboxylate (100 mg, yield: 94.3%) as a yellow solid. MS ($ES^+$): m/z=695 $(M+H)^+$.

Step 3c: Preparation of 4-((S)-4-acryloyl-2-methylpiperazin-1-yl)-6-fluoro-7-(2-fluoro-6-(2-hydroxyethoxy)phenyl)-1-(2-isopropyl-4-methylpyridin-3-yl)pyrido[2,3-d]pyrimidin-2(1H)-one (compound 6): The synthetic method was similar to Step 1h and Step 1i of EXAMPLE 1, except that compound 0113-1 therein was replaced by tert-butyl (3S)-4-(6-fluoro-7-(2-fluoro-6-(2-(methoxymethoxy)ethoxy)phenyl)-1-(2-isopropyl-4-methylpyridin-3-yl)-2-oxo-1,2-dihydropyrido[2,3-d]pyrimidin-4-yl)-3-methylpiperazine-1-carboxylate (0113-6) (100 mg, 0.14 mmol, 1.0 eq.), 4-((S)-4-acryloyl-2-methylpiperazin-1-yl)-6-fluoro-7-(2-fluoro-6-(2-hydroxyethoxy)phenyl)-1-(2-isopropyl-4-methylpyridin-3-yl)pyrido[2,3-d]pyrimidin-2(1H)-one was obtained (45 mg, yield: 51.7%) as a yellow solid. MS (ES+): m/z=605 (M+H)+. Melting point: 150~155°C; $^1$H NMR (500 MHz, DMSO) δ 8.33 (dd, *J* = 37.4, 6.4 Hz, 2H), 7.43 (dd, *J* = 15.4, 8.4 Hz, 1H), 7.17 (d, *J* = 7.7 Hz, 1H), 6.98 (d, *J* = 8.5 Hz, 1H), 6.85 (dd, *J* = 19.2, 10.4 Hz, 2H), 6.20 (d, *J*= 16.3 Hz, 1H), 5.76 (dd, *J*= 10.4, 2.3 Hz, 1H), 4.95 (s, 1H), 4.62 (t, *J* = 5.1 Hz, 1H), 4.36 (m, 2H), 4.05 (m, 3H), 3.70 (d, *J* = 31.2 Hz, 2H), 3.50 (d, *J*= 7.8 Hz, 2H), 3.14 (s, 1H), 2.70 (s, 1H), 1.90 (s, 3H), 1.32 (dd, *J*= 14.6, 8.6 Hz, 3H), 1.07 (d, *J* = 6.7 Hz, 3H), 0.88 (m, 3H).

## Example 4: 4-((S)-4-acryloyl-2-methylpiperazin-1-yl)-6-fluoro-7-(2-fluoro-6-(2-methoxyethoxy)phenyl)-1-(2-isopropyl-4-methylpyridin-3-yl)pyrido[2,3-d]pyrimidin-2(1H)-one (compound 7) (prepared according to Scheme 1)

Step 4a: Preparation of (2-fluoro-6-(2-methoxyethoxy)phenyl)boronic acid (compound 0105-7): To a solution of 2-bromo-1-fluoro-3-(2-methoxyethoxy)benzene (250 mg, 1.0 mmol, 1.0 eq.) in tetrahydrofuran (6 mL) was added 1.6 M n-BuLi (1.0 mL, 1.5 mmol, 1.5 eq.) dropwise at -70 °C under the protection of nitrogen,. After addition, the mixture was stirred for 30 min. A solution of trimethyl borate (166 mg, 1.6 mmol, 1.6 eq.) in tetrahydrofuran (0.5 mL) was added dropwise to the mixture, and the mixture was continued stirring for 30 min. The reaction solution was quenched with aqueous ammonium chloride and then ethyl acetate was added to extract. The organic layer was dried over anhydrous sodium sulfate and concentrated in vacuo to give (2-fluoro-6-(2-methoxyethoxy)phenyl)boronic acid (180 mg, yield: 84.1%) as a yellow solid.

Step 4b: Preparation of tert-butyl (3S)-4-(6-fluoro-7-(2-fluoro-6-(2-methoxyethoxy)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0113-7):

The synthetic method was similar to Step 1g of EXAMPLE 1, except that compound 0105-1 therein was replaced by (2-fluoro-6-(2-methoxyethoxy)phenyl)boronic acid (0105-7) (81 mg, 0.38 mmol, 2.0 eq.), tert-butyl (3S)-4-(6-fluoro-7-(2-fluoro-6-(2-methoxyethoxy)phenyl)-1-(2-isopropyl-4-methylpyridin-3-yl)-2-oxo-1,2-dihydropyrido[2,3-d]pyrimidin-4-yl)-3-methylpiperazine-1-carboxylate was obtained (84 mg, yield: 67.2%) as a yellow solid. MS ($ES^+$): m/z=665 $(M+H)^+$.

Step 4c: Preparation of 6-fluoro-7-(2-fluoro-6-(2-methoxyethoxy)phenyl)-1-(2-isopropyl-4-methylpyridin-3-yl)-4-((S)-2-methylpiperazin-1-yl)pyrido[2,3-d]pyrimidin-2(I1)-one (compound 0114-7): The synthetic method was similar to Step 1h of EXAMPLE 1, except that compound 0113-1 therein was replaced by tert-butyl (3S)-4-(6-fluoro-7-(2-fluoro-6-(2-methoxyethoxy)phenyl)-1-(2-isopropyl-4-methylpyridin-3-yl)-2-oxo-1,2-dihydropyrido[2,3-d]pyrimidin-4-yl)-3-methylpiperazine-1-carboxylate (0113-7) (80 mg, 0.12 mmol, 1.0 eq.), 6-fluoro-7-(2-fluoro-6-(2-methoxyethoxy)phenyl)-1-(2-isopropyl-4-methylpyridin-3-yl)-4-((S)-2-methylpiperazin-1-yl)pyrido[2,3-d]pyrimidin-2(1H)-one was obtained (43 mg, yield: 63.2%) as a yellow solid. MS ($ES^+$): m/z=565 $(M+H)^+$.

Step 4d: Preparation of 4-((S)-4-acryloyl-2-methylpiperazin-1-yl)-6-fluoro-7-(2-fluoro-6-(2-methoxyethoxy)phenyl)-1-(2-isopropyl-4-methylpyridin-3-yl)pyrido[2,3-d]pyrimidin-2(1H)-one (compound 7): The synthetic method was similar to Step 1i of EXAMPLE 1, except that compound 0114-1 therein was replaced by 6-fluoro-7-(2-fluoro-6-(2-methoxyethoxy)phenyl)-1-(2-isopropyl-4-methylpyridin-3-yl)-4-((S)-2-methylpiperazin-1-yl)pyrido[2,3-d]pyrimidin-2(1H)-one (0114-7) (43 mg, 0.08 mmol, 1.0 eq.), 4-((S)-4-acryloyl-2-methylpiperazin-1-yl)-6-fluoro-7-(2-fluoro-6-(2-methoxyethoxy)phenyl)-1-(2-isopropyl-4-methylpyridin-3-yl)pyrido[2,3-d]pyrimidin-2(1H)-one was obtained (23 mg, yield: 48.9%) as a yellow solid. MS (ES+): m/z=565 (M+H)+. Melting point: 110~115 °C; $^1$H NMR (500 MHz, DMSO) δ 8.35 (dd, *J*= 27.6, 7.3 Hz, 2H), 7.44 (dd, *J* = 15.5, 7.2 Hz, 1H), 7.18 (s, 1H), 6.99 (d, *J* = 8.5 Hz, 1H), 6.87 (t, *J* = 8.6 Hz, 2H), 6.20 (d, *J* = 15.9 Hz, 1H), 5.76 (d, *J* = 10.5 Hz, 1H), 4.89 (s, 1H), 4.32 (m, 2H), 4.06 (s, 3H), 3.67 (s, 2H), 3.44 (s, 2H), 3.11 (s, 4H), 2.69 (s, 1H), 1.89 (s, 3H), 1.32 (dd, *J* = 11.7, 6.4 Hz, 3H), 1.07 (d, *J* = 6.5 Hz, 3H), 0.90 (m, 3H).

**Example 5: 4-((S)-4-acryloyl-2-methylpiperazin-1-yl)-6-fluoro-7-(2-fluoro-6-(2,2,2-trifluoroethoxy)phenyl)-1-(2-isopropyl-4-methylpyridin-3-yl)pyrido[2,3-d]pyrimidin-2(1H)-one (compound 8) (prepared according to Scheme 1)**

Step 5a: Preparation of tert-butyl (3S)-4-(6-fluoro-7-(2-fluoro-6-(2,2,2-trifluoroethoxy)phenyl)-1-(2-isopropyl-4-methylpyridin-3-yl)-2-oxo-1,2-dihydropyrido[2,3-*d*]pyrimidin-4-yl)-3-methylpiperazine-1-carboxylate (compound 0113-8): Under the protection of nitrogen, (2-fluoro-6-hydroxyphenyl)boronic acid (0105-8) (522 mg, 3.0 mmol, 18.0 eq.), tert-butyl (S)-4-(7-chloro-6-fluoro-1-(4-isopropyl-2-methylpyridin-3-yl)-2-oxo-1,2-dihydropyrido[2,3-d]pyrimidin-4-yl)-3-methylpiperazine-1-carboxylate (0112-1) (120 mg, 0.226 mmol, 1.0 eq.), 1, 1'-bis(diphenylphosphino)ferrocene palladium(II)dichloride (16.5 mg, 0.0226 mmol, 0.1 eq.) and potassium carbonate (93 mg, 0.678 mmol, 3.0 eq.) were added to a mixed solvent of 10 mL dioxane and 1 mL water. The mixture was stirred for 3 h at 90°C. The reaction was cooled to room temperature and extracted with ethyl acetate and water. The organic phase was washed with water and concentrated under reduced pressure. The residue was purified by column chromatography on silica gel ( dichloromethane / methanol =150 / 1 to 30 / 1) to give tert-butyl (3S)-4-(6-fluoro-7-(2-fluoro-6-hydroxyphenyl)-1-(2-isopropyl-4-methylpyridin-3-yl)-2-oxo-1,2-dihydropyrido[2,3-d]pyrimidin-4-yl)-3-methylpiperazine-1-carboxylate (112 mg, yield: 81.7%) as a yellow solid. MS ($ES^+$): m/z=607 $(M+H)^+$. The mixture of above tert-butyl (3S)-4-(6-fluoro-7-(2-fluoro-6-hydroxyphenyl)-1-(2-isopropyl-4-methylpyridin-3-yl)-2-oxo-1,2-dihydropyrido[2,3-d]pyrimidin-4-yl)-3-methylpiperazine-1-carboxylate (100 mg, 0.165 mmol, 1.0 eq.), 2,2,2-trifluoroethyl trifluoromethanesulfonate (382 mg, 1.65 mmol, 10.0 eq.) and potassium carbonate (45.5 mg, 0.33 mmol, 2.0 eq.) in acetonitrile (10 mL) was stirred at 85°C for 3.0 h. The mixture was cooled to room temperature and extracted with ethyl acetate and water. The organic phase was dried and concentrated. The residue was purified by prep-TLC (silica gel, dichloromethane/methanol=30:1) to give tert-butyl (3S)-4-(6-fluoro-7-(2-fluoro-6-(2,2,2-trifluoroethoxy)phenyl)-1-(2-isopropyl-4-methylpyridin-3-yl)-2-oxo-1,2-dihydropyrido[2,3-d]pyrimidin-4-yl)-3-methylpiperazine-1-carboxylate (40 mg, yield: 35.1%) as a yellow solid. MS ($ES^+$): m/z=689 $(M+H)^+$.

Step 5b: Preparation of 6-fluoro-7-(2-fluoro-6-(2,2,2-trifluoroethoxy)phenyl)-1-(2-isopropyl-4-methylpyridin-3-yl)-4-((S)-2-methylpiperazin-1-yl)pyrido[2,3-d]pyrimidin-2(1H)-one (compound 0114-8): The synthetic method was similar to Step 1h of EXAMPLE 1, except that compound 0113-1 therein was replaced by tert-butyl (3S)-4-(6-fluoro-7-(2-fluoro-6-(2,2,2-trifluoroethoxy)phenyl)-1-(2-isopropyl-4-methylpyridin-3-yl)-2-oxo-1,2-dihydropyrido[2,3-d]pyrimidin-4-yl)-3-methylpiperazine-1-carboxylate (0113-8) (40 mg, 0.058 mmol, 1.0 eq.), 6-fluoro-7-(2-fluoro-6-(2,2,2-trifluoroethoxy)phenyl)-1-(2-isopropyl-4-methylpyridin-3-yl)-4-((S)-2-methylpiperazin-1-yl)pyrido[2,3-d]pyrimidin-2(1H)-one was obtained which was used directly in the next step without further purification. MS ($ES^+$): m/z=589 $(M+H)^+$.

Step 5c: Preparation of 4-((S)-4-acryloyl-2-methylpiperazin-1-yl)-6-fluoro-7-(2-fluoro-6-(2,2,2-trifluoroethoxy)phenyl)-1-(2-isopropyl-4-methylpyridin-3-yl)pyrido[2,3-d]pyrimidin-2(1H)-one (compound 8): The synthetic method was similar to Step 1i of EXAMPLE 1, except that compound 0114-1 therein was replaced by 6-fluoro-7-(2-fluoro-6-(2,2,2-trifluoroethoxy)phenyl)-1-(2-isopropyl-4-methylpyridin-3-yl)-4-((S)-2-methylpiperazin-1-yl)pyrido[2,3-d] pyrimidin-2(1H)-one (0114-8), 4-((S)-4-acryloyl-2-methylpiperazin-1-yl)-6-fluoro-7-(2-fluoro-6-(2,2,2-trifluoroethoxy)phenyl)-1-(2-isopropyl-4-methylpyridin-3-yl)pyrido[2,3-d]pyrimidin-2(1H)-one was obtained (20 mg, yield: 53.7%) as a yellow solid. MS (ES+): m/z=643 (M+H)+. Melting point: 118~122 °C; 1H NMR (500 MHz, DMSO) $\delta$ 8.37 (d, *J* = 4.7 Hz, 2H), 7.52 (dd, *J* = 15.5, 7.9 Hz, 1H), 7.14 (dd, *J* = 32.8, 12.5 Hz, 2H), 7.01 (t, *J* = 8.0 Hz, 1H), 6.85 (m, 1H), 6.21 (d, *J* = 16.6 Hz, 1H), 5.76 (d, *J* = 10.9 Hz, 1H), 4.92 (d, *J* = 41.5 Hz, 1H), 4.72 (d, *J* = 7.8 Hz, 2H), 4.35 (m, 2H), 4.09 (dd, *J* = 56.9, 12.8 Hz, 1H), 3.65 (dd, *J* = 66.8, 54.3 Hz, 2H), 3.16 (d, *J* = 46.2 Hz, 1H), 2.67 (d, *J* = 62.2 Hz, 1H), 1.89 (d, *J* = 60.3 Hz, 3H), 1.33 (s, 3H), 1.08 (t, *J* = 16.2 Hz, 3H), 0.89 (dd, *J* = 52.6, 11.7 Hz, 3H).

**Example 6: 4-((S)-4-acryloyl-2-methylpiperazin-1-yl)-6-fluoro-7-(2-fluoro-6-mercaptophenyl)-1-(2-isopropyl-4-methylpyridin-3-yl)pyrido[2,3-d]pyrimidin-2(1H)-one (compound 13) (prepared according to Scheme 2)**

Step 6a: Preparation of (2-fluoro-6-((methoxymethyl)thio)phenyl)boronic acid (compound 0202-13): 2-bromo-3-fluoroaniline (950 mg, 5.0 mmol, 1.0 eq.) was added into a diluted hydrochloric acid solution (19 mL concentrated hydrochloric acid in 19 mL water), and stirred at 90°C till the solid disappeared. The mixture was cooled to 5°C and then. sodium nitrite (380 mg, 5.5 mmol, 1.1 eq.), pre-dissolved in 1.0 mL water, was added dropwise into the mixture above. The mixture was stirred at 5°C for 1 h, and then added dropwise into a mixture of potassium ethyldithicxarbonate (960 mg, 6.0 mmol, 1.2 eq.) and potassium hydroxide (336 mg, 6.0 mmol, 1.2 eq.) in 20 mL water at 5°C. The mixture was stirred for 1 h and dichloromethane was added to extract. The organic phase was washed with water and brine, dried over anhydrous sodium sulfate, and concentrated under vacuum. The residue was stirred at room temperature in a potassium hydroxide solution (560 mg, 10.0 mmol, 2.0 eq. in 25 mL ethanol) for 1 h. The mixture was extracted with dichloromethane, and the organic phase was washed with water, dried over anhydrous sodium sulfate, and concentrated under vacuum. The residue was used in the next step without further purification. MS ($ES^-$): m/z=205 $(M-H)^-$. 2-Bromo-3-fluorobenzenethiol obtained above was dissolved in 32 mL dichloromethane, and DIPEA (1.03 g, 8.0 mmol, 1.6 eq.)

was added. The mixture was cooled to 0°C, and bromomethyl methyl ether (750 mg, 6.0 mmol, 1.2 eq.) was added dropwise. After the mixture was stirred at 0°C for 1 h. water was added and the layers were separated. The organic phase was dried over anhydrous sodium sulfate and concentrated under vacuum. The residue was purified bycolumn chromatography (silica gel, petroleum ether/ethyl acetate=10/1 to 5/1) to give (2-bromo-3-fluorophenyl) (methoxymethyl)sulfane (382 mg, yield: 30%) as a yellow oil. Under the protection of nitrogen, (2-bromo-3-fluorophenyl) (methoxymethyl)sulfane (150 mg, 0.60 mmol, 1.0 eq.) obtained above was dissolved in 8 mL anhydrous tetrahydrofuran. The solution was cooled to -70°C and n-BuLi (1.6M in hexane, 0.68 mL, 1.08 mmol, 1.8 eq.) was added dropwise. The mixture was stirred at -70°C for 30 min and then trimethyl borate (113 mg, 1.08 mmol, 1.8 eq.), pre-dissolved in 1 mL anhydrous tetrahydrofuran, was added dropwise into the mixture above. The mixture was stirred at -70°C for 1 h and was then quenched with aqueous ammonium chloride solution and extracted with ethyl acetateThe organic phase was washed with brine, dried over anhydrous sodium sulfate and concentrated under vacuum. The residue was used directly in the next step without further purification.

Step 6b: Preparation of tert-butyl (3*S*)-4-(6-fluoro-7-(2-fluoro-6-((methoxymethyl)thio)phenyl)-1-(2-isopropyl-4-methylpyridin-3-yl)-2-oxo-1,2-dihydropyrido[2,3-*d*]pyrimidin-4-yl)-3-methylpiperazine-1-carboxylate (compound 0204-13): Under the protection of nitrogen, the crude (2-fluoro-6-((methoxymethyl)thio)phenyl)boronic acid (0202-13), tert-butyl (*S*)-4-(7-chloro-6-fluoro-1-(2-isopropyl-4-methylpyridin-3-yl)-2-oxo-1,2-dihydropyrido[2,3-*d*]pyrimidin-4-yl)-3-methylpiperazine-1-carboxylate (0112-1) (100 mg, 0.19 mmol, 1.0 eq.), tetrakis(triphenylphosphine)palladium (50 mg, 0.048 mmol, 0.4 eq.), and sodium carbonate (101 mg, 0.95 mmol, 5.0 eq.) were added into a mixed solvent of 15 mL acetonitrile and 1.5 mL water. The mixture was heated and stirred at 80°C for 12 h. The mixture was cooled to room temperature, and extracted with ethyl acetate and water. The organic phase was washed with brine and concentrated under vacuum. The residue was purified by prep-TLC (silica gel, dichloromethane/methanol=30/1) to give tert-butyl (3*S*)-4-(6-fluoro-7-(2-fluoro-6-((methoxymethyl)thio)phenyl)-1-(2-isopropyl-4-methylpyridin-3-yl)-2-oxo-1,2-dihydropyrido[2,3-*d*]pyrimidin-4-yl)-3-methylpiperazine-1-carboxylate as a yellow solid (25 mg, yield: 19%). MS ($ES^+$): m/z=667 $(M+H)^+$.

Step 6c: Preparation of 6-fluoro-7-(2-fluoro-6-mercaptophenyl)-1-(2-isopropyl-4-methylpyridin-3-yl)-4-((*S*)-2-methylpiperazin-1-yl)pyrido[2,3-*d*]pyrimidin-2(1*H*)-one (compound 0205-13): tert-butyl (3*S*)-4-(6-fluoro-7-(2-fluoro-6-((methoxymethyl)thio)phenyl)-1-(2-isopropyl-4-methylpyridin-3-yl)-2-oxo-1,2-dihydropyrido[2,3-*d*]pyrimidin-4-yl)-3-methylpiperazine-1-carboxylate (0204-13) (25 mg, 0.038 mmol, 1.0 eq.) was dissolved in 10 mL dichloromethane. TFA (1.0 mL) was added dropwise, and the mixture was stirred at room temperature for 1 h. The mixture was concentrated under vacuum to dryness. The residue obtained was used directly in the next step without further purification. MS ($ES^+$): m/z=523 $(M+H)^+$.

Step 6d: Preparation of 4-((*S*)-4-acryloyl-2-methylpiperazin-1-yl)-6-fluoro-7-(2-fluoro-6-mercaptophenyl)-1-(2-isopropyl-4-methylpyridin-3-yl)pyrido[2,3-*d*]pyrimidin-2(1*H*)-one (compound 13): The crude 6-fluoro-7-(2-fluoro-6-mercaptophenyl)-1-(2-isopropyl-4-methylpyridin-3-yl)-4-((*S*)-2-methylpiperazin-1-yl)pyrido[2,3-*d*]pyrimidin-2(1*H*)-one (0205-13) was dissolved in 8 mL tetrahydrofuran, and 1.5 mL DIPEA was added to it. The mixture was cooled in an ice-water bath. Acryloyl chloride (4.0 mg, 0.042 mmol, 1.1 eq.), pre-dissolved in 0.5 mL tetrahydrofuran, was added dropwise into the mixture above. The mixture was stirred for 30 minand water was added and extracted with ethyl acetate. The organic phase was washed with water and concentrated under vacuum to give a residue which was purified by prep-HPLC to give 4-((*S*)-4-acryloyl-2-methylpiperazin-1-yl)-6-fluoro-7-(2-fluoro-6-mercaptophenyl)-1-(2-isopropyl-4-methylpyridin-3-yl)pyrido[2,3-*d*]pyrimidin-2(1*H*)-one as a yellow solid (8 mg, yield: 36%). MS ($ES^+$): m/z=577 $(M+H)^+$. $^1H$ NMR (500 MHz, DMSO) δ 8.29 (d, *J*= 4.8 Hz, 1H), 8.12 (d, *J* = 10.4 Hz, 1H), 7.16 (m, 2H), 7.00 (m, 3H), 6.84 (d, *J* = 10.1 Hz, 1H), 6.19 (d, *J*= 16.9 Hz, 1H), 5.74 (dd, *J* = 10.4, 2.3 Hz, 1H), 4.81 (d, *J* = 22.1 Hz, 1H), 4.25 (m, 2H), 4.04 (dd, *J* = 63.2, 11.9 Hz, 1H), 3.61 (s, 2H), 3.07 (d, *J* = 11.5 Hz, 1H), 2.45 (m, 1H), 1.73 (s, 3H), 1.28 (dd, *J* = 11.1, 4.4 Hz, 3H), 0.97 (d, *J* = 6.0 Hz, 3H), 0.71 (d, *J* = 6.6 Hz, 3H).

### Example 7: 4-((S)-4-acryloyl-2-methylpiperazin-1-yl)-6-fluoro-7-(2-fluoro-6-(methylthio)phenyl)-1-(2-isopropyl-4-methylpyridin-3-yl)pyrido[2,3-d]pyrimidin-2(1H)-one (compound 14) (prepared according to Scheme 2)

Step 7a: Preparation of (2-fluoro-6-(methylthio)phenyl)boronic acid (compound 0202-14): t-BuONO (8.24 g ,80 mmol, 8 eq.) was added slowly to a solution of 2-bromo-3-fluoroaniline (1900 mg, 10 mmol, 1 eq.) in 1,2-dimethyldisulfane (10 mL) at 50°C. The mixture was heat to 95°C and stirred at 95 °C for 3 h. The reaction was cooled to room temperature. The mixture was extracted with petroleum ether. The organic phase was washed with brine and dried over sodium sulfate. The organic phase was concentrated and purified by column chromatography on silica gel (eluent: petroleum ether) to give (2-bromo-3-fluorophenyl) (methyl) sulfane as a yellow oil (1.982 g, crude). MS (ESI): m/z 221$(M+H)^+$. Under the protection of nitrogen, n-BuLi (3.3 mL, 1.6 mol/L in hexane,5.34 mmol, 1.2 eq.) was added dropwise to a solution of (2-bromo-3-fluorophenyl) (methyl)sulfane (980 mg, 4.45 mmol, 1 eq.) in tetrahydrofuran (5 mL) at -70°C. The mixture was stirred at -70°C for 30 min. Trimethyl borate (601 mg, 5.78 mmol, 1.3 eq.) was added dropwise to the mixture. The mixture was stirred at -70 °C for another 30 min. The reaction was then quenched with 2M sodium hydroxide

and extracted with petroleum ether. The PH of the aqueous phase was adjust with 2M HCl to 2. The mixture was extracted with ethyl acetate and water. The organic phase was dried over sodium sulfate and concentrated to give (2-fluoro-6-(methylthio)phenyl)boronic acid (580 mg, 70.05%) as a yellow solid. MS (ESI): m/z 187$(M+H)^+$.

Step 7b: Preparation of tert-butyl (3S)-4-(6-fluoro-7-(2-fluoro-6-(methylthio)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0204-14): Under the protection of nitrogen, tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (150 mg, 0.283 mmol, 1 eq.), (2-fluoro-6-(methylthio)phenyl)boronic acid (0202-14) (78 mg, 0.424 mmol, 1.5 eq.), tetrakis(triphenylphosphine)palladium (33 mg, 0.0283 mmol, 0.1 eq.) and sodium carbonate (90 mg, 0.849 mmol, 3 eq.) were added into the mixture solution of acetonitrile (10 mL) and water(1 mL). The mixture was stirred at 82°C for 16 h. Then, the mixture was cooled to room temperature and extracted with ethyl acetate and water. The organic phase was washed with brine and dried over anhydrous sodium sulfate and concentrated under vacuum. The residue was purified by perp-TLC (eluent: dichloromethane/methanol=15/1) to give tert-butyl (3S)-4-(6-fluoro-7-(2-fluoro-6-(methylthio)phenyl)-1-(2-isopropyl-4-methylpyridin-3-yl)-2-oxo-1,2-dihydropyrido[2,3-d]pyrimidin-4-yl)-3-methylpiperazine-1-carboxylate as a yellow solid (152 mg, crude). MS (ESI): *m/z* 637$(M+H)^+$.

Step 7c: Preparation of 4-((S)-4-acryloyl-2-methylpiperazin-1-yl)-6-fluoro-7-(2-fluoro-6-(methylthio)phenyl)-1-(2-isopropyl-4-methylpyridin-3-yl)pyrido[2,3-d]pyrimidin-2(1H)-one (compound 14): tert-butyl (3S)-4-(6-fluoro-7-(2-fluoro-6-(methylthio)phenyl)-1-(2-isopropyl-4-methylpyridin-3-yl)-2-oxo-1,2-dihydropyrido[2,3-d]pyrimidin-4-yl)-3-methylpiperazine-1-carboxylate (0204-14) (152 mg, 0.239 mmol, 1.0 eq.) was dissolved in 4 mL dichloromethane and then 1 mL TFA was added. The mixture was stirred at room temperature for 2 h. and then concentrated. The residue was dissolved in tetrahydrofuran (5 mL) and DIPEA (92.5 mg, 0.717 mmol, 3 eq.) was added into the mixture followed by the addition of acryloyl chloride (21.6 mg, 0.239 mmol, 1.0 eq.). The mixture was stirred at room temperature for 15 min and was then quenched with ammonium hydroxide. The mixture was extracted with dichloromethane. The organic phase was concentrated under reduced pressure and purified by prep-HPLC to give 4-((S)-4-acryloyl-2-methylpiperazin-1-yl)-6-fluoro-7-(2-fluoro-6-(methylthio)phenyl)-1-(2-isopropyl-4-methylpyridin-3-yl)pyrido[2,3-d]pyrimidin-2(1H)-one as a yellow solid (69.4 mg, 49.22%). MS (ESI): *m/z* 591$(M+H)^+$. Melting point: 134~135°C; $^1H$ NMR (500 MHz, DMSO) $\delta$ 8.37 (d, *J*= 4.8 Hz, 2H), 7.49 (td, *J*= 8.2, 6.2 Hz, 1H), 7.22 (d, *J*= 8.0 Hz, 1H), 7.18 (d, *J* = 4.8 Hz, 1H), 7.11 (t, *J* = 8.8 Hz, 1H), 6.84 (s, 1H), 6.21 (d, *J* = 15.8 Hz, 1H), 5.76 (dd, *J* = 10.4, 2.1 Hz, 1H), 4.94 (d, *J* = 32.7 Hz, 1H), 4.34 (dd, *J* = 35.9, 22.0 Hz, 2H), 4.07 (d, *J* = 58.3 Hz, 1H), 3.67 (dd, *J*= 80.0, 46.7 Hz, 2H), 3.16 (m, 1H), 2.71 (s, 1H), 2.35 (s, 3H), 1.92 (s, 3H), 1.33 (m, 3H), 1.07 (d, *J*= 6.6 Hz, 3H), 0.89 (d, *J*= 20.3 Hz, 3H).

**Example 8: 4-((S)-4-acryloyl-2-methylpiperazin-1-yl)-7-(2-(ethylthio)-6-fluorophenyl)-6-fluoro-1-(2-isopropyl-4-methylpyridin-3-yl)pyrido[2,3-d]pyrimidin-2(1H)-one (compound 15) (prepared according to Scheme 2)**

Step 8a: Preparation of (2-(ethylthio)-6-fluorophenyl)boronic acid (compound 0202-15): To a solution of 2-bromo-3-fluoroaniline (1 g, 5.26 mmol, 1.0 eq.) in 1,2-diethyldisulfane (1.3 g, 10.63 mmol, 2.0 eq.) was added tert-butyl nitrite (1.15 g, 9.22 mmol, 1.2 eq.) dropwise at 50°C. The mixture was stirred at 50 °C for 1 h. Then the temperature of reaction was adjusted to 95°C, and the mixture was stirred at 95 °C for 2 h. The reaction was cooled to room temparature and extracted with water and ethyl acetate. The organic phase was dried and concentrated under vacuum. The residue was purified by column chromatography on silica gel (petroleum ether) to give product (2-bromo-3-fluorophenyl) (ethyl)sulfane (800 mg, yield: 64.7%) as a yellow oil. Under the protection of nitrogen, to a solution of the above (2-bromo-3-fluorophenyl) (ethyl)sulfane (740 mg, 3.15 mmol, 1.0 eq.) in toluene (10 mL) was added n-butyllithium (1.6 M, 2.36 ml, 3.73 mmol, 1.2 eq.) dropwise at -70°C, the mixture was stirred at -70 °C for 0.5 h. Then a solution of trimethyl borate (425 mg, 4.09 mmol, 1.3 eq.) in toluene (1 mL) was added at -70 °C and the mixture was stirred for 1 h. The reaction was extracted with water and ethyl acetate, and the organic phase was dried and concentrated under vacuum. The residue was used directly in the next step without further purification.

Step 8b: Preparation of tert-butyl (3S)-4-(7-(2-(ethylthio)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0204-15): The synthetic method was similar to Step 7b of EXAMPLE 7, except that compound 0202-14 therein was replaced by (2-(ethylthio)-6-fluorophenyl)boronic acid (0202-15), tert-butyl (3S)-4-(7-(2-(ethylthio)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate was obtained as a yellow solid (92 mg, yield: 83.6%). MS $(ES^+)$: m/z=651 $(M+H)^+$.

Step 8c: Preparation of 7-(2-(ethylthio)-6-fluorophenyl)-6-fluoro-1-(2-isopropyl-4-methylpyridin-3-yl)-4-((S)-2-methylpiperazin-1-yl)pyrido[2,3-d]pyrimidin-2(1H)-one (compound 0205-15): tert-butyl (3S)-4-(7-(2-(ethylthio)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0204-15) (92 mg, 0.14mmol, 1.0 eq.) was dissolved in 6 mL dichloromethane and TFA (1mL) was added dropwise. The mixture was stirred at room temperature for 1.0 h and was then concentrated under vacuum to dryness. The residue was used directly in the next step without further purification. MS $(ES^+)$: m/z=551 $(M+H)^+$.

Step 8d: Preparation of 4-((S)-4-acryloyl-2-methylpiperazin-1-yl)-7-(2-(ethylthio)-6-fluorophenyl)-6-fluoro-1-(2-isopropyl-4-methylpyridin-3-yl)pyrido[2,3-d]pyrimidin-2(1H)-one (compound 15): The crude 7-(2-(ethylthio)-6-fluorophenyl)-6-fluoro-1-(2-isopropyl-4-methylpyridin-3-yl)-4-((S)-2-methylpiperazin-1-yl)pyrido[2,3-d]pyrimidin-2(1H)-one (0205-15) was dissolved in 10 mL tetrahydrofuran, and 2.0 mL DIPEA was then added. The reaction was cooled in an ice-water bath and acryloyl chloride (25.3 mg, 0.28 mmol, 2.0 eq.), pre-dissolved in 0.5 mL tetrahydrofuran, was added dropwise into the mixture above. The mixture was stirred for 0.5 h. Water and ethyl acetate was added to extract the product. The organic phase was washed with brine and concentrated in vacuo. The residue was purified by prep-TLC (silica gel, eluent: dichloromethane/methanol=13/1) to give 4-((S)-4-acryloyl-2-methylpiperazin-1-yl)-7-(2-(ethylthio)-6-fluorophenyl)-6-fluoro-1-(2-isopropyl-4-methylpyridin-3-yl)pyrido[2,3-d]pyrimidin-2(1H)-one as a yellow solid (42 mg, yield: 49.7%). MS ($ES^+$): m/z=605 $(M+H)^+$. Melting point: 132~136°C; $^1H$ NMR (500 MHz, DMSO) δ 8.37 (d, *J* = 3.9 Hz, 2H), 7.47 (d, *J* = 6.7 Hz, 1H), 7.28 (d, *J* = 7.7 Hz, 1H), 7.14 (m, 2H), 6.84 (s, 1H), 6.20 (d, *J* = 15.6 Hz, 1H), 5.76 (d, *J* = 10.2 Hz, 1H), 4.94 (d, *J* = 36.4 Hz, 1H), 4.32 (m, 2H), 4.08 (d, *J* = 62.3 Hz, 1H), 3.65 (m, 2H), 3.17 (s, 1H), 2.84 (d, *J* = 5.6 Hz, 2H), 2.71 (s, 1H), 1.94 (s, 3H), 1.33 (dd, *J*= 18.2, 5.7 Hz, 3H), 1.07 (d, *J*= 6.2 Hz, 6H), 0.88 (s, 3H).

**Example 9: 4-((S)-4-acryloyl-2-methylpiperazin-1-yl)-6-fluoro-7-(2-fluoro-6-(isopropylthio)phenyl)-1-(2-isopropyl-4-methylpyridin-3-yl)pyrido [2,3-d] pyrimidin-2(1H)-one (compound 16) (prepared according to Scheme 2)**

Step 9a: Preparation of 2-(2-fluoro-6-(isopropylthio)phenyl)-4,4,5,5-tetramethyl-1,3,2-dioxaborolane (compound 0203-16): To a mixture of 2-bromo-3-fluoroaniline(0.7 g, 3.68 mmol, 1.0 eq.)in 1,2-diisopropyldisulfane (1.5 mL, 9.41 mmol, 2.56 eq.) was added tert-butyl nitrite(2.63 mL, 22.10 mmol, 6.0 eq.)dropwise at 50°C. The mixture was stirred at 50°C for 1 h and then the mixture was heated to 75°C and stirred for 2 h. The solvent was removed under reduced pressure. The residue was purified by column chromatography on silica gel (petroleum ether) to give (2-bromo-3-fluorophenyl) (isopropyl)sulfane (0.426 g, yield: 46%) as a pale yellow oil. TLC: Rf 0.8(petroleum ether). To a mixture of the above (2-bromo-3-fluorophenyl) (isopropyl)sulfane(0.426 g, 1.711 mmol, 1.0 eq.)in tetrahydrofuran(9 mL) was added n-BuLi(1.6M solution in hexane, 1.18 mL, 1.882 mmol, 1.1 eq.)at -78°C under $N_2$ atmosphere. The mixture was stirred for 1 h and 2-methoxy-4,4,5,5-tetramethyl-1,3,2-dioxaborolane (0.56 mL, 3.422 mmol, 2.0 eq.) was added and then the reaction was stirred at -78°C for 1 h. Saturated ammonium chloride solution (20mL) was added to quench the reaction. The aqueous layer was extracted with ethyl acetate(20 mL×3). The combined organic layers were washed with brine (20 mL×1), dried over anhydrous sodium sulfate and concentrated to give 2-(2-fluoro-6-(isopropylthio)phenyl)-4,4,5,5-tetramethyl-1,3,2-dioxaborolane (0.607 g, crude) as a pale yellow oil. LCMS(ESI): m/z 297$[M+1]^+$; TLC: Rf 0.5 (petroleum ether:ethyl acetate=60:1).

Step 9b: Preparation of tert-butyl (3S)-4-(6-fluoro-7-(2-fluoro-6-(isopropylthio)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0204-16) : To a mixture of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate(0112-1) (80 mg, 0.151 mmol, 1.0 eq.), tetrakis(triphenylphosphine)palladium(17.4 mg, 0.0151 mmol, 0.1 eq.) and sodium carbonate(32 mg, 0.302 mmol, 2.0 eq.) in acetonitrile(8 mL)and water(0.8 mL) was added 2-(2-fluoro-6-(isopropylthio)phenyl)-4,4,5,5-tetramethyl-1,3,2-dioxaborolane(0203-16) (67.1 mg, 0.227 mmol, 1.5 eq.). The mixture was heated to 82°C and stirred overnight under $N_2$ atmosphere. The solvent was removed under reduced pressure. The residue was purified by column chromatography on silica gel(dichloromethane:methanol 40:1) to get tert-butyl (3S)-4-(6-fluoro-7-(2-fluoro-6-(isopropylthio)phenyl)-1-(2-isopropyl-4-methylpyridin-3-yl)-2-oxo-1,2-dihydropyrido[2,3-d]pyrimidin-4-yl)-3-methylpiperazine-1-carboxylate(88 mg, yield: 88%) as a pale yellow solid. LC-MS(ESI): *m/z* 665$[M+1]^+$.

Step 9c: Preparation of 6-fluoro-7-(2-fluoro-6-(isopropylthio)phenyl)-1-(2-isopropyl-4-methylpyridin-3-yl)-4-((S)-2-methylpiperazin-1-yl)pyrido[2,3-d]pyrimidin-2(1H)-one (0205-16):The synthetic method was similar to Step 8c of EXAMPLE 8, except that compound 0204-15 therein was replaced by tert-butyl (3S)-4-(6-fluoro-7-(2-fluoro-6-(isopropylthio)phenyl)-1-(2-isopropyl-4-methylpyridin-3-yl)-2-oxo-1,2-dihydropyrido[2,3-d]pyrimidin-4-yl)-3-methylpiperazine-1-carboxylate (0204-16) (88 mg, 0.132 mmol, 1.0 eq.), 6-fluoro-7-(2-fluoro-6-(isopropylthio)phenyl)-1-(2-isopropyl-4-methylpyridin-3-yl)-4-((S)-2-methylpiperazin-1-yl)pyrido[2,3-d]pyrimidin-2(1H)-one was obtained (95 mg, crude) as a light yellow oil. LCMS ($ES^+$): m/z=565 $(M+H)^+$.

Step 9d: Preparation of 4-((S)-4-acryloyl-2-methylpiperazin-1-yl)-6-fluoro-7-(2-fluoro-6-(isopropylthio)phenyl)-1-(2-isopropyl-4-methylpyridin-3-yl)pyrido[2,3-d]pyrimidin-2(1H)-one (compound 16):The synthetic method was similar to Step 8d of EXAMPLE 8, except that compound 0205-15 therein was replaced by 6-fluoro-7-(2-fluoro-6-(isopropylthio)phenyl)-1-(2-isopropyl-4-methylpyridin-3-yl)-4-((S)-2-methylpiperazin-1-yl)pyrido[2,3-d]pyrimidin-2(1H)-one(0205-16) (75 mg, 0.132 mmol, 1.0 eq.), 4-((S)-4-acryloyl-2-methylpiperazin-1-yl)-6-fluoro-7-(2-fluoro-6-(isopropylthio)phenyl)-1-(2-isopropyl-4-methylpyridin-3-yl)pyrido[2,3-d]pyrimidin-2(1H)-one was obtained (30 mg, yield: 37%) as a pale yellow solid. LCMS(ESI): *m/z* 619$[M+1]^+$; Melting point: 142-146°C. $^1H$ NMR (500 MHz, DMSO) δ 8.37 (d, *J* = 4.8 Hz, 2H), 7.49 (dd, *J* = 14.3, 8.0 Hz, 1H), 7.36 (d, *J* = 7.7 Hz, 1H), 7.17 (t, *J* = 6.0 Hz, 2H), 6.86 (d, *J* = 13.4 Hz, 1H), 6.21 (d, *J* = 16.5 Hz, 1H), 5.77 (d, *J* = 12.0 Hz, 1H), 4.95 (d, *J*= 40.6 Hz, 1H), 4.33 (m, 2H), 4.11 (m, 1H), 3.66

(m, 2H), 3.40 (m, 1H), 3.17 (dd, *J*= 22.9, 17.6 Hz, 1H), 2.75 (d, *J* = 38.9 Hz, 1H), 1.92 (m, 3H), 1.32 (m, 3H), 1.09 (m, 9H), 0.94 (dd, *J* = 60.2, 5.7 Hz, 3H).

**Example 10: 4-((S)-4-acryloyl-2-methylpiperazin-1-yl)-6-fluoro-7-(2-fluoro-6-(methylsulfonyl)phenyl)-1-(2-isopropyl-4-methylpyridin-3-yl)pyrido[2,3-d]pyrimidin-2(1H)-one (compound 31) (prepared according to Scheme 2)**

Step 10a: Preparation of (S)-4-(6-fluoro-7-(2-fluoro-6-(methylsulfonyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0204-31): Under the protection of nitrogen, tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (100 mg, 0.188 mmol, 1 eq.), (2-(methylsulfonyl)phenyl)boronic acid (0202-31) (45.3 mg, 0.226 mmol, 1.2 eq.), tetrakis(triphenylphosphine)palladium (22 mg, 0.018 mmol, 0.1 eq.) and sodium carbonate (40 mg, 0.378 mmol,2 eq.) were added into a mixture of acetonitrile (10 mL) and water(1 mL). The mixture was stirred at 82°C for 16 h. Then, the mixture was cooled to room temparature. The mixture was extracted with ethyl acetate and water. The organic phase was washed with brine and dried over anhydrous sodium sulfate, and concentrated under vacuum. The residue was purified by perp-TLC (eluent: dichloromethane/methanol=15/1) to give (S)-4-(6-fluoro-7-(2-fluoro-6-(methylsulfonyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (60 mg, crude) as a yellow solid. MS (ESI): *m*/*z* 651$(M+H)^+$.

Step 10b: Preparation of 4-((S)-4-acryloyl-2-methylpiperazin-1-yl)-6-fluoro-7-(2-fluoro-6-(methylsulfonyl)phenyl)-1-(2-isopropyl-4-methylpyridin-3-yl)pyrido[2,3-d]pyrimidin-2(1H)-one (compound 31): (S)-4-(6-fluoro-7-(2-fluoro-6-(methylsulfonyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (0204-31) (60 mg, 0.092 mmol, 1.0 eq.) was dissolved in 4 mL dichloromethane and then 1 mL TFA was added. The mixture was stirred at room temperature for 2h and then was concentrated. The residue was dissolved in tetrahydrofuran (5 mL) and DIPEA (35.7 mg, 0.277 mmol, 3 eq.) was added into the mixture followed by the addition of acryloyl chloride (8.3 mg, 0.092 mmol, 1.0 eq.). The mixture was stirred at room temperature for 15 min. Then, the reaction was quenched with ammonium hydroxide. The mixture was extracted with dichloromethane. The organic phase was concentrated and purified by prep-HPLC to 4-((S)-4-acryloyl-2-methylpiperazin-1-yl)-6-fluoro-7-(2-fluoro-6-(methylsulfonyl)phenyl)-1-(2-isopropyl-4-methylpyridin-3-yl)pyrido[2,3-d]pyrimidin-2(1H)-one (9.3 mg, 16.68%) as a yellow solid. MS (ESI): *m*/*z* 605$(M+H)^+$. Melting point: 160~161°C; $^1$H NMR (500 MHz, DMSO) $\delta$ 8.35 (d, *J* = 4.7 Hz, 2H), 8.01 (d, *J* = 7.8 Hz, 1H), 7.79 (dt, *J* = 29.9, 7.5 Hz, 2H), 7.45 (d, *J* = 7.5 Hz, 1H), 7.15 (d, *J* = 4.8 Hz, 1H), 6.85 (s, 1H), 6.20 (d, *J* = 15.9 Hz, 1H), 5.76 (d, *J* = 10.8 Hz, 1H), 4.93 (s, 1H), 4.34 (d, *J* = 64.7 Hz, 2H), 4.08 (m, 1H), 3.64 (m, 2H), 3.15 (m, 1H), 2.66 (d, *J* = 27.1 Hz, 4H), 1.95 (s, 3H), 1.30 (dd, *J* = 30.2, 12.6 Hz, 3H), 1.04 (d, *J* = 5.4 Hz, 3H), 0.85 (s, 3H).

**Example 11: N-(2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorophenyl)acetamide (compound 33) (prepared according to Scheme 3)**

Step 11a: Preparation of 3-fluoro-2-(4,4,5,5-tetramethyl-1,3,2-dioxaborolan-2-yl)aniline (compound 0303-33): Under the protection of nitrogen, a mixture of 2-bromo-3-fluoroaniline (1.00 g, 5.26 mmol, 1.0 eq.), bis(pinacolato)diboron (2.00 g, 7.89 mmol, 2.0 eq.), potassium acetate (1.55 g, 15.8 mmol) mol, 3.0 eq.) and tetrakis(triphenylphosphine)palladium (150 mg, 0.027 mmol, 0.05 eq.) in toluene (10 mL) was stirred at 115°C overnight. After cooling to room temperature, the mixture was filtered, and the filtrate was concentrated under reduced pressure to give 3-fluoro-2-(4,4,5,5-tetramethyl-1,3,2-dioxaborolan-2-yl)aniline (920 mg, yield: 73.8%) as a colorless oil..

Step 11b: Preparation of tert-butyl (3S)-4-(7-(2-acetamido-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0304-33): Under the protection of nitrogen, a mixture of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (150 mg, 0.283 mmol, 1.00 eq.), 3-fluoro-2-(4,4,5,5-tetramethyl-1,3,2-dioxaborolan-2-yl)aniline (0303-33) (135 mg, 0.566 mmol, 2.00 eq.), sodium carbonate (90 mg , 0.849 mmol, 3.00 eq.) and tetrakis(triphenylphosphine)palladium (32 mg, 0.028 mmol, 0.10 eq.) in acetonitrile (10 mL) and water (1 mL) was stirred at 80°C overnight. After cooling to room temperature, water and ethyl acetate were added to extract. The organic layer was washed with brine, and the solvent was removed under reduced pressure. The residue was purified by prep-TLC (silica gel, eluent: dichloromethane/methanol=12/1) to obtain tert-butyl (3S)-4-(7-(2-amino-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (102 mg, yield: 59.6%) as a pale yellow solid. MS $(ES^+)$: m/z=606 $(M+H)^+$. To the above prepared tert-butyl (3S)-4-(7-(2-amino-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (150 mg, 0.247 mmol, 1.0 eq.) in dichloromethane (10 mL) was added dropwise acetyl chloride (58.1 mg, 0.741 mmol, 1.0 eq.) and the reaction was stirred at room temperature for 2 h. Water and dichloromethane were added to extract, and the organic layer was washed with brine and concentrated

under reduced pressure to obtain tert-butyl (3S)-4-(7-(2-acetamido-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate(130 mg, yield: 81.2%) as a yellow solid . MS ($ES^+$): m/z=648$(M+H)^+$.

Step 11c: Preparation of N-(3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)phenyl)acetamide (compound 0305-33): To a solution of the tert-butyl (3S)-4-(7-(2-acetamido-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0304-33) (130 mg, 0.201 mmol, 1.0 eq.) in dichloromethane (5 mL) was added TFA (0.5mL). The reaction was stirred at room temperature for 30 min. The mixture was concentrated in vacuo to give N-(3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)phenyl)acetamide (110 mg, crude) as a yellow oil. MS ($ES^+$): m/z=548 $(M+H)^+$

Step 11d: Preparation of N-(2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorophenyl)acetamide (compound 33): To a mixture of N-(3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)phenyl)acetamide (0305-33) (110 mg, crude) and DIPEA(0.5mL) in acetonitrile(5mL) was added dropwise acryloyl chloride(27.2 mg, 0.301 mmol, 1.50 eq.), the reaction was stirred at room temperature for 30 min. Water and ethyl acetate were added to extract and the organic layer was washed with brine and concentrated in vacuo. The residue was purified by prep-TLC (silica gel, eluent: dichloromethane/methanol=10/1) to give N-(2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorophenyl)acetamide (66 mg, yield: 54.6%) as a yellow solid. MS ($ES^+$): m/z=602 $(M+H)^+$, m. p: 143-154°C. $^1H$ NMR (500 MHz, DMSO) $\delta$ 8.40 (d, $J$ = 4.8 Hz, 1H), 8.31 (dd, $J$ = 20.3, 9.8 Hz, 1H), 7.31 (dd, $J$ = 14.8, 7.4 Hz, 1H), 7.14 (dd, $J$ = 26.3, 18.5 Hz, 2H), 6.85 (t, $J$ = 10.4 Hz, 3H), 6.19 (d, $J$ = 15.8 Hz, 1H), 5.75 (d, $J$ = 10.6 Hz, 1H), 4.88 (d, $J$= 40.1 Hz, 1H), 4.28 (m, 2H), 4.06 (m, 1H), 3.58 (m, 2H), 3.14 (m, 1H), 2.60 (dd, $J$= 13.0, 6.9 Hz, 1H), 1.91 (s, 3H), 1.70 (s, 3H), 1.30 (dd, $J$= 19.5, 6.6 Hz, 3H), 1.02 (d, $J$= 6.6 Hz, 3H), 0.70 (d, $J$= 6.0 Hz, 3H).

Example 12: **4-((S)-4-acryloyl-2-methylpiperazin-1-yl)-6-fluoro-7-(2-fluoro-6-methylphenyl)-1-(2-isopropyl-4-methylpyridin-3-yl)pyrido[2,3-d]pyrimidin-2(1H)-one** (compound 34) (prepared according to Scheme 4)

Step 12a: Preparation of (2-fluoro-6-methylphenyl)boronic acid (compound 0402-34): to a solution of 2-bromo-1-fluoro-3-methylbenzene (500 mg, 2.6 mmol, 1.0 eq.) in tetrahydrofuran (10 mL) was added 1.6 M n-BuLi (2.3 mL, 3.6 mmol, 1.4 eq.) dropwise at -70 °C under the protection of nitrogen. After addition, the mixture was stirred for 30 min. A solution of trimethyl borate (406 mg, 3.9 mmol, 1.5 eq.) in tetrahydrofuran (0.5 mL) was added dropwise to the mixture, and the mixture was continued stirring for 30 min. The reaction solution was quenched with aqueous ammonium chloride, then ethyl acetate was added to extract. The organic layer was dried over anhydrous sodium sulfate and concentrated in vacuo to give (2-fluoro-6-methylphenyl)boronic acid (300 mg, yield: 73.7%) as a yellow solid.

Step 12b: Preparation of tert-butyl (3S)-4-(6-fluoro-7-(2-fluoro-6-methylphenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0404-34): Under the protection of nitrogen, a mixture of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (100 mg, 0.19 mmol, 1.0 eq.), (2-fluoro-6-methylphenyl)boronic acid (58 mg, 0.38 mmol, 2.0 eq.), sodium carbonate(60 mg, 0.57 mmol, 3.0 eq.) and tetrakis(triphenylphosphine)palladium (22 mg, 0.019 mmol, 0.1 eq.) in acetonitrile/water=10/1 (11 mL) was stirred at 82°C overnight. After cooling to room temperature, the mixture was poured into water, and ethyl acetate was added to extracted. The organic layer was concentrated in vacuo. The residue was purified by column chromatography on silica gel (eluent: petroleum ether/ethyl acetate =2/1to ethyl acetate) to give tert-butyl (3S)-4-(6-fluoro-7-(2-fluoro-6-methylphenyl)-1-(2-isopropyl-4-methylpyridin-3-yl)-2-oxo-1,2-dihydropyrido[2,3-d]pyrimidin-4-yl)-3-methylpiperazine-1-carboxylate (120 mg, yield: 106%) as a yellow solid. MS ($ES^+$): m/z=605 $(M+H)^+$.

Step 12c: Preparation of 4-((S)-4-acryloyl-2-methylpiperazin-1-yl)-6-fluoro-7-(2-fluoro-6-methylphenyl)-1-(2-isopropyl-4-methylpyridin-3-yl)pyrido[2,3-d]pyrimidin-2(1H)-one (compound 34): To a solution of tert-butyl (3S)-4-(6-fluoro-7-(2-fluoro-6-methylphenyl)-1-(2-isopropyl-4-methylpyridin-3-yl)-2-oxo-1,2-dihydropyrido[2,3-d]pyrimidin-4-yl)-3-methylpiperazine-1-carboxylate (0404-34) (120 mg, 0.2 mmol, 1.0 eq.) in dichloromethane (8 mL) was added TFA (4 mL), and the mixture was stirred at room temperature for 1 h. The solvent was removed in vacuo. The residue was dissolved in tetrahydrofuran (10 mL), and DIPEA (77 mg, 0.6 mmol, 3.0 eq.) was added. A solution of acryloyl chloride (22 mg, 0.24 mmol, 1.2 eq.) in tetrahydrofuran (0.5 mL) was added dropwise to the mixture in an ice-water bath. The mixture was stirred for 30 min. Water was added, then dichloromethane was added to extract. The organic layer was concentrated in vacuo. The residue was purified by prep-TLC (silica gel, eluent: dichloromethane/methanol=12/1) to give 4-((S)-4-acryloyl-2-methylpiperazin-1-yl)-6-fluoro-7-(2-fluoro-6-methylphenyl)-1-(2-isopropyl-4-methylpyridin-3-yl)pyrido[2,3-d]pyrimidin-2(1H)-one (75 mg, yield: 67.6%) as a yellow solid. MS ($ES^+$): m/z=559 $(M+H)^+$. Melting point: 140~145°C; $^1H$ NMR (500 MHz, DMSO) $\delta$ 8.38 (t, $J$ = 7.6 Hz, 2H), 7.39 (dd, $J$ = 14.2, 7.8 Hz, 1H), 7.19 (m, 1H), 7.11 (m, 2H), 6.86 (d, $J$ = 12.5 Hz, 1H), 6.21 (d, $J$ = 15.9 Hz, 1H), 5.76 (m, 1H), 4.94 (d, $J$ = 34.1 Hz, 1H), 4.33 (dd, $J$ = 26.7,

12.9 Hz, 2H), 4.10 (m, 1H), 3.67 (dd, *J* = 74.6, 58.9 Hz, 2H), 3.15 (m, 1H), 2.72 (s, 1H), 1.93 (d, *J* = 17.0 Hz, 6H), 1.33 (dt, *J*= 15.1, 7.6 Hz, 3H), 1.07 (d, *J*= 6.3 Hz, 3H), 0.90 (m, 3H).

**Example 13: 4-((S)-4-acryloyl-2-methylpiperazin-1-yl)-6-fluoro-7-(2-fluoro-6-isopropylphenyl)-1-(2-isopropyl-4-methylpyridin-3-yl)pyrido[2,3-d]pyrimidin-2(1H)-one (compound 35) (prepared according to Scheme 4)**

Step 13a: Preparation of (2-fluoro-6-isopropylphenyl)boronic acid (17-096-5) (compound 0402-35): To a mixture of 2-fluoro-6-isopropylaniline(0.55 g, 3.59 mmol, 1.0 eq.)and $CuBr_2$ (0.96 g, 4.31 mmol, 1.2 eq.) in acetonitrile (10 mL) was added tert-butyl nitrite(1.48 g, 14.36 mmol, 4 eq.)at 45°C under $N_2$ atmosphere. The mixture was stirred for 20 min. The solvent was removed under reduced pressure. The residue was purified by column chromatography on silica gel (petroleum ether) to give 2-bromo-1-fluoro-3-isopropylbenzene(0.526 g, yield: 68%) as a pale yellow oil. MS ($ESI^+$): m/z 217 $[M+1]^+$. To a solution of the above 2-bromo-1-fluoro-3-isopropylbenzene (0.526 g, 2.42 mmol, 1.0 eq.) in tetrahydrofuran (10 mL) was added dropwise n-butyllithium (1.6 M in hexane, 1.82 mL, 2.91 mmol, 1.2 eq.). The mixture was stirred for 1 h. Trimethyl borate(0.67 mL, 6.05 mmol, 2.5 eq.) was added and stirred at -78°C for another 1 h. Saturated ammonium chloride solution (20 mL) was added to quench the reaction. The aqueous layer was extracted with ethyl acetate(20 mL×3). The combined organic layers were washed with brine(20 mL×1), dried over anhydrous sodium sulfate and concentrated to give mixture of (2-fluoro-6-isopropylphenyl)boronic acid (0.405 g, crude) as a pale yellow oil.

Step 13b: Preparation of tert-butyl (3S)-4-(6-fluoro-7-(2-fluoro-6-isopropylphenyl)-1-(2-isopropyl-4-methylpyridin-3-yl)-2-oxo-1,2-dihydropyrido[2,3-d]pyrimidin-4-yl)-3-methylpiperazine-1-carboxylate (0404-35): The synthetic method was similar to Step 12b of EXAMPLE 12, except that compound 0402-34 therein was replaced by (2-fluoro-6-isopropylphenyl)boronic acid (0402-35) (123 mg, 0.678 mmol, 6.0 eq.), tert-butyl (3S)-4-(6-fluoro-7-(2-fluoro-6-isopropylphenyl)-1-(2-isopropyl-4-methylpyridin-3-yl)-2-oxo-1,2-dihydropyrido[2,3-d]pyrimidin-4-yl)-3-methylpiperazine-1-carboxylate (60 mg, crude) was obtained. MS($ESI^+$): *m/z* 633$[M+1]^+$.

Step 13c: Preparation of 6-fluoro-7-(2-fluoro-6-isopropylphenyl)-1-(2-isopropyl-4-methylpyridin-3-yl)-4-((S)-2-methylpiperazin-1-yl)pyrido[2,3-d]pyrimidin-2(1H)-one (0405-35): To a solution of tert-butyl (3S)-4-(6-fluoro-7-(2-fluoro-6-isopropylphenyl)-1-(2-isopropyl-4-methylpyridin-3-yl)-2-oxo-1,2-dihydropyrido[2,3-d]pyrimidin-4-yl)-3-methylpiperazine-1-carboxylate (0404-35) (60 mg, 0.095 mmol, 1.0 eq.) in dichloromethane (7.5 mL) was added TFA (2 mL). The mixture was stirred at room temperature for 0.5 h. The solvent was removed under reduced pressure. The residue was dried under vacuum to give 6-fluoro-7-(2-fluoro-6-isopropylphenyl)-1-(2-isopropyl-4-methylpyridin-3-yl)-4-((S)-2-methylpiperazin-1-yl)pyrido[2,3-d]pyrimidin-2(1H)-one (55 mg, crude) as a pale yellow oil . MS($ESI^+$): m/z 533$[M+1]^+$; TLC: Rf 0.3 (dichloromethane:methanol=10:1).

Step 13d: Preparation of 4-((S)-4-acryloyl-2-methylpiperazin-1-yl)-6-fluoro-7-(2-fluoro-6-isopropylphenyl)-1-(2-isopropyl-4-methylpyridin-3-yl)pyrido[2,3-d]pyrimidin-2(1H)-one (compound 35): To a mixture of 6-fluoro-7-(2-fluoro-6-isopropylphenyl)-1-(2-isopropyl-4-methylpyridin-3-yl)-4-((S)-2-methylpiperazin-1-yl)pyrido[2,3-d]pyrimidin-2(1H)-one (0405-35) (55 mg, 0.103 mmol, 1.0 eq.) and DIPEA (27 mg, 0.207 mmol, 2.0 eq.) in dichloromethane (6 mL) was added a solution of acryloyl chloride (19 mg, 0.207 mmol, 2.0 eq.) in dichloromethane (1 mL). The mixture was stirred for 20 min. The mixture was diluted with water (20 mL) and extracted with dichloromethane (10 mL × 2). The combined organic layers were washed with brine (20 mL×1), dried over anhydrous sodium sulfate and concentrated. The residue was purified by prep-HPLC (acetonitrile-0.1% formic acid solution system) to give 4-((S)-4-acryloyl-2-methylpiperazin-1-yl)-6-fluoro-7-(2-fluoro-6-isopropylphenyl)-1-(2-isopropyl-4-methylpyridin-3-yl)pyrido[2,3-d]pyrimidin-2(1H)-one (1st peak, 13 mg, yield: 22%) as a white solid. MS ($ESI^+$): m/z 587 $[M+1]^+$; TLC: Rf 0.5 (dichloromethane:methanol=10:1); m. p. : 113-116°C. $^1$HNMR (DMSO-d6, 500MHz): δ8.36-8.34 (m, 2H), 7.46-7.42 (m, 1H), 7.24-7.22 (m, 1H), 7.18-7.15 (m, 1H), 7.12-7.07 (m, 1H), 6.88-6.81 (m, 1H), 6.21-6.17 (m, 1H), 5.76-5.74 (m, 1H), 4.590-4.85 (m, 1H), 4.39-4.26 (m, 2H), 4.14-3.99(m, 1H), 3.75-3.50(m, 2H), 3.17-3.12(m, 1H), 2.82-2.78(m, 1H), 2.66-2.62(m, 1H), 1.94-1.83(m, 3H), 1.28-1.24 (m, 3H), 1.09-0.99 (m, 6H), 0.85-0.74 (m, 6H).

**Example 14: Preparation of 4-((S)-4-acryloyl-2-methylpiperazin-1-yl)-7-(2-(difluoromethyl)-6-fluorophenyl)-6-fluoro-1-(2-isopropyl-4-methylpyridin-3-yl)pyrido[2,3-d]pyrimidin-2(1H)-one (compound 37) (prepared according to Scheme 4)**

Step 14a: Preparation of 2-(2-(difluoromethyl)-6-fluorophenyl)-4,4,5,5-tetramethyl-1,3,2-dioxaborolane (compound 0403-37): Under the protection of nitrogen, to a solution of 2-bromo-3-fluorobenzaldehyde (1.5 g, 7.39 mmol, 1.0 eq.) in dichloromethane (50 mL) was added dropwise diethylaminosulfur trifluoride (2.97 g, 18.5 mmol, 2.5 eq.) at 0°C, the mixture was stirred at room temperature overnight. The mixture was poured into ice water and extracted with dichloromethane, and the organic phase was dried and concentrated under vacuum. The residue was purified by column chromatography on silica gel (eluent: pure petroleum ether) to give 2-bromo-1-(difluoromethyl)-3-fluorobenzene as a colorless oil (1.4 g, yield: 84.2%). Under the protection of nitrogen, to a solution of the above 2-bromo-1-(difluoromethyl)-3-fluorobenzene (1.2 g, 5.33 mmol, 1.0 eq.) in tetrahydrofuran (10 mL) was added dropwise n-butyllithium (1.6 M, 4.33

ml, 6.93 mmol, 1.3 eq.) at -70°C, and the mixture was stirred at -70°C for 40 min. Then a solution of 2-methoxy-4,4,5,5-tetramethyl-1,3,2-dioxaborolane (1.35 g, 8.5 mmol, 1.6 eq.) in tetrahydrofuran (2.5 mL) was added dropwise at -70°C and the mixture was stirred for 1 h. Aqueous ammonium chloride solution was added to quenched the reaction and the mixture was extracted with water and ethyl acetate. The organic phase was dried and concentrated under vacuum. The residue (1.3 g, crude) was used directly in the next step without further purification.

Step 14b: Preparation of tert-butyl (3S)-4-(7-(2-(difluoromethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0404-37): Under the protection of nitrogen, 2-(2-(difluoromethyl)-6-fluorophenyl)-4,4,5,5-tetramethyl-1,3,2-dioxaborolane (0403-37) (1.3 g, crude), tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate(0112-1) (150 mg, 0.282 mmol, 1.0 eq.), (1, 1'-bis(diphenylphosphino)ferrocene)dichloropalladium (20.6 mg, 0.0282 mmol, 0.1 eq.), and potassium carbonate (78 mg, 0.564 mmol, 2.0 eq.) were added into a mixed solvent of 10 mL dioxane and 1 mL water. The mixture was stirred at 90°C for 5 h. The reaction solution was cooled to room temparature and extracted with ethyl acetate and water. The organic phase was washed with brine and concentrated under vacuum. The residue was purified by column chromatography on silica gel (eluent: dichloromethane/methanol=100/1 to 40/1) to give tert-butyl (3S)-4-(7-(2-(difluoromethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (100 mg, yield: 55.3%) as a yellow solid. MS ($ES^+$): m/z=641 $(M+H)^+$.

Step 14c. Preparation of 7-(2-(difluoromethyl)-6-fluorophenyl)-6-fluoro-1-(2-isopropyl-4-methylpyridin-3-yl)-4-((S)-2-methylpiperazin-1-yl)pyrido[2,3-d]pyrimidin-2(1H)-one (compound 0405-37): Tert-butyl (3S)-4-(7-(2-(difluoromethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0404-37) (100 mg, 0.156 mmol, 1.0 eq.) was dissolved in 10 mL dichloromethane and TFA (2 mL) was then added dropwise. The mixture was stirred at room temperature for 1 h, and was concentrated under vacuum. The residue was dissolved in dichloromethane and water, and the pH was adjusted to 8 by the addition of 2 molar sodium hydroxide. The mixture was extracted with dichloromethane and the organic phase was dried, concentrated and purified by prep-TLC (silica gel,dichloromethane/methanol=10/1) to give 7-(2-(difluoromethyl)-6-fluorophenyl)-6-fluoro-1-(2-isopropyl-4-methylpyridin-3-yl)-4-((S)-2-methylpiperazin-1-yl)pyrido[2,3-d]pyrimidin-2(1H)-one (55 mg, yield: 65.2%) as a yellow solid. MS ($ES^+$): m/z=541 $(M+H)^+$.

Step 14d: Preparation of 4-((S)-4-acryloyl-2-methylpiperazin-1-yl)-7-(2-(difluoromethyl)-6-fluorophenyl)-6-fluoro-1-(2-isopropyl-4-methylpyridin-3-yl)pyrido[2,3-d]pyrimidin-2(1H)-one (compound 37): 7-(2-(difluoromethyl)-6-fluorophenyl)-6-fluoro-1-(2-isopropyl-4-methylpyridin-3-yl)-4-((S)-2-methylpiperazin-1-yl)pyrido[2,3-d]pyrimidin-2(1H)-one (0405-37) (55 mg, 0.102 mmol, 1.0 eq.) was dissolved in 10 mL tetrahydrofuran, and DIPEA (39 mg, 0.306 mmol, 3.0 eq.) was added. The reaction system was cooled in an ice-water bath. Acryloyl chloride (13.8 mg, 0.153 mmol, 1.5 eq.), pre-dissolved in 0.5 mL tetrahydrofuran, was added dropwise into the mixture above. The mixture was stirred for 0.5 h and water and ethyl acetate were added to extract. The organic phase was washed with brine, and concentrated in vacuo. The residue was purified by prep-TLC (silica gel, dichloromethane/methanol=12/1) to give 4-((S)-4-acryloyl-2-methylpiperazin-1-yl)-7-(2-(difluoromethyl)-6-fluorophenyl)-6-fluoro-1-(2-isopropyl-4-methylpyridin-3-yl)pyrido[2,3-d]pyrimidin-2(1H)-one as a yellow solid (38 mg, yield: 62.5%). MS ($ES^+$): m/z=595 $(M+H)^+$. $^1$H NMR (500 MHz, DMSO) $\delta$ 8.40 (dd, $J$ = 18.7, 8.8 Hz, 2H), 7.70 (dd, $J$= 13.6, 8.0 Hz, 1H), 7.55 (t, $J$ = 9.9 Hz, 2H), 7.18 (d, $J$ = 4.8 Hz, 1H), 6.85 (s, 1H), 6.59 (t, $J$ = 54.4 Hz, 1H), 6.21 (d, $J$ = 16.1 Hz, 1H), 5.76 (m, 1H), 4.94 (d, $J$ = 46.0 Hz, 1H), 4.33 (m, 2H), 4.11 (m, 1H), 3.63 (m, 2H), 3.17 (dd, $J$ = 31.8, 21.7 Hz, 1H), 2.68 (d, $J$ = 48.7 Hz, 1H), 1.96 (d, $J$ = 44.0 Hz, 3H), 1.34 (dd, $J$= 20.8, 6.6 Hz, 3H), 1.06 (d, $J$= 6.4 Hz, 3H), 0.88 (s, 3H).

**Example 15: Preparation of 4-((S)-4-acryloyl-2-methylpiperazin-1-yl)-7-(2-fluoro-6-(fluoromethyl)phenyl)-6-fluoro-1-(2-isopropyl-4-methylpyridin-3-yl)pyrido[2,3-d]pyrimidin-2(1H)-one (compound 38) (prepared according to Scheme 4)**

Step 15a: Preparation of 2-(2-fluoro-6-(fluoromethyl)phenyl)-4,4,5,5-tetramethyl-1,3,2-dioxaborolane (compound 0403-38) : Under the protection of nitrogen, to a solution of (2-bromo-3-fluorophenyl)methanol (0.9 g, 4.39 mmol, 1.0 eq.) in dichloromethane (30 mL) was added dropwise diethylaminosulfur trifluoride (1.06 g, 6.58 mmol, 1.5 eq.) at 0°C, and the mixture was stirred at room temperature overnight. The mixture was poured into ice water and extracted with dichloromethane. The organic phase was dried and concentrated under reduced pressure. The residue was purified by column chromatography on silica gel (pure petroleum ether) to give 2-bromo-1-fluoro-3-(fluoromethyl)benzene (0.55 g, yield: 60.5%) as a colorless oil. Under the protection of nitrogen, to a solution of the above 2-bromo-1-fluoro-3-(fluoromethyl)benzene (0.55 g, 2.66 mmol, 1.0 eq.) in tetrahydrofuran (10 mL) was added dropwise n-butyllithium (1.6 mol, 2.2 mL, 3.45 mmol, 1.3 eq.) at -70°C, and the mixture was stirred at -70°C for 40 min. A solution of 2-methoxy-4,4,5,5-tetramethyl-1,3,2-dioxaborolane (0.67 g, 4.26 mmol, 1.6 eq.) in tetrahydrofuran (1.5 mL) was then added dropwise at -70°C and the mixture was stirred for 1 h. Aqueous ammonium chloride solution was added to quench the reaction solution, and the mixture was extracted with water and ethyl acetate. The organic phase was dried and concentrated

under reduced pressure. The residue (0.5 g, crude) was used directly in the next step without further purification.

Step 15b: Preparation of tert-butyl (3S)-4-(7-(2-fluoro-6-(fluoromethyl)phenyl)-6-fluoro-1-(2-isopropyl-4-methylpyridin-3-yl)-2-oxo-l,2-dihydropyrido[2,3-d]pyrimidin-4-yl)-3-methylpiperazine-1-carboxylate (0404-38): The synthetic method was similar to Step 14b of EXAMPLE 14, except that compound 0403-37 therein was replaced by 2-(2-fluoro-6-(fluoromethyl)phenyl)-4,4,5,5-tetramethyl-1,3,2-dioxaborolane (0403-38) (1.3 g, crude), tert-butyl (3S)-4-(7-(2-fluoro-6-(fluoromethyl)phenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (80 mg, yield: 85.6%) was obtained as a yellow liquid. MS ($ES^+$): m/z=623 $(M+H)^+$.

Step 15c: Preparation of 4-((S)-4-acryloyl-2-methylpiperazin-1-yl)-7-(2-fluoro-6-(fluoromethyl)phenyl)-6-fluoro-1-(2-isopropyl-4-methylpyridin-3-yl)pyrido[2,3-d]pyrimidin-2(1H)-one (compound 38): The synthetic method was similar to Step 12c of EXAMPLE 12, except that compound 0404-34 therein was replaced by tert-butyl (3S)-4-(7-(2-fluoro-6-(fluoromethyl)phenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0404-38) (80 mg, 0.128 mmol, 1.0 eq.), 4-((S)-4-acrloyl-2-methylpiperazin-1-yl)-7-(2-fluoro-6-(fluoromethyl)phenyl)-6-fluoro-1-(2-isopropyl-4-methylpyridin-3-yl)pyrido[2,3-d]pyrimidin-2(1H)-one (25 mg, yield: 33.8%) was obtained as a yellow solid. MS ($ES^+$): m/z=557 $(M+H)^+$. $^1H$ NMR (500 MHz, DMSO) $\delta$ 8.41 (s, 2H), 7.58 (s, 1H), 7.29 (m, 3H), 6.84 (s, 1H), 6.20 (d, *J* = 15.2 Hz, 1H), 5.76 (d, *J* = 9.4 Hz, 1H), 5.13 (ddd, *J* = 131.5, 82.1, 47.5 Hz, 3H), 4.33 (m, 2H), 4.08 (d, *J* = 56.0 Hz, 1H), 3.73 (d, *J* = 61.5 Hz, 2H), 3.07 (m, 1H), 2.74 (s, 1H), 1.92 (s, 3H), 1.34 (d, *J* = 19.4 Hz, 3H), 1.06 (s, 3H), 0.91 (d, *J* = 21.1 Hz, 3H).

**Example 16: Preparation of 4-((S)-4-acryloyl-2-methylpiperazin-1-yl)-6-fluoro-7-(2-fluoro-6-(trifluoromethyl)phenyl)-1-(2-isopropyl-4-methylpyridin-3-yl)pyrido[2,3-d]pyrimidin-2(1H)-one (compound 39) (prepared according to Scheme 4)**

Step 16a: Preparation of tert-butyl (3S)-4-(6-fluoro-7-(2-fluoro-6-(trifluoromethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate(compound 0404-39) The synthetic method was similar to Step 12b of Example 12, except that compound 0402-34 was replaced by (2-fluoro-6-(trifluoromethyl)phenyl)boronic acid (0402-39) (235 mg, 1.13 mmol), 6.0 eq.), tert-butyl (3 S)-4-(6-fluoro-7-(2-fluoro-6-(trifluoromethyl)phenyl)-1-(2-isopropyl-4-methylpyridin-3-yl)-2-oxo-l,2-dihydropyrido[2,3-d]pyrimidin-4-yl)-3-methylpiperazine-1-carboxylate (45 mg, yield: 36.3%) was obtained as a pale yellow solid. MS ($ES^+$): m/z=659 $(M+H)^+$

Step 16b: Preparation of 6-fluoro-7-(2-fluoro-6-(trifluoromethyl)phenyl)-1-(2-isopropyl-4-methylpyridin-3-yl)-4-((S)-2-methylpiperazin-1-yl)pyrido[2,3-d]pyrimidin-2(1H)-one (compound 0405-39) : The synthetic method was similar to Step 14c of Example 14, except that compound 0404-37 was replaced by tert-butyl (3S)-4-(6-fluoro-7-(2-fluoro-6-(trifluoromethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (0404-39) (45 mg, 0.224 mmol, 1.0 eq.), 6-fluoro-7-(2-fluoro-6-(trifluoromethyl)phenyl)-1-(2-isopropyl-4-methylpyridin-3-yl)-4-((S)-2-methylpiperazin-1-yl)pyrido[2,3-d]pyrimidin-2(1H)-one (28 mg, crude) was obtained as a yellow oil. MS ($ES^+$): m/z=559 $(M+H)^+$

Step 16c: Preparation of 4-((S)-4-acryloyl-2-methylpiperazin-1-yl)-6-fluoro-7-(2-fluoro-6-(trifluoromethyl)phenyl)-1-(2-isopropyl-4-methylpyridin-3-yl)pyrido[2,3-d]pyrimidin-2(1H)-one (compound 39) : The synthetic method was similar to Step 14d of Example 14, except that compound 0405-37 was replaced by 6-fluoro-7-(2-fluoro-6-(trifluoromethyl)phenyl)-1-(2-isopropyl-4-methylpyridin-3-yl)-4-((S)-2-methylpiperazin-1-yl)pyrido[2,3-d]pyrimidin-2(1H)-one (0405-39) (28 mg, crude), 4-((S)-4-acryloyl-2-methylpiperazin-1-yl)-6-fluoro-7-(2-fluoro-6-(trifluoromethyl)phenyl)-1-(2-isopropyl-4-methylpyridin-3-yl)pyrido[2,3-d]pyrimidin-2(1H)-one (21 mg, yield: 66.6%) was obtained as a yellow solid. MS ($ES^+$): m/z=613 $(M+H)^+$. m. p: 154-162°C; $^1H$ NMR (500 MHz, DMSO) $\delta$ 8.39 (m, 2H), 7.74 (d, *J* = 25.3 Hz, 3H), 7.15 (s, 1H), 6.83 (s, 1H), 6.19 (d, *J* = 15.4 Hz, 1H), 5.75 (d, *J*= 9.9 Hz, 1H), 4.93 (t, *J*= 40.8 Hz, 1H), 4.33 (m, 2H), 4.07 (d, *J*= 59.8 Hz, 1H), 3.70 (d, *J* = 35.5 Hz, 2H), 3.15 (m, 1H), 2.72 (d, *J* = 50.1 Hz, 1H), 1.85 (dd, *J*= 25.3, 5.2 Hz, 3H), 1.32 (t, *J*= 13.9 Hz, 3H), 1.05 (m, 3H), 0.88 (dd, *J*= 43.1, 5.9 Hz, 3H).

**Example 17: Preparation of (S)-4-(4-acryloyl-2-methylpiperazin-1-yl)-6-fluoro-7-(2-(hydroxymethyl)phenyl)-1-(2-isopropyl-4-methylpyridin-3-yl)pyrido[2,3-d]pyrimidin-2(1H)-one (compound 43) (prepared according to Scheme 5)**

Step 17a: Preparation of tert-butyl (S)-4-(6-fluoro-7-(2-(hydroxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0504-43): Under the protection of nitrogen, a mixture of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (120 mg, 0.226 mmol, 1.0 eq.), (2-(hydroxymethyl)phenyl)boronic acid (0502-43) (55 mg, 0.339 mmol, 1.50 eq.), sodium carbonate (72 mg, 0.679 mmol, 3.0 eq.) and tetrakis(triphenylphosphine)palladium (4 mg, 0.0028 mmol, 0.05 eq.) in acetonitrile (5 mL) and water(1mL) was stirred at 80°C overnight. After cooling to room temperature, water and ethyl acetate were added to extract and the organic layer was washed with brine and the solvent was removed in vacuo. The residue was purified by prep-TLC

(silica gel, eluent: dichloromethane/methanol=10/1) to give tert-butyl (S)-4-(6-fluoro-7-(2-(hydroxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (135 mg , yield: 99.2%) as a light-yellow solid. MS (ES$^+$): m/z=603 (M+H)$^+$.

Step 17b: Preparation of (S)-6-fluoro-7-(2-(hydroxymethyl)phenyl)-1-(2-isopropyl-4-methylpyridin-3-yl)-4-(2-methylpiperazin-1-yl)pyrido[2,3-d]pyrimidin-2(1H)-one (compound 0505-43): To a solution of tert-butyl (S)-4-(6-fluoro-7-(2-(hydroxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate(0504-43) (135 mg, 0.224 mmol, 1.00 eq.) in dichloromethane (5 mL) was added TFA (1mL) and the reaction was stirred at room temperature for 30 min. The mixture was concentrated in vacuo to give (S)-6-fluoro-7-(2-(hydroxymethyl)phenyl)-1-(2-isopropyl-4-methylpyridin-3-yl)-4-(2-methylpiperazin-1-yl)pyrido[2,3-d]pyrimidin-2(1H)-one (105 mg, crude) as a yellow oil. MS (ES$^+$): m/z=503 (M+H)$^+$.

Step 17c: Preparation of (S)-4-(4-acryloyl-2-methylpiperazin-1-yl)-6-fluoro-7-(2-(hydroxymethyl)phenyl)-1-(2-isopropyl-4-methylpyridin-3-yl)pyrido[2,3-d]pyrimidin-2(1H)-one (compound 43): To a mixture of (S)-6-fluoro-7-(2-(hydroxymethyl)phenyl)-1-(2-isopropyl-4-methylpyridin-3-yl)-4-(2-methylpiperazin-1-yl)pyrido[2,3-d]pyrimidin-2(1H)-one (0505-43) (105 mg crude)and DIPEA(0.5mL) in acetonitrile(5mL) was added dropwise acryloyl chloride (28.3 mg, 0.313 mmol, 1.50 eq.). The reaction was stirred at room temperature for 30 min and water and ethyl acetate were added to extract. The organic layer was washed with brine and concentrated in vacuo. The residue was purified by prep-TLC (silica gel, eluent: dichloromethane/methanol=10/1) to give (S)-4-(4-acryloyl-2-methylpiperazin-1-yl)-6-fluoro-7-(2-(hydroxymethyl)phenyl)-1-(2-isopropyl-4-methylpyridin-3-yl)pyrido[2,3-d]pyrimidin-2(1H)-one as a yellow solid (65 mg, yield: 56.0%). MS (ES$^+$): m/z=557 (M+H)$^+$. m. p: 145-157°C; $^1$H NMR (500 MHz, DMSO) $\delta$ 8.40 (d, *J* = 4.8 Hz, 1H), 8.31 (dd, *J* = 18.9, 9.9 Hz, 1H), 7.53 (d, *J*= 7.7 Hz, 1H), 7.43 (t, *J*= 7.5 Hz, 1H), 7.31 (t, *J*= 7.4 Hz, 1H), 7.25 (d, *J*= 7.6 Hz, 1H), 7.20 (d, *J* = 4.8 Hz, 1H), 6.85 (m, 1H), 6.21 (d, *J* = 16.5 Hz, 1H), 5.76 (d, *J* = 10.6 Hz, 1H), 4.92 (dd, *J* = 19.8, 13.9 Hz, 2H), 4.32 (m, 2H), 4.12 (m, 3H), 3.67 (m, 2H), 3.15 (t, *J* = 19.3 Hz, 1H), 2.72 (m, 1H), 1.93 (d, *J* = 4.0 Hz, 3H), 1.33 (dd, *J* = 19.5, 6.6 Hz, 3H), 1.06 (d, *J* = 6.6 Hz, 3H), 0.92 (d, *J* = 6.6 Hz, 3H).

**Example 18: Preparation of 4-((S)-4-acryloyl-2-methylpiperazin-1-yl)-6-fluoro-7-(2-fluoro-6-(hydroxymethyl)phenyl)-1-(2-isopropyl-4-methylpyridin-3-yl)pyrido[2,3-d]pyrimidin-2(1H)-one (compound 44) (prepared according to Scheme 5)**

Step 18a: Preparation of 2-(2-fluoro-6-((methoxymethoxy)methyl)phenyl)-4,4,5,5-tetramethyl-1,3,2-dioxaborolane (compound 0503-44): sodium borohydride (280 mg, 7.4 mmol, 1.5 eq.) was added slowly to a solution of 2-bromo-3-fluorobenzaldehyde (1 g, 4.9 mmol, 1 eq.) in methanol (20 mL). The mixture was stirred at room temperature for 2 h. The reaction was quenched with water and the mixture was filtered. The residue was washed with water, and dried to give (2-bromo-3-fluorophenyl)methanol (900 mg, 89.1%) as a white solid. MS (ESI): *m/z* 206(M+H)$^+$. Under the protection of nitrogen and in an ice bath, sodium hydride (263 mg,6.58 mmol, 1.5 eq.) was added to a solution of the above (2-bromo-3-fluorophenyl) methanol (900 mg,4.39 mmol, 1 eq.) in DMF (10 mL). The mixture was stirred at room temperature for 30 min. MOMBr (653 mg, 5.27 mmol, 1.2 eq.) in tetrahydrofuran (1 mL) was added dropwise to the mixture. The mixture was stirred at room temperature for another 1h. The reaction was quenched with saturated ammonium chloride and extracted with ethyl acetate. The organic phase was washed with brine, dried over sodium sulfate and concentrated and purified by column chromatography on silica gel (eluent: Petroleum ether: Ethyl acetate = 200:1) to give 2-bromo-1-fluoro-3-((methoxymethoxy)methyl)benzene (825 mg, 75.2%) as a clear oil. MS (ESI): *m/z* 251(M+H)$^+$. Under the protection of nitrogen, the above 2-bromo-1-fluoro-3-((methoxymethoxy)methyl)benzene (250 mg, 1 mmol, 1 eq.), bis(pinacolato)diboron (381 mg, 1.5 mmol, 1.5 eq.), tetrakis(triphenylphosphine)palladium (115 mg, 0.1 mmol, 0.1 eq.) and potassium acetate (294 mg,3 mmol,3 eq.) were added into toluene (10 mL). The mixture was stirred at 110°C for 16 h. The mixture was then cooled to room temparature and filtered. The filtrate was concentrated under vacuum to give 2-(2-fluoro-6-((methoxymethoxy)methyl)phenyl)-4,4,5,5-tetramethyl-1,3,2-dioxaborolane (358 mg, crude) as a black oil. MS (ESI): *m/z* 297(M+H)$^+$.

Step 18b: Preparation of tert-butyl (3S)-4-(6-fluoro-7-(2-fluoro-6-((methoxymethoxy)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0504-44): Under the protection of nitrogen, tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (150 mg, 0.283 mmol, 1 eq.), 2-(2-fluoro-6-((methoxymethoxy)methyl)phenyl)-4,4,5,5-tetramethyl-1,3,2-dioxaborolane (0503-44) (126 mg, 0.424 mmol, 1.5 eq.), 1, 1'-bis(diphenylphosphino)ferrocene palladium(II)dichloride (20 mg, 0.0283 mmol, 0.1 eq.) and potassium carbonate (118 mg, 0.849 mmol,3 eq.) were added into a mixture of 1,4-dioxane (10 mL) and water(1 mL). The mixture was stirred at 92°C for 3 h. The mixture was then cooled to room temparature and extracted with ethyl acetate and water. The organic phase was washed with brine, dried over anhydrous sodium sulfate, and concentrated under vacuum. The residue was purified by perp-TLC (eluent: dichloromethane/methanol=15/1) to give tert-butyl (3S)-4-(6-fluoro-7-(2-fluoro-6-((methoxymethoxy)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate as a yellow solid (173 mg, 92.02%). MS (ESI): *m/z* 665(M+H)$^+$.

Step 18c: Preparation of 6-fluoro-7-(2-fluoro-6-(hydroxymethyl)phenyl)-1-(2-isopropyl-4-methylpyridin-3-yl)-4-((S)-2-methylpiperazin-1-yl)pyrido[2,3-d]pyrimidin-2(1H)-one (compound 0505-44): tert-butyl (3 S)-4-(6-fluoro-7-(2-fluoro-6-((methoxymethoxy)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (0505-44) (170 mg, 0.251 mmol, 1.0 eq.) was dissolved in 4 mL dichloromethane, and 1 mL TFA was then added. The mixture was stirred at room temperature for 16 h. The mixture was then concentrated and the PH of the residue was adjust with sodium hydroxide to pH 14. The mixture was extracted with dichloromethane and the organic phase was concentrated and purified by prep-TLC (silica gel, eluent: dichloromethane/methanol=10/1) to give 6-fluoro-7-(2-fluoro-6-(hydroxymethyl)phenyl)-1-(2-isopropyl-4-methylpyridin-3-yl)-4-((S)-2-methylpiperazin-1-yl)pyrido[2,3-d]pyrimidin-2(1H)-one (85.5 mg, 64.22%) as a yellow solid. MS (ESI): *m/z* 521$(M+H)^+$.

Step 18d: Preparation of 4-((S)-4-acryloyl-2-methylpiperazin-1-yl)-6-fluoro-7-(2-fluoro-6-(hydroxymethyl)phenyl)-1-(2-isopropyl-4-methylpyridin-3-yl)pyrido[2,3-d]pyrimidin-2(1H)-one (compound 44): 6-fluoro-7-(2-fluoro-6-(hydroxymethyl)phenyl)-1-(2-isopropyl-4-methylpyridin-3-yl)-4-((S)-2-methylpiperazin-1-yl)pyrido[2,3-d]pyrimidin-2(1H)-one (0505-44) (85.5 mg, 0.164 mmol, 1.0 eq.) and DIPEA (64 mg, 0.492 mmol, 3.0 eq.) were dissolved in 4 mL tetrahydrofuran, and acryloyl chloride (18 mg, 0.197 mmol, 1.2 eq.) was then added into the mixture. The mixture was stirred at room temperature for 15 min. The reaction was quenched with ammonium hydroxide and extracted with dichloromethane and water. The organic phase was concentrated and purified by prep-TLC (silica gel, eluent: dichloromethane/methanol=15/1) to give 4-((S)-4-acryloyl-2-methylpiperazin-1-yl)-6-fluoro-7-(2-fluoro-6-(hydroxymethyl)phenyl)-1-(2-isopropyl-4-methylpyridin-3-yl)pyrido[2,3-d]pyrimidin-2(1H)-one (49 mg, 52.13%) as a yellow solid. MS (ESI): *m/z* 575$(M+H)^+$. Melting point: 170~171°C. $^1$H NMR (500 MHz, DMSO) δ 8.36 (m, 2H), 7.49 (m, 1H), 7.35 (d, *J*= 7.8 Hz, 1H), 7.19 (t, *J* = 8.9 Hz, 2H), 6.86 (d, *J* = 11.6 Hz, 1H), 6.21 (d, *J* = 16.6 Hz, 1H), 5.77 (dd, *J* = 10.4, 2.2 Hz, 1H), 5.08 (s, 1H), 4.96 (dd, *J* = 25.8, 22.7 Hz, 1H), 4.33 (m, 2H), 4.06 (m, 3H), 3.60 (m, 2H), 3.15 (m, 1H), 2.72 (d, *J* = 11.5 Hz, 1H), 1.95 (s, 3H), 1.33 (dt, *J* = 18.2, 6.1 Hz, 3H), 1.07 (d, *J*= 6.0 Hz, 3H), 0.85 (d, *J*= 7.0 Hz, 3H).

**Example 19: Preparation of 4-((S)-4-acryloyl-2-methylpiperazin-1-yl)-6-fluoro-7-(2-chloro-6-(hydroxymethyl)phenyl)-1-(2-isopropyl-4-methylpyridin-3-yl)pyrido[2,3-d]pyrimidin-2(1H)-one (compound 45) (prepared according to Scheme 5)**

Step 19a: Preparation of 2-(2-chloro-6-((methoxymethoxy)methyl)phenyl)-4,4,5,5-tetramethyl-1,3,2-dioxaborolane (compound 0502-45): sodium borohydride (260 mg, 6.83 mmol, 1.5 eq.) was added slowly to a solution of 2-bromo-3-chlorobenzaldehyde (1 g, 4.55 mmol, 1 eq.) in methanol (20 mL). The mixture was stirred at room temperature for 2h. The reaction was quenched with water and the mixture was filtered. The residue was washed with water and dried to give (2-bromo-3-chlorophenyl)methanol as a white solid (826 mg, 82.6%). MS (ESI): *m/z* 221$(M+H)^+$. Under the protection of nitrogen and in an ice bath, sodium hydride (298 mg,7.46 mmol,2 eq.) was added to a solution of (2-bromo-3-chlorophenyl)methanol (826 mg,3.73 mmol, 1 eq.) prepared above in tetrahydrofuran (10 mL). The mixture was stirred at room temperature for 30 min. MOMBr (694 mg, 5.60 mmol, 1.5 eq.) in tetrahydrofuran (1 mL) was added dropwise to the mixture. The mixture was stirred at room temperature for another 1h. The reaction was quenched with saturated ammonium chloride and extracted with ethyl acetate and water. The organic phase was washed with brine, dried over sodium sulfate and concentrated and purified by column chromatography on silica gel ( eluent: Petroleum ether: Ethyl acetate= 200:1) to give 2-bromo-1-chloro-3-((methoxymethoxy)methyl)benzene as a clear oil (864 mg, 87.62%). MS (ESI): *m/z* 265$(M+H)^+$. Under the protection of nitrogen, 2-bromo-1-chloro-3-((methoxymethoxy)methyl)benzene (400 mg, 1.5 mmol, 1 eq.) prepared above, bis(pinacolato)diboron (581 mg,2.29 mmol, 1.5 eq.), tetrakis(triphenylphosphine)palladium (175 mg, 0.15 mmol, 0.1 eq.) and potassium acetate (449 mg,4.58 mmol,3 eq.) were added into toluene (10 mL). The mixture was stirred at 110°C for 16 h. Then, the mixture was cooled to room temperature and filtered. The filtrate was concentrated under vacuum to give 2-(2-chloro-6-((methoxymethoxy)methyl)phenyl)-4,4,5,5-tetramethyl-1,3,2-dioxaborolane as a black oil (360 mg, crude). MS (ESI): *m/z* 313$(M+H)^+$.

Step 19b: Preparation of tert-butyl (3S)-4-(6-fluoro-7-(2-chloro-6-((methoxymethoxy)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0504-45) : The synthetic method was similar to Step 18b of Example 18, except that compound 0503-44 was replaced by 2-(2-chloro-6-((methoxymethoxy)methyl)phenyl)-4,4,5,5-tetramethyl-1,3,2-dioxaborolane (0502-45), tert-butyl (3 S)-4-(6-fluoro-7-(2-chloro-6-((methoxymethoxy)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate(130 mg, crude) was obtained as a yellow solid. MS $(ES^+)$: m/z=681 $(M+H)^+$.

Step 19c: Preparation of 6-fluoro-7-(2-chloro-6-(hydroxymethyl)phenyl)-1-(2-isopropyl-4-methylpyridin-3-yl)-4-((S)-2-methylpiperazin-1-yl)pyrido[2,3-d]pyrimidin-2(1H)-one (compound 0505-45) : The synthetic method was similar to Step 18c of Example 18, except that compound 0504-44 was replaced by tert-butyl (3S)-4-(6-fluoro-7-(2-chloro-6-((methoxymethoxy)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (0504-45), 6-fluoro-7-(2-chloro-6-(hydroxymethyl)phenyl)-1-(2-isopropyl-4-methylpyridin-3-yl)-4-((S)-2-methylpiperazin-1-yl)pyrido[2,3-d]pyrimidin-2(1H)-one (45 mg, yield: 44.12%) was obtained as a

yellow solid. MS ($ES^+$): m/z=537 $(M+H)^+$.

Step 19d: Preparation of 4-((S)-4-acryloyl-2-methylpiperazin-1-yl)-6-fluoro-7-(2-chloro-6-(hydroxymethyl)phenyl)-1-(2-isopropyl-4-methylpyridin-3-yl)pyrido[2,3-d]pyrimidin-2(1H)-one (compound 45) : The synthetic method was similar to Step 18d of Example 18, except that compound 0505-44 was replaced by 6-fluoro-7-(2-chloro-6-(hydroxymethyl)phenyl)-1-(2-isopropyl-4-methylpyridin-3-yl)-4-((S)-2-methylpiperazin-1-yl)pyrido[2,3-d]pyrimidin-2(1H)-one (0505-45) (45 mg, 0.08 mmol, 1.0 eq.), 4-((S)-4-acryloyl-2-methylpiperazin-1-yl)-6-fluoro-7-(2-chloro-6-(hydroxymethyl)phenyl)-1-(2-isopropyl-4-methylpyridin-3-yl)pyrido[2,3-d]pyrimidin-2(1H)-one (26.5 mg, yield: 53.53%) was obtained as a yellow solid. MS ($ES^+$): m/z=537 $(M+H)^+$. Melting point: 174~175°C; $^1$H NMR (500 MHz, DMSO) $\delta$ 8.36 (t, *J* = 4.6 Hz, 2H), 7.45 (tdd, *J* = 9.7, 6.6, 3.5 Hz, 3H), 7.18 (dd, *J*= 13.6, 4.9 Hz, 1H), 6.85 (s, 1H), 6.21 (d, *J*= 16.9 Hz, 1H), 5.76 (dd, *J*= 9.1, 7.1 Hz, 1H), 5.19 (ddd, *J* = 30.7, 10.9, 5.5 Hz, 1H), 4.96 (m, 1H), 4.33 (m, 2H), 4.07 (m, 3H), 3.74 (m, 2H), 3.17 (d, *J* = 5.3 Hz, 1H), 2.74 (m, 1H), 1.90 (dd, *J* = 55.4, 6.0 Hz, 3H), 1.34 (ddd, *J* = 22.9, 12.4, 6.0 Hz, 3H), 1.07 (m, 3H), 0.94 (m, 3H).

**Example 20: Preparation of 4-((S)-4-acryloyl-2-methylpiperazin-1-yl)-6-fluoro-7-(2-fluoro-6-(1-hydroxyethyl)phenyl)-1-(2-isopropyl-4-methylpyridin-3-yl)pyrido[2,3-d]pyrimidin-2(1H)-one (compound 46) (prepared according to Scheme 5)**

Step 20a: Preparation of 2-(2-fluoro-6-(1-(methoxymethoxy)ethyl)phenyl)-4,4,5,5-tetramethyl-1,3,2-dioxaborolane (compound 0503-46): Under the protection of nitrogen, MeMgBr (1.6 mL, 3 mol/L in diethyl ether ,4.73 mmol, 1.2 eq.) was added dropwise to a solution of 2-bromo-3-fluorobenzaldehyde (800 mg, 3.94 mmol, 1 eq.) in tetrahydrofuran (10 mL). The mixture was stirred at room temperature for 1 h. The reaction was quenched with saturated ammonium chloride and extracted with ethyl acetate and water. The organic phase was washed with brine, dried over sodium sulfate and concentrated to give 1-(2-bromo-3-fluorophenyl)ethan-1-ol (1.09 g, crude) as a yellow oil. MS (ESI): *m*/*z* 219$(M+H)^+$. Under the protection of nitrogen and in an ice bath, sodium hydride (398 mg,9.95 mmol,2 eq.) was added to a solution of 1-(2-bromo-3-fluorophenyl)ethan-1-ol (1090 mg,4.97 mmol, 1 eq.) obtained above in tetrahydrofuran (10 mL). The mixture was stirred at room temperature for 30 min. MOMBr (926 mg, 7.46 mmol, 1.5 eq.) in tetrahydrofuran (1 mL) was added dropwise to the mixture. The mixture was stirred at room temperature for another 1h. The reaction was quenched with saturated ammonium chloride and extracted with ethyl acetate and water. The organic phase was washed with brine, dried over sodium sulfate and concentrated and purified by column chromatography on silica gel ( eluent: petroleum ether: ethyl acetate= 200:1) to give 2-bromo-1-fluoro-3-(1-(methoxymethoxy)ethyl)benzene (977 mg, 75.15%) as a clear oil. MS (ESI): *m*/*z* 263$(M+H)^+$. Under the protection of nitrogen, 2-bromo-1-fluoro-3-(1-(methoxymethoxy)ethyl)benzene (400 mg, 1.52 mmol, 1 eq.) obtained above, bis(pinacolato)diboron (581 mg, 2.29 mmol, 1.5 eq.), tetrakis(triphenylphosphine)palladium (175 mg, 0.152 mmol, 0.1 eq.) and potassium acetate (449 mg, 4.58 mmol,3 eq.) were added into toluene (10 mL). The mixture was stirred at 110°C for 16 h. The mixture was then cooled to room temparature and filtered. The filtrate was concentrated under vacuum to give 2-(2-fluoro-6-(1-(methoxymethoxy)ethyl)phenyl)-4,4,5,5-tetramethyl-1,3,2-dioxaborolane (539 mg, crude) as a black oil. MS (ESI): *m*/*z* 311$(M+H)^+$.

Step 20b: Preparation of tert-butyl (3S)-4-(6-fluoro-7-(2-fluoro-6-(1-(methoxymethoxy)ethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate(compound 0504-46) : The synthetic method was similar to Step 18b of Example 18, except that compound 0503-44 was replaced by 2-(2-fluoro-6-(1-(methoxymethoxy)ethyl)phenyl)-4,4,5,5-tetramethyl-1,3,2-dioxaborolane (0503-46) (105 mg, 0.34 mmol, 1.2 eq.), tert-butyl (3S)-4-(6-fluoro-7-(2-fluoro-6-(1-(methoxymethoxy)ethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (176 mg, crude) was obtained as a yellow solid. MS (ESI): *m*/*z* 679$(M+H)^+$.

Step 20c: Preparation of 6-fluoro-7-(2-fluoro-6-(1-hydroxyethyl)phenyl)-1-(2-isopropyl-4-methylpyridin-3-yl)-4-((S)-2-methylpiperazin-1-yl)pyrido[2,3-d]pyrimidin-2(1H)-one (compound 0505-46) : The synthetic method was similar to Step 18c of Example 18, except that compound 0504-44 was replaced by tert-butyl (3S)-4-(6-fluoro-7-(2-fluoro-6-(1-(methoxymethoxy)ethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (0504-46), 6-fluoro-7-(2-fluoro-6-(1-hydroxyethyl)phenyl)-1-(2-isopropyl-4-methylpyridin-3-yl)-4-((S)-2-methylpiperazin-1-yl)pyrido[2,3-d]pyrimidin-2(1H)-one (27 mg, yield: 20.15%) was obtained as a yellow solid. MS (ESI): *m*/*z* 535$(M+H)^+$.

Step 20d: Preparation of 4-((S)-4-acryloyl-2-methylpiperazin-1-yl)-6-fluoro-7-(2-fluoro-6-(1-hydroxyethyl)phenyl)-1-(2-isopropyl-4-methylpyridin-3-yl)pyrido[2,3-d]pyrimidin-2(1H)-one (compound 46) : The synthetic method was similar to Step 18d of Example 18, except that compound 0505-44 was replaced by 6-fluoro-7-(2-fluoro-6-(1-hydroxyethyl)phenyl)-1-(2-isopropyl-4-methylpyridin-3-yl)-4-((S)-2-methylpiperazin-1-yl)pyrido[2,3-d]pyrimidin-2(1H)-one (0505-46) (27 mg, 0.05 mmol, 1.0 eq.), 4-((S)-4-acryloyl-2-methylpiperazin-1-yl)-6-fluoro-7-(2-fluoro-6-(1-hydroxyethyl)phenyl)-1-(2-isopropyl-4-methylpyridin-3-yl)pyrido[2,3-d]pyrimidin-2(1H)-one (14 mg, yield: 47.14%) was obtained as a yellow solid. MS (ESI): *m*/*z* 589$(M+H)^+$. Melting point: 154~155°C; $^1$H NMR (500 MHz, DMSO) $\delta$ 8.36 (dd, *J*= 6.8, 3.7 Hz, 2H), 7.50 (m, 1H), 7.41 (dt, *J*= 13.0, 6.7 Hz, 1H), 7.17 (dd, *J* = 15.3, 6.7 Hz, 2H), 6.85 (s, 1H), 6.21 (d, *J* = 16.8

Hz, 1H), 5.77 (dd, *J* = 10.5, 2.0 Hz, 1H), 5.02 (m, 2H), 4.36 (m, 3H), 4.08 (d, *J*= 60.8 Hz, 1H), 3.70 (d, *J* = 34.1 Hz, 2H), 3.27 (s, 3H), 3.18 (s, 1H), 2.76 (m, 1H), 1.92 (m, 3H), 1.35 (m, 3H), 1.04 (m, 3H), 0.84 (dt, *J* = 14.9, 6.9 Hz, 3H).

**Example 21: Preparation of 4-((S)-4-acryloyl-2-methylpiperazin-1-yl)-6-fluoro-7-(2-fluoro-6-(methoxymethyl)phenyl)-1-(2-isopropyl-4-methylpyridin-3-yl)pyrido[2,3-d]pyrimidin-2(1H)-one (compound 48) (prepared according to Scheme 5)**

Step 21a: Preparation of 2-(2-fluoro-6-(methoxymethyl)phenyl)-4,4,5,5-tetramethyl-1,3,2-dioxaborolane (compound 0503-48): sodium methanolate (3.38 mL, 1 mol/L in methanol ,3.38 mmol, 1.5 eq.) was added to a solution of 2-bromo-1-(bromomethyl)-3-fluorobenzene (600 mg, 2.25 mmol, 1 eq.) in methanol (10 mL). The mixture was stirred at room temperature for 1h. The reaction was quenched with water and the mixture was extracted with Petroleum ether. The organic phase was washed with brine, dried over sodium sulfate and concentrated and purified by column chromatography on silica gel ( eluent: Petroleum ether: Ethyl acetate= 200:1) to give 2-bromo-1-fluoro-3-(methoxymethyl)benzene (415 mg, 84.35) as a yellow oil. MS (ESI): *m/z* 219(M+H)$^+$. Under the protection of nitrogen, 2-bromo-1-fluoro-3-(methoxymethyl)benzene (415 mg, 1.89 mmol, 1 eq.) obtained above, bis(pinacolato)diboron (722 mg,2.84 mmol, 1.5 eq.), tetrakis(triphenylphosphine)palladium (109 mg, 0.09 mmol, 0.1 eq.) and potassium acetate (557 mg,5.68 mmol,3 eq.) were added into toluene (10 mL). The mixture was stirred at 110°C for 16 h. Then, the mixture was cooled to room temperature and filtered. The filtrate was concentrated under vacuum to give 2-(2-fluoro-6-(methoxymethyl)phenyl)-4,4,5,5-tetramethyl-1,3,2-dioxaborolanel (545 mg, crude) as a black oi. MS (ESI): *m/z* 267(M+H)$^+$.

Step 21b: Preparation of tert-butyl (3S)-4-(6-fluoro-7-(2-fluoro-6-(methoxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0504-48) : The synthetic method was similar to Step 18b of Example 18, except that compound 0503-44 was replaced by 2-(2-fluoro-6-(methoxymethyl)phenyl)-4,4,5,5-tetramethyl-1,3,2-dioxaborolane (0503-48) (90 mg, 0.34 mmol, 1.2 eq.), tert-butyl (3S)-4-(6-fluoro-7-(2-fluoro-6-(methoxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate(125 mg, crude) was obtained as a yellow solid. MS (ESI): *m/z* 635(M+H)$^+$.

Step 21c: Preparation of 4-((S)-4-acryloyl-2-methylpiperazin-1-yl)-6-fluoro-7-(2-fluoro-6-(methoxymethyl)phenyl)-1-(2-isopropyl-4-methylpyridin-3-yl)pyrido[2,3-d]pyrimidin-2(1H)-one (compound 48): tert-butyl (3S)-4-(6-fluoro-7-(2-fluoro-6-(methoxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate(0504-48) (125 mg, 0.197 mmol, 1.0 eq.) was dissolved in 4 mL dichloromethane, and 1 mL TFA was then added. The mixture was stirred at room temperature for 2 h. The mixture was then concentrated. The residue was dissolved in tetrahydrofuran (5 mL) and DIPEA (76 mg, 0.591 mmol, 3 eq.) was added followed by the addition of acryloyl chloride (21 mg, 0.236 mmol, 1.2 eq.). The mixture was stirred at room temperature for 15 min and was then quenched with ammonium hydroxide. The mixture was extracted with dichloromethane. The organic phase was concentrated and purified by HPLC to give 4-((S)-4-acryloyl-2-methylpiperazin-1-yl)-6-fluoro-7-(2-fluoro-6-(methoxymethyl)phenyl)-1-(2-isopropyl-4-methylpyridin-3-yl)pyrido[2,3-d]pyrimidin-2(1H)-one as a yellow solid (26 mg, 22.41%). MS (ESI): *m/z* 589(M+H)$^+$. Melting point: 134~135°C; $^1$H NMR (500 MHz, DMSO) δ 8.37 (t, *J*= 17.7 Hz, 2H), 7.50 (dd, *J*= 13.8, 7.8 Hz, 1H), 7.26 (m, 3H), 6.84 (s, 1H), 6.20 (d, *J* = 16.1 Hz, 1H), 5.76 (d, *J*= 10.5 Hz, 1H), 4.93 (d, *J* = 53.7 Hz, 1H), 4.33 (m, 2H), 4.06 (d, *J* = 34.5 Hz, 3H), 3.63 (dd, *J* = 105.5, 36.5 Hz, 2H), 3.07 (d, *J* = 40.6 Hz, 4H), 2.73 (s, 1H), 1.97 (d, *J* = 34.4 Hz, 3H), 1.34 (m, 3H), 1.07 (s, 3H), 0.84 (d, *J* = 6.6 Hz, 3H).

**Example 22: Preparation of 4-((S)-4-acryloyl-2-methylpiperazin-1-yl)-7-(2-(ethoxymethyl)-6-fluorophenyl)-6-fluoro-1-(2-isopropyl-4-methylpyridin-3-yl)pyrido[2,3-d]pyrimidin-2(1H)-one (compound 49) (prepared according to Scheme 5)**

Step 22a: Preparation of (2-(ethoxymethyl)-6-fluorophenyl)boronic acid (compound 0502-49): Under the protection of nitrogen, sodium hydride (1.5 g, 37.6 mmol, 10.0 eq.) was added to EtOH (15 mL) at 0°C, the mixture was stirred at room temparature for 1 h. 2-Bromo-1- (bromomethyl) -3-fluorobenzene was then added to the above reaction, and the mixture was stirred at room temparature for 2 h. The reaction solution was extracted with water and ethyl acetate, and the organic phase was dried and concentrated under vacuum. The residue was purified by column chromatography on silica gel (petroleum ether: ethyl acetate=10:1) to give product 2-bromo-1-(ethoxymethyl)-3-fluorobenzene (610 mg, yield: 69.6%) as a yellow oil. Under the protection of nitrogen, to a solution of 2-bromo-1-(ethoxymethyl)-3-fluorobenzene (508 mg, 2.18 mmol, 1.0 eq.) obtained above in tuluene (10 mL) was added n-butyllithium (1.6 ml, 2.61 mmol, 1.2 eq.) dropwise under -70°C and the mixture was stirred at -70 °C for 0.5 h. Then the solution of trimethyl borate (340 mg, 3.27 mmol, 1.5 eq.) in toluene (1 mL) was added under -70 °C and the mixture was stirred for 1 h. A solution of 2M sodium hydroxide was added to the reaction solution to adjust pH = 10, and the mixture was extracted with petroleum ether. After separation, the aqueous phase was adjusted to pH = 5 with 1M hydrochloric acid and extracted with ethyl acetate. The obtained organic phase was dried and concentrated under vacuum. The residue was used directly in the next step

without further purification.

Step 22b: Preparation of tert-butyl (3 S)-4-(7-(2-(ethoxymethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0504-49): Under the protection of nitrogen, (2-(ethoxymethyl)-6-fluorophenyl)boronic acid (0502-49) (100 mg, 0.508 mmol, 3.0 eq.), tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (90 mg, 0.169 mmol, 1.0 eq.), tetrakis(triphenylphosphine)palladium (19.5 mg, 0.0169 mmol, 0.1 eq.), and sodium carbonate (36 mg, 0.338 mmol, 2.0 eq.) were added into a mixed solvent of 10 mL acetonitrile and 2 mL water. The mixture was stirred at 80°C overnight. The reaction solution was cooled to room temperature, and extracted with ethyl acetate and water. The organic phase was washed with brine, and concentrated under vacuum. The residue was purified by column chromatography on silica gel (eluent: dichloromethane/methanol=100/1 to 20/1) to give tert-butyl (3S)-4-(7-(2-(ethoxymethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxyl (90 mg, yield: 82.3%) as a yellow solid. MS ($ES^+$): m/z=649 $(M+H)^+$.

Step 22c: Preparation of 7-(2-(ethoxymethyl)-6-fluorophenyl)-6-fluoro-1-(2-isopropyl-4-methylpyridin-3-yl)-4-((S)-2-methylpiperazin-1-yl)pyrido[2,3-d]pyrimidin-2(1H)-one (compound 0505-49) : The synthetic method was similar to Step 18c of Example 18, except that compound 0504-44 was replaced by tert-butyl (3S)-4-(7-(2-(ethoxymethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxyl (0504-49) (90 mg, 0.14 mmol, 1.0 eq.). The product was prepared and used directly in the next step without further purification. MS (ESI): *m/z* 549$(M+H)^+$.

Step 22d: Preparation of 4-((S)-4-acryloyl-2-methylpiperazin-1-yl)-7-(2-(ethoxymethyl)-6-fluorophenyl)-6-fluoro-1-(2-isopropyl-4-methylpyridin-3-yl)pyrido[2,3-d]pyrimidin-2(1H)-one (compound 49) : The synthetic method was similar to Step 18d of Example 18, except that compound 0505-44 was replaced by 7-(2-(ethoxymethyl)-6-fluorophenyl)-6-fluoro-1-(2-isopropyl-4-methylpyridin-3-yl)-4-((S)-2-methylpiperazin-1-yl)pyrido[2,3-d]pyrimidin-2(1H)-one (0505-49), 4-((S)-4-acryloyl-2-methylpiperazin-1-yl)-7-(2-(ethoxymethyl)-6-fluorophenyl)-6-fluoro-1-(2-isopropyl-4-methylpyridin-3-yl)pyrido[2,3-d]pyrimidin-2(1H)-one (51mg, yield: 60.5%) was obtained as a yellow solid. MS ($ES^+$): *m/z* 603 $(M+H)^+$. Melting point: 115~120°C; $^1H$ NMR (500 MHz, DMSO) δ 8.37 (dd, *J* = 23.4, 7.5 Hz, 2H), 7.50 (dd, J = 13.9, 7.9 Hz, 1H), 7.26 (m, 3H), 6.86 (d, *J* = 11.0 Hz, 1H), 6.21 (d, *J* = 16.3 Hz, 1H), 5.77 (dd, *J* = 10.5, 2.0 Hz, 1H), 4.93 (d, *J* = 49.1 Hz, 1H), 4.34 (dd, *J* = 40.7, 27.4 Hz, 2H), 4.09 (dd, *J* = 42.2, 10.8 Hz, 3H), 3.62 (dd, *J* = 115.8, 30.8 Hz, 2H), 3.17 (dd, *J* = 13.3, 7.3 Hz, 3H), 2.68 (d, *J* = 38.8 Hz, 1H), 1.99 (d, *J* = 26.2 Hz, 3H), 1.35 (m, 3H), 1.06 (d, *J* = 3.9Hz, 3H), 0.91 (m, 6H).

**Example 23: Preparation of 4-((S)-4-acryloyl-2-methylpiperazin-1-yl)-6-fluoro-7-(2-fluoro-6-(isopropoxymethyl)phenyl)-1-(2-isopropyl-4-methylpyridin-3-yl)pyrido[2,3-d]pyrimidin-2(1H)-one (compound 50) (prepared according to Scheme 5)**

Step 23a: Preparation of 2-(2-fluoro-6-(isopropoxymethyl)phenyl)-4,4,5,5-tetramethyl-1,3,2-dioxaborolane (compound 0503-50): To a mixture of sodium hydride (60%, 178 mg, 4.48 mmol, 2.0 eq.)in tetrahydrofuran (8mL) was added propan-2-ol(0.34 mL, 4.48 mmol, 2.0 eq.)and the mixture was stirred at room temperature for 20 min. 2-Bromo-1-(bromomethyl)-3-fluorobenzene(0.6 g, 2.24 mmol, 1.0 eq.)was added. The mixture was stirred for additional 2.5 h. Saturated ammonium chloride aqueous solution (20 mL) was added to quench the reaction. The aqueous layer was extracted with ethyl acetate (20 mL×3). The combined organic layers were washed with brine(20 mL×1),dried over anhydrous sodium sulfate and concentrated. The residue was purified by column chromatography on silica gel (petroleum ether:ethyl acetate 20:1) to get 2-bromo-1-fluoro-3-(isopropoxymethyl)benzene(270 mg, yield: 49%) as a pale yellow oil. LCMS(ESI): *m/z* 248$[M+1]^+$. To a mixture of 2-bromo-1-fluoro-3-(isopropoxymethyl)benzene(0.27 g, 1.093 mmol, 1.0 eq.)obtained above in tetrahydrofuran(7 mL) was added n-BuLi(1.6M solution in hexane, 0.75 mL, 1.202 mmol, 1.1 eq.)at -78°C under $N_2$ atmosphere. The mixture was stirred for 40 min. 2-methoxy-4,4,5,5-tetramethyl-1,3,2-dioxaborolane(0.36 mL, 2.186 mmol, 2.0 eq.) was added and the mixture was stirred at -78°C for additional 1 h. Saturated ammonium chloride solution (20mL) was added to quench the reaction. The aqueous layer was extracted with ethyl acetate(20 mL×3). The combined organic layers were washed with brine(20 mL×1), dried over anhydrous sodium sulfate and concentrated to get 2-(2-fluoro-6-(isopropoxymethyl)phenyl)-4,4,5,5-tetramethyl-1,3,2-dioxaborolane(0.354 g, crude) as a pale yellow oil. LCMS(ESI): *m/z* 295$[M+1]^+$; TLC: Rf 0.5(petroleum ether:ethyl acetate=10:1).

Step 23b: Preparation of tert-butyl (3S)-4-(6-fluoro-7-(2-fluoro-6-(isopropoxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0504-50) : The synthetic method was similar to Step 18b of Example 18, except that compound 0503-44 was replaced by 2-(2-fluoro-6-(isopropoxymethyl)phenyl)-4,4,5,5-tetramethyl-1,3,2-dioxaborolane (0503-50) (74.9 mg, 0.255 mmol, 1.5 eq.), tert-butyl (3S)-4-(6-fluoro-7-(2-fluoro-6-(isopropoxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate was obtained as a yellow solid. LCMS (ESI): m / z 663$([M + 1)^+$; TLC: Rf 0.5 (dichloromethane: Methanol =20:1).

Step 23c: Preparation of 6-fluoro-7-(2-fluoro-6-(isopropoxymethyl)phenyl)-1-(2-isopropyl-4-methylpyridin-3-yl)-4-((S)-2-methylpiperazin-1-yl)pyrido[2,3-d]pyrimidin-2(1H)-one (compound 0505-50) : The synthetic method was similar to Step 18c of Example 18, except that compound 0504-44 was replaced by tert-butyl (3S)-4-(6-fluoro-7-(2-fluoro-6-(isopropoxymethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (0504-50) (50 mg, 0.075 mmol, 1.0 eq.), 6-fluoro-7-(2-fluoro-6-(isopropoxymethyl)phenyl)-1-(2-isopropyl-4-methylpyridin-3-yl)-4-((S)-2-methylpiperazin-1-yl)pyrido[2,3-d]pyrimidin-2(1H)-one (25 mg, yield: 60%) was obtained as a colorless oil. LCMS (ESI): m / z 563([M + 1) $^+$; TLC: Rf 0.3 (dichloromethane: Methanol =10:1).

Step 23d: Preparation of 4-((S)-4-acryloyl-2-methylpiperazin-1-yl)-6-fluoro-7-(2-fluoro-6-(isopropoxymethyl)phenyl)-1-(2-isopropyl-4-methylpyridin-3-yl)pyrido[2,3-d]pyrimidin-2(1H)-one (compound 50) : The synthetic method was similar to Step 18d of Example 18, except that compound 0505-44 was replaced by 6-fluoro-7-(2-fluoro-6-(isopropoxymethyl)phenyl)-1-(2-isopropyl-4-methylpyridin-3-yl)-4-((S)-2-methylpiperazin-1-yl)pyrido[2,3-d]pyrimidin-2(1H)-one (0505-50), 4-((S)-4-acryloyl-2-methylpiperazin-1-yl)-6-fluoro-7-(2-fluoro-6-(isopropoxymethyl)phenyl)-1-(2-isopropyl-4-methylpyridin-3-yl)pyrido[2,3-d]pyrimidin-2(1H)-one (8 mg, yield: 30%) was obtained as a white solid. LCMS (ESI): m / z 617([M + 1) $^+$; TLC: Rf 0.6 (dichloromethane: Methanol =10:1). Melting point: 118-122°C. $^1$H NMR (500 MHz, DMSO) δ 8.38 (t, *J* = 12.3 Hz, 2H), 7.48 (dd, *J* = 14.0, 7.7 Hz, 1H), 7.26 (m, 3H), 6.87 (m, 1H), 6.21 (d, *J* = 15.6 Hz, 1H), 5.77 (d, *J* = 10.5 Hz, 1H), 4.91 (d, *J* = 31.6 Hz, 1H), 4.19 (m, 5H), 3.72 (d, *J* = 37.0 Hz, 2H), 3.23 (dd, *J* = 31.5, 25.2 Hz, 2H), 2.66 (s, 1H), 1.96 (m, 3H), 1.29 (m, 9H), 0.87 (dd, *J* = 13.0, 6.4 Hz, 6H).

**Example 24: Preparation of 4-((S)-4-acryloyl-2-methylpiperazin-1-yl)-6-fluoro-7-(2-fluoro-6-(2-hydroxyethyl)phenyl)-1-(2-isopropyl-4-methylpyridin-3-yl)pyrido[2,3-d]pyrimidin-2(1H)-one (compound 52) (prepared according to Scheme 5)**

Step 24a: Preparation of 2-(2-fluoro-6-(2-(methoxymethoxy)ethyl)phenyl)-4,4,5,5-tetramethyl-1,3,2-dioxaborolane (compound 0503-52): A mixture of 2-(2-bromo-3-fluorophenyl)ethan-1-ol (450 mg, 2.0 mmol, 1.0 eq.), MOMBr (325 mg, 2.6 mmol, 1.3 eq.) and DIPEA (516 mg, 4.0 mmol, 2.0 eq.) in dichloromethane (8 mL) was stirred at room temperature overnight. The mixture was washed with brine and concentrated in vacuo. The residue was purified by column chromatography on silica gel (eluent: petroleum ether/ethyl acetate=50/1 to 25/1) to give 2-bromo-1-fluoro-3-(2-(methoxymethoxy)ethyl)benzene (360 mg, yield: 66.7%) as a colorless oil. Under the protection of nitrogen, a mixture of 2-bromo-1-fluoro-3-(2-(methoxymethoxy)ethyl)benzene (200 mg, 0.76 mmol, 1.0 eq.) obtained above, bis(pinacolato)diboron (290 mg, 1.14 mmol, 1.5 eq.), potassium acetate(223 mg, 2.28 mmol, 3.0 eq.) and tetrakis(triphenylphosphine)palladium (88 mg, 0.076 mmol, 0.1 eq.) in toluene (10 mL) was refluxed overnight. After cooling to room temperature, the reaction solution was filtered, and the filtrate was concentrated in vacuo to give the crude product 2-(2-fluoro-6-(2-(methoxymethoxy)ethyl)phenyl)-4,4,5,5-tetramethyl-1,3,2-dioxaborolane which was used directly in the next step without further purification.

Step 24b: Preparation of tert-butyl (3S)-4-(6-fluoro-7-(2-fluoro-6-(2-(methoxymethoxy)ethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0504-52) : The synthetic method was similar to Step 18b of Example 18, except that compound 0503-44 was replaced by 2-(2-fluoro-6-(2-(methoxymethoxy)ethyl)phenyl)-4,4,5,5-tetramethyl-1,3,2-dioxaborolane (0503-52) (93 mg, 0.30 mmol, 2.0 eq.), tert-butyl (3S)-4-(6-fluoro-7-(2-fluoro-6-(2-(methoxymethoxy)ethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (75 mg, yield: 73.5%) was obtained as a yellow solid. MS ($ES^+$): m/z=679 $(M+H)^+$.

Step 24c: Preparation of 4-((S)-4-acryloyl-2-methylpiperazin-1-yl)-6-fluoro-7-(2-fluoro-6-(2-hydroxyethyl)phenyl)-1-(2-isopropyl-4-methylpyridin-3-yl)pyrido[2,3-d]pyrimidin-2(1H)-one (compound 52): To a solution of tert-butyl (3S)-4-(6-fluoro-7-(2-fluoro-6-(2-(methoxymethoxy)ethyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (0504-52) (75 mg, 0.11 mmol, 1.0 eq.) in dichloromethane (6 mL) was added TFA (3 mL), and the mixture was stirred at room temperature overnight. The solvent was removed in vacuo. The residue was dissolved in tetrahydrofuran (6 mL), and DIPEA (50 mg, 0.39 mmol, 3.0 eq.) was then added. A solution of acryloyl chloride (12 mg, 0.13 mmol, 1.2 eq.) in tetrahydrofuran (0.5 mL) was added dropwise to the mixture in an ice-water bath. The mixture was stirred for 30 min and water was added and extracted with dichloromethane. The organic layer was concentrated in vacuo. The residue was purified by prep-TLC (silica gel, eluent: dichloromethane/methanol=12/1) to give 4-((S)-4-acryloyl-2-methylpiperazin-1-yl)-6-fluoro-7-(2-fluoro-6-(2-hydroxyethyl)phenyl)-1-(2-isopropyl-4-methylpyridin-3-yl)pyrido[2,3-d]pyrimidin-2(1H)-one (40 mg, yield: 61.5%) as a yellow solid. MS ($ES^+$): m/z=589 $(M+H)^+$. $^1$H NMR (500 MHz, DMSO) δ 8.36 (t, *J* = 8.2 Hz, 2H), 7.40 (dd, *J* = 13.9, 7.7 Hz, 1H), 7.15 (m, 3H), 6.84 (m, 1H), 6.20 (d, *J* = 17.9 Hz, 1H), 5.75 (m, 1H), 4.93 (t, *J* = 32.9 Hz, 1H), 4.36 (m, 3H), 4.08 (dd, *J* = 58.8, 11.1 Hz, 1H), 3.65 (m, 2H), 3.18 (dd, *J* = 16.8, 9.6 Hz, 2H), 2.75 (m, 1H), 2.54 (m, 1H), 2.37 (ddd, *J* = 48.3, 18.3, 7.2 Hz, 1H), 1.89 (dd, *J* = 68.8, 5.8 Hz, 3H), 1.32 (m, 3H), 1.06 (dd, *J* = 24.1, 5.7 Hz, 3H), 0.92 (m, 3H).

**Example 25: Preparation of 4-((S)-4-acryloyl-2-methylpiperazin-1-yl)-6-fluoro-7-(2-fluoro-6-((methylthio)methyl)phenyl)-1-(2-isopropyl-4-methylpyridin-3-yl)pyrido[2,3-d]pyrimidin-2(1H)-one (compound 58) (prepared according to Scheme 6)**

Step 25a: Preparation of (2-fluoro-6-((methylthio)methyl)phenyl)boronic acid (compound 0602-58): A mixture of 2-bromo-1-(bromomethyl)-3-fluorobenzene(1.0 g, 3.73 mmol, 1.0 eq.) and $NaSCH_3$ (783 mg, 11.2 mmol, 3.0 eq.) in acetonitrile (20 mL) was stirred at room temperature for 3 h. Water and ethyl acetate were added to extract, and the organic layer was washed with brine and concentrated in vacuo. The residue was purified by column chromatography on silica gel (eluent: petroleum ether/ethyl acetate=1000/1 to 500/1) to give (2-bromo-3-fluorobenzyl)(methyl)sulfane as a colourless oil (600 mg, yield: 68.4%). Under the protection of nitrogen, to a solution of (2-bromo-3-fluorobenzyl)(methyl)sulfane (300 mg, 1.27 mmol, 1.0 eq.) obtained above in tetrahydrofuran (15 mL) was added n-butyllithium (98 mg, 1.53 mmol, 1.2 eq.) dropwise at -72°C. After addition, the mixture was stirred at -72°C for 1 h and then trimethyl borate (172 mg, 1.65 mmol, 1.3 eq.) in tetrahydrofuran (1 mL) was added dropwise. After addition, the mixture was stirred at -72°C for 30 minutes. The reaction mixture was quenched with aqueous ammonium chloride solution and stirred for 10 min. Water and ethyl acetate was added to extract, and the organic layer was washed with brine and concentrated in vacuo to give (2-fluoro-6-((methylthio)methyl)phenyl)boronic acid (120 mg, yield: 47.0%) as a white solid.

Step 25b: Preparation of tert-butyl (3S)-4-(6-fluoro-7-(2-fluoro-6-((methylthio)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (compound 0604-58): Under the protection of nitrogen, a mixture of tert-butyl(S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (98 mg, 0.184 mmol, 1.00 eq.), (2-fluoro-6-((methylthio)methyl)phenyl)boronic acid (0602-58) (120 mg, 0.6 mmol, 3.20 eq.), sodium carbonate (80 mg, 0.754 mmol, 3.00 eq.) and tetrakis(triphenylphosphine)palladium (10.6 mg, 0.0092 mmol, 0.05 eq.) in acetonitrile (20 mL) and water (2 mL) was stirred at 80°C overnight. After cooling to room temperature, water and ethyl acetate were added to extract and the organic layers were washed with brine. The solvent was removed in vacuo and the residue was purified by prep-TLC (silica gel, eluent: petroleum ether/ethyl acetate=1/2)to give tert-butyl (3S)-4-(6-fluoro-7-(2-fluoro-6-((methylthio)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate as a light-yellow solid (43 mg, yield: 35.8%). MS ($ES^+$): m/z=651 $(M+H)^+$

Step 25c: Preparation of 6-fluoro-7-(2-fluoro-6-((methylthio)methyl)phenyl)-1-(2-isopropyl-4-methylpyridin-3-yl)-4-((S)-2-methylpiperazin-1-yl)pyrido[2,3-d]pyrimidin-2(1H)-one (compound 0605-58): To a solution of tert-butyl (3S)-4-(6-fluoro-7-(2-fluoro-6-((methylthio)methyl)phenyl)-1-(2-isopropyl-4-methylpyridin-3-yl)-2-oxo-1,2-dihydropyrido[2,3-d]pyrimidin-4-yl)-3-methylpiperazine-1-carboxylate (0604-58) (43 mg, 0.066 mmol, 1.00 eq.) in dichloromethane (5 mL) was added TFA (0.5mL), and the reaction was stirred at room temperature for 30 min. The mixture was concentrated in vacuo to give 6-fluoro-7-(2-fluoro-6-((methylthio)methyl)phenyl)-1-(2-isopropyl-4-methylpyridin-3-yl)-4-((S)-2-methylpiperazin-1-yl)pyrido[2,3-d]pyrimidin-2(1H)-one (29 mg, crude) as a yellow oil, and which was used directly in the next step without further purification. MS ($ES^+$): m/z=551 $(M+H)^+$

Step 25d: Preparation of 4-((S)-4-acryloyl-2-methylpiperazin-1-yl)-6-fluoro-7-(2-fluoro-6-((methylthio)methyl)phenyl)-1-(2-isopropyl-4-methylpyridin-3-yl)pyrido[2,3-d]pyrimidin-2(1H)-one (compound 58): To a mixture of 6-fluoro-7-(2-fluoro-6-((methylthio)methyl)phenyl)-1-(2-isopropyl-4-methylpyridin-3-yl)-4-((S)-2-methylpiperazin-1-yl)pyrido[2,3-d]pyrimidin-2(1H)-one(0605-58) (29 mg, crude)and DIPEA(0.5mL) in acetonitrile(5mL) was added dropwise acryloyl chloride (7.1 mg, 0.079 mmol, 1.50 eq.). The reaction was stirred at room temperature for 30 min. Water and ethyl acetate were added to extract, and the organic layer was washed with brine and concentrated in vacuo. The residue was purified by prep-TLC (silica gel, eluent: ethyl acetate/methanol=20/1) to give 4-((S)-4-acryloyl-2-methylpiperazin-1-yl)-6-fluoro-7-(2-fluoro-6-((methylthio)methyl)phenyl)-1-(2-isopropyl-4-methylpyridin-3-yl)pyrido[2,3-d]pyrimidin-2(1H)-one as a yellow solid (21 mg, yield: 65.6%). MS ($ES^+$): m/z=605 $(M+H)^+$; m. p: 142-151°C; $^1H$ NMR (500 MHz, DMSO) $\delta$ 8.35 (d, *J* = 37.0 Hz, 2H), 7.45 (d, *J* = 6.8 Hz, 1H), 7.22 (t, *J* = 8.9 Hz, 3H), 6.84 (s, 1H), 6.20 (d, *J* = 16.9 Hz, 1H), 5.76 (d, *J* = 10.6 Hz, 1H), 4.93 (d, *J* = 60.0 Hz, 1H), 4.33 (m, 2H), 4.09 (d, *J* = 66.4 Hz, 1H), 3.73 (d, *J* = 59.1 Hz, 2H), 3.49 (d, *J* = 12.8 Hz, 2H), 3.11 (s, 1H), 2.77 (d, *J*= 45.6 Hz, 1H), 1.90 (t, *J*= 32.9 Hz, 3H), 1.68 (s, 3H), 1.33 (m, 3H), 1.05 (s, 3H), 0.84 (d, *J* = 6.2 Hz, 3H).

**Example 26: Preparation of ethyl 2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorobenzoate (compound 63) (prepared according to Scheme 7)**

Step 26a: Preparation of ethyl 3-fluoro-2-(4,4,5,5-tetramethyl-1,3,2-dioxaborolan-2-yl)benzoate (compound 0703-63): A mixture of 2-bromo-3-fluorobenzoic acid (1.0 g, 4.58 mmol, 1.0 eq.) and thionyl chloride (546 mg, 4.58 mmol, 1.0 eq.) in EtOH (20 mL) was stirred at 85°C for 3 h. Water and ethyl acetate were added to extract, and the organic layer was washed with brine and concentrated in vacuo. The residue was purified by column chromatography on silica gel (eluent: petroleum ether/ethyl acetate=500/1 to 100/1) to give ethyl 2-bromo-3-fluorobenzoate (1.05 g, yield: 93.7%)

as a colourless oil. Under the protection of nitrogen, a mixture of ethyl 2-bromo-3-fluorobenzoate (600 mg, 2.44 mmol, 1.00 eq.) obtained above, bis(pinacolato)diboron (1.12 g, 4.39 mmol, 1.80 eq.), potassium acetate (717 mg, 7.32 mmol, 3.00 eq.) and tetrakis(triphenylphosphine)palladium (141 mg, 0.122 mmol, 0.05 eq.) in toluene (30 mL) was stirred at 115°C overnight. After cooling to room temperature, the mixture was filtered and the filtrate was concentrated in vacuo to give ethyl 3-fluoro-2-(4,4,5,5-tetramethyl-1,3,2-dioxaborolan-2-yl)benzoate (500 mg, crude) as a brown oil.

Step 26b: Preparation of tert-butyl (3S)-4-(7-(2-(ethoxycarbonyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0704-63): Under the protection of nitrogen, a mixture of tert-butyl(S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (80 mg, 0.151 mmol, 1.00 eq.), ethyl 3-fluoro-2-(4,4,5,5-tetramethyl-1,3,2-dioxaborolan-2-yl)benzoate (0703-63) (177 mg, 0.604 mmol, 4.00 eq.), sodium carbonate (48 mg, 0.453 mmol, 3.00 eq.) and tetrakis(triphenylphosphine)palladium (9 mg, 0.0075 mmol, 0.05 eq.) in acetonitrile (15 mL) and water(1.5mL) was stirred at 80°C overnight. After cooling to room temperature, water and ethyl acetate were added to extract and the organic layer was washed with brine. The solvent was removed in vacuo and the residue was purified by prep-TLC (silica gel, eluent: dichloromethane/methanol=18/1)to give tert-butyl (3S)-4-(7-(2-(ethoxycarbonyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (74 mg , yield: 74.1%) as a light-yellow solid. MS ($ES^+$): m/z=663 $(M+H)^+$.

Step 26c: Preparation of ethyl 3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)benzoate (compound 0705-63): To a solution of the tert-butyl (3S)-4-(7-(2-(ethoxycarbonyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0704-63) (74 mg, 0.112 mmol, 1.00 eq.) in dichloromethane (5 mL) was added TFA (0.5mL), and the reaction was stirred at room temperature for 30 min. The mixture was concentrated in vacuo to give ethyl 3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)benzoate) (35 mg, crude) as a yellow oil which was used directly in the next step without further purification. MS ($ES^+$): m/z=563 $(M+H)^+$.

Step 26d: Preparation of ethyl 2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorobenzoate (compound 63): To a mixture of ethyl 3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)benzoate (0705-63) (35 mg, crude)and DIPEA(0.5mL) in acetonitrile(5mL) was added dropwise acryloyl chloride(8.4 mg, 0.093 mmol, 1.50 eq.). The reaction was stirred at room temperature for 30 min. Water and ethyl acetate were added to extract, and the organic layer was washed with brine and concentrated in vacuo. The residue was purified by prep-TLC (silica gel, eluent: dichloromethane/methanol=16/1) to give ethyl 2-(4-((S)-4-acryloyl-2-methylpiperazin-1-yl)-6-fluoro-1-(2-isopropyl-4-methylpyridin-3 -yl)-2-oxo-1,2-dihydropyrido[2,3-d]pyrimidin-7-yl)-3-fluorobenzoate (13 mg, yield: 34.2%) as a yellow solid. MS ($ES^+$): m/z=617 $(M+H)^+$ , m. p: 121-133°C; $^1H$ NMR (500 MHz, DMSO) $\delta$ 8.36 (d, *J* = 4.6 Hz, 2H), 7.79 (dd, *J* = 31.7, 7.5 Hz, 1H), 7.63 (m, 2H), 7.16 (s, 1H), 6.86 (d, *J* = 10.3 Hz, 1H), 6.21 (d, *J* = 16.0 Hz, 1H), 5.76 (d, *J* = 10.5 Hz, 1H), 4.91 (d, *J* = 28.5 Hz, 1H), 4.18 (m, 5H), 3.69 (s, 2H), 3.16 (s, 1H), 2.69 (d, *J* = 6.1 Hz, 1H), 1.88 (s, 3H), 1.32 (dd, *J* = 15.1, 8.1 Hz, 3H), 1.02 (m, 6H), 0.86 (d, *J* = 6.5 Hz, 3H).

**Example 27: Preparation of 2-(2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorophenyl)acetamide (compound 65) (prepared according to Scheme 4)**

Step 27a: Preparation of 2-(3-fluoro-2-(4,4,5,5-tetramethyl-1,3,2-dioxaborolan-2-yl)phenyl)acetonitrile (compound 0403-65): Under the protection of nitrogen, 2-(2-bromo-3-fluorophenyl)acetonitrile (432 mg,2 mmol, 1 eq.), Bis(pinacolato)diboron (762 mg,3 mmol, 1.5 eq.), tetrakis(triphenylphosphine)palladium (115 mg, 0.1 mmol, 0.05 eq.) and potassium acetate (588 mg,6 mmol,3 eq.) were added into toluene (10 mL). The mixture was stirred at 110°C for 16h and was cooled to room temperature and filtered. The filtrate was concentrated under vacuum to give 2-(3-fluoro-2-(4,4,5,5-tetramethyl-1,3,2-dioxaborolan-2-yl)phenyl)acetonitrile (524 mg, crude) as a black oil. MS (ESI): *m/z* 262$(M+H)^+$.

Step 27b: Preparation of tert-butyl (3S)-4-(7-(2-(2-amino-2-oxoethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0404-65): Under the protection of nitrogen, tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (150 mg, 0.283 mmol, 1 eq.), 2-(3-fluoro-2-(4,4,5,5-tetramethyl-1,3,2-dioxaborolan-2-yl)phenyl)acetonitrile (0403-65) (89 mg, 0.34 mmol, 1.2 eq.), tetrakis(triphenylphosphine)palladium (33 mg, 0.0283 mmol, 0.1 eq.) and sodium carbonate (90 mg, 0.849 mmol,3 eq.) were added into a mixture solution of acetonitrile (10 mL) and water(1 mL). The mixture was stirred at 82°C for 16 h and. cooled to room temperature. The mixture was extracted with ethyl acetate and water. The organic phase was washed with brine, dried over anhydrous sodium sulfate, and concentrated under vacuum. The residue was purified by

perp-TLC (eluent: dichloromethane/methanol=15/1) to give tert-butyl (3S)-4-(7-(2-(2-amino-2-oxoethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (56 mg, 30.77%) as a yellow solid. MS (ESI): *m/z* 648(M+H)$^+$.

Step 27c: Preparation of 2-(2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorophenyl)acetamide (compound 65): tert-butyl (3S)-4-(7-(2-(2-amino-2-oxoethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (56 mg, 0.086 mmol, 1.0 eq.) was dissolved in 4 mL dichloromethane, and 1 mL TFA was then added. The mixture was stirred at room temperature for 2 h and was then concentrated. The residue was dissolved in tetrahydrofuran (5 mL). DIPEA (33 mg, 0.258 mmol, 3 eq.) was then added into the mixture followed by the addition of acryloyl chloride (7.8 mg, 0.086 mmol, 1.0 eq.). The mixture was stirred at room temperature for 15 min and was then quenched with ammonium hydroxide. The mixture was extracted with dichloromethane. The organic phase was concentrated and purified by perp-TLC (eluent: dichloromethane/methanol=15/1) to give 2-(2-(4-((S)-4-acryloyl-2-methylpiperazin-1-yl)-6-fluoro-1-(2-isopropyl-4-methylpyridin-3 -yl)-2-oxo-1,2-dihydropyrido[2,3-d]pyrimidin-7-yl)-3-fluorophenyl)acetamide (50 mg, 96.15%) as a yellow solid. MS (ESI): *m/z* 602(M+H)$^+$. Melting point: 164~165°C; $^1$H NMR (500 MHz, DMSO) $\delta$ 8.34 (m, 2H), 7.42 (m, 1H), 7.15 (dd, *J*= 34.2, 24.9 Hz, 4H), 6.84 (s, 1H), 6.66 (s, 1H), 6.20 (d, *J*= 17.1 Hz, 1H), 5.76 (d, *J* = 10.5 Hz, 1H), 4.92 (d, *J*= 43.6 Hz, 1H), 4.32 (dd, *J* = 38.8, 25.4 Hz, 2H), 4.09 (d, *J* = 65.6 Hz, 1H), 3.69 (dd, *J* = 42.0, 28.2 Hz, 2H), 3.14 (dd, *J* = 36.4, 15.0 Hz, 3H), 2.72 (m, 1H), 1.90 (t, *J*= 32.8 Hz, 3H), 1.33 (m, 3H), 1.06 (dd, *J*= 20.2, 6.0 Hz, 3H), 0.83 (d, *J* = 6.3 Hz, 3H).

**Example 28: Preparation of N-(2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorobenzyl)acetamide (compound 67) (prepared according to Scheme 8)**

Step 28a: Preparation of N-(3-fluoro-2-(4,4,5,5-tetramethyl-1,3,2-dioxaborolan-2-yl)benzyl)acetamide (compound 0803-67): To a mixture of 2-bromo-1-(bromomethyl)-3-fluorobenzene (1.00 g, 3.73 mmol, 1.00 eq.) in dichloromethane (20 mL) was added hexamethylenetetramine (783 mg, 5.59 mmol, 1.50 eq.), and the reaction was stirred at room temperature overnight. methanol/hydrogen chloride solution was then added dropwise. After 30 min, water and ethyl acetate were added to extract, and the organic layer was washed with brine and concentrated in vacuo to give (2-bromo-3-fluorophenyl) methanamine as a white solid (500 mg, yield: 65.5%). MS (ES$^+$): m/z=204 (M+H)$^+$. A mixture of (2-bromo-3-fluorophenyl) methanamine (300 mg, 1.47 mmol, 1.00 eq.) obtained above, acetic acid(135 mg, 2.20 mmol, 1.50 eq.), TEA (299 mg, 2.94 mmol, 2.00 eq.) and HATU (612 mg, 1.62 mmol, 1.10 eq.) in DMF (15 mL) was stirred at room temperature for 2 h. Water and ethyl acetate were added to extract, and the organic layer was washed with brine and concentrated in vacuo to give N-(2-bromo-3-fluorobenzyl)acetamide as a colourless oil. (160 mg, yield: 44.4%). MS (ES$^+$): m/z=246 (M+H)$^+$. Under the protection of nitrogen, a mixture of N-(2-bromo-3-fluorobenzyl)acetamide (160 mg, 0.653 mmol, 1.00 eq.) obtained above, bis(pinacolato)diboron (298 mg, 1.17 mmol, 1.80 eq.), potassium acetate (192 mg, 1.96 mmol, 3.00 eq.) and tetrakis(triphenylphosphine)palladium (40 mg, 0.03 mmol, 0.05 eq.) in toluene (15 mL) was stirred at 115°C overnight. After cooling to room temperature, the mixture was filtered, and the filtrate was concentrated in vacuo to give N-(3-fluoro-2-(4,4,5,5-tetramethyl-1,3,2-dioxaborolan-2-yl)benzyl)acetamide (140 mg crude as a brown oil).

Step 28b: Preparation of tert-butyl (3S)-4-(7-(2-(acetamidomethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0804-67): Under the protection of nitrogen, a mixture of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (125 mg, 0.239 mmol, 1.00 eq.), N-(3-fluoro-2-(4,4,5,5-tetramethyl-1,3,2-dioxaborolan-2-yl)benzyl)acetamide (0803-67) (140 mg, 0.478 mmol, 2.00 eq.), sodium carbonate (76 mg, 0.717 mmol, 3.00 eq.) and tetrakis(triphenylphosphine)palladium (14 mg, 0.012 mmol, 0.05 eq.) in acetonitrile (15 mL) and water(2mL) was stirred at 80°C overnight. After cooling to room temperature, water and ethyl acetate were added to extract and the organic layer was washed with brine. The solvent was removed in vacuo and the residue was purified by prep-TLC (silica gel, eluent: dichloromethane/methanol=10/1) to give tert-butyl (3S)-4-(7-(2-(acetamidomethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (30 mg , yield: 18.9%) as a light-yellow solid MS (ES$^+$): m/z=662 (M+H)$^+$.

Step 28c: Preparation of N-(3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)benzyl)acetamide (compound 0805-67): To a solution of the tert-butyl (3S)-4-(7-(2-(acetamidomethyl)-6-fluorophenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0804-67) (30 mg, 0.045 mmol, 1.00 eq.) in dichloromethane (5 mL) was added TFA (0.5mL), and the reaction was stirred at room temperature for 30 min. The mixture was concentrated in vacuo to give N-(3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)benzyl)acetamide (27 mg, crude) as a yellow oil. MS (ES$^+$): m/z=562 (M+H)$^+$.

Step 28d: Preparation of N-(2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorobenzyl)acetamide (compound 67): To a mixture of the N-(3-fluoro-2-(6-fluoro-1-(2-isopropyl-4-methylpyridin-3-yl)-4-((S)-2-methylpiperazin-1-yl)-2-oxo-1,2-dihydropyrido[2,3-d]pyrimidin-7-yl)benzyl)acetamide(0805-67) (27 mg, crude)and DIPEA(0.5mL) in acetonitrile(5mL) was added dropwise acryloyl chloride(6.5 mg, 0.072 mmol, 1.50 eq.). The mixture was stirred at room temperature for 30 min. Water and ethyl acetate were added to extract, and the organic layer was washed with brine and concentrated in vacuo. The residue was purified by prep-TLC (silica gel, eluent: dichloromethane/methanol=10/1) to give N-(2-(4-((S)-4-acryloyl-2-methylpiperazin-1-yl)-6-fluoro-1-(2-isopropyl-4-methylpyridin-3-yl)-2-oxo-1,2-dihydropyrido[2,3-d]pyrimidin-7-yl)-3-fluorobenzyl)acetamide (17 mg, yield: 58.6%) as a yellow solid. MS ($ES^+$): m/z=616 $(M+H)^+$. m. p: 121-133°C; $^1H$ NMR (500 MHz, DMSO) $\delta$ 8.23 (m, 3H), 7.44 (m, 1H), 7.21 (ddd, *J* = 19.3, 14.8, 5.3 Hz, 2H), 6.92 (m, 2H), 6.20 (d, *J* = 15.3 Hz, 1H), 5.76 (dd, *J*= 10.4, 2.3 Hz, 1H), 5.00 (d, *J*= 90.6 Hz, 1H), 4.26 (m, 3H), 3.98 (dd, J= 23.0, 7.4 Hz, 2H), 3.69 (dd, *J*= 59.3, 23.6 Hz, 2H), 3.08 (m, 1H), 2.59 (d, *J*= 48.9 Hz, 1H), 2.00 (dd, *J* = 13.5, 6.0 Hz, 3H), 1.77 (d, *J* = 31.6 Hz, 3H), 1.33 (dd, *J* = 16.8, 10.9 Hz, 3H), 1.02 (dd, *J* = 36.9, 5.7 Hz, 3H), 0.77 (dt, *J* = 11.7, 6.5 Hz, 3H).

**Example 29: Preparation of 4-((S)-4-acryloyl-2-methylpiperazin-1-yl)-6-fluoro-7-(6-fluorobenzofuran-7-yl)-1-(2-isopropyl-4-methylpyridin-3-yl)pyrido[2,3-d]pyrimidin-2(1H)-one (compound 70) (prepared according to Scheme 9)**

Step 29a: Preparation of (6-fluorobenzofuran-7-yl)boronic acid (compound 0902-70): A mixture of 2-bromo-3-fluorophenol (1 g, 5.2 mmol, 1.0 eq.), 2-bromo-1, 1-dimethoxyethane (1.3 g, 7.8 mmol, 1.5 eq.) and potassium carbonate (1 g, 7.8 mmol, 2.0 eq.) in DMF (20 mL) was stirred at 135°C for 3 h. After cooling to room temperature, the mixture was poured into water, and ethyl acetate was added to extracted. The organic layer was washed with brine, dried over anhydrous sodium sulfate and concentrated in vacuo to give 2-bromo-1-(2,2-dimethoxyethoxy)-3-fluorobenzene as a yellow oil (1.4 g, yield: 95.9%). A mixture of 2-bromo-1-(2,2-dimethoxyethoxy)-3-fluorobenzene (1.4 g, 5.0 mmol, 1.0 eq.) obtained above and PPA (1.7 g, 5.0 mmol, 1.0 eq.) in toluene (30 mL) was refluxed overnight. After cooling to room temperature, the mixture was washed with water. The organic layer was concentrated in vacuo. The residue was purified by silica gel chromatogaphy(eluent: petroleum ether) to give 7-bromo-6-fluorobenzofuran as a colorless oil (1.0 g, yield: 92.6%). Under the protection of nitrogen, to a solution of 7-bromo-6-fluorobenzofuran (215 mg, 1.0 mmol, 1.0 eq.) obtained above in toluene (6 mL) was added 1.6 M n-BuLi (0.9 mL, 1.4 mmol, 1.4 eq.) dropwise at -70°C. After addition, the mixture was stirred for 1 h. A solution of trimethyl borate (156 mg, 1.5 mmol, 1.5 eq.) in toluene (0.5 mL) was added dropwise to the mixture, and the mixture was stirred for another 30 min. The reaction solution was quenched with 2 M sodium hydroxide and the mixture was separated. 2M HCl was added to the aqueous layer to adjust PH<2, then ethyl acetate was added to extract. The organic layer was dried over anhydrous sodium sulfate and concentrated in vacuo to give (6-fluorobenzofuran-7-yl)boronic acid (170 mg, yield: 94.4%) as a yellow solid..

Step 29b: Preparation of tert-butyl (3S)-4-(6-fluoro-7-(6-fluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-70): Under the protection of nitrogen, a mixture of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (80 mg, 0.15 mmol, 1.0 eq.), (6-fluorobenzofuran-7-yl)boronic acid (0902-70) (82 mg, 0.45 mmol, 3.0 eq.), sodium carbonate(48 mg, 0.45 mmol, 3.0 eq.) and tetrakis(triphenylphosphine)palladium (17 mg, 0.015 mmol, 0.1 eq.) in acetonitrile/water=10/1 (11 mL) was stirred at 82°C overnight. After cooling to room temperature, the mixture was poured into water, and ethyl acetate was added to extracted. The organic layer was concentrated in vacuo. The residue was purified by prep-TLC (silica gel, eluent: ethyl acetate/methanol=100/1) to give tert-butyl (3S)-4-(6-fluoro-7-(6-fluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate as a yellow solid (75 mg, yield: 78.9%). MS ($ES^+$): m/z=631 $(M+H)^+$.

Step 29c: Preparation of 4-((S)-4-acryloyl-2-methylpiperazin-1-yl)-6-fluoro-7-(6-fluorobenzofuran-7-yl)-1-(2-isopropyl-4-methylpyridin-3-yl)pyrido[2,3-d]pyrimidin-2(1H)-one (compound 70): To a solution of tert-butyl (3S)-4-(6-fluoro-7-(6-fluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (0904-70) (75 mg, 0.12 mmol, 1.0 eq.) in dichloromethane (6 mL) was added TFA (2 mL), and the mixture was stirred at room temperature overnight. The solvent was removed in vacuo. The residue was dissolved in tetrahydrofuran (6 mL), DIPEA (46 mg, 0.36 mmol, 3.0 eq.) was then added. A solution of acryloyl chloride (16 mg, 0.18 mmol, 1.5 eq.) in tetrahydrofuran (0.5 mL) was added dropwise to the mixture in an ice-water bath. The mixture was stirred for 30 min and then water was added and extracted with. The organic layer was concentrated in vacuo. The residue was purified by prep-TLC (silica gel, eluent: dichloromethane/methanol=12/1) to give 4-((S)-4-acryloyl-2-methylpiperazin-1-yl)-6-fluoro-7-(6-fluorobenzofuran-7-yl)-1-(2-isopropyl-4-methylpyridin-3-yl)pyrido[2,3-d]pyrimidin-2(1H)-one as a yellow solid (36 mg, yield: 51.4%). MS ($ES^+$): m/z=585 $(M+H)^+$. $^1H$ NMR (500 MHz, DMSO) $\delta$ 8.40 (m, 2H), 7.94 (d, *J*= 1.7 Hz, 1H), 7.79 (dd, *J* = 8.5, 5.2 Hz, 1H), 7.26 (m, 1H), 7.18 (d, *J*= 4.9 Hz, 1H), 7.01 (m, 1H), 6.86 (d, *J*= 11.7 Hz, 1H), 6.21 (d, *J*= 15.8 Hz, 1H), 5.76 (m, 1H), 4.96 (d, *J* = 40.4 Hz, 1H), 4.35 (dd, *J* = 28.6, 13.3 Hz, 2H),

4.11 (m, 1H), 3.65 (m, 2H), 3.16 (m, 1H), 2.77 (m, 1H), 1.94 (d, *J*= 2.5 Hz, 3H), 1.35 (dd, *J*= 21.1, 6.6 Hz, 3H), 1.08 (d, *J* = 6.7 Hz, 3H), 0.94 (d, *J* = 6.7 Hz, 3H).

**Example 30: Preparation of 4-((S)-4-acryloyl-2-methylpiperazin-1-yl)-7-(2,3-difluoro-6-(methylthio)phenyl)-6-fluoro-1-(2-isopropyl-4-methylpyridin-3-yl)pyrido[2,3-d]pyrimidin-2(1H)-one (compound 22) (prepared according to Scheme 2)**

Step 30a: Preparation of (2-bromo-3,4-difluorophenyl) (methyl)sulfane (compound 0201-22): tert-butyl nitrite (3.95 g, 38.4 mmol, 8 eq.) was slowly added dropwise to a solution of 2-bromo-3,4-difluoroaniline (1 g, 4.8 mmol, 1 eq.) in dimethyl disulfide (542 mg, 5.77 mmol, 1.2 eq.). The mixture was heated to 95°C and stirred at 95°C for 3 h. The mixture was cooled to room temperature. The mixture was extracted with petroleum ether. The organic phase was washed with brine and dried over anhydrous sodium sulfate. The organic phase was concentrated and purified by column chromatography on silica gel (eluent: petroleum ether) to give (2-bromo-3,4-difluorophenyl) (methyl)sulfane (767 mg, crude) as a yellow oil. MS (ESI): m/z 239$(M+H)^+$.

Step 30b: Preparation of (2,3-difluoro-6-(methylthio)phenyl)boronic acid (compound 0202-22): n-butyllithium (1.5 mL, 1.6 mol/L in n-hexane, 2.4 mmol, 1.2 eq.) was added dropwise to a solution of (2-bromo-3,4-difluorophenyl) (methyl)sulfane (0201-22) (476 mg, 2 mmol, 1 eq.) in toluene (5 mL). The mixture was stirred at -70°C for 30 min. Trimethyl borate (270 mg, 2.6 mmol, 1.3 eq.) was added dropwise to the mixture. The mixture was stirred at - 70°C for an additional 30 min. Then, the reaction was quenched with 2 mol/L aqueous sodium hydroxide solution. The mixture was extracted with petroleum ether. The pH of the aqueous phase was adjusted to 2 with 2 mol/L hydrochloric acid. The mixture was extracted with ethyl acetate and water. The organic phase was dried over anhydrous sodium sulfate and concentrated to give (2,3-difluoro-6-(methylthio)phenyl)boronic acid (254 mg, crude) as a yellow solid. MS (ESI): m/z 205$(M+H)^+$.

Step 30c: Preparation of tert-butyl (3S)-4-(7-(2,3-difluoro-6-(methylthio)phenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0204-22): tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (100 mg, 0.188 mmol, 1 eq.), (2,3-difluoro-6-(methylthio)phenyl)boronic acid (0202-22) (254 mg, 1.257 mmol, 6 eq.), tetrakis(triphenylphosphine)palladium (22 mg, 0.0188 mmol, 0.1 eq.) and sodium carbonate (40 mg, 0.376 mmol, 2 eq.) were added to a mixed solution of acetonitrile (10 mL) and water (1 mL). The mixture was stirred at 82°C for 16 h and was then cooled to room temperature and extracted with ethyl acetate and water. The organic phase was washed with saturated brine, dried over anhydrous sodium sulfate, and concentrated under reduced pressure. The residue was purified by TLC (eluent: dichloromethane/methanol = 20/1) to give tert-butyl (3S)-4-(7-(2,3-difluoro-6-(methylthio)phenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (90 mg, crude) as a yellow solid. MS (ESI): m/z 655$(M+H)^+$.

Step 30d: Preparation of 4-((S)-4-acryloyl-2-methylpiperazin-1-yl)-7-(2,3-difluoro-6-(methylthio)phenyl)-6-fluoro-1-(2-isopropyl-4-methylpyridin-3-yl)pyrido[2,3-d]pyrimidin-2(1H)-one (compound 22): tert-butyl (3S)-4-(7-(2,3-difluoro-6-(methylthio)phenyl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0204-22) (90 mg, 0.137 mmol , 1.0 eq.) was dissolved in dichloromethane (4 mL), and TFA (1 mL) was then added. The mixture was stirred at room temperature for 2 h and concentrated. The residue was dissolved in tetrahydrofuran (5 mL). DIPEA (53 mg, 0.413 mmol, 3.0 eq.) was then added to the mixture followed by the addition of acryloyl chloride (12.4 mg, 0.137 mmol, 1.0 eq.). The mixture was stirred at room temperature for 15 min and quenched with aqueous ammonia. The mixture was extracted with dichloromethane and the organic phase was concentrated under reduced pressure. The residue was purified by prep-TLC (silica gel, eluent: dichloromethane/methanol=20/1) to give 4-((S)-4-acryloyl-2-methylpiperazin-1-yl)-7-(2,3-difluoro-6-(methylthio)phenyl)-6-fluoro-1-(2-isopropyl-4-methylpyridin-3-yl)pyrido[2,3-d]pyrimidin-2(1H)-one (50 mg, yield: 59.76%) as a yellow solid. MS (ESI): m/z 609$(M+H)^+$. $^1$HNMR (500 MHz, DMSO) $\delta$ 8.40 (s, 2H), 7.58 (s, 1H), 7.23 (d, *J* = 35.5 Hz, 2H), 6.87 (s, 1H), 6.22 (s, 1H), 5.77 (s, 1H), 4.95 (d, *J* = 29.4 Hz, 1H), 4.32 (s, 2H), 4.09 (d, *J* = 59.2 Hz, 1H), 3.85-3.50 (m, 2H), 3.21-3.05 (m, 1H), 2.73 (s, 1H), 2.33 (s, 3H), 1.93 (s, 3H), 1.35 (s, 3H), 1.08 (s, 3H), 0.92 (s, 3H).

**Example 31: Preparation of 4-((S)-4-Acryloyl-2-methylpiperazin-1-yl)-6-fluoro-7-(6-fluorobenzo[b]thiophen-7-yl)-1-(2-isopropyl-4-methylpyridin-3-yl)pyrido[2,3-d]pyrimidin-2(1H)-one (compound 71) (prepared according to Scheme 9)**

Step 31a: Preparation of 7-bromo-6-fluorobenzo[b]thiophene (compound 0901-71): Under the protection of nitrogen, 2-bromo-3-fluorobenzenethiol (940 mg, 5.0 mmol, 1.0 eq.), 2-bromo-1, 1-dimethoxyethane (1.1 g, 6.5 mmol, 1.3 eq.) and potassium carbonate (966 mg, 7.0 mmol, 1.4 eq.) were added into DMF (7 mL), and the mixture was stirred at room temperature for 2 h. The mixture was diluted with ethyl acetate, and washed with water and brine. The organic phase was concentrated under vacuum. The residue was purified by column chromatography on silica gel (eluent:

Petroleum ether/ethyl acetate=30/1 to 20/1) to give (2-bromo-3-fluorophenyl) (2,2-dimethoxyethyl)sulfane (841 mg, yield: 57%) as a yellow oil. MS (ES$^+$): *m*/*z*=295 $(M+H)^+$. Under the protection of nitrogen, (2-bromo-3-fluorophenyl) (2,2-dimethoxyethyl)sulfane (841 mg, 2.86 mmol, 1.0 eq.) obtained above, polyphosphoric acid (1.64 g, 4.86 mmol, 1.7 eq.) were added into dry toluene (40 mL), and the mixture was stirred at 120°C overnight. The mixture was diluted with ethyl acetate, and washed with water. The organic phase was concentrated under vacuum. The residue was purified by column chromatography on silica gel (eluent: Petroleum ether) to give 7-bromo-6-fluorobenzo[*b*]thiophene (386mg, yield: 58%) as a yellow oil. MS (ES$^+$): *m*/*z*=231 $(M+H)^+$.

Step 31b: Preparation of tert-butyl (*S*)-4-(6-fluoro-1-(2-isopropyl-4-methylpyridin-3-yl)-2-oxo-7-(trimethylstannyl)-1,2-dihydropyrido[2,3-*d*]pyrimidin-4-yl)-3-methylpiperazine-1-carboxylate (compound 0906-71): Under nitrogen atmosphere, tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (300 mg, 0.566 mmol, 1 eq.), hexamethyldistannane (278 mg, 1.132 mmol, 2 eq.) and tetrakis(triphenylphosphine)palladium (65 mg, 0.05 mmol, 0.1 eq.) were added to toluene (10 mL). The mixture was stirred at 110 °C for 2 h and cooled to room temperature. The mixture was concentrated and purified by column chromatography on silica gel (eluent: petroleum ether/ethyl acetate = 5/1-1/2) to give tert-butyl (*S*)-4-(6-fluoro-1-(2-isopropyl-4-methylpyridin-3-yl)-2-oxo-7-(trimethylstannyl)-1,2-dihydropyrido[2,3-*d*]pyrimidin-4-yl)-3-methylpiperazine-1-carboxylate (321 mg, yield: 85.94%) as a yellow solid. MS (ESI): m/z 661$(M+H)^+$.

Step 31c: Preparation of tert-butyl (3*S*)-4-(6-fluoro-7-(6-fluorobenzo[*b*]thiophen-7-yl)-1-(2-isopropyl-4-methylpyridin-3-yl)-2-oxo-1,2-dihydropyrido[2,3-*d*]pyrimidin-4-yl)-3-methylpiperazine-1-carboxylate (compound 0904-71): Under the protection of nitrogen, tert-butyl (*S*)-4-(6-fluoro-1-(2-isopropyl-4-methylpyridin-3-yl)-2-oxo-7-(trimethylstannyl)-1,2-dihydropyrido[2,3-*d*]pyrimidin-4-yl)-3-methylpiperazine-1-carboxylate (0906-71) (150 mg, 0.23 mmol, 1.0 eq.), 7-bromo-6-fluorobenzo[*b*]thiophene (0901-71) (116 mg, 0.5 mmol, 2.2 eq.), tetrakis(triphenylphosphine)palladium (27 mg, 0.023 mmol, 0.1 eq.) and CuI (4.4 mg, 0.023 mmol, 0.1 eq.) were added into dry toluene (50 mL). The mixture was stirred at 110°C overnight and diluted with ethyl acetate. The organic phase was washed with water and concentrated under vacuum. The residue was purified by column chromatography on silica gel (eluent: ethyl acetate/methanol=70/1 to 50/1) to give tert-butyl (3*S*)-4-(6-fluoro-7-(6-fluorobenzo[*b*]thiophen-7-yl)-1-(2-isopropyl-4-methylpyridin-3-yl)-2-oxo-1,2-dihydropyrido[2,3-*d*]pyrimidin-4-yl)-3-methylpiperazine-1-carboxylate as a yellow solid (57 mg, yield: 38%). MS (ES$^+$): *m*/*z*=647 $(M+H)^+$.

Step 31d: Preparation of 4-((*S*)-4-acryloyl-2-methylpiperazin-1-yl)-6-fluoro-7-(6-fluorobenzo[*b*]thiophen-7-yl)-1-(2-isopropyl-4-methylpyridin-3-yl)pyrido[2,3-*d*]pyrimidin-2(1*H*)-one (compound 71): tert-butyl (3*S*)-4-(6-fluoro-7-(6-fluorobenzo[*b*]thiophen-7-yl)-1-(2-isopropyl-4-methylpyridin-3-yl)-2-oxo-1,2-dihydropyrido[2,3-*d*]pyrimidin-4-yl)-3-methylpiperazine-1-carboxylate (60 mg, 0.093 mmol, 1.0 eq.) was dissolved in 12 mL dichloromethane. TFA (1.5 mL) was then added dropwise, and the mixture was stirred at room temperature for 2 h. The mixture was concentrated under vacuum to dryness. The residue was dissolved in 20 mL tetrahydrofuran, and 2 mL DIPEA was added. The mixture was cool in an ice-water bath and acryloyl chloride (25 mg, 0.28 mmol, 3.0 eq.), pre-dissolved in 0.5 mL tetrahydrofuran, was added dropwise into the mixture above. The mixture was stirred for 30 min and diluted with ethyl acetate. The organic phase was washed with water and concentrated under vacuum. The residue was purified by prep-TLC (silica gel, eluent: ethyl acetate/methanol=20/1) to give 4-((*S*)-4-acryloyl-2-methylpiperazin-1-yl)-6-fluoro-7-(6-fluorobenzo[*b*]thiophen-7-yl)-1-(2-isopropyl-4-methylpyridin-3-yl)pyrido[2,3-*d*]pyrimidin-2(1*H*)-one as a yellow solid (35 mg, yield: 62%). MS (ES$^+$): *m*/*z*=601 $(M+H)^+$. Melting point: 148°C ~150°C. $^1$H NMR (500 MHz, DMSO) $\delta$ 8.39 (t, *J* = 19.2 Hz, 2H), 8.02 (dd, *J* = 8.4, 4.8 Hz, 1H), 7.63 (d, *J* = 5.3 Hz, 1H), 7.41 (dd, *J* = 19.2, 7.5 Hz, 2H), 7.21 (d, *J* = 4.7 Hz, 1H), 6.85 (d, *J* = 11.7 Hz, 1H), 6.20 (d, *J* = 16.3 Hz, 1H), 5.75 (d, *J* = 10.5 Hz, 1H), 4.95 (d, *J* = 48.0 Hz, 1H), 4.35 (m, 2H), 4.10 (m, 1H), 3.73 (d, *J* = 51.9 Hz, 2H), 3.09 (s, 1H), 2.76 (s, 1H), 2.01 (d, *J* = 4.5 Hz, 3H), 1.34 (dd, *J* = 24.5, 6.3 Hz, 3H), 1.05 (d, *J* = 6.5 Hz, 3H), 0.87 (d, *J* = 6.5 Hz, 3H).

**Example 32: Preparation of 4-((*S*)-4-acryloyl-2-methylpiperazin-1-yl)-6-fluoro-7-(5-fluorobenzo[d][1,3]dioxol-4-yl)-1-(2-isopropyl-4-methylpyridin-3-yl)pyrido[2,3-d]pyrimidin-2(1H)-one (compound 69) (prepared according to Scheme 9)**

Step 32a: Preparation of 5-fluoro-4-iodobenzo[d][1,3]dioxole (compound 0901-69): Under the protection of nitrogen, a mixture of 4-fluoro-3-iodobenzene-1,2-diol (500 mg, 2.0 mmol, 1.0 eq.), bromochloromethane (390 mg, 3.0 mmol, 1.5 eq.) and cesium carbonate (1.3 g, 4.0 mmol, 2.0 eq.) in DMF (20 mL) was stirred at 100°C overnight. After cooling to room temperature, the mixture was poured into water and extracted with petroleum ether/ethyl acetate=1/1. The organic layer was washed with brine and concentrated in vcuo, and the residue was purified by column chromatography on silica gel (eluent: petroleum ether/ethyl acetate=100/1 to 50/1) to give 5-fluoro-4-iodobenzo[d][1,3]dioxole (5 24 mg, yield: 80.2%) as a white solid.

Step 32b: Preparation oftert-butyl (3S)-4-(6-fluoro-7-(5-fluorobenzo[d][1,3]dioxol-4-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-69): Under the protection of nitrogen, a mixture of tert-butyl (S)-4-(6-fluoro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (0906-71) (100 mg, 0.15 mmol, 1.0 eq.), 5-fluoro-4-iodobenzo[d][1,3]dioxole (0901-69) (42 mg, 0.15 mmol, 1.0 eq.), CuI(2.9 mg, 0.015 mmol, 0.1 eq.) and tetrakis(triphenylphosphine)palladium (17.5 mg, 0.015 mmol, 0.1 eq.) in toluene (8 mL) was stirred at 110°C overnight. After cooling to room temperature, the solvent was removed in vacuo. The residue was purified by prep-TLC (silica gel, eluent: dichloromethane/methanol=20/1) to give tert-butyl (3S)-4-(6-fluoro-7-(5-fluorobenzo[d][1,3]dioxol-4-yl)-1-(2-isopropyl-4-methylpyridin-3-yl)-2-oxo-1,2-dihydropyrido[2,3-d]pyrimidin-4-yl)-3-methylpiperazine-1-carboxylate as a yellow solid (70 mg, yield: 72.8%). MS ($ES^+$): m/z=635 $(M+H)^+$.

Step 32c; Preparation of 4-((S)-4-acryloyl-2-methylpiperazin-1-yl)-6-fluoro-7-(5-fluorobenzo[d][1,3]dioxol-4-yl)-1-(2-isopropyl-4-methylpyridin-3-yl)pyrido[2,3-d]pyrimidin-2(1H)-one (compound 69): To a solution of tert-butyl (3S)-4-(6-fluoro-7-(5-fluorobenzo[d][1,3]dioxol-4-yl)-1-(2-isopropyl-4-methylpyridin-3-yl)-2-oxo-1,2-dihydropyrido[2,3-d]pyrimidin-4-yl)-3-methylpiperazine-1-carboxylate (0904-69) (70 mg, 0.11 mmol, 1.0 eq.) in dichloromethane (6 mL) was added TFA (3 mL), and the mixture was stirred at room temperature for 1h. The solvent was removed in vacuo. The residue was dissolved in tetrahydrofuran (6 mL), and DIPEA (43 mg, 0.33 mmol, 3.0 eq.) was then added. A solution of acryloyl chloride (15 mg, 0.17 mmol, 1.5 eq.) in tetrahydrofuran (0.5 mL) was added dropwise to the mixture in an ice-water bath. The mixture was stirred for 30 min. Water was added, and then ethyl acetate was added to extract. The organic layer was concentrated in vacuo and the residue was purified by prep-TLC (silica gel, eluent: ethyl acetate/methanol=12/1) to give 4-((S)-4-acryloyl-2-methylpiperazin-1-yl)-6-fluoro-7-(5-fluorobenzo[d][1,3]dioxol-4-yl)-1-(2-isopropyl-4-methylpyridin-3-yl)pyrido[2,3-d]pyrimidin-2(1H)-one (47 mg, yield: 72.3%) as a yellow solid. MS ($ES^+$): m/z=589 $(M+H)^+$. $^1H$ NMR (500 MHz, DMSO) $\delta$ 8.38 (m, 2H), 7.19 (d, $J$= 4.9 Hz, 1H), 7.00 (dd, $J$= 8.5, 4.1 Hz, 1H), 6.84 (m, 1H), 6.74 (dd, $J$= 10.3, 9.0 Hz, 1H), 6.20 (d, $J$ = 16.6 Hz, 1H), 6.00 (d, $J$ = 0.7 Hz, 2H), 5.76 (m, 1H), 4.94 (dd, $J$ = 21.6, 18.6 Hz, 1H), 4.33 (m, 2H), 4.09 (m, 1H), 3.62 (m, 2H), 3.13 (dd, $J$ = 36.5, 11.5 Hz, 1H), 2.73 (m, 1H), 1.90 (d, $J$ = 8.5 Hz, 3H), 1.33 (dd, $J$ = 19.8, 6.6 Hz, 3H), 1.07 (d, $J$ = 6.7 Hz, 3H), 0.94 (d, $J$ = 6.6 Hz, 3H).

### Example 33: Preparation of 4-((S)-4-acryloyl-2-methylpiperazin-1-yl)-6-fluoro-7-(6-fluoro-2,3-dihydrobenzo[b][1,4]dioxin-5-yl)-1-(2-isopropyl-4-methylpyridin-3-yl)pyrido[2,3-d]pyrimidin-2(1H)-one(compound 72) (prepared according to Scheme 9)

Step 33a: Preparation of 6-fluoro-5-iodo-2,3-dihydrobenzo[b][1,4]dioxine (compound 0901-72): Under the protection of nitrogen, to a solution of 4-fluoro-1,2-dimethoxybenzene (1.0 g, 6.4 mmol, 1.0 eq.) and TMEDA (743 mg, 6.4 mmol, 1.0 eq.) in tetrahydrofuran (20 mL) was added 1.6 M n-BuLi (5.6 mL, 8.96 mmol, 1.4 eq.) dropwise at -70°C. After addition, the mixture was stirred for 2 h. A solution of Iodine (2.0 g, 7.68 mmol, 1.2 eq.) in tetrahydrofuran (20 mL) was added dropwise to the mixture, and the mixture was stirred for 1 h. The reaction solution was quenched with aqueous ammonium chloride solution, and then extracted with ethyl acetate. The organic layer was concentrated in vacuo, and the residue was purified by column chromatography on silica gel (eluent: petroleum ether/ethyl acetate=200/1 to 50/1) to give 1-fluoro-2-iodo-3,4-dimethoxybenzene (1.2 g, yield: 66.7%) as a colorless oil. Under the protection of nitrogen, to a solution of 1-fluoro-2-iodo-3,4-dimethoxybenzene (1.2 g, 4.3 mmol, 1.0 eq.) prepared above in dichloromethane (20 mL) was added boron tribromide (3.2 g, 12.9 mmol, 3.0 eq.) at 0°C, and the mixture was stirred at room temperature for 3 h. The reaction solution was quenched with water, filtered and the filtrate was extracted with ethyl acetate. The organic layer was dried and concentrated in vacuo to give 4-fluoro-3-iodobenzene-1,2-diol (1.1 g, yield: 100%) as a brown solid. MS ($ES^+$): m/z=253 $(M+H)^-$. Under the protection of nitrogen, a mixture of 4-fluoro-3-iodobenzene-1,2-diol (500 mg, 2.0 mmol, 1.0 eq.) prepared above, 1,2-dibromoethane (560 mg, 3.0 mmol, 1.5 eq.) and cesium carbonate (1.3 g, 4.0 mmol, 2.0 eq.) in DMF (20 mL) was stirred at 100°C overnight. After cooling to room temperature, the mixture was poured into water and petroleum ether/ethyl acetate=1/1 was added to extract. The organic layer was washed with brine and concentrated in vcuo, and the residue was purified by column chromatography on silica gel (eluent: petroleum ether/ethyl acetate=100/1 to 50/1) to give 6-fluoro-5-iodo-2,3-dihydrobenzo[b][1,4]dioxine (551 mg, yield: 67.2%) as a white solid.

Step 33b: Preparation of (6-fluoro-2,3-dihydrobenzo[b][1,4]dioxin-5-yl)boronic acid (compound 0902-72): Under the protection of nitrogen, to a solution of 6-fluoro-5-iodo-2,3-dihydrobenzo[b][1,4]dioxine (0901-72) (280 mg, 1.0 mmol, 1.0 eq.) in toluene (8 mL) was added 1.6 M n-BuLi (0.75 mL, 1.2 mmol, 1.2 eq.) dropwise at -70°C. After addition, the mixture was stirred for 1 h. A solution of trimethyl borate (125 mg, 1.2 mmol, 1.2 eq.) in toluene (0.5 mL) was added dropwise to the mixture, and the mixture was stirred for 30 min. The reaction solution was quenched with 2 M sodium hydroxide, and the mixture was then separated. 2M HCl was added to the aqueous layer to adjust PH<2, and ethyl acetate was added to extract. The organic layer was dried over anhydrous sodium sulfate and concentrated in vacuo to give (6-fluoro-2,3-dihydrobenzo[b][1,4]dioxin-5-yl)boronic acid. (160 mg, yield: 80.8%) as a colorless oil.

Step 33c: Preparation of tert-butyl (3S)-4-(6-fluoro-7-(6-fluoro-2,3-dihydrobenzo[b][1,4]dioxin-5-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-72): Under the protection of nitrogen, a mixture of tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (100 mg, 0.19 mmol, 1.0

eq.), (6-fluoro-2,3-dihydrobenzo[b][1,4]dioxin-5-yl)boronic acid (0902-72) (113 mg, 0.57 mmol, 3.0 eq.), sodium carbonate(60 mg, 0.57 mmol, 3.0 eq.) and tetrakis(triphenylphosphine)palladium (22 mg, 0.019 mmol, 0.1 eq.) in acetonitrile/water=10/1 (11 mL) was stirred at 82°C overnight. After cooling to room temperature, the mixture was poured into water, and then extracted with ethyl acetate.. The organic layer was concentrated in vacuo and the residue was purified by prep-TLC (silica gel, eluent: ethyl acetate/methanol=100/1) to give tert-butyl (3S)-4-(6-fluoro-7-(6-fluoro-2,3-dihydrobenzo[b][1,4]dioxin-5-yl)-1-(2-isopropyl-4-methylpyridin-3-yl)-2-oxo-1,2-dihydropyrido[2,3-d]pyrimidin-4-yl)-3-methylpiperazine-1-carboxylate (65 mg, yield: 53.3%) as a white solid. MS ($ES^+$): m/z=649 $(M+H)^+$.

Step 33d: Preparation of 4-((S)-4-acryloyl-2-methylpiperazin-1-yl)-6-fluoro-7-(6-fluoro-2,3-dihydrobenzo[b][1,4]dioxin-5-yl)-1-(2-isopropyl-4-methylpyridin-3-yl)pyrido[2,3-d]pyrimidin-2(1H)-one (compound 72): To a solution of tert-butyl (3S)-4-(6-fluoro-7-(6-fluoro-2,3-dihydrobenzo[b][1,4]dioxin-5-yl)-1-(2-isopropyl-4-methylpyridin-3-yl)-2-oxo-1,2-dihydropyrido[2,3-d]pyrimidin-4-yl)-3-methylpiperazine-1-carboxylate (0904-72) (65 mg, 0.1 mmol, 1.0 eq.) in dichloromethane (6 mL) was added TFA (2 mL), and the mixture was stirred at room temperature for 1h. The solvent was removed in vacuo. The residue was dissolved in tetrahydrofuran (4 mL), and DIPEA (39 mg, 0.3 mmol, 3.0 eq.) was added. A solution of acryloyl chloride (14 mg, 0.15 mmol, 1.5 eq.) in tetrahydrofuran (0.5 mL) was added dropwise to the mixture in an ice-water bath. The mixture was stirred for 30 min. Water was added and extracted with dichloromethane. The organic layer was concentrated in vacuo and the residue was purified by prep-TLC (silica gel, eluent: dichloromethane/methanol=12/1) to give 4-((S)-4-acryloyl-2-methylpiperazin-1-yl)-6-fluoro-7-(6-fluoro-2,3-dihydrobenzo[b][1,4]dioxin-5-yl)-1-(2-isopropyl-4-methylpyridin-3-yl)pyrido[2,3-d]pyrimidin-2(1H)-one (32 mg, yield: 53.3%) as a yellow solid. MS ($ES^+$): m/z=603 $(M+H)^+$. $^1H$ NMR (500 MHz, DMSO) $\delta$ 8.36 (dd, $J$ = 41.3, 6.5 Hz, 2H), 7.20 (d, $J$ = 4.8 Hz, 1H), 6.98 (dd, $J$ = 9.0, 5.5 Hz, 1H), 6.85 (m, 1H), 6.77 (t, $J$ = 9.0 Hz, 1H), 6.21 (d, $J$ = 16.0 Hz, 1H), 5.77 (dd, $J$ = 10.5, 2.0 Hz, 1H), 4.94 (d, $J$= 30.0 Hz, 1H), 4.22 (m, 7H), 3.65 (m, 2H), 3.14 (m, 1H), 2.73 (m, 1H), 1.90 (s, 3H), 1.34 (dd, $J$= 16.8, 6.6 Hz, 3H), 1.09 (d, $J$= 6.6 Hz, 3H), 0.94 (d, $J$= 6.6 Hz, 3H).

**Example 34: Preparation of 4-((S)-4-acryloyl-2-methylpiperazin-1-yl)-6-chloro-7-(6-fluorobenzofuran-7-yl)-1-(2-isopropyl-4-methylpyridin-3-yl)pyrido[2,3-d]pyrimidin-2(1H)-one (compound 73) (prepared according to Scheme 1 and Scheme 9)**

Step 34: Preparation of 2-(6-fluorobenzofuran-7-yl)-4,4,5,5-tetramethyl-1,3,2-dioxaborolane (compound 0903-73): A mixture of (6-fluorobenzofuran-7-yl)boronic acid (0902-70) (3.0 g, 16.7 mmol, 1.0 eq.), pinacol (2.4 g, 20.0 mmol, 1.2 eq.) and magnesium sulfate (1.0 g) in ethyl acetate (50 mL) was stirred at room temperature overnight. The mixture was filtered, and the filtrate was concentrated in vacuo to give 2-(6-fluorobenzofuran-7-yl)-4,4,5,5-tetramethyl-1,3,2-dioxaborolane (4.4 g, yield: 100%) as a yellow solid.

Step 34b: Preparation of 2,5,6-trichloronicotinamide (compound 0108-73): A solution of 2,5,6-trichloronicotinic acid (0107-73) (2.0 g, 8.8 mmol, 1.0 eq.) in thionyl chloride (20 mL) was refluxed for 3 h protected form moisture. After cooling to room temperature, the solvent was removed in vacuo. The residue was dissolved in tetrahydrofuran (10 mL), and the solution was added dropwise to ammonium hydroxide solution (10 mL) in an ice-water bath. After addition, the mixture was stirred for 30 min. Water was added, and the mixture was filtered, then the filter cake was washed with water and dried in vacuo to give 2,5,6-trichloronicotinamide (1.56 g, yield: 78%) as a white solid. MS ($ES^+$): m/z=225 $(M+H)^+$.

Step 34c: Preparation of 2,5,6-trichloro-N-((2-isopropyl-4-methylpyridin-3-yl)carbamoyl)nicotinamide (compound 0109-73): Under the protection of nitrogen, a mixture of 2,5,6-trichloronicotinamide (0108-73) (1.56 g, 6.9 mmol, 1.0 eq.) and oxalyl chloride(1.76 g, 13.8 mmol, 2.0 eq.) in tetrahydrofuran (30 mL) was stirred at 75°C for 2 h. After cooling to room temperature, the solvent was removed in vacuo. The residue was dissolved in tetrahydrofuran (20 mL), and a solution of 2-isopropyl-4-methylpyridin-3-amine (0103-1) (1.0 g, 6.9 mmol, 1.0 eq.) in tetrahydrofuran (10 mL) was added dropwsie in an ice-water bath. After addition, the mixture was stirred for 30 min. ethyl acetate was added and the mixture was washed with aqueous sodium carbonate solution, dried over anhydrous sodium sulfate and concentrated in vacuo to give crude product 2,5,6-trichloro-N-((2-isopropyl-4-methylpyridin-3-yl)carbamoyl)nicotinamide and which was used directly in the next step without further purification. MS ($ES^+$): m/z=401 $(M+H)^+$.

Step 34d: 6,7-dichloro-1-(2-isopropyl-4-methylpyridin-3-yl)pyrido[2,3-d]pyrimidine-2,4(1H,3H)-dione (compound 0110-73): Under the protection of nitrogen, to a solution of 2,5,6-trichloro-N-((2-isopropyl-4-methylpyridin-3-yl)carbamoyl)nicotinamide (2.6 g, 6.5 mmol, 1.0 eq.) in DMF (50 mL) was added 60% sodium hydride (520 mg, 13.0 mmol, 2.0 eq.) portionwise in an ice-water bath, and the mixture was stirred for 1 h. The reaction solution was quenched with water and washed with petroleum ether. 2M HCl was added to the aqueous layer to adjust PH=7, and then ethyl acetate was added to extract. The organic layer was dried and concentrated in vacuo to give 6,7-dichloro-1-(2-isopropyl-4-methylpyridin-3-yl)pyrido[2,3-d]pyrimidine-2,4(1H,3H)-dione as a yellow solid (1.4 g, yield: 58.3%). MS ($ES^+$): m/z=365 $(M+H)^+$.

Step 34e: Preparation of tert-butyl (S)-4-(6,7-dichloro-1-(2-isopropyl-4-methylpyridin-3-yl)-2-oxo-1,2-dihydropyrido[2,3-d]pyrimidin-4-yl)-3-methylpiperazine-1-carboxylate (compound 0112-73): Under the protection of nitrogen, a

mixture of 6,7-dichloro-1-(2-isopropyl-4-methylpyridin-3-yl)pyrido[2,3-d]pyrimidine-2,4(1H,3H)-dione(0110-73) (1.4 g, 3.8 mmol, 1.0 eq.), DIPEA (2.5 g, 19.0 mmol, 5.0 eq.) and phosphorus oxychloride (872 mg, 5.7 mmol, 1.5 eq.) in acetonitrile (20 mL) was stirred at 85°C for 2 h. After cooling to room temperature, the solvent was removed in vacuo. The residue was dissolved in tetrahydrofuran (20 mL), DIPEA (1.5 g, 11.4 mmol, 3.0 eq.) and tert-butyl (S)-3-methylpiperazine-1-carboxylate (0111-1) (760 mg, 3.8 mmol, 1.0 eq.) were added in an ice-water bath, and the mixture was stirred for 1 h. ethyl acetate was added, and then the mixture was washed with water and brine. The organic layer was concentrated in vacuo. The residue was purified by column chromatography on silica gel (eluent: petroleum ether/ethyl acetate=5/1 to 1/1) to give tert-butyl (S)-4-(6,7-dichloro-1-(2-isopropyl-4-methylpyridin-3-yl)-2-oxo-1,2-dihydropyrido[2,3-d]pyrimidin-4-yl)-3-methylpiperazine-1-carboxylate (870 mg, yield: 41.4%) as a yellow solid. MS ($ES^+$): m/z=547 $(M+H)^+$.

Step 34f: Preparation of tert-butyl (3S)-4-(6-chloro-7-(6-fluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-73): Under the protection of nitrogen, a mixture of tert-butyl (S)-4-(6,7-dichloro-1-(2-isopropyl-4-methylpyridin-3-yl)-2-oxo-1,2-dihydropyrido[2,3-d]pyrimidin-4-yl)-3-methylpiperazine-1-carboxylate (0112-73) (150 mg, 0.27 mmol, 1.0 eq.), 2-(6-fluorobenzofuran-7-yl)-4,4,5,5-tetramethyl-1,3,2-dioxaborolane (0903-73) (108 mg, 0.41 mmol, 1.5 eq.), sodium carbonate(57 mg, 0.54 mmol, 2.0 eq.) and tetrakis(triphenylphosphine)palladium (31 mg, 0.027 mmol, 0.1 eq.) in acetonitrile/water=10/1 (11 mL) was stirred at 82°C overnight. After cooling to room temperature, the mixture was poured into water, and then extracted with ethyl acetate. The organic layer was washed with brine and concentrated in vacuo. The residue was purified by prep-TLC (silica gel, eluent: petroleum ether/ethyl acetate=1/2) to give tert-butyl (3 S)-4-(6-chloro-7-(6-fluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (130 mg, yield: 73.4%) as a yellow solid. MS ($ES^+$): m/z=647 $(M+H)^+$.

Step 34g: Preparation of 4-((S)-4-acryloyl-2-methylpiperazin-1-yl)-6-chloro-7-(6-fluorobenzofuran-7-yl)-1-(2-isopropyl-4-methylpyridin-3-yl)pyrido[2,3-d]pyrimidin-2(1H)-one (compound 73): To a solution of tert-butyl (3S)-4-(6-chloro-7-(6-fluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (0904-73) (130 mg, 0.2 mmol, 1.0 eq.) in dichloromethane (6 mL) was added TFA (2 mL), and the mixture was stirred at room temperature for 1 h. The solvent was removed in vacuo. The residue was dissolved in tetrahydrofuran (6 mL), and then DIPEA (77.4 mg, 0.6 mmol, 3.0 eq.) was added. A solution of acryloyl chloride (22 mg, 0.24 mmol, 1.2 eq.) in tetrahydrofuran (0.5 mL) was added dropwise to the mixture in an ice-water bath. The mixture was stirred for 30 min. Water was added, and then ethyl acetate was added to extract. The organic layer was concentrated in vacuo. The residue was purified by prep-TLC (silica gel, eluent: ethyl acetate/methanol=12/1) to give 4-((S)-4-acryloyl-2-methylpiperazin-1-yl)-6-chloro-7-(6-fluorobenzofuran-7-yl)-1-(2-isopropyl-4-methylpyridin-3-yl)pyrido[2,3-d]pyrimidin-2(1H)-one (72 mg, yield: 59.5%) as a yellow solid. MS ($ES^+$): m/z=601 $(M+H)^+$. $^1H$ NMR (500 MHz, DMSO) $\delta$ 8.54 (d, $J$ = 13.0 Hz, 1H), 8.33 (d, $J$ = 4.9 Hz, 1H), 7.95 (m, 1H), 7.76 (dd, $J$ = 8.6, 5.3 Hz, 1H), 7.24 (dd, $J$ = 10.2, 8.7 Hz, 1H), 7.15 (d, $J$ = 4.9 Hz, 1H), 7.01 (d, $J$ = 2.1 Hz, 1H), 6.85 (m, 1H), 6.21 (dd, $J$ = 13.3, 7.4 Hz, 1H), 5.77 (dd, $J$ = 10.4, 2.3 Hz, 1H), 4.99 (d, $J$ = 28.3 Hz, 1H), 4.34 (dd, $J$ = 31.4, 16.0 Hz, 2H), 4.10 (dd, $J$ = 58.7, 11.7 Hz, 1H), 3.70 (m, 2H), 3.17 (dd, $J$ = 42.6, 31.8 Hz, 1H), 2.75 (s, 1H), 1.94 (d, $J$ = 25.7 Hz, 3H), 1.36 (dd, $J$ = 13.4, 6.7 Hz, 3H), 1.07 (d, $J$ = 6.7 Hz, 3H), 0.92 (d, $J$ = 6.7 Hz, 3H).

**Example 35: Preparation of 4-((S)-4-acryloyl-2-methylpiperazin-1-yl)-7-(5,6-difluorobenzofuran-7-yl)-6-fluoro-1-(2-isopropyl-4-methylpyridin-3-yl)pyrido[2,3-d]pyrimidin-2(1H)-one (compound 74) (prepared according to Scheme 9)**

Step 35a: Preparation of 5,6-difluoro-7-iodobenzofuran (compound 0901-74): Under nitrogen atmosphere, n-butyllithium (6.94 mL, 1.6 mol/L of n-hexane solution, 11.1 mmol, 1.6 eq.) was added dropwise to 1,2-difluoro-4-methoxybenzene (1 g, 6.94 mmol, 1 eq.) and N,N,N',N'-Tetramethylethylenediamine (805 mg, 6.94 mmol, 1 eq.) in tetrahydrofuran (20 mL). The mixture was stirred at -70 °C for 2 h. A solution of iodine (2.1 g, 8.3 mmol, 1.2 eq.) in tetrahydrofuran (10 mL) was added dropwise to the mixture. The mixture was stirred for 1 h at -70°C. Then, the reaction was quenched with aqueous ammonium chloride. The mixture was extracted with ethyl acetate. The organic phase was washed with aqueous sodium bisulfite. The organic phase was dried over anhydrous sodium sulfate and concentrated to give 1,2-difluoro-3-iodo-4-methoxybenzene (1.94 g, crude) as a yellow oil. MS (ESI): m/z 271$(M+H)^+$. Boron tribromide (6 g, 24.15 mmol, 3 eq.) was slowly added dropwise to a solution of 1,2-difluoro-3-iodo-4-methoxybenzene (1.94 g, 8.05 mmol, 1 eq.) prepared above in dichloromethane (10 mL) at 0 °C under nitrogen atmosphere. The mixture was stirred at room temperature for 3 h. The reaction was quenched with water. The mixture was extracted with ethyl acetate. The organic phase was washed with brine and dried over anhydrous sodium sulfate. The organic phase was concentrated to give 3,4-difluoro-2-iodophenol(2 g, crude) as a yellow oil. MS (ESI): m/z 256 $(M+H)^+$. Under nitrogen atmosphere, 3,4-difluoro-2-iodophenol (2 g, 7.84 mmol, 1 eq.) prepared above, 2-Bromo-1,1-dimethoxyethane (1.99 g, 11.76 mmol, 1.5 eq.), potassium carbonate (1.62 g, 11.76 mmol, 1.5 eq.) and potassium iodide (200 mg, 10% mass fraction)) were added to DMF (20 mL). The mixture was stirred at 120 °C for 16 h. The reaction was cooled to room temperature. The

mixture was extracted with ethyl acetate. The organic phase was washed with brine and dried over anhydrous sodium sulfate. The organic phase was concentrated to give 1-(2,2-dimethoxyethoxy)-3,4-difluoro-2-iodobenzene (2.9 g, crude) as a yellow oil. MS (ESI): m/z 345 $(M+H)^+$. Under nitrogen atmosphere, 1-(2,2-dimethoxyethoxy)-3,4-difluoro- 2-iodobenzene (2 g, 5.83 mmol, 1 eq.) prepared above and polyphosphoric acid (3.94 g, 11.66 mmol, 1 eq.) were added to toluene (100 mL). The mixture was stirred at 125 °C for 16 h. The reaction was cooled to room temperature. The mixture was extracted with ethyl acetate. The organic phase was washed with brine and dried over anhydrous sodium sulfate. The organic phase was concentrated and purified by column chromatography on silica gel (eluent: petroleum ether) to give 5,6-difluoro-7-iodobenzofuran (620 mg, yield: 38.13%) as a white solid. MS (ESI): m/z 281$(M+H)^+$.

Step 35b: Preparation oftert-butyl (3S)-4-(7-(5,6-difluorobenzofuran-7-yl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0904-74): Under nitrogen, tert-butyl (S)-4-(6-fluoro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (0906-71) (100 mg, 0.15 mmol, 1 eq.), 5,6-difluoro-7-iodobenzofuran (0901-74) (84 mg, 0.3 mmol, 2 eq.), CuI (3 mg, 0.015 mmol, 0.1 eq.) and tetrakis(triphenylphosphine)palladium (17 mg, 0.015 mmol, 0.1 eq.) were added into toluene (10 mL). The mixture was stirred at 110°C for 16h. The reaction was cooled to room temperature. The mixture was concentrated and purified by prep-TLC (silica gel, petroleum ether / ethyl acetate =1 / 3) to give tert-butyl (3S)-4-(7-(5,6-difluorobenzofuran-7-yl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (59 mg, 60.09%) as a yellow solid. MS (ESI): *m/z* 649$(M+H)^+$.

Step 35c: Preparation of 4-((S)-4-acryloyl-2-methylpiperazin-1-yl)-7-(5,6-difluorobenzofuran-7-yl)-6-fluoro-1-(2-isopropyl-4-methylpyridin-3-yl)pyrido[2,3-d]pyrimidin-2(1H)-one (Compound 74): tert-butyl (3S)-4-(7-(5,6-difluorobenzofuran-7-yl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0904-074) (59 mg, 0.09 mmol, 1.0 eq.) was dissolved in 4 mL dichloromethane, and 1 mL TFA was then added into the mixture. The mixture was stirred at room temperature for 2h and was concentrated. The residue was dissolved in tetrahydrofuran (5 mL), andDIPEA (35 mg, 0.27 mmol, 3 eq.) was then added into the mixture followed by the addition of acryloyl chloride (8 mg, 0.09 mmol, 1.0 eq.). The mixture was stirred at room temperature for 15 min and was quenched with ammonium hydroxide and extracted with dichloromethane. The organic phase was concentrated and purified by prep-TLC (silica gel, eluent: ethyl acetate/methanol=12/1) to give 4-((S)-4-acryloyl-2-methylpiperazin-1-yl)-7-(5,6-difluorobenzofuran-7-yl)-6-fluoro-1-(2-isopropyl-4-methylpyridin-3-yl)pyrido[2,3-d]pyrimidin-2(1H)-one (41 mg, 74.8%) as a yellow solid. MS (ESI): *m/z* 603$(M+H)^+$. $^1$H NMR (500 MHz, DMSO) $\delta$ 8.39 (d, *J* = 41.9 Hz, 2H), 8.01 (s, 1H), 7.86 (s, 1H), 7.18 (s, 1H), 7.01 (s, 1H), 6.84 (s, 1H), 6.19 (d, *J* = 13.9 Hz, 1H), 5.75 (d, *J* = 9.3 Hz, 1H), 4.95 (d, *J* = 42.2 Hz, 1H), 4.34 (m, 2H), 4.08 (d, *J* = 63.5 Hz, 1H), 3.71 (d, *J* = 50.9 Hz, 2H), 3.12 (m, 1H), 2.76 (s, 1H), 1.93 (s, 3H), 1.34 (d, *J*= 21.0 Hz, 3H), 1.06 (s, 3H), 0.92 (s, 3H).

**Example 36: Preparation of 4-((S)-4-acryloyl-2-methylpiperazin-1-yl)-6-fluoro-7-(6-fluorobenzo[d]thiazol-7-yl)-1-(2-isopropyl-4-methylpyridin-3-yl)pyrido[2,3-d]pyrimidin-2(1H)-one (compound 78) (prepared according to Scheme 9)**

Step 36a: Preparation of 7-bromo-6-fluorobenzo[d]thiazole (compounds 0901-78) : To the mixture of 6-fluorobenzo[d]thiazol-2-amine(1.0 g, 5.95 mmol, 1.0 eq.)and sodium acetate(0.98 g, 11.90 mmol, 2.0 eq.)in AcOH (6.5 mL) was added a solution of $Br_2$(1.05 g, 6.55 mmol, 1.1 eq.)in AcOH (5mL). The mixture was stirred at room temperature for 2.5h. The mixture was diluted with water(100 mL) and filtered. The solid was washed with water and dried in vacuo to give 7-bromo-6-fluorobenzo[d]thiazol-2-amine(1.46 g, yield: 99%) as a brown solid. LCMS(ESI): *m/z* 247$[M+1]^+$; TLC: Rf 0.5 (petroleum ether: ethyl acetate = 2:1). To a mixture of 7-bromo-6-fluorobenzo[d]thiazol-2-amine(1.46 g, 5.91 mmol, 1.0 eq.)prepared above in tetrahydrofuran (40mL) was added tert-butyl nitrite(4.57 mL, 38.42 mmol, 6.5 eq.). The mixture was heated at 80°C for 8h. The solvent was removed under reduced pressure. The residue was purified by column chromatography on silica gel (petroleum ether: ethyl acetate 20:1) to get 7-bromo-6-fluorobenzo[d]thiazole(918 mg, yield: 67%) as a yellow solid. LCMS(ESI): *m/z* 232$[M+1]^+$; TLC: Rf 0.5(petroleum ether:ethyl acetate = 10:1).

Step 36b: Preparation of tert-butyl (3S)-4-(6-fluoro-7-(6-fluorobenzo[d]thiazol-7-yl)-1-(2-isopropyl-4-methylpyridin-3-yl)-2-oxo-1,2-dihydropyrido[2,3-d]pyrimidin-4-yl)-3-methylpiperazine-1-carboxylate (compounds 0904-78) To a mixture of 7-bromo-6-fluorobenzo[d]thiazole(compounds 0901-78) (114 mg, 0.49 mmol, 2.5 eq.), tetrakis(triphenylphosphine)palladium(23 mg, 0.020 mmol, 0.1 eq.) and CuI(3.8 mg, 0.02 mmol, 0.1 eq.) in toluene(15 mL) was added tert-butyl (S)-4-(6-fluoro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (compound 0906-71) (130 mg, 0.20 mmol, 1.0 eq.). The mixture was heated to 115°C under $N_2$ atmosphere overnight. The solvent was removed under reduced pressure. The residue was purified by column chromatography on silica gel (dichloromethane:methanol 20:1) to give tert-butyl (3S)-4-(6-fluoro-7-(6-fluorobenzo[d]thiazol-7-yl)-1-(2-isopropyl-4-methylpyridin-3-yl)-2-oxo-1,2-dihydropyrido[2,3-d]pyrimidin-4-yl)-3-methylpiperazine-1-carboxylate(102 mg, yield: 80%) as a brown oil. LCMS(ESI): *m/z* 648$[M+1]^+$; TLC: Rf0.5(dichloromethane:methanol = 10:1).

Step 36c: Preparation of 6-fluoro-7-(6-fluorobenzo[d]thiazol-7-yl)-1-(2-isopropyl-4-methylpyridin-3-yl)-4-((S)-2-methylpiperazin-1-yl)pyrido[2,3-d]pyrimidin-2(1H)-one (compound 0905-78): To a mixture of tert-butyl (3S)-4-(6-fluoro-7-(6-fluorobenzo[d]thiazol-7-yl)-1-(2-isopropyl-4-methylpyridin-3-yl)-2-oxo-1,2-dihydropyrido[2,3-d]pyrimidin-4-yl)-3-methylpiperazine-1-carboxylate(compound 0904-78) (102 mg, 0.157 mmol, 1.0 eq.)in dichloromethane (6 mL) was added TFA(1.5 mL). The mixture was stirred at room temperature for 0.5h. The solvent was removed under reduced pressure. The residue was dried in vacuo to give 6-fluoro-7-(6-fluorobenzo[d]thiazol-7-yl)-1-(2-isopropyl-4-methylpyridin-3-yl)-4-((S)-2-methylpiperazin-1-yl)pyrido[2,3-d]pyrimidin-2(1H)-one(110 mg, crude) as a brown oil. LCMS (ESI): *m/z* 548 $[M + 1]^+$; TLC: Rf0.3 (dichloromethane: Methanol =10:1).

Step 36d: Preparation of 4-((S)-4-acryloyl-2-methylpiperazin-1-yl)-6-fluoro-7-(6-fluorobenzo[d]thiazol-7-yl)-1-(2-isopropyl-4-methylpyridin-3-yl)pyrido[2,3-d]pyrimidin-2(1H)-one (compound 78): To a mixture of 6-fluoro-7-(6-fluorobenzo[d]thiazol-7-yl)-1-(2-isopropyl-4-methylpyridin-3-yl)-4-((S)-2-methylpiperazin-1-yl)pyrido[2,3-d]pyrimidin-2(1H)-one (compound 0905-78) (86 mg, 0.157 mmol, 1.0 eq.) andDIPEA(41 mg, 0.314 mmol, 2.0 eq.) in dichloromethane(5 mL) was added a solution of acryloyl chloride(21 mg, 0.236 mmol, 1.5 eq.)in dichloromethane(1 mL) at 0°C. The mixture was stirred for 15min and was then diluted with water(15 mL) and extracted with dichloromethane(15 mL×2). The combined organic layer was washed with brine(20 mL×1), dried over anhydrous sodium sulfate and concentrated. The residue was purified by prep-TLC(ethyl acetate:methanol=10:1) to get 4-((S)-4-acryloyl-2-methylpiperazin-1-yl)-6-fluoro-7-(6-fluorobenzo[d]thiazol-7-yl)-1-(2-isopropyl-4-methylpyridin-3-yl)pyrido[2,3-d]pyrimidin-2(1H)-one(45 mg, yield: 48%) as a white solid. LCMS(ESI): *m/z* $602[M+1]^+$; TLC: Rf 0.6(dichloromethane: methanol=10: 1); Melting point: 158-162°C. $^1$HNMR(DMSO-$d_6$, 500MHz): δ9.38(s, 1H), 8.40-8.35(m, 2H), 8.26-8.23(m, 1H), 7.20-7.15(m, 2H), 6.90-6.82(m, 1H), 6.21(d, *J* = 16.5Hz, 1H),5.78-5.75(m, 1H), 4.95(s, 1H), 4.44-4.29(m, 2H), 4.17-4.02(m, 1H), 3.76-3.74(m, 1H),3.68-3.50(m, 1H), 3.17-3.11(m, 1H), 2.78-2.73(m, 1H), 1.96(s, 3H), 1.36-1.34(m, 3H), 1.09-1.07(m, 3H), 0.97-0.94(m, 3H).

**Example 37: Preparation of 4-((S)-4-acryloyl-2-methylpiperazin-1-yl)-6-fluoro-7-(6-fluoro-2,3-dihydrobenzofuran-7-yl)-1-(2-isopropyl-4-methylpyridin-3-yl)pyrido[2,3-d]pyrimidin-2(1H)-one (compound 79) (prepared according to Scheme 9)**

Step 37a: Preparation of tert-butyl (3 S)-4-(6-fluoro-7-(6-fluoro-2,3-dihydrobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-79): A mixture of tert-butyl (3 S)-4-(6-fluoro-7-(6-fluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (0904-70) (50 mg, 0.08 mmol, 1.0 eq.) and Pd/C (10 mg) in methanol (4 mL) was stirred at room temperature under hydrogen atmosphere overnight. The mixture was filtered, and the filtrate was concentrated in vacuo to give tert-butyl (3S)-4-(6-fluoro-7-(6-fluoro-2,3-dihydrobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate.(50 mg, yield: 100%) as a yellow solid. MS ($ES^+$): m/z=633 $(M+H)^+$.

Step 37b: Preparation of 4-((S)-4-acryloyl-2-methylpiperazin-1-yl)-6-fluoro-7-(6-fluoro-2,3-dihydrobenzofuran-7-yl)-1-(2-isopropyl-4-methylpyridin-3-yl)pyrido[2,3-d]pyrimidin-2(1H)-one (compound 79): To a solution of tert-butyl (3S)-4-(6-fluoro-7-(6-fluoro-2,3-dihydrobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-79) (50 mg, 0.08 mmol, 1.0 eq.) in dichloromethane (4 mL) was added TFA(2 mL), and the mixture was stirred at room temperature for 1 h. The solvent was removed in vacuo. The residue was dissolved in tetrahydrofuran (4 mL), and DIPEA (31 mg, 0.24 mmol, 3.0 eq.) was then added. A solution of acryloyl chloride (11 mg, 0.12 mmol, 1.5 eq.) in tetrahydrofuran (0.5 mL) was added dropwise to the mixture in an ice-water bath. The mixture was stirred for 30 min. Water was added and extracted with ethyl acetate. The organic layer was concentrated in vacuo. The residue was purified by prep-TLC (silica gel, eluent: dichloromethane/methanol=10/1) to give 4-((S)-4-acryloyl-2-methylpiperazin-1-yl)-6-fluoro-7-(6-fluoro-2,3-dihydrobenzofuran-7-yl)-1-(2-isopropyl-4-methylpyridin-3-yl)pyrido[2,3-d]pyrimidin-2(1H)-one (27 mg, yield: 58.7%) as a yellow solid. MS ($ES^+$): m/z=587 $(M+H)^+$. $^1$H NMR (500 MHz, DMSO) δ 8.39 (d, *J* = 4.9 Hz, 1H), 8.31 (dd, *J* = 18.0, 9.0 Hz, 1H), 7.29 (dd, *J* = 8.2, 5.6 Hz, 1H), 7.19 (d, *J* = 4.9 Hz, 1H), 6.84 (m, 1H), 6.70 (dd, *J* = 10.3, 8.2 Hz, 1H), 6.20 (d, *J* = 16.3 Hz, 1H), 5.76 (dd, *J* = 10.4, 2.3 Hz, 1H), 4.93 (dd, *J* = 21.4, 18.1 Hz, 1H), 4.50 (m, 2H), 4.32 (m, 2H), 4.09 (m, 1H), 3.70 (m, 2H), 3.14 (m, 3H), 2.72 (m, 1H), 1.89 (m, 3H), 1.33 (dd, *J* = 19.3, 6.7 Hz, 3H), 1.07 (d, *J* = 6.7 Hz, 3H), 0.94 (d, *J* = 6.7 Hz, 3H).

**Example 38: Preparation of (S)-4-(4-acryloyl-2-methylpiperazin-1-yl)-6-fluoro-7-(1H-indol-7-yl)-1-(2-isopropyl-4-methylpyridin-3-yl)pyrido[2,3-d]pyrimidin-2(1H)-one (compound 82) (prepared according to Scheme 9)**

Step 38a: Preparation of tert-butyl 7-bromo-1H-indole-1-carboxylate (compound 0906-71): a mixture of 7-bromo-1H-indole (0.8 g, 4.08 mmol, 1.0 eq.), $(Boc)_2O$ (1.8 g, 8.16 mmol, 2.0 eq.), DMAP (49 mg, 0.4 mmol, 0.1 eq.) and DIPEA (1.6 g, 12.24 mmol, 3.0 eq.) in tetrahydrofuran (30 mL) was stirred at room temparature overnight. The reaction solution was extracted with dichloromethane and water. The organic phase was dried and concentrated to give tert-butyl

7-bromo-1H-indole-1-carboxylate (1.2 g, crude) as a colourless oil. MS (ESI): *m/z* 296(M+H) +.

Step 38b: Preparation of tert-butyl (S)-7-(4-(4-(tert-butoxycarbonyl)-2-methylpiperazin-1-yl)-6-fluoro-1-(2-isopropyl-4-methylpyridin-3-yl)-2-oxo-1,2-dihydropyrido[2,3-d]pyrimidin-7-yl)-1H-indole-1-carboxylate (compound 0904-82): Under the protection of nitrogen, tert-butyl (S)-4-(6-fluoro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (compound 0906-71) (100 mg, 0.151 mmol, 1.0 eq.), tert-butyl 7-bromo-1H-indole-1-carboxylate(compound 0901-82) (89.7 mg, 0.303mmol, 2 eq.), CuI (2.8 mg, 0.015 mmol, 0.1 eq.) and tetrakis(triphenylphosphine)palladium (17.5 mg, 0.015 mmol, 0.1 eq.) were added into toluene (10 mL). The mixture was stirred at 110°C overnight. The reaction was cooled to room temperature. The mixture was concentrated and purified by prep-TLC (silica gel, eluent: petroleum ether/ethyl acetate=1/3) to give tert-butyl (S)-7-(4-(4-(tert-butoxycarbonyl)-2-methylpiperazin-1-yl)-6-fluoro-1-(2-isopropyl-4-methylpyridin-3 -yl)-2-oxo-1,2-dihydropyrido[2,3-d]pyrimidin-7-yl)-1H-indole-1-carboxylate (100 mg, 93.6%) as a yellow solid. MS (ESI): *m/z* 712(M+H) +.

Step 38c: Preparation of (S)-4-(4-acryloyl-2-methylpiperazin-1-yl)-6-fluoro-7-(1H-indol-7-yl)-1-(2-isopropyl-4-methylpyridin-3-yl)pyrido[2,3-d]pyrimidin-2(1H)-one (compound 82): Tert-butyl (S)-7-(4-(4-(tert-butoxycarbonyl)-2-methylpiperazin-1-yl)-6-fluoro-1-(2-isopropyl-4-methylpyridin-3-yl)-2-oxo-1,2-dihydropyrido[2,3-d]pyrimidin-7-yl)-1H-indole-1-carboxylate (100 mg, 0.14 mmol, 1.0 eq.) was dissolved in 5 mL dichloromethane, and 1 mL TFA was then added into the mixture. The mixture was stirred at room temperature for 2 h. The mixture was then concentrated. The residue was dissolved in tetrahydrofuran (5 mL) and DIPEA (0.5 mL) was then added into the mixture. Acryloyl chloride (12 mg, 0.13 mmol, 0.9 eq.) was added into the mixture at 0°C. The mixture was stirred at room temperature for 15 min. The reaction was quenched with ammonium hydroxide and extracted with dichloromethane. The organic phase was concentrated and purified by prep-TLC (silica gel, eluent: ethyl acetate/methanol=10/1) to give (S)-4-(4-acryloyl-2-methylpiperazin-1-yl)-6-fluoro-7-(1H-indol-7-yl)-1-(2-isopropyl-4-methylpyridin-3-yl)pyrido[2,3-d]pyrimidin-2(1H)-one (27 mg, 34.1%) as a yellow solid. MS (ESI): *m/z* 566(M+H)+. $^1$H NMR (500 MHz, DMSO) $\delta$ 9.36 (s, 1H), 8.75 (d, *J* = 4.6 Hz, 1H), 8.35 (d, *J* = 8.9 Hz, 1H), 8.02 (d, *J* = 7.5 Hz, 1H), 7.76 (d, *J* = 7.5 Hz, 1H), 7.46 (s, 1H), 7.16 (t, *J* = 7.7 Hz, 1H), 6.85 (s, 2H), 6.47 (s, 1H), 6.20 (d, *J*= 16.1 Hz, 1H), 5.75 (d, *J* = 10.2 Hz, 1H), 4.96 (s, 1H), 4.18 (m, 3H), 3.70 (d, *J* = 32.7 Hz, 2H), 3.10 (m, 1H), 2.81 (s, 1H), 1.99 (d, *J* = 5.3 Hz, 3H), 1.34 (t, *J* = 7.2 Hz, 3H), 1.11 (d, *J* = 4.6 Hz, 3H), 0.83 (d, *J* = 3.5 Hz, 3H).

**Example 39: Preparation of 4-((2S,5R)-4-acryloyl-2,5-dimethylpiperazin-1-yl)-6-fluoro-7-(6-fluorobenzofuran-7-yl)-1-(2-isopropyl-4-methylpyridin-3-yl)pyrido[2,3-d]pyrimidin-2(1H)-one (compound 89) (prepared according to Scheme 9)**

Step 39a: Preparation of potassium trifluoro(6-fluorobenzofuran-7-yl)borate (compound 0907-89): To a solution of 6-fluorobenzofuran-7-boronic acid (compound 0902-70) (180 mg, 1.0 mmol, 1.0 eq.) in methanol/water = 3/1 (10 mL) was added an a solution of potassium hydrogen fluoride (234 mg, 3.0 mmol, 3.0 eq.) in water (1 mL). The mixture was stirred at room temperature for 2 h. The solid was filtered and dried in vacuo to give potassium trifluoro(6-fluorobenzofuran-7-yl)borate (240 mg, yield: 99.2%) as a white solid. MS (ES-): m/z=203 (M-H)-.

Step 39b: Preparation of tert-butyl (2*R*,5*S*)-4-(7-chloro-6-fluoro-1-(2-isopropyl-4-methylpyridin-3-yl)-2-oxo-1,2-dihydropyrido[2,3-*d*]pyrimidin-4-yl)-2,5-dimethylpiperazine-1-carboxylate (compound 0112-89): Under the protection of nitrogen, a mixture of 7-chloro-6-fluoro-1-(2-isopropyl-4-methylpyridin-3-yl)pyrido[2,3-d]pyrimidine-2,4(1*H*,3*H*)-dione (0110-1) (395 mg, 1.13 mmol, 1.0 eq.), DIPEA (729 mg, 5.65 mmol, 5.0 eq.) and phosphorus oxychloride (348 mg, 2.27 mmol, 2.0 eq.) in acetonitrile (20 mL) was stirred at 85°C for 2 h. After cooling to room temperature, the solvent was removed in vacuo. The residue was dissolved in tetrahydrofuran (15 mL) and cooled in an ice-water bath. DIPEA (442 mg, 3.42 mmol, 3.0 eq.) and tert-butyl (2*R*,5*S*)-2,5-dimethylpiperazine-1-carboxylate (0111-89) (242 mg, 1.14 mmol, 1.0 eq.) were added. The mixture was stirred for 30 min. ethyl acetate was added and the mixture was washed with water and brine. The organic phase was concentrated in vacuo. The residue was purified by column chromatography on silica gel (eluent: dichloromethane/methanol=200/1 to 50/1) to give tert-butyl (2*R*,5*S*)-4-(7-chloro-6-fluoro-1-(2-isopropyl-4-methylpyridin-3-yl)-2-oxo-1,2-dihydropyrido[2,3-*d*]pyrimidin-4-yl)-2,5-dimethylpiperazine-1-carboxylate (435 mg, yield: 70%) as a yellow solid. MS (ES+): *m/z*=545 (M+H)+.

Step 39c: Preparation of tert-butyl (2*R*,5*S*)-4-(6-fluoro-7-(6-fluorobenzofuran-7-yl)-1-(2-isopropyl-4-methylpyridin-3-yl)-2-oxo-1,2-dihydropyrido[2,3-d]pyrimidin-4-yl)-2,5-dimethylpiperazine-1-carboxylate (compound 0904-89): Under the protection of nitrogen, potassium trifluoro(6-fluorobenzofuran-7-yl)borate (compound 0907-89) (80 mg, 0.33 mmol, 1.5 eq.), tert-butyl (2*R*,5*S*)-4-(7-chloro-6-fluoro-1-(2-isopropyl-4-methylpyridin-3-yl)-2-oxo-1,2-dihydropyrido[2,3-*d*]pyrimidin-4-yl)-2,5-dimethylpiperazine-1-carboxylate (0112-89) (120 mg, 0.22 mmol, 1.0 eq.), 1, 1'-bis(diphenylphosphino)ferrocene palladium(II)dichloride (16 mg, 0.02 mmol, 0.1 eq.) and potassium fluoride (39 mg, 0.66 mmol, 3.0 eq.) were added into a mixed solvent of 25 mL dioxane and 2.5 mL water. The mixture was heated at 90°C for 2 h. The mixture was cooled to room temparature and extracted with ethyl acetate. The organic phase was washed with water and concentrated under vacuum. The residue was purified by prep-TLC (silica gel, eluent: dichloromethane/methanol=50/1) to give tert-butyl (2*R*,5*S*)-4-(6-fluoro-7-(6-fluorobenzofuran-7-yl)-1-(2-isopropyl-4-methylpyridin-3-yl)-2-oxo-1,2-dihydropyrido[2,3-*d*]pyrimidin-4-yl)-2,5-dimethylpiperazine-1-carboxylate (116 mg, yield: 81%) as a yellow solid. MS ($ES^+$): *m/z*=645$(M+H)^+$.

Step 39d: Preparation of 4-((2*S*,5*R*)-4-acryloyl-2,5-dimethylpiperazin-1-yl)-6-fluoro-7-(6-fluorobenzofuran-7-yl)-1-(2-isopropyl-4-methylpyridin-3-yl)pyrido[2,3-*d*]pyrimidin-2(1*H*)-one (compound 89): tert-butyl (2*R*,5*S*)-4-(6-fluoro-7-(6-fluorobenzofuran-7-yl)-1-(2-isopropyl-4-methylpyridin-3-yl)-2-oxo-1,2-dihydropyrido[2,3-*d*]pyrimidin-4-yl)-2,5-dimethylpiperazine-1-carboxylate (compound 0904-89) (116 mg, 0.18 mmol, 1.0 eq.) was dissolved in 10 mL dichloromethane.and TFA (1.5 mL) was then added dropwise. The mixture was stirred at room temperature for 2 h andconcentrated under vacuum to dryness. The residue was dissolved in 10 mL tetrahydrofuran, and 1.5 mL DIPEA was then added. The mixture was cool in an ice-water bath and acryloyl chloride (20 mg, 0.22 mmol, 1.2 eq.), pre-dissolved in 0.5 mL tetrahydrofuran, was added dropwise into the mixture above. The mixture was stirred for 30 min and diluted with ethyl acetate. The organic phase was washed with water and concentrated under vacuum. The residue was purified by prep-TLC (silica gel, eluent: dichloromethane/methanol=25/1) to give 4-((2*S*,5*R*)-4-acryloyl-2,5-dimethylpiperazin-1-yl)-6-fluoro-7-(6-fluorobenzofuran-7-yl)-1-(2-isopropyl-4-methylpyridin-3-yl)pyrido[2,3-d]pyrimidin-2(1H)-one (22 mg, yield: 20%) as a yellow solid. MS ($ES^+$): *m/z*=599 $(M+H)^+$. Melting point: 147°C ~149°C. $^1H$ NMR (500 MHz, DMSO) $\delta$ 8.40 (m, 2H), 7.95 (dd, *J* = 9.0, 1.9 Hz, 1H), 7.80 (dd, *J* = 8.4, 5.2 Hz, 1H), 7.26 (t, *J* = 9.5 Hz, 1H), 7.19 (dd, *J* = 8.0, 4.9 Hz, 1H), 7.02 (s, 1H), 6.84 (ddd, *J* = 20.4, 16.8, 9.0 Hz, 1H), 6.19 (dd, *J* = 16.7, 1.9 Hz, 1H), 5.75 (dd, *J* = 10.1, 3.5 Hz, 1H), 4.72 (m, 2H), 3.93 (m, 4H), 2.75 (dd, *J* = 12.8, 6.2 Hz, 1H), 1.96 (d, *J* = 14.5 Hz, 3H), 1.32 (m, 3H), 1.23 (t, *J* = 8.4 Hz, 3H), 1.07 (d, *J* = 6.6 Hz, 3H), 0.94 (d, *J* = 6.4 Hz, 3H).

**Example 40: Preparation of 4-((2S,5R)-4-acryloyl-2,5-dimethylpiperazin-1-yl)-6-chloro-7-(6-fluorobenzofuran-7-yl)-1-(2-isopropyl-4-methylpyridin-3-yl)pyrido[2,3-d]pyrimidin-2(1H)-one (compound 90) (prepared according to Scheme 9)**

Step 40a: Preparation of tert-butyl (2*R*,5*S*)-4-(6,7-dichloro-1-(2-isopropyl-4-methylpyridin-3-yl)-2-oxo-1,2-dihydropyrido[2,3-*d*]pyrimidin-4-yl)-2,5-dimethylpiperazine-1-carboxylate (compound 0112-90): Under the protection of nitrogen, a mixture of 6,7-dichloro-1-(2-isopropyl-4-methylpyridin-3-yl)pyrido[2,3-*d*]pyrimidin-2(1*H*)-one (0110-73) (225 mg, 0.62 mmol, 1.0 eq.), DIPEA (400 mg, 3.10 mmol, 5.0 eq.) and phosphorus oxychloride (190 mg, 1.24 mmol, 2.0 eq.) in acetonitrile (15 mL) was stirred at 85°C for 2 h. After cooling to room temperature, the solvent was removed in vacuo. The residue was dissolved in tetrahydrofuran (20 mL) and cooled in an ice-water bath. DIPEA (240 mg, 1.86 mmol, 3.0 eq.) and tert-butyl (2*R*, 5*S*)-2, 5-dimethylpiperazine-1-carboxylate (0111-89) (133 mg, 0.62 mmol, 1.0 eq.) were added. The mixture was stirred for 30 min. ethyl acetate was added, and the mixture was washed with water and brine. The organic phase was concentrated in vacuo. The residue was purified by column chromatography on silica gel (eluent: dichloromethane/methanol=200/1 to 50/1) to give tert-butyl (2*R*,5*S*)-4-(6,7-dichloro-1-(2-isopropyl-4-methylpyridin-3-yl)-2-oxo-1,2-dihydropyrido[2,3-*d*]pyrimidin-4-yl)-2,5-dimethylpiperazine-1-carboxylate (277 mg, yield: 79%) as a yellow solid. MS ($ES^+$): *m/z*=561 $(M+H)^+$.

Step 40b: Preparation of tert-butyl (2*R*,5*S*)-4-(6-chloro-7-(6-fluorobenzofuran-7-yl)-1-(2-isopropyl-4-methylpyridin-3-yl)-2-oxo-1,2-dihydropyrido[2,3-*d*]pyrimidin-4-yl)-2,5-dimethylpiperazine-1-carboxylate (compound 0904-90): Under the protection of nitrogen, 2-(6-fluorobenzofuran-7-yl)-4,4,5,5-tetramethyl-1,3,2-dioxaborolane (compound 0903-73) (140 mg, 0.54 mmol, 2.0 eq.), tert-butyl (2*R*,5*S*)-4-(6,7-dichloro-1-(2-isopropyl-4-methylpyridin-3-yl)-2-oxo-1,2-dihydropyrido[2,3-*d*]pyrimidin-4-yl)-2,5-dimethylpiperazine-1-carboxylate (0112-90) (150 mg, 0.27 mmol, 1.0 eq.), tetrakis(triphenylphosphine)palladium (32 mg, 0.027 mmol, 0.1 eq.) and sodium carbonate (57 mg, 0.54 mmol, 2.0 eq.) were added into a mixed solvent of 50 mL acetonitrile and 5 mL water. The mixture was stirred at 80°C for 16 h. The mixture was cooled to room temparature and extracted with ethyl acetate. The organic phase was washed with water and concentrated under vacuum. The residue was purified by prep-TLC (silica gel, eluent: dichloromethane/methanol=50/1) to give tert-butyl (2*R*,5*S*)-4-(6-chloro-7-(6-fluorobenzofuran-7-yl)-1-(2-isopropyl-4-methylpyridin-3-yl)-2-oxo-1,2-dihydropyrido[2,3-*d*]pyrimidin-4-yl)-2,5-dimethylpiperazine-1-carboxylate (86 mg, yield: 48%) as a yellow solid. MS ($ES^+$): *m/z*=661$(M+H)^+$.

Step 40c: Preparation of 4-((2*S*,5*R*)-4-acryloyl-2,5-dimethylpiperazin-1-yl)-6-chloro-7-(6-fluorobenzofuran-7-yl)-1-(2-isopropyl-4-methylpyridin-3-yl)pyrido[2,3-*d*]pyrimidin-2(1*H*)-one (compound 90): tert-butyl (2*R*,5*S*)-4-(6-chloro-7-(6-fluorobenzofuran-7-yl)-1-(2-isopropyl-4-methylpyridin-3-yl)-2-oxo-1,2-dihydropyrido[2,3-*d*]pyrimidin-4-yl)-2,5-dimethylpiperazine-1-carboxylate (compound 0904-90) (86 mg, 0.13 mmol, 1.0 eq.) was dissolved in 15 mL dichloromethane. TFA (1.5 mL) was then added dropwise and the mixture was stirred at room temperature for 2 h. The mixture was concentrated under vacuum to dryness. The residue was dissolved in 15 mL tetrahydrofuran and 1.5 mL DIPEA was then added. The mixture was cool in an ice-water bath and acryloyl chloride (35 mg, 0.39 mmol, 3.0 eq.), pre-dissolved in 0.5 mL tetrahydrofuran, was added dropwise into the mixture above. The mixture was stirred for 30 min and diluted with ethyl acetate. The organic phase was washed with water and concentrated under vacuum. The residue was purified by prep-HPLC to give 4-((2*S*,5*R*)-4-acryloyl-2,5-dimethylpiperazin-1-yl)-6-chloro-7-(6-fluorobenzofuran-7-yl)-1-(2-isopropyl-4-methylpyridin-3-yl)pyrido[2,3-*d*]pyrimidin-2(1*H*)-one (24 mg, yield: 25%) as a yellow solid. MS ($ES^+$):

*m/z*=615 (M+H)+. Melting point: 167°C~169°C. $^1$H NMR (500 MHz, DMSO) δ 8.51 (m, 1H), 8.32 (d, *J* = 4.9 Hz, 1H), 7.94 (m, 1H), 7.75 (ddd, *J* = 8.5, 5.3, 1.4 Hz, 1H), 7.23 (t, *J* = 9.4 Hz, 1H), 7.14 (t, *J* = 40 Hz, 1H), 7.00 (d, *J* = 1.4 Hz, 1H), 6.83 (ddd, *J* = 27.4, 13.7, 8.4 Hz, 1H), 6.17 (dd, *J* = 16.7, 2.2 Hz, 1H), 5.74 (m, 1H), 4.71 (m, 2H), 3.89 (m, 4H), 2.70 (s, 1H), 1.95 (m, 3H), 1.34 (dd, *J* = 14.6, 7.6 Hz, 3H), 1.24 (ddd, *J* = 18.5, 10.9, 5.2 Hz, 3H), 1.04 (dd, *J* = 6.7, 2.1 Hz, 3H), 0.90 (d, *J* = 6.7 Hz, 3H).

**Example 41: Preparation of 4-((2S,5R)-4-acryloyl-2,5-dimethylpiperazin-1-yl)-7-(5,6-difluorobenzofuran-7-yl)-6-fluoro-1-(2-isopropyl-4-methylpyridin-3-yl)pyrido[2,3-d]pyrimidin-2(1H)-one (compound 91) (prepared according to Scheme 9)**

Step 41a: Preparation oftert-butyl (2R,5S)-4-(6-fluoro-1-(2-isopropyl-4-methylpyridin-3-yl)-2-oxo-7-(trimethylstannyl)-1,2-dihydropyrido[2,3-d]pyrimidin-4-yl)-2,5-dimethylpiperazine-1-carboxylate (compound 0906-91): Under nitrogen, tert-butyl (2R,5S)-4-(7-chloro-6-fluoro-1-(2-isopropyl-4-methylpyridin-3-yl)-2-oxo-1,2-dihydropyrido[2,3-d]pyrimidin-4-yl)-2,5-dimethylpiperazine-1-carboxylate (0112-89) (150 mg, 0.275 mmol, 1 eq.), hexamethyldistannane (270 mg, 0.825 mmol, 3 eq.)and tetrakis(triphenylphosphine)palladium (32 mg, 0.03 mmol, 0.1 eq.) were added into toluene (10 mL). The mixture was stirred at 110°C for 2h. The reaction was cooled to room temperature. The mixture was concentrated and purified by column chromatography on silica gel ( eluent: petroleum ether/ethyl acetate=5/1-1/2) to give tert-butyl (2R,5S)-4-(6-fluoro-1-(2-isopropyl-4-methylpyridin-3-yl)-2-oxo-7-(trimethylstannyl)-1,2-dihydropyrido[2,3-d]pyrimidin-4-yl)-2,5-dimethylpiperazine-1-carboxylate (145 mg, 78.16%) as a yellow solid. MS (ESI): *m/z* 675(M+H) +.

Step 41b: Preparation of tert-butyl (2R,5S)-4-(7-(5,6-difluorobenzofuran-7-yl)-6-fluoro-1-(2-isopropyl-4-methylpyridin-3-yl)-2-oxo-1,2-dihydropyrido[2,3-d]pyrimidin-4-yl)-2,5-dimethylpiperazine-1-carboxylate (compound 0904-91): Under nitrogen, tert-butyl (2R,5S)-4-(6-fluoro-1-(2-isopropyl-4-methylpyridin-3-yl)-2-oxo-7-(trimethylstannyl)-1,2-dihydropyrido[2,3-d]pyrimidin-4-yl)-2,5-dimethylpiperazine-1-carboxylate(compound 0906-91) (140 mg, 0.208 mmol, 1 eq.), 5,6-difluoro-7-iodobenzofuran (compound 0901-74) (116 mg, 0.416 mmol, 2 eq.), CuI (4 mg, 0.02 mmol, 0.1 eq.) and tetrakis(triphenylphosphine)palladium (24 mg, 0.02 mmol, 0.1 eq.) were added into toluene (10 mL). The mixture was stirred at 110°C for 16h. The reaction was cooled to room temperature. The mixture was concentrated and purified by prep-TLC (silica gel, eluent: petroleum ether/ethyl acetate=1/3) to give tert-butyl (2R,5S)-4-(7-(5,6-difluorobenzofuran-7-yl)-6-fluoro-1-(2-isopropyl-4-methylpyridin-3-yl)-2-oxo-1,2-dihydropyrido[2,3-d]pyrimidin-4-yl)-2,5-dimethylpiperazine-l-carboxylate (51.6 mg, 37.53%) as a yellow solid. MS (ESI): *m/z* 663(M+H)+.

Step 41c. Preparation of 4-((2S,5R)-4-acryloyl-2,5-dimethylpiperazin-1-yl)-7-(5,6-difluorobenzofuran-7-yl)-6-fluoro-1-(2-isopropyl-4-methylpyridin-3-yl)pyrido[2,3-d]pyrimidin-2(1H)-one (compound 91): tert-butyl (2R,5S)-4-(7-(5,6-difluorobenzofuran-7-yl)-6-fluoro-1-(2-isopropyl-4-methylpyridin-3 -yl)-2-oxo-1,2-dihydropyrido[2,3 -d]pyrimidin-4-yl)-2,5-dimethylpiperazine-1-carboxylate (compound 0904-91) (51.6 mg, 0.078 mmol, 1.0 eq.) was dissolved in 4 mL dichloromethane, and 1 mL TFA was then added into the mixture. The mixture was stirred at room temperature for 2h and then concentrated. The residue was dissolved in tetrahydrofuran (5 mL). and DIPEA (30 mg, 0.234 mmol, 3 eq.) was then added into the mixture. Acryloyl chloride (7 mg, 0.078 mmol, 1.0 eq.) was added into the mixture. The mixture was stirred at room temperature for 15 min, quenched with ammonium hydroxide and extracted with dichloromethane. The organic phase was concentrated and purified by prep-TLC (silica gel, eluent: ethyl acetate/methanol=12/1) to give 4-((2S,5R)-4-acryloyl-2,5-dimethylpiperazin-1-yl)-7-(5,6-difluorobenzofuran-7-yl)-6-fluoro-1-(2-isopropyl-4-methylpyridin-3-yl)pyrido[2,3-d]pyrimidin-2(1H)-one (25 mg, 52.08%) as a yellow solid. MS (ESI): *m/z* 617(M+H)+. $^1$H NMR (500 MHz, DMSO) δ 8.42 (m, 2H), 8.03 (dd, *J* = 8.7, 2.1 Hz, 1H), 7.89 (m, 1H), 7.20 (dd, *J* = 8.4, 5.0 Hz, 1H), 7.03 (d, *J* = 2.1 Hz, 1H), 6.84 (m, 1H), 6.19 (dd, *J* = 16.6, 2.1 Hz, 1H), 5.75 (ddd, *J* = 10.4, 5.5, 2.2 Hz, 1H), 4.76 (m, 2H), 3.85 (m, 4H), 2.75 (dt, *J* = 13.6, 6.8 Hz, 1H), 1.96 (d, *J* = 14.5 Hz, 3H), 1.33 (ddd, *J* = 14.1, 10.7, 6.5 Hz, 3H), 1.21 (m, 3H), 1.07 (dd, *J* = 6.7, 2.2 Hz, 3H), 0.94 (dd, *J* = 7.7, 3.6 Hz, 3H).

**Example 42: Preparation of 4-((S)-4-acryloyl-2-methylpiperazin-1-yl)-6-chloro-7-(5-fluorobenzo[d][1,3]dioxol-4-yl)-1-(2-isopropyl-4-methylpyridin-3-yl)pyrido[2,3-d]pyrimidin-2(1H)-one (compound 97) (prepared according to Scheme 9)**

Step 42a: Preparation of 2-(5-fluorobenzo[d][1,3]dioxol-4-yl)-4,4,5,5-tetramethyl-1,3,2-dioxaborolane (compound 0903-97): Under the protection of nitrogen, 5-fluoro-4-iodobenzo[d][1,3]dioxole (compound 0901-69) (554 mg, 2.08 mmol, 1.0 eq.) was dissolved in 15 mL dry toluene. The mixture was cooled to -75°C and 2.5M n-BuLi in n-hexane solution (1 mL, 2.5 mmol, 1.2 eq.) was added dropwise. The mixture was stirred at -75°C for 2 h. Trimethyl borate (281 mg, 2.7 mmol, 1.3 eq.) was dissolved in dry toluene, and added dropwise into the mixture above. The mixture was stirred at -75°C for 1 h. The PH of the mixture was adjust to 1 with 2M HCl solution, and the mixture was warmed up to room temperature. ethyl acetate was added to extract. The organic phase was dried and concentrated under vacuum to give a yellow solid (250 mg, crude). The solid (250 mg, 1.358 mmol, 1.0 eq.), pinacol (192 mg, 1.63 mmol, 1.2 eq.) and anhydrous magnesium sulfate (244 mg, 2.037 mmol, 1.5 eq.) was added into ethyl acetate. The mixture was stirred at

room temperature for 2 h, filtered through a celite pad, and the filtrate was concentrated under vacuum to give 2-(5-fluorobenzo[d][1,3]dioxol-4-yl)-4,4,5,5-tetramethyl-1,3,2-dioxaborolane (480 mg, crude) as a white solid. MS ($ES^+$): *m*/*z*=267$(M+H)^+$.

Step 42b: tert-butyl (3S)-4-(6-chloro-7-(5-fluorobenzo[d][1,3]dioxol-4-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-97): Under nitrogen, tert-butyl (S)-4-(6,7-dichloro-1-(2-isopropyl-4-methylpyridin-3-yl)-2-oxo-1,2-dihydropyrido[2,3-d]pyrimidin-4-yl)-3-methylpiperazine-1-carboxylate (0112-73) (300 mg, 0.549 mmol, 1 eq.), 2-(5-fluorobenzo[d][1,3]dioxol-4-yl)-4,4,5,5-tetramethyl-1,3,2-dioxaborolane (compound 0903-97) (417 mg, 1.567 mmol,2.85 eq.), tetrakis(triphenylphosphine)palladium (63 mg, 0.055 mmol, 0.1 eq.), copper(I) chloride (54 mg, 0.055 mmol, 0.1 eq.) and sodium carbonate (116 mg, 1.098 mmol,2 eq.) were added into the mixture of acetonitrile (10 mL) and water(1 mL). The mixture was stirred at 80°C for 16h and then cooled to room temparature. The mixture was extracted with ethyl acetate. The organic phase was washed with brine and dried over anhydrous sodium sulfate, and concentrated under vacuum. The residue was purified by column chromatography on silica gel ( eluent: dichloromethane/methanol=100/1-30/1) to give tert-butyl (3S)-4-(6-chloro-7-(5-fluorobenzo[d][1,3]dioxol-4-yl)-1-(2-isopropyl-4-methylpyridin-3-yl)-2-oxo-1,2-dihydropyrido[2,3-d]pyrimidin-4-yl)-3-methylpiperazine-1-carboxylate (344 mg, 96.35%) as a yellow solid. MS (ESI): *m*/*z* 651$(M+H)^+$.

Step 42c: 3.4-((S)-4-acryloyl-2-methylpiperazin-1-yl)-6-chloro-7-(5-fluorobenzo[d][1,3]dioxol-4-yl)-1-(2-isopropyl-4-methylpyridin-3-yl)pyrido[2,3-d]pyrimidin-2(1H)-one (compound 97): tert-butyl (3S)-4-(6-chloro-7-(5-fluorobenzo[d][1,3]dioxol-4-yl)-1-(2-isopropyl-4-methylpyridin-3-yl)-2-oxo-1,2-dihydropyiido[2,3-d]pyrimidin-4-yl)-3-methylpiperazine-1-carboxylate (compound 0904-97) (344 mg, 0.528 mmol, 1.0 eq.) was dissolved in 4 mL dichloromethane, and 1 mL TFA was added into the mixture. The mixture was stirred at room temperature for 2h and then concentrated. The residue was dissolved in tetrahydrofuran (5 mL),and DIPEA (204 mg, 1.584 mmol, 3 eq.) was then added into the mixture. acryloyl chloride (48 mg, 0.528 mmol, 1.0 eq.) was added into the mixture. The mixture was stirred at room temperature for 15 min, quenched with ammonium hydroxide and extracted with dichloromethane. The organic phase was concentrated and purified by column chromatography on silica gel ( eluent: dichloromethane/methanol=100/1-30/1) to give 4-((S)-4-acryloyl-2-methylpiperazin-1-yl)-6-chloro-7-(5-fluorobenzo[d][1,3]dioxol-4-yl)-1-(2-isopropyl-4-methylpyridin-3-yl)pyrido[2,3-d]pyrimidin-2(1H)-one (208 mg, 65.2%) as a yellow solid. MS (ESI): *m*/*z* 605$(M+H)^+$. $^1H$ NMR (500 MHz, DMSO) $\delta$ 8.49 (d, *J* = 13.8 Hz, 1H), 8.41 (d, *J* = 4.9 Hz, 1H), 7.20 (d, *J* = 4.9 Hz, 1H), 6.99 (dd, *J* = 8.6, 4.3 Hz, 1H), 6.93 - 6.80 (m, 1H), 6.74 (dd, *J* = 10.3, 8.7 Hz, 1H), 6.22 (d, *J* = 17.0 Hz, 1H), 6.02 (s, 2H), 5.77 (dd, *J* = 10.4, 2.2 Hz, 1H), 4.96 (d, *J* = 29.5 Hz, 1H), 4.34 (dd, *J* = 32.0, 17.9 Hz, 2H), 4.19 - 3.99 (m, 1H), 3.84 - 3.50 (m, 2H), 3.26 - 3.05 (m, 1H), 2.79 - 2.67 (m, 1H), 1.91 (d, *J* = 2.9 Hz, 3H), 1.35 (dd, *J* = 14.2, 6.7 Hz, 3H), 1.08 (d, *J* = 6.7 Hz, 3H), 0.95 (d, *J* = 6.6 Hz, 3H).

**Example 43: Preparation of 4-((S)-4-acryloyl-2-methylpiperazin-1-yl)-6-chloro-7-(6-fluoro-2,3-dihydrobenzo[b][1,4]dioxin-5-yl)-11-(2-isopropyl-4-methylpyridin-3-yl)pyrido[2,3-d]pyrimidin-2(1H)-one (compound 98) (prepared according to Scheme 9)**

Step 43a: Preparation of tert-butyl (S)-4-(6-chloro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylat (compound 0906-98): Under the protection of nitrogen, tert-butyl (S)-4-(6,7-dichloro-1-(2-isopropyl-4-methylpyridin-3-yl)-2-oxo-1,2-dihydropyrido[2,3-d]pyrimidin-4-yl)-3-methylpiperazine-1-carboxylate (0112-73) (500 mg, 0.91 mmol, 1.0 eq.), hexamethyldistannane (629 mg, 1.92 mmol, 2.1 eq.) and tetrakis(triphenylphosphine)palladium (106 mg, 0.091 mmol, 0.1 eq.) were added to toluene (100 mL), and the mixture was stirred at 110°C for 3 h. The mixture was diluted with ethyl acetate, filtered through celite, and the filtrate was concentrated under reduced pressure. The residue was purified by column chromatography on silica gel (eluent: petroleum ether/ethyl acetate = 1/2 to 1/3) to get tert-butyl (S)-4-(6-chloro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylat (661 mg, crude) as a yellow solid . MS (ES+): m/z=677 $(M+H)^+$.

Step 43b: Preparation of tert-butyl (3S)-4-(6-chloro-7-(6-fluoro-2,3-dihydrobenzo[b][1,4]dioxin-5-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-98): Under the protection of nitrogen, 5-bromo-6-fluoro-2,3-dihydrobenzo[b][1,4]dioxine (compound 0901-98) (51.72 mg, 0.222 mmol, 1.5 eq.), tert-butyl (S)-4-(6-chloro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (compound 0906-98) (100mg, 0.148 mmol, 1.0 eq.), tetrakis(triphenylphosphine)palladium (17.1 mg, 0.0148 mmol, 0.1 eq.) were added into 10 mL toluene. The mixture was stirred at 120°C overnight. The reaction system was cooled to room temperature, and extracted with ethyl acetate and water. The organic phase was washed with water, and concentrated under vacuum. The residue was purified by column chromatography on silica gel (eluent: dichloromethane/methanol=150/1 to 30/1) to give tert-butyl (3S)-4-(6-chloro-7-(6-fluoro-2,3-dihydrobenzo[b][1,4]dioxin-5-yl)-1-(2-isopropyl-4-methylpyridin-3-yl)-2-oxo-1,2-dihydropyrido[2,3-d]pyrimidin-4-yl)-3-methylpiperazine-1-carboxylate (30 mg, yield: 30.6%) as a yellow solid. MS ($ES^+$): m/z=664 $(M+H)^+$.

Step 43c: Preparation of 6-chloro-7-(6-fluoro-2,3-dihydrobenzo[b][1,4]dioxin-5-yl)-1-(2-isopropyl-4-methylpyridin-3-yl)-4-((S)-2-methylpiperazin-1-yl)pyrido[2,3-d]pyrimidin-2(1H)-one (compound 0905-98): tert-butyl (3S)-4-(6-chloro-7-(6-fluoro-2,3-dihydrobenzo[b][1,4]dioxin-5-yl)-1-(2-isopropyl-4-methylpyridin-3-yl)-2-oxo-1,2-dihydropyrido[2,3-d]pyrimidin-4-yl)-3-methylpiperazine-1-carboxylate (compounds 0904-98) (30 mg, 0.045 mmol, 1.0 eq.) was dissolved in 6 mL dichloromethane and TFA (1mL) was added dropwise. The mixture was stirred at room temperature for 1 h and was concentrated under vacuum to dryness. The residue was used directly in the next step without further purification. MS (ES+): m/z=564 $(M+H)^+$.

Step 43d: Preparation of 4-((S)-4-acryloyl-2-methylpiperazin-1-yl)-6-chloro-7-(6-fluoro-2,3-dihydrobenzo[b][1,4]dioxin-5-yl)-1-(2-isopropyl-4-methylpyridin-3-yl)pyrido[2,3-d]pyrimidin-2(1H)-one (compound 98): The crude 6-chloro-7-(6-fluoro-2,3-dihydrobenzo[b][1,4]dioxin-5-yl)-1-(2-isopropyl-4-methylpyridin-3-yl)-4-((S)-2-methylpiperazin-1-yl)pyrido[2,3-d]pyrimidin-2(1H)-one (compound 0905-98) was dissolved in 10 mL tetrahydrofuran, and 2.0 mL DIPEA was then added. The reaction was cooled in an ice-water bath and acryloyl chloride (8.1 mg, 0.09 mmol, 2.0 eq.), pre-dissolved in 0.5 mL tetrahydrofuran, was added dropwise into the mixture above. The mixture was stirred for 0.5 h and water and ethyl acetate were added to extract the product. The organic phase was washed with water and concentrated in vacuo. The residue was purified by prep-TLC (silica gel, eluent: dichloromethane/methanol=12/1) to give 4-((S)-4-acryloyl-2-methylpiperazin-1-yl)-6-chloro-7-(6-fluoro-2,3-dihydrobenzo[b][1,4]dioxin-5-yl)-1-(2-isopropyl-4-methylpyridin-3-yl)pyrido[2,3-d]pyrimidin-2(1H)-one(20 mg, yield: 71.9%) as a yellow solid . MS (ES+): m/z=618 (M+H)+. Melting point: 155~158°C; $^1$H NMR (500 MHz, DMSO) $\delta$ 8.50 - 8.30 (m, 2H), 7.18 (d, *J* = 3.6 Hz, 1H), 6.94 (dd, *J* = 9.1, 5.5 Hz, 1H), 6.89 - 6.79 (m, 1H), 6.74 (t, *J* = 9.0 Hz, 1H), 6.20 (d, *J* = 16.2 Hz, 1H), 5.76 (dd, *J* = 10.5, 2.1 Hz, 1H), 4.94 (d, *J* = 27.4 Hz, 1H), 4.34 (d, *J* = 38.4Hz, 2H), 4.22 - 3.96 (m, 5H), 3.87 - 3.51 (m, 2H), 3.13 (d, *J* = 10.1 Hz, 1H), 2.71 (s, 1H), 1.95 - 1.83 (m, 3H), 1.34 (dd, *J* = 12.9, 6.5 Hz, 3H), 1.07 (dd, *J* = 6.6, 2.9 Hz, 3H), 0.92 (t, *J* = 7.1 Hz, 3H).

**Example 44: Preparation of 4-((S)-4-acryloyl-2-ethylpiperazin-1-yl)-6-chloro-7-(6-fluorobenzofuran-7-yl)-1-(2-isopropyl-4-methylpyridin-3-yl)pyrido[2,3-d]pyrimidin-2(1H)-one (compound 99) (prepared according to Scheme 9)**

Step 44a: Preparation of tert-butyl (S)-4-(6,7-dichloro-1-(2-isopropyl-4-methylpyridin-3-yl)-2-oxo-1,2-dihydropyrido[2,3-d]pyrimidin-4-yl)-3-ethylpiperazine-1-carboxylate (compound 0112-99): Under the protection of nitrogen, a mixture of 6,7-dichloro-1-(2-isopropyl-4-methylpyridin-3-yl)-415-pyrido[3,2-e]pyrimidine-2,4(1H)-dione (400 mg, 1.09 mmol, 1.0 eq.), DIPEA (703 mg, 5.45 mmol, 5.0 eq.) and phosphorus oxychloride (500 mg, 3.27 mmol, 3.0 eq.) in acetonitrile (12 mL) was stirred at 85°C for 2 h. After cooling to room temperature, the solvent was removed in vacuo. The residue was dissolved in tetrahydrofuran (10 mL), DIPEA (422 mg, 3.27 mmol, 3.0 eq.) and tert-butyl (S)-3-ethylpiperazine-1-carboxylate (135 mg, 1.09 mmol, 1.0 eq.) were added in an ice_-water bath, and the mixture was stirred for 1 h. ethyl acetate was added and the mixture was washed with water and brine. The organic layer was concentrated in vacuo. The residue was purified by column chromatography on silica gel (eluent: dichloromethane/methanol=150/1 to 60/1) to give tert-butyl (S)-4-(6,7-dichloro-1-(2-isopropyl-4-methylpyridin-3-yl)-2-oxo-1,2-dihydropyrido[2,3-d]pyrimidin-4-yl)-3-ethylpiperazine-1-carboxylate (360 mg, yield: 59.0% as a yellow solid). MS (ES+): m/z=561 $(M+H)^+$.

Step 44b: Preparation of tert-butyl (3S)-4-(6-chloro-7-(6-fluorobenzofuran-7-yl)-1-(2-isopropyl-4-methylpyridin-3-yl)-2-oxo-1,2-dihydropyrido[2,3-d]pyrimidin-4-yl)-3-ethylpiperazine-1-carboxylate (compound 0904-99): Under the protection of nitrogen, trifluoro(6-fluorobenzofuran-7-yl)borate (compound 0907-89) (195mg, 0.803 mmol, 3.0 eq.), tert-butyl (S)-4-(6,7-dichloro-1-(2-isopropyl-4-methylpyridin-3-yl)-2-oxo-1,2-dihydropyrido[2,3-d]pyrimidin-4-yl)-3-ethylpiperazine-1-carboxylate (0112-99) (150mg, 0.26 mmol, 1.0 eq.), tetrakis(triphenylphosphine)palladium (30 mg, 0.026 mmol, 0.1 eq.), and sodium bicarbonate (26 mg, 0.312 mmol, 1.2 eq.) were added into a mixed solvent of 10 mL acetonitrile and 2 mL water. The mixture was stirred at 80°C overnight. The reaction solution was cooled to room temperature, and extracted with ethyl acetate. The organic phase was washed with water and concentrated under vacuum. The residue was purified by column chromatography on silica gel (eluent: dichloromethane/methanol=150/1 to 20/1) to give tert-butyl (3S)-4-(6-chloro-7-(6-fluorobenzofuran-7-yl)-1-(2-isopropyl-4-methylpyridin-3-yl)-2-oxo-1,2-dihydropyrido[2,3-d]pyrimidin-4-yl)-3-ethylpiperazine-1-carboxylate (168 mg, yield: 94.9%) as a yellow solid. MS $(ES^+)$: m/z=661 $(M+H)^+$.

Step 44c: Preparation of 6-chloro-4-((S)-2-ethylpiperazin-1-yl)-7-(6-fluorobenzofuran-7-yl)-1-(2-isopropyl-4-methylpyridin-3-yl)pyrido[2,3-d]pyrimidin-2(1H)-one (compound 0905-99): tert-butyl (3S)-4-(6-chloro-7-(6-fluorobenzofuran-7-yl)-1-(2-isopropyl-4-methylpyridin-3-yl)-2-oxo-1,2-dihydropyrido[2,3-d]pyrimidin-4-yl)-3-ethylpiperazine-1-carboxylate (compound 0904-99) (168mg, 0.25mmol, 1.0 eq.) was dissolved in 6 mL dichloromethane, and TFA (1mL) was added dropwise. The mixture was stirred at room temperature for 2 h. The reaction solution was concentrated under vacuum to dryness. The residue was used directly in the next step without further purification. MS $(ES^+)$: m/z=561 $(M+H)^+$.

Step 44d: Preparation of 4-((S)-4-acryloyl-2-ethylpiperazin-1-yl)-6-chloro-7-(6-fluorobenzofuran-7-yl)-1-(2-isopropyl-4-methylpyridin-3-yl)pyrido[2,3-d]pyrimidin-2(1H)-one (compound 99): The crude 6-chloro-4-((S)-2-ethylpiperazin-1-yl)-7-(6-fluorobenzofuran-7-yl)-1-(2-isopropyl-4-methylpyridin-3-yl)pyrido[2,3-d]pyrimidin-2(1H)-one (compound 0905-99) was dissolved in 10 mL tetrahydrofuran, and 2.0 mL DIPEA was added. The reaction was cooled in an ice-water bath and acryloyl chloride (33.9 mg, 0.37 mmol, 1.5 eq.), pre-dissolved in 0.5 mL tetrahydrofuran, was added dropwise into the mixture above. The mixture was stirred for 0.5 h. Water and ethyl acetate were added to extract the product. The organic phase was washed with water and concentrated in vacuo. The residue was purified by prep-TLC (silica gel, eluent: dichloromethane/methanol=12/1) to give 4-((S)-4-acryloyl-2-ethylpiperazin-1-yl)-6-chloro-7-(6-fluorobenzofuran-7-yl)-1-(2-isopropyl-4-methylpyridin-3-yl)pyrido[2,3-d]pyrimidin-2(1H)-one (60 mg, yield: 53.5%) as a yellow solid. MS ($ES^+$): m/z=615 $(M+H)^+$. Melting point: 115~120°C; $^1H$ NMR (500 MHz, DMSO) δ 8.54 (d, *J* = 16.2 Hz, 1H), 8.32 (d, *J* = 4.5 Hz, 1H), 7.94 (d, *J* = 22.4 Hz, 1H), 7.75 (dd, *J* = 8.0, 5.3 Hz, 1H), 7.23 (t, *J* = 9.3 Hz, 1H), 7.14 (s, 1H), 7.00 (s, 1H), 6.86 (s, 1H), 6.20 (d, *J* = 11.6 Hz, 1H), 5.76 (d, *J* = 10.4 Hz, 1H), 4.81 (d, *J* = 23.6 Hz, 1H), 4.37 (m, 3H), 3.68 (d, *J* = 60.2 Hz, 2H), 3.04 (s, 1H), 2.72 (t, *J* = 37.5 Hz, 1H), 1.83 (m, 5H), 1.06 (t, *J* = 5.9 Hz, 3H), 0.92 (m, 6H).

**Example 45: Preparation of 4-((S)-4-acryloyl-2-methylpiperazin-1-yl)-6-chloro-7-(4,6-difluorobenzofuran-7-yl)-1-(2-isopropyl-4-methylpyridin-3-yl)pyrido [2,3-d]pyrimidin-2(1H)-one (compound 102) (prepared according to Scheme 9)**

Step 45a: 7-bromo-4,6-difluorobenzofuran (compound 0901-102): Under nitrogen, 2-bromo-3,5-difluorophenol (911 mg, 4.35 mmol, 1 eq.), 2-bromo-1,1-diethoxyethane (1.03 g, 5.23 mmol, 1.2 eq.), potassium carbonate (900 mg, 6.525 mmol, 1.5 eq.) and potassium iodide (91.1 mg, W=10%) were added into DMF (20 mL). The mixture was stirred at 120°C for 16h. The reaction was cooled to room temperature and extracted with ethyl acetate. The organic phase was washed with brine and dried over sodium sulfate. The organic phase was concentrated to give 2-bromo-1-(2,2-diethoxyethoxy)-3,5-difluorobenzene (1.47 g, crude) as a yellow oil. MS (ESI): *m*/*z* 325$(M+H)^+$. Under nitrogen, 2-bromo-1-(2,2-diethoxyethoxy)-3,5-difluorobenzene(1.42 g, 4.35 mmol, 1 eq.) prepared above, and PPA(1.46 g, 4.35 mmol, 1 eq.) were added into 1,2-dichloroethane (20 mL). The mixture was stirred at 85°C for 16 h. The reaction was cooled to room temperature. The mixture was extracted with ethyl acetate. The organic phase was washed with brine and dried over sodium sulfate. The organic phase was concentrated and purified by column chromatography on silica gel ( eluent: Petroleum ether) to give 7-bromo-4,6-difluorobenzofuran (650 mg, 64.35%) as a white solid. MS (ESI): *m*/*z* 233$(M+H)^+$.

Step 45b. Preparation of 2-(4,6-difluorobenzofuran-7-yl)-4,4,5,5-tetramethyl-1,3,2-dioxaborolane (compound 0903-102): Under the protection of nitrogen, 7-bromo-4,6-difluorobenzofuran (compound 0901-102) (309 mg, 1.33 mmol, 1.0 eq.) was dissolved in 15 mL dry toluene. The mixture was cooled to -75°C, and 1.6M n-BuLi in n-hexane solution (1 mL, 1.6 mmol, 1.2 eq.) was added dropwise. The mixture was stirred at -75°C for 2.0 h. Trimethyl borate (180 mg, 1.73 mmol, 1.3 eq.) was dissolved in dry toluene, and added dropwise into the mixture above. The mixture was stirred at -75°C for 1.0 h. The PH of the mixture was adjust to 1 with 2M HCl solution, and the mixture was warmed up to room temperature. ethyl acetate was added to extract. The organic phase was concentrated under vacuum to give a yellow solid (200 mg, crude). The solid (200 mg, 1.015 mmol, 1.0 eq.), pinacol (144 mg, 1.218 mmol, 1.2 eq.) and anhydrous magnesium sulfate (183 mg, 1.52 mmol, 1.5 eq.) were added into ethyl acetate. The mixture was stirred at room temperature for 2 h and was filtered through a celite pad. The filtrate was concentrated under vacuum to give 2-(4,6-difluorobenzofuran-7-yl)-4,4,5,5-tetramethyl-1,3,2-dioxaborolane (291 mg, crude) as a white solid. MS ($ES^+$): *m*/*z*=281$(M+H)^+$.

Step 45c: tert-butyl (3S)-4-(6-chloro-7-(4,6-difluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-102): Under nitrogen, tert-butyl (S)-4-(6,7-dichloro-1-(2-isopropyl-4-methylpyridin-3-yl)-2-oxo-1,2-dihydropyrido[2,3-d]pyrimidin-4-yl)-3-methylpiperazine-1-carboxylate (0112-73) (100 mg, 0.183 mmol, 1 eq.), 2-(4,6-difluorobenzofuran-7-yl)-4,4,5,5-tetramethyl-1,3,2-dioxaborolane (compound 0903-102) (102 mg, 0.366 mmol,2 eq.), tetrakis(triphenylphosphine)palladium (21 mg, 0.018 mmol, 0.1 eq.), copper(I) chloride (18 mg, 0.018 mmol, 0.1 eq.) and sodium carbonate (38.8 mg, 0.366 mmol,2 eq.) were added into the mixed solvent of acetonitrile (10 mL) and water(1 mL). The mixture was stirred at 80°C for 16h and was then cooled to room temparature. The mixture was extracted with ethyl acetate. The organic phase was washed with brine, dried over anhydrous sodium sulfate, and concentrated under vacuum. The residue was purified by column chromatography on silica gel ( eluent: dichloromethane/methanol=100/1-30/1) to give tert-butyl (3S)-4-(6-chloro-7-(4,6-difluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (87 mg, 71.9%) as a yellow solid. MS (ESI): *m*/*z* 665$(M+H)^+$.

Step 45d. Preparation of 4-((S)-4-acryloyl-2-methylpiperazin-1-yl)-6-chloro-7-(4,6-difluorobenzofuran-7-yl)-1-(2-isopropyl-4-methylpyridin-3-yl)pyrido[2,3-d]pyrimidin-2(1H)-one (compound 102): tert-butyl (3S)-4-(6-chloro-7-(4,6-difluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-102) (87 mg, 0.131 mmol, 1.0 eq.) was dissolved in 4 mL dichloromethane, and 1 mL TFA was added into the mixture. The mixture was stirred at room temperature for 2h and then concentrated. The residue was dissolved in tetrahydrofuran (5 mL) and DIPEA (50 mg, 0.393 mmol, 3 eq.) was then added into the mixture followed by the addition of acryloyl chloride (12 mg, 0.131 mmol, 1.0 eq.). The mixture was stirred at room temperature for 15 min. The reaction was quenched with ammonium hydroxide and was extracted with dichloromethane. The organic phase was concentrated and purified by prep-TLC (silica gel, eluent: ethyl acetate/methanol=12/1) to give

4-((S)-4-acryloyl-2-methylpiperazin-1-yl)-6-chloro-7-(4,6-difluorobenzofuran-7-yl)-1-(2-isopropyl-4-methylpyridin-3-yl)pyrido[2,3-d]pyrimidin-2(1H)-one (75 mg, 92.59%) as a yellow solid. MS (ESI): *m/z* 619(M+H)$^+$. $^1$H NMR (500 MHz, DMSO) δ 8.54 (d, *J* = 14.4 Hz, 1H), 8.34 (d, *J* = 4.8 Hz, 1H), 8.02 (d, *J* = 20.6 Hz, 1H), 7.32 (t, *J* = 10.2 Hz, 1H), 7.14 (dd, *J* = 15.0, 3.6 Hz, 2H), 6.92 - 6.78 (m, 1H), 6.19 (dd, *J* = 15.0, 11.8 Hz, 1H), 5.76 (dd, *J*= 10.4, 2.2 Hz, 1H), 4.97 (d, *J* = 32.7 Hz, 1H), 4.32 (dd, *J* = 34.7, 17.3 Hz, 2H), 4.09 (dd, *J* = 58.4, 10.4 Hz, 1H), 3.88 - 3.46 (m, 2H), 3.27 - 3.02 (m, 1H), 2.74 (d, *J* = 6.1 Hz, 1H), 1.94 (s, 3H), 1.35 (dd, *J* = 14.5, 6.7 Hz, 3H), 1.06 (d, *J* = 6.7 Hz, 3H), 0.91 (d, *J* = 6.7 Hz, 3H).

**Example 46: Preparation of 4-((S)-4-acryloyl-2-methylpiperazin-1-yl)-6-chloro-7-(5,6-difluorobenzofuran-7-yl)-1-(2-isopropyl-4-methylpyridin-3-yl)pyrido[2,3-d]pyrimidin-2(1H)-one (compound 103) (prepared according to Scheme 9)**

Step 46a: Preparation of tert-butyl (3S)-4-(6-chloro-7-(5,6-difluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-103): Under nitrogen, tert-butyl (S)-4-(6-chloro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (compound 0906-98) (150 mg, 0.221 mmol, 1 eq.), 5,6-difluoro-7-iodobenzofuran (compound 0901-74) (121 mg, 0.442 mmol, 2 eq.), CuI (4 mg, 0.02 mmol, 0.1 eq.) and tetrakis(triphenylphosphine)palladium (25 mg, 0.02 mmol, 0.1 eq.) were added into toluene (10 mL). The mixture was stirred at 110°C for 16h. The reaction was cooled to room temperature. The mixture was concentrated and purified by prep-TLC (silica gel, eluent: petroleum ether/ethyl acetate=1/3) to give tert-butyl (3S)-4-(6-chloro-7-(5,6-difluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (90 mg, 61.22%) as a yellow solid. MS (ESI): *m/z* 665(M+H)$^+$.

Step 46b: Preparation of 4-((S)-4-acryloyl-2-methylpiperazin-1-yl)-6-chloro-7-(5,6-difluorobenzofuran-7-yl)-1-(2-isopropyl-4-methylpyridin-3-yl)pyrido[2,3-d]pyrimidin-2(1H)-one (compound 103): tert-butyl (3S)-4-(6-chloro-7-(5,6-difluorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-103) (90 mg, 0.135 mmol, 1.0 eq.) was dissolved in 4 mL dichloromethane, and 1 mL TFA was then added into the mixture. The mixture was stirred at room temperature for 2h and was then concentrated. The residue was dissolved in tetrahydrofuran (5 mL), and DIPEA (53 mg, 0.406 mmol, 3 eq.) was added into the mixture followed by the addition of acryloyl chloride (12 mg, 0.135 mmol, 1.0 eq.). The mixture was stirred at room temperature for 15 min. The reaction was quenched with ammonium hydroxide and extracted with dichloromethane. The organic phase was concentrated and purified by prep-TLC (silica gel, eluent: ethyl acetate/methanol=12/1) to give 4-((S)-4-acryloyl-2-methylpiperazin-1-yl)-6-chloro-7-(5,6-difluorobenzofuran-7-yl)-1-(2-isopropyl-4-methylpyridin-3-yl)pyrido[2,3-d]pyrimidin-2(1H)-one (35 mg, 41.81%) as a yellow solid. MS (ESI): *m/z* 619(M+H)$^+$. $^1$H NMR (500 MHz, DMSO) δ 8.57 (d, *J* = 14.0 Hz, 1H), 8.34 (d, *J* = 4.9 Hz, 1H), 8.03 (d, *J* = 23.9 Hz, 1H), 7.86 (dd, *J* = 10.1, 7.9 Hz, 1H), 7.16 (d, *J* = 4.6 Hz, 1H), 7.03 (d, *J* = 2.2 Hz, 1H), 6.85 (m, 1H), 6.20 (m, 1H), 5.77 (dd, *J* = 10.4, 2.3 Hz, 1H), 4.99 (d, *J* = 31.7 Hz, 1H), 4.36 (m, 2H), 4.16 (d, *J* = 13.2 Hz, 1H), 3.75 (m, 2H), 3.16 (dd, *J* = 39.9, 28.8 Hz, 1H), 2.75 (m, 1H), 1.94 (s, 3H), 1.36 (dd, *J* = 13.8, 6.7 Hz, 3H), 1.07 (d, *J* = 6.7 Hz, 3H), 0.92 (d, *J* = 6.5 Hz, 3H).

**Example 47: Preparation of 4-((2S,5R)-4-acryloyl-2,5-dimethylpiperazin-1-yl)-6-chloro-7-(5,6-difluorobenzofuran-7-yl)-1-(2-isopropyl-4-methylpyridin-3-yl)pyrido[2,3-d]pyrimidin-2(1H)-one (compound 104) (prepared according to Scheme 9)**

Step 47a: tert-butyl (2R,5S)-4-(6-chloro-7-(5,6-difluorobenzofuran-7-yl)-1-(2-isopropyl-4-methylpyridin-3-yl)-2-oxo-1,2-dihydropyrido[2,3-d]pyrimidin-4-yl)-2,5-dimethylpiperazine-1-carboxylate (compound 0904-104): Under nitrogen, tert-butyl (2R,5S)-4-(6-chloro-1-(2-isopropyl-4-methylpyridin-3-yl)-2-oxo-7-(trimethylstannyl)-1,2-dihydropyrido[2,3-d]pyrimidin-4-yl)-2,5-dimethylpiperazine-1-carboxylate (0906-104) (150 mg, 0.217 mmol, 1 eq.), 5,6-difluoro-7-iodobenzofuran (0901-74) (121 mg, 0.434 mmol, 2 eq.), CuI (4 mg, 0.02 mmol, 0.1 eq.) and tetrakis(triphenylphosphine)palladium (25 mg, 0.02 mmol, 0.1 eq.) were added into toluene (10 mL). The mixture was stirred at 110°C for 16h. The reaction was cooled to room temperature andwas concentrated and purified by prep-TLC (silica gel, eluent: petroleum ether/ethyl acetate=1/3) to give tert-butyl (2R,5S)-4-(6-chloro-7-(5,6-difluorobenzofuran-7-yl)-1-(2-isopropyl-4-methylpyridin-3-yl)-2-oxo-1,2-dihydropyrido[2,3-d]pyrimidin-4-yl)-2,5-dimethylpiperazine-1-carboxylate (95 mg, 64.62%) as a yellow solid. MS (ESI): *m/z* 679(M+H)$^+$.

Step 47b: Preparation of 4-((2S,5R)-4-acryloyl-2,5-dimethylpiperazin-1-yl)-6-chloro-7-(5,6-difluorobenzofuran-7-yl)-1-(2-isopropyl-4-methylpyridin-3-yl)pyrido[2,3-d]pyrimidin-2(1H)-one (compound 104): tert-butyl (2R,5S)-4-(6-chloro-7-(5,6-difluorobenzofuran-7-yl)-1-(2-isopropyl-4-methylpyridin-3-yl)-2-oxo-1,2-dihydropyrido[2,3-d]pyrimidin-4-yl)-2,5-dimethylpiperazine-1-carboxylate (0904-104) (95 mg, 0.14 mmol, 1.0 eq.) was dissolved in 4 mL dichloromethane, and 1 mL TFA was then added into the mixture. The mixture was stirred at room temperature for 2h and concentrated. The residue was dissolved in tetrahydrofuran (5 mL) and DIPEA (54 mg, 0.42 mmol, 3 eq.) was added into the mixture followed by the addition of acryloyl chloride (13 mg, 0.14 mmol, 1.0 eq.). The mixture was stirred at room temperature

for 15 min and was then quenched with ammonium hydroxide and extracted with dichloromethane. The organic phase was concentrated and purified by prep-TLC (silica gel, eluent: ethyl acetate/methanol=12/1) to give 4-((2S,SR)-4-acryloyl-2,5-dimethylpiperazin-1-yl)-6-chloro-7-(5,6-difluorobenzofuran-7-yl)-1-(2-isopropyl-4-methylpyridin-3-yl)pyrido[2,3-d] pyrimidin-2(1H)-one (50 mg, 56.46%) as a yellow solid. MS (ESI): *m/z* 633(M+H)$^+$. $^1$H NMR (500 MHz, DMSO) δ 8.58 (dd, *J* = 16.2, 8.0 Hz, 1H), 8.47 (dd, *J* = 8.7, 5.3 Hz, 1H), 8.06 (s, 1H), 7.87 (t, *J* = 8.9 Hz, 1H), 7.45 (d, *J* = 15.4 Hz, 1H), 7.03 (d, *J* = 2.1 Hz, 1H), 6.84 (m, 1H), 6.19 (d, *J* = 16.7 Hz, 1H), 5.75 (m, 1H), 4.73 (m, 2H), 4.10 (m, 4H), 2.87 (m, 1H), 2.05 (s, 3H), 1.36 (m, 3H), 1.25 (ddd, *J* = 22.0, 10.8, 7.0 Hz, 3H), 1.14 (dd, *J* = 19.8, 11.5 Hz, 3H), 0.99 (d, *J* = 6.8 Hz, 3H).

**Example 48: Preparation of 4-((S)-4-acryloyl-2-methylpiperazin-1-yl)-6-chloro-7-(6-chlorobenzofuran-7-yl)-1-(2-isopropyl-4-methylpyridin-3-yl)pyrido[2,3-d]pyrimidin-2(1H)-one (compound 105) (prepared according to Scheme 9)**

Step 48a: Preparation of 7-bromo-6-chlorobenzofuran (compound 0901-105): Under the protection of nitrogen, 2-bromo-3-chlorophenol (1.0g, 4.83 mmol, 1.0 eq.) was dissolved in DMF(20 mL), and 2-bromo-1, 1-diethoxyethane (1.43g, 7.24 mmol, 1.5 eq.) and potassium carbonate (999mg, 7.24 mmol, 1.5 eq.) were added. The mixture was stirred at 120°C overnight. The reaction solution was cooled to room temperature, poured into 15 mL water and extracted with ethyl acetate. The organphase was washed with saturated sodium chloride solution and concentrated under vacuum. The residue was used directly in the next step without further purification. Under the protection of nitrogen, the crude 2-bromo-1-chloro-3-(2,2-diethoxyethoxy)benzene obtained above was dissolved in 30 mL 1,2-dichloroethane, and polyphosphoric acid (1.95 g, 5.79 mmol, 1.2 eq.) was then added. The mixture was stirred at 85°C overnight. Water was added to the reaction solution and the mixture was extracted with dichloromethane. The organic phase was dried and concentrated under vacuum. The residue was purified by column chromatography on silica gel (petroleum ether) to give 7-bromo-6-chlorobenzofuran as a yellow oil (500 mg, yield: 45.0%) as a yellow oil.

Step 48b: Preparation of 2-(6-chlorobenzofuran-7-yl)-4,4,5,5-tetramethyl-1,3,2-dioxaborolane (compound 0903-105): Under the protection of nitrogen, to a solution of 7-bromo-6-chlorobenzofuran (0901-105) (450 mg, 1.95 mmol, 1.0 eq.) in tuluene (15 mL) was added n-butyllithium (1.01 ml, 2.53 mmol, 1.3 eq.) dropwise at -70 °C. The mixture was stirred at -70 °C for 1.0 h and then 2-methoxy-4,4,5,5-tetramethyl-1,3,2-dioxaborolane (462 mg, 2.92mmol, 1.5 eq.) in tuluene (2 mL) was added to the reaction solution at -70 °C and stirred for 2 h. Water was added to the reaction solution and the mixture was extracted with ethyl acetate. The organic phase was concentrated under vacuum to dryness. The residue was used directly in the next step without further purification.

Step 48c: Preparation of tert-butyl (3S)-4-(6-chloro-7-(6-chlorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-105): Under the protection of nitrogen, 2-(6-chlorobenzofuran-7-yl)-4,4,5,5-tetramethyl-1,3,2-dioxaborolane (0903-105) (219mg, 0.789 mmol, 3.0 eq.), tert-butyl (S)-4-(6,7-dichloro-1-(2-isopropyl-4-methylpyridin-3-yl)-2-oxo-1,2-dihydropyrido[2,3-d]pyrimidin-4-yl)-3-methylpiperazine-1-carboxylate (0112-73) (150 mg, 0.263 mmol, 1.0 eq.), tetrakis(triphenylphosphine)palladium (30.41 mg, 0.0263 mmol, 0.1 eq.), and sodium bicarbonate (44.18 mg, 0.526 mmol, 2.0 eq.) were added into a mixed solvent of 10 mL acetonitrile and 2 mL water. The mixture was stired at 80°C overnight. The reaction solution was cooled to room temperature and extracted with ethyl acetate. The organic phase was washed with water and concentrated under vacuum. The residue was purified by column chromatography on silica gel (eluent: dichloromethane/methanol=100/1 to 20/1) to give tert-butyl (3S)-4-(6-chloro-7-(6-chlorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (140 mg, yield: 80.4%) as a yellow solid. MS (ES$^+$): m/z=662 (M+H)$^+$.

Step 48d: Preparation of 6-chloro-7-(6-chlorobenzofuran-7-yl)-1-(2-isopropyl-4-methylpyridin-3-yl)-4-((S)-2-methylpiperazin-1-yl)pyrido[2,3-d]pyrimidin-2(1H)-one (compound 0905-105): tert-butyl (3S)-4-(6-chloro-7-(6-chlorobenzofuran-7-yl)- 1-(2-isopropyl-4-methylpyridin-3-yl)-2-oxo-1,2-dihydropyrido[2,3-d]pyrimidin-4-yl)-3-methylpiperazine-1-carboxylate (0904-105) (140 mg, 0.211 mmol, 1.0 eq.) was dissolved in 6 mL dichloromethane, and TFA (1mL) was then added dropwise. The mixture was stirred at room temperature for 1 h. The reaction solution was concentrated under vacuum to dryness to abtain a residue which was used directly in the next step without further purification. MS (ES$^+$): m/z=562(M+H)$^+$.

Step 48e: Preparation of 4-((S)-4-acryloyl-2-methylpiperazin-1-yl)-6-chloro-7-(6-chlorobenzofuran-7-yl)-1-(2-isopropyl-4-methylpyridin-3-yl)pyrido[2,3-d]pyrimidin-2(1H)-one (compound 105): The crude 6-chloro-7-(6-chlorobenzofuran-7-yl)-1-(2-isopropyl-4-methylpyridin-3-yl)-4-((S)-2-methylpiperazin-1-yl)pyrido[2,3-d]pyrimidin-2(1H)-one (0905-105) was dissolved in 10 mL tetrahydrofuran, and 2.0 mL DIPEA was then added. The reaction was cooled in an ice-water bath and acryloyl chloride (38.1 mg, 0.422 mmol, 2.0 eq.), pre-dissolved in 0.5 mL tetrahydrofuran, was added dropwise into the mixture above. The mixture was stirred for 0.5 h. Water and ethyl acetate was added to extract the product. The organic phase was washed with water and concentrated in vacuo. The residue was purified by prep-TLC (silica gel, eluent: dichloromethane/methanol=12/1) to give 4-((S)-4-acryloyl-2-methylpiperazin-1-yl)-6-chloro-7-(6-chlorobenzofuran-7-yl)-1-(2-isopropyl-4-methylpyridin-3-yl)pyrido[2,3-d]pyrimidin-2(1H)-one (45 mg, yield: 34.6%) as a yellow solid. MS ($ES^+$): m/z=616 $(M+H)^+$. Melting point: 169~174°C; $^1H$ NMR (500 MHz, DMSO) δ 8.56 (d, *J* = 15.4 Hz, 1H), 8.33 (t, *J*= 4.5 Hz, 1H), 7.96 (d, *J*= 20.2 Hz, 1H), 7.75 (d, *J*= 8.4 Hz, 1H), 7.41 (d, *J* = 8.3 Hz, 1H), 7.16 (dd, *J* = 24.4, 4.3 Hz, 1H), 7.03 (d, *J* = 3.1 Hz, 1H), 6.86 (d, *J* = 11.3 Hz, 1H), 6.21 (d, *J* = 15.9 Hz, 1H), 5.77 (d, *J* = 10.1 Hz, 1H), 4.98 (d, *J* = 40.1 Hz, 1H), 4.50 - 4.23 (m, 2H), 4.09 (d, *J* = 56.1 Hz, 1H), 3.74 (dd, *J* = 66.5, 21.4 Hz, 2H), 3.20 - 3.10 (m, 1H), 2.77 (d, *J*= 6.2 Hz, 1H), 1.92 (s, 3H), 1.43 - 1.31 (m, 3H), 1.07 (d, *J*= 5.7 Hz, 3H), 0.99 - 0.84 (m, 3H).

**Example 49: Preparation of 4-((S)-4-acryloyl-2-methylpiperazin-1-yl)-6-fluoro-7-(6-fluorobenzofuran-7-yl)-1-(2-isopropyl-6-methylphenyl)pyrido[2,3-d]pyrimidin-2(1H)-one (compound 106) (prepared according to Scheme 9)**

Step 49a: Preparation of 2-isopropyl-6-methylaniline (compound 0103-106): Under the protection of nitrogen, a mixture of 2-bromo-6-methylaniline (1.86 g, 10.0 mm mol, 1.0 eq.), 4,4,5,5-tetramethyl-2-(prop-1-en-2-yl)-1,3,2-dioxaborolane (5.04 g, 30.0 mmol , 3.0 eq.), sodium carbonate (2.12 g, 20.0 mmol, 2.0 eq.) and 1, 1'-bis(diphenylphosphino)ferrocene palladium(II)dichloride (0.73 g, 1.0 mmol, 0.1 eq.) in dioxane/water = 5/1 (120 mL) was refluxed overnight. After cooling to room temperature, the mixture was filtered and concentrated in vacuo. The residue was purified by column chromatography on silica gel (petroleum ether:ethyl acetate=20:1) to give 2-methyl-6-(prop-1-en-2-yl)aniline (1.5 g, yield: ~100.0%) as a colorless oil. MS ($ES^+$): M/z = 148 $(M+H)^+$. A mixture of 2-methyl-6-(prop-1-en-2-yl)aniline (1.47 g, 10 mmol, 1.0 eq.) prepared above and palladium carbon (0.5 g, 34% w/w) in ethyl acetate (50 mL) was stirred at room temperature under hydrogen atmosphere overnight. The reaction solution was filtered and the filtrate was concentrated under reduced pressure to obtain 2-isopropyl-6-methylaniline (1.4 g, yield: 94%) as a pale yellow oil. MS ($ES^+$): m/z=150 $(M+H)^+$.

Step 49b: Preparation of 2,6-dichloro-5-fluoro-N-((2-isopropyl-6-methylphenyl)carbamoyl)nicotinamide (compound 0109-106): Under the protection of nitrogen, to a mixture of 2,6-dichloro-5-fluoronicotinamide (0108-1) (1.4 g, 6.7 mmol, 1.0 eq.) in tetrahydrofuran (50 mL) was added oxalyl chloride (0.94 g, 7.4 g) mmol, 1.1 eq.) at 0°C. The mixture was warmed to room temperature, and then heated to 60°C and stirred for 1 h. After cooling to room temperature, the solvent was removed under reduced pressure. The residue was dissolved in tetrahydrofuran (50 mL) and cooled to 0°C, and a solution of 2-isopropyl-6-methylaniline (0103-106) (1.1 g, 7.4 mmol, 1.1 eq.) in tetrahydrofuran was added dropwise (10 ml). The mixture was warmed to room temperature and stirred overnight. The mixture was concentrated under reduced pressure to give crude 2,6-dichloro-5-fluoro-N-((2-isopropyl-6-methylphenyl)carbamoyl)nicotinamide which was used directly in the next step without further purification. MS($ES^+$): M/z = 384(M + H)$^+$.

Step 49c: Preparation of 7-chloro-6-fluoro-1-(2-isopropyl-6-methylphenyl)pyrido[2,3-d]pyrimidine-2,4(1H,3H)-dione (compound 0110-106): Under the protection of nitrogen, to a solution of 2,6-dichloro-5-fluoro-N-((2-isopropyl-6-methylphenyl)carbamoyl)nicotinamide (compound 0109-106) (3.84 g, 10 mmol, 1.0 eq.) in DMF (22 mL) was added portionwise 60% sodium hydride (1.2 g, 30 mmol, 3.0 eq.)) in an ice-water bath and the mixture was stirred at room temperature for 1 h. The reaction solution was quenched with saturated aqueous ammonium chloride solution and stirred for 30 min. The mixture was filtered, and the solid was washed with water (10 mL*3), dried under reduced pressure to give 7-chloro-6-fluoro-1-(2-isopropyl-6-methylphenyl)pyrido[2,3-d]pyrimidine-2,4(1H,3H)-dione (1.92 g, yield: 55%) as an off-white solid. MS ($ES^+$): M/z = 348 $(M+H)^+$.

Step 49d: Preparation of tert-butyl (S)-4-(7-chloro-6-fluoro-1-(2-isopropyl-6-methylphenyl)-2-oxo-1,2-dihydropyrido[2,3-d]pyrimidin-4-yl)-3-methylpiperazine-1-carboxylate (compound 0112-106): A mixture of 7-chloro-6-fluoro-1-(2 -isopropyl-6-methylphenyl)pyridine[2,3-d]pyrimidine-2,4(1H,3H)-dione (0110-106) (1.57 g, 4.5 mmol, 1.0 eq.), DIPEA(2.9 g, 22.5 mmol, 5.0 eq.) and phosphorus oxychloride (1.38 g, 9.0 mmol, 2.0 eq.) in acetonitrile (50 mL) was stirred at 75°C for 1.5 h. After cooling to room temperature, the solvent was removed under reduced pressure. The residue was dissolved in tetrahydrofuran (50 mL), and DIPEA (1.74 g, 13.5 mmol, 3.0 eq.) and tert-butyl (S)-3-methylpiperazine-1-carboxylate (0111-1) (0.9 g, 4.5 mmol, 1.0 eq.) were then added in an ice-water bath, and the mixture was stirred for 0.5 h. Dichloromethane was added and the mixture was washed with water and brine. The organic layer was concentrated under reduced pressure. The residue was purified by silica gel (eluent: dichloromethane/methanol = 40/1) to give tert-butyl (S)-4-(7-chloro-6-fluoro-1-(2-isopropyl-6-methylphenyl)-2-oxo-1,2-dihydropyrido[2,3-d]pyrimidin-4-yl)-3-methylpiperazine-1-carboxylate (1.97 g, yield: 68.0%) as a brown solid. MS ($ES^+$): M/z = 530 $(M+H)^+$.

Step 49e: Preparation of tert-butyl (3S)-4-(6-fluoro-7-(6-fluorobenzofuran-7-yl)-1-(2-isopropyl-6-methylphenyl)-2-oxo-1,2-dihydropyrido[2,3-d]pyrimidin-4-yl)-3-methylpiperazine-1-carboxylate (compound 0904-106): Under the protection of nitrogen, 2-(6-fluorobenzofuran-7-yl)-4,4,5,5-tetramethyl-1,3,2-dioxaborolane (compound 0903-73) (248mg, 0.945 mmol, 2.0 eq.), tert-butyl (S)-4-(7-chloro-6-fluoro-1-(2-isopropyl-6-methylphenyl)-2-oxo-1,2-dihydropyrido[2,3-d] pyrimidin-4-yl)-3-methylpiperazine-1-carboxylate (compound 0112-106) (250mg, 0.472 mmol, 1.0 eq.), tetrakis(triphenylphosphine)palladium (55 mg, 0.047 mmol, 0.1 eq.), and sodium bicarbonate (79 mg, 0.945 mmol, 2.0 eq.) were added into a mixed solvent of 10 mL acetonitrile and 2 mL water. The mixture was stirred at 80°C overnight. The reaction solution was cooled to room temperature and extracted with ethyl acetate. The organic phase was washed with water and concentrated under vacuum. The residue was purified by column chromatography on silica gel (eluent: dichloromethane/methanol=150/1 to 30/1) to give tert-butyl (3S)-4-(6-fluoro-7-(6-fluorobenzofuran-7-yl)-1 -(2-isopropyl-6-methylphenyl)-2-oxo-1,2-dihydropyrido[2,3-d]pyrimidin-4-yl)-3-methylpiperazine-1-carboxylate (168 mg, yield: 64.3%) as a yellow solid. MS ($ES^+$): m/z=660 $(M+H)^+$.

Step 49f: Preparation of 6-fluoro-4-((S)-2-methylpiperazin-1-yl)-7-(6-fluorobenzofuran-7-yl)-1-(2-isopropyl-6-methylphenyl)pyrido[2,3-d]pyrimidin-2(1H)-one (compound 0905-106): tert-butyl (3S)-4-(6-chloro-7-(6-fluorobenzofuran-7-yl)-1-(2-isopropyl-6-methylphenyl)-2-oxo-1,2-dihydropyrido[2,3-d]pyrimidin-4-yl)-3-methylpiperazine-1-carboxylate (compound 0904-106) (200 mg, 0.30 mmol, 1.0 eq.) was dissolved in 6 mL dichloromethane, TFA (1mL) was then added dropwise, and the mixture was stirred at room temperature for 2 h. The reaction solution was concentrated under vacuum to dryness. The residue was used directly in the next step without further purification. MS ($ES^+$): m/z=560 $(M+H)^+$.

Step 49g: Preparation of 4-((S)-4-acryloyl-2-methylpiperazin-1-yl)-6-fluoro-7-(6-fluorobenzofuran-7-yl)-1-(2-isopropyl-6-methylphenyl)pyrido[2,3-d]pyrimidin-2(1H)-one (compound 106): 6-fluoro-4-((S)-2-methylpiperazin-1-yl)-7-(6-fluorobenzofuran-7-yl)-1-(2-isopropyl-6-methylphenyl)pyrido[2,3-d]pyrimidin-2(1H)-one (compound 0905-106) was dissolved in 10 mL tetrahydrofuran, and 2.0 mL DIPEA was then added. The reaction was cooled in an ice-water bath. Acryloyl chloride (40.7mg, 0.45 mmol, 1.5 eq.), pre-dissolved in 0.5 mL tetrahydrofuran, was added dropwise into the mixture above. The mixture was stirred for 0.5 h. Water and ethyl acetate was added to extract the product. The organic phase was washed with water and concentrated in vacuo. The residue was purified by prep-TLC (silica gel, eluent: dichloromethane/methanol=12/1) to give 4-((S)-4-acryloyl-2-methylpiperazin-1-yl)-6-fluoro-7-(6-fluorobenzofuran-7-yl)-1-(2-isopropyl-6-methylphenyl)pyrido[2,3-d]pyrimidin-2(1H)-one (10 mg, yield: 5.4%) as a yellow solid. MS ($ES^+$): m/z=614 $(M+H)^+$. $^1H$ NMR (500 MHz, DMSO) $\delta$ 8.40 (s, 1H), 7.95 (s, 1H), 7.79 (dd, $J$ = 8.4, 5.2 Hz, 1H), 7.24 (m, 3H), 7.11 (s, 1H), 7.02 (s, 1H), 6.87 (d, $J$= 10.8 Hz, 1H), 6.21 (d, $J$= 15.9 Hz, 1H), 5.77 (d, $J$ = 10.5 Hz, 1H), 4.93 (d, $J$ = 31.1 Hz, 1H), 4.35 (dd, $J$ = 39.9, 26.0 Hz, 2H), 4.08 (d, $J$ = 62.1 Hz, 1H), 3.65 (m, 2H), 3.15 (m, 1H), 2.59 (d, $J$= 6.3 Hz, 1H), 1.89 (s, 3H), 1.34 (dd, $J$ = 17.0, 6.5 Hz, 3H), 1.24 (s, 3H), 1.07 (d, $J$= 6.7 Hz, 3H), 0.94 (d, $J$= 6.5 Hz, 3H).

**Example 50: Preparation of (S)-4-(4-acryloyl-2-methylpiperazin-1-yl)-7-(benzofuran-7-yl)-6-fluoro-1-(2-isopropyl-4-methylpyridin-3-yl)pyrido[2,3-d]pyrimidin-2(1H)-one (compound 107) (prepared according to Scheme 9)**

Step 50a: Preparation of 2-(benzofuran-7-yl)-4,4,5,5-tetramethyl-1,3,2-dioxaborolane (compound 0903-107): Under the protection of nitrogen, to a solution of 7-bromobenzofuran (compound 0901-107) (300 mg, 1.53 mmol, 1.0 eq.) in tetrahydrofuran (10 mL) was added n-butyllithium (1.6 M, 1.24 ml, 1.99 mmol, 1.3 eq.) dropwise at -70°C. The mixture was stirred at -70°C for 40 min, anda solution of 2-methoxy-4,4,5,5-tetramethyl-1,3,2-dioxaborolane (387 mg, 2.45 mmol, 1.6 eq.) in tetrahydrofuran (1.5 mL) was then added dropwise at -70°C. The mixture was stirred for 1 h and the reaction was quenched by dropwise addition of aqueous ammonium chloride solution. The mixture was extracted with water and ethyl acetate. The organic phase was dried and concentrated under vacuum to obtain a . residue (0.4 g, crude) which was used directly in the next step without further purification.

Step 50b: Preparation of tert-butyl (S)-4-(7-(benzofuran-7-yl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0904-107): Under the protection of nitrogen, 2-(benzofuran-7-yl)-4,4,5,5-tetramethyl-1,3,2-dioxaborolane (0.4 g, crude), tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (100 mg, 0.19 mmol, 1.0 eq.), tetrakis(triphenylphosphine)palladium (21.8 mg, 0.019 mmol, 0.1 eq.), and sodium carbonate (40.0 mg, 0.38 mmol, 2.0 eq.) were added into a mixed solvent of 10 mL acetonitrile and 1 mL water. The mixture was stirred at 80°C overnighr. The reaction solution was cooled to room temperature, and extracted with ethyl acetate and water. The organic phase was washed with brine and concentrated under vacuum. The residue was purified by column chromatography on silica gel (eluent: dichloromethane/methanol=100/1 to 40/1) to give tert-butyl (S)-4-(7-(benzofuran-7-yl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (110 mg, yield: 94.6%) as a yellow oil. MS ($ES^+$): m/z=613$(M+H)^+$.

Step 50c: Preparation of (S)-4-(4-acryloyl-2-methylpiperazin-1-yl)-7-(benzofuran-7-yl)-6-fluoro-1-(2-isopropyl-4-methylpyridin-3-yl)pyrido[2,3-d]pyrimidin-2(1H)-one (compound 107): Tert-butyl (S)-4-(7-(benzofuran-7-yl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0904-107) (120 mg, 0.196 mmol, 1.0 eq.) was dissolved in 10 mL dichloromethane, and TFA (2 mL) was then added dropwise. The mixture was stirred at room temperature for 1 h. The reaction solution was concentrated under vacuum. The residue was dissolved in 10 mL tetrahydrofuran, and DIPEA (2 mL) was then added. The reaction was cooled in an ice-water bath and acryloyl chloride (35.4 mg, 0.39 mmol, 2.0 eq.), pre-dissolved in 0.5 mL tetrahydrofuran, was added dropwise into the mixture above. The mixture was stirred for 0.5 h. Water and ethyl acetate was added to extract the product. The organic phase was washed with brine and concentrated in vacuo. The residue was purified by prep-TLC (silica gel,dichloromethane/methanol=10/1) to give (S)-4-(4-acryloyl-2-methylpiperazin-1-yl)-7-(benzofuran-7-yl)-6-fluoro-1-(2-isopropyl-4-methylpyridin-3-yl)pyrido[2,3-d]pyrimidin-2(1H)-one (80 mg, yield: 72.0%) as a yellow solid. MS ($ES^+$): m/z=567 $(M+H)^+$. $^1H$ NMR(500 MHz, DMSO) $\delta$ 8.43 (s, 1H), 8.37 (s, 1H), 7.94 (d, $J$ = 1.7 Hz, 1H), 7.79 (d, $J$ = 7.4 Hz, 1H), 7.30 (dt, $J$ = 14.9, 7.4 Hz, 2H), 7.22 (d, $J$ = 4.6 Hz, 1H), 7.02 (d, $J$ = 1.9 Hz, 1H), 6.85 (m, 1H), 6.21 (d, $J$= 16.3 Hz, 1H), 5.77 (d, $J$= 10.4 Hz, 1H), 4.95 (s, 1H), 4.38 (dd, $J$= 39.2, 12.2 Hz, 2H), 4.09 (dd, $J$=

61.7, 12.4 Hz, 1H), 3.64 (dd, *J* = 64.5, 51.3 Hz, 2H), 3.15 (dd, *J*= 17.1, 8.2 Hz, 1H), 2.74 (d, *J* = 5.9 Hz, 1H), 1.96 (d, *J* = 4.3 Hz, 3H), 1.35 (t, *J* = 6.7 Hz, 3H), 1.08 (d, *J* = 6.4 Hz, 3H), 0.95 (dd, *J* = 6.4, 3.5 Hz, 3H).

**Example 51: Preparation of 4-((S)-4-acryloyl-2-methylpiperazin-1-yl)-6-fluoro-1-(2-isopropyl-4-methylpyridin-3-yl)-7-(6-methylbenzofuran-7-yl)pyrido[2,3-d]pyrimidin-2(1H)-one (compound 108) (prepared according to Scheme 9)**

Step 51a: Preparation of tert-butyl (3S)-4-(6-fluoro-1-(2-isopropyl-4-methylpyridin-3-yl)-7-(6-methylbenzofuran-7-yl)-2-oxo-1,2-dihydropyrido[2,3-d]pyrimidin-4-yl)-3-methylpiperazine-1-carboxylate (compound 0904-108): Under the protection of nitrogen, a mixture of tert-butyl (S)-4-(6-fluoro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (compound 0906-71) (120 mg, 0.181 mmol, 1.0 eq.), 7-bromo-6-methylbenzofuran(compound 0901-108) (77 mg, 0.362 mmol, 2.0 eq.), CuI (5 mg, 0.027 mmol, 0.15 eq.) and tetrakis(triphenylphosphine)palladium (21 mg, 0.018 mmol, 0.1 eq.) in toluene (10 mL) was refluxed overnight. After cooling to room temperature, water and ethyl acetate were added to extract and the organic layer was washed with brine. The solvent was removed in vacuo and the residue was purified by prep-TLC (silica gel, eluent: petroleum ether/ethyl acetate=1/3) to give tert-butyl (3S)-4-(6-fluoro-1-(2-isopropyl-4-methylpyiidin-3-yl)-7-(6-methylbenzofuran-7-yl)-2-oxo-1,2-dihydropyrido[2,3-d]pyrimidin-4-yl)-3-methylpiperazine-1-carboxylate (43 mg , yield: 37.7%) as a light-yellow solid. MS ($ES^+$): m/z=627 $(M+H)^+$

Step 51b: Preparation of 6-fluoro-1-(2-isopropyl-4-methylpyridin-3-yl)-7-(6-methylbenzofuran-7-yl)-4-((S)-2-methylpiperazin-1-yl)pyrido[2,3-d]pyrimidin-2(1H)-one (compound 0905-108): To a solution of tert-butyl (3S)-4-(6-fluoro-1-(2-isopropyl-4-methylpyridin-3-yl)-7-(6-methylbenzofuran-7-yl)-2-oxo-1,2-dihydropyrido[2,3-d]pyrimidin-4-yl)-3-methylpiperazine-1-carboxylate (compound 0904-108) (43 mg, 0.068 mmol, 1.0 eq.) in dichloromethane (5 mL) was added TFA (0. 5mL). The reaction was stirred at room temperature for 30 min. The mixture was concentrated in vacuo to give 6-fluoro-1-(2-isopropyl-4-methylpyridin-3-yl)-7-(6-methylbenzofuran-7-yl)-4-((S)-2-methylpiperazin-1-yl)pyrido[2,3-d]pyrimidin-2(1H)-one (32 mg, crude) as a yellow oil which was used directly in the next step without further purification. MS ($ES^+$): m/z=527 $(M+H)^+$

Step 51c: Preparation of 4-((S)-4-acryloyl-2-methylpiperazin-1-yl)-6-fluoro-1-(2-isopropyl-4-methylpyridin-3-yl)-7-(6-methylbenzofuran-7-yl)pyrido[2,3-d]pyrimidin-2(1H)-one (compound 108): To a mixture of 6-fluoro-1-(2-isopropyl-4-methylpyridin-3-yl)-7-(6-methylbenzofuran-7-yl)-4-((S)-2-methylpiperazin-1-yl)pyrido[2,3-d]pyrimidin-2(1H)-one (compound 0905-108) (30 mg, crude) and DIPEA(0.5mL) in acetonitrile(5mL) was added dropwise acryloyl chloride(7.7 mg, 0.085 mmol, 1.50 eq.). The reaction was stirred at room temperature for 30 min. Water and ethyl acetate were added to extract, and the organic layer was washed with brine and concentrated in vacuo. The residue was purified by prep-TLC (silica gel, eluent: ethyl acetate/methanol=15/1) to give 4-((S)-4-acryloyl-2-methylpiperazin-1-yl)-6-fluoro-1-(2-isopropyl-4-methylpyridin-3-yl)-7-(6-methylbenzofuran-7-yl)pyrido[2,3-d]pyrimidin-2(1H)-one (16 mg, yield: 48.4%) as a yellow solid. MS ($ES^+$): m/z=581 $(M+H)^+$, m. p: 151-163°C. $^1H$ NMR (500 MHz, DMSO) $\delta$ 8.38 (t, *J*= 12.2 Hz, 2H), 7.85 (d, *J*= 1.9 Hz, 1H), 7.61 (d, *J*= 7.9 Hz, 1H), 7.18 (dd, *J*= 25.8, 6.1 Hz, 2H), 6.90 (dd, *J*= 36.1, 7.0 Hz, 2H), 6.21 (d, *J*= 16.6 Hz, 1H), 5.83 - 5.71 (m, 1H), 4.96 (d, *J*= 47.2 Hz, 1H), 4.48 - 4.24 (m, 2H), 4.21 - 3.99 (m, 1H), 3.74 (d, *J* = 55.0 Hz, 2H), 3.14 (d, *J* = 24.8 Hz, 1H), 2.85 - 2.72 (m, 1H), 2.05 (s, 3H), 1.93 (d, *J*= 4.4 Hz, 3H), 1.35 (dd, *J*= 24.1, 6.6 Hz, 3H), 1.07 (d, *J*= 6.5 Hz, 3H), 0.88 (d, *J*= 4.4 Hz, 3H).

**Example 52: Preparation of 4-((S)-4-acryloyl-2-methylpiperazin-1-yl)-6-fluoro-1-(2-isopropyl-4-methylpyridin-3-yl)-7-(6-methoxybenzofuran-7-yl)pyrido[2,3-*d*]pyrimidin-2(1*H*)-one (compound 109) (prepared according to Scheme 9)**

Step 52a: Preparation of 7-bromo-6-methoxybenzofuran (compound 0901-109): Under the protection of nitrogen, 2-bromo-3-methoxyphenol (812 mg, 4.0 mmol, 1.0 eq.), 2-bromo-1,1-dimethoxyethane (1.01 g, 6.0 mmol, 1.5 eq.), potassium iodide (80 mg, 0.48 mmol, 0.1 eq.) and potassium carbonate (1.38 g, 10.0 mmol, 2.5 eq.) were added into DMF (7 mL), and the mixture was stirred at 120°C for 16 h. The mixture was diluted with ethyl acetate, and washed with water and brine. The organic phase was concentrated under vacuum. The residue was purified by column chromatography on silica gel (eluent: Petroleum ether/ethyl acetate=30/1 to 20/1) to give 2-bromo-1-(2,2-dimethoxyethoxy)-3-methoxybenzene (1.14 g, yield: 98%) as a yellow oil. MS ($ES^+$): *m*/*z*=291 $(M+H)^+$. Under the protection of nitrogen, 2-bromo-1-(2,2-dimethoxyethoxy)-3-methoxybenzene (1.14 g, 4.0 mmol, 1.0 eq.) obtained above, polyphosphoric acid (2.64 g, 8.0 mmol, 2.0 eq.) were added into dry toluene (35 mL), and the mixture was stirred at 120°C overnight. The mixture was diluted with ethyl acetate, and washed with water. The organic phase was concentrated under vacuum. The residue was purified by column chromatography on silica gel (eluent: Petroleum ether) to give 7-bromo-6-methoxybenzofuran (830mg, yield: 93%) as a white solid. MS ($ES^+$): *m*/*z*=227 $(M+H)^+$.

Step 52b: Preparation of (6-methoxybenzofuran-7-yl)boronic acid (compound 0902-109): Under the protection of nitrogen, 7-bromo-6-methoxybenzofuran (compound 0901-109) (713 mg, 3.16 mmol, 1.0 eq.) was dissolved in 63 mL

dry toluene. The mixture was cooled to -75°C, and 1.6M n-BuLi in n-hexane solution (2.37 mL, 3.79 mmol, 1.2 eq.) was then added dropwise. The mixture was stirred at -75°C for 2.0 h. Trimethyl borate (427 mg, 4.11 mmol, 1.3 eq.) was dissolved in dry toluene, and added dropwise into the mixture above. The mixture was stirred at -75°C for 1 h. 2.0 mL 2M sodium hydroxide solution and 20 mL water was added, and the mixture was warmed up to room temperature. ethyl acetate was added, and the layers were separated. 1M HCl solution was added into the aqueous phase to adjust the pH to 1, and ethyl acetate was added to extract. The organic phase was concentrated under vacuum to give (6-methoxybenzofuran-7-yl)boronic acid (389 mg, crude) as a brown solid. MS ($ES^+$): *m*/*z*=193 $(M+H)^+$.

Step 52c: Preparation of 2-(6-methoxybenzofuran-7-yl)-4,4,5,5-tetramethyl-1,3,2-dioxaborolane (compound 0903-109): (6-methoxybenzofuran-7-yl)boronic acid (compound 0902-109) (389 mg, 2.03 mmol, 1.0 eq.), pinacol (359 mg, 3.04 mmol, 1.5 eq.) and anhydrous magnesium sulfate (731 mg, 6.0 mmol, 3.0 eq.) was added into ethyl acetate. The mixture was stirred at room temperature for 2 h. The mixture was filtered through a celite pad, and the filtrate was concentrated under vacuum to give 2-(6-methoxybenzofuran-7-yl)-4,4,5,5-tetramethyl-1,3,2-dioxaborolane (640 mg, crude) as a yellow solid. MS ($ES^+$): *m*/*z*=275$(M+H)^+$.

Step 52d: Preparation of tert-butyl (3S)-4-(6-fluoro-1-(2-isopropyl-4-methylpyridin-3-yl)-7-(6-methoxybenzofuran-7-yl)-2-oxo-1,2-dihydropyrido[2,3-*d*]pyrimidin-4-yl)-3-methylpiperazine-1-carboxylate (compound 0904-109): Under the protection of nitrogen, 2-(6-methoxybenzofuran-7-yl)-4,4,5,5-tetramethyl-1,3,2-dioxaborolane (compound 0903-109) (640 mg, 2.03 mmol, 4.0 eq.), tert-butyl (*S*)-4-(7-chloro-6-fluoro-1-(2-isopropyl-4-methylpyridin-3-yl)-2-oxo-1,2-dihydropyrido[2,3-*d*]pyrimidin-4-yl)-3-methylpiperazine-1-carboxylate (0112-1) (265 mg, 0.5 mmol, 1.0 eq.), tetrakis(triphenylphosphine)palladium (232 mg, 0.2 mmol, 0.4 eq.), and sodium carbonate (530 mg, 5.0 mmol, 10.0 eq.) were added into a mixed solvent of 66 mL acetonitrile and 6.6 mL water. The mixture was heated at 80°C for 2.0 h. The mixture was cooled to room temperature, and extracted with ethyl acetate. The organic phase was washed with water and concentrated under vacuum. The residue was purified by column chromatography on silica gel (eluent: dichloromethane/methanol=100/1 to 30/1) to give tert-butyl (3*S*)-4-(6-fluoro-1-(2-isopropyl-4-methylpyridin-3-yl)-7-(6-methoxybenzofuran-7-yl)-2-oxo-1,2-dihydropyrido[2,3-*d*]pyrimidin-4-yl)-3-methylpiperazine-1-carboxylate (366 mg, crude) as a yellow solid. MS ($ES^+$): *m*/*z*=643$(M+H)^+$.

Step 52e: Preparation of 4-((*S*)-4-acryloyl-2-methylpiperazin-1-yl)-6-fluoro-1-(2-isopropyl-4-methylpyridin-3-yl)-7-(6-methoxybenzofuran-7-yl)pyrido[2,3-*d*]pyrimidin-2(1*H*)-one (compound 109): tert-butyl (3*S*)-4-(6-fluoro-1-(2-isopropyl-4-methylpyridin-3-yl)-7-(6-methoxybenzofuran-7-yl)-2-oxo-1,2-dihydropyrido[2,3-*d*]pyrimidin-4-yl)-3-methylpiperazine-1-carboxylate(compound 0904-109) (366 mg, 0.57 mmol, 1.0 eq.) was dissolved in 10 mL dichloromethane, and TFA (2.0 mL) was then added dropwise The mixture was stirred at room temperature for 2.0 h and was concentrated under vacuum to dryness. The residue was dissolved in 15 mL tetrahydrofuran, and DIPEA (221 mg, 1.71 mmol, 3.0 eq.) was then added. The mixture was cool in an ice-water bath, and acryloyl chloride (103 mg, 1.14 mmol, 2.0 eq.), pre-dissolved in 0.5 mL tetrahydrofuran, was added dropwise into the mixture above. The mixture was stirred for 30 min and diluted with ethyl acetate. The organic phase was washed with water and concentrated under vacuum. The residue was purified by prep-TLC (silica gel, eluent: dichloromethane/methanol= 30/1) to give 4-((*S*)-4-acryloyl-2-methylpiperazin-1-yl)-6-fluoro-1-(2-isopropyl-4-methylpyridin-3-yl)-7-(6-methoxybenzofuran-7-yl)pyrido[2,3-*d*]pyrimidin-2(1*H*)-one (200 mg, yield: 58%) as a yellow solid. MS ($ES^+$): *m*/*z*=597 $(M+H)^+$. Melting point: 156°C~158°C. $^1$H NMR (500 MHz, DMSO) $\delta$ 8.32 (dd, *J* = 14.1, 7.8 Hz, 2H), 7.76 (d, *J* = 2.1 Hz, 1H), 7.69 (d, *J* = 8.6 Hz, 1H), 7.16 (d, *J* = 4.9 Hz, 1H), 7.11 (d, *J* = 8.7 Hz, 1H), 6.88 (dd, *J* = 18.2, 7.1 Hz, 2H), 6.21 (d, *J* = 16.9 Hz, 1H), 5.77 (dd, *J* = 10.4, 2.3 Hz, 1H), 4.97 (dd, *J* = 25.5, 22.4 Hz, 1H), 4.35 (m, 2H), 4.11 (m, 1H), 3.62 (m, 5H), 3.15 (dd, *J* = 39.7, 12.1 Hz, 1H), 2.77 (m, 1H), 1.94 (d, *J* = 2.6 Hz, 3H), 1.35 (dd, *J* = 23.7, 6.7 Hz, 3H), 1.08 (d, *J* = 6.7 Hz, 3H), 0.93 (d, *J* = 6.7 Hz, 3H).

**Example 53: Preparation of 4-((S)-4-acryloyl-2-methylpiperazin-1-yl)-6-chloro-7-(6-fluoro-5-hydroxybenzofuran-7-yl)-1-(2-isopropyl-4-methylpyridin-3-yl)pyrido [2,3-d]pyrimidin-2(1H)-one (compound 110) (prepared according to Scheme 9)**

Step 53a: Preparation of 6-fluoro-7-iodobenzofuran-5-ol (compound 0901-110): Under the protection of nitrogen, to a solution of 6-fluoro-7-iodo-5-methoxybenzofuran (300 mg, 1.02 mmol, 1.0 eq.) in dichloromethane (10 mL) was added dropwise boron tribromide (386 mg, 1.54 mmol, 1.5 eq.) in an ice-water bath. After addition, the mixture was stirred at room temperature for 1 h. The reaction mixture was quenched with aqueous ammonium chloride solution and stirred for 10 min. water and dichloromethane was added to extract, and the organic layer was washed with brine and concentrated in vacuo to give 6-fluoro-7-iodobenzofuran-5-ol (240 mg, yield: 96.1%) as a white solid. MS ($ES^+$): m/z=279 $(M+H)^+$.

Step 53b: Preparation of tert-butyl (3S)-4-(6-chloro-7-(6-fluoro-5-hydroxybenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-110): Under the protection of nitrogen, a mixture of tert-butyl (S)-4-(6-chloro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (0906-98) (120 mg, 0.177 mmol, 1.0 eq.), 6-fluoro-7-iodobenzofuran-5-ol (0901-110) (98 mg, 0.355 mmol, 2.0 eq.), CuI (3.5 mg, 0.017 mmol,

0.1 eq.) and tetrakis(triphenylphosphine)palladium (20 mg, 0.017 mmol, 0.1 eq.) in toluene (15 mL) was refluxed overnight. After cooling to room temperature, water and ethyl acetate were added to extract and the organic layer was washed with brine. The solvent was removed in vacuo and the residue was purified by prep-TLC (silica gel, eluent: petroleum ether/ethyl acetate=1/2) to give tert-butyl (3S)-4-(6-chloro-7-(6-fluoro-5-hydroxybenzofuran-7-yl)-1-(2-isopropyl-4-methylpyridin-3-yl)-2-oxo-1,2-dihydropyiido[2,3-d]pyrimidin-4-yl)-3-methylpiperazine-1-carboxylate (35 mg , yield: 29.9%) as a light-yellow solid. MS ($ES^+$): m/z=663 $(M+H)^+$

Step 53c: Preparation of 6-chloro-7-(6-fluoro-5-hydroxybenzofuran-7-yl)-1-(2-isopropyl-4-methylpyridin-3-yl)-4-((S)-2-methylpiperazin-1-yl)pyrido[2,3-d]pyrimidin-2(1H)-one (compound 0905-110): To a solution of tert-butyl (3S)-4-(6-chloro-7-(6-fluoro-5-hydroxybenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (0904-110) (35 mg, 0.053 mmol, 1.00 eq.) in dichloromethane (5 mL) was added TFA (0.5mL). The mixture was stirred at room temperature for 30 min, and concentrated in vacuo to give 6-chloro-7-(6-fluoro-5-hydroxybenzofuran-7-yl)-1-(2-isopropyl-4-methylpyridin-3-yl)-4-((S)-2-methylpiperazin-1-yl)pyrido[2,3-d]pyrimidin-2(1H)-one (20 mg, crude) which was used directly in the next step without further purification. MS ($ES^+$): m/z=563 $(M+H)^+$

Step 53d: Preparation of 4-((S)-4-acryloyl-2-methylpiperazin-1-yl)-6-chloro-7-(6-fluoro-5-hydroxybenzofuran-7-yl)-1-(2-isopropyl-4-methylpyridin-3-yl)pyrido[2,3-d]pyrimidin-2(1H)-one (compound 110): To a mixture of 6-chloro-7-(6-fluoro-5-hydroxybenzofuran-7-yl)-1-(2-isopropyl-4-methylpyridin-3-yl)-4-((S)-2-methylpiperazin-1-yl)pyrido[2,3-d]pyrimidin-2(1H)-one (0905-110) (20 mg, crude) in acetonitrile (5mL) was added DIPEA (0.5mL) and followed by dropwise addition of acryloyl chloride(4.8 mg, 0.053 mmol, 1.50 eq.). The mixture was stirred at room temperature for 30 min. Water and ethyl acetate was added to extract, and the organic layer was washed with brine and concentrated in vacuo. The residue was purified by prep-TLC (silica gel, eluent: dichloromethane/methanol=10/1) to give 4-((S)-4-acryloyl-2-methylpiperazin-1-yl)-6-chloro-7-(6-fluoro-5-hydroxybenzofuran-7-yl)-1-(2-isopropyl-4-methylpyridin-3-yl)pyrido[2,3-d]pyrimidin-2(1H)-one (9 mg, yield: 40.9%) as a yellow solid. MS ($ES^+$): m/z=617 $(M+H)^+$. m. p: 92-104°C; $^1H$ NMR (500 MHz, DMSO) $\delta$ 9.85 (s, 1H), 8.52 (d, $J$ = 12.7 Hz, 1H), 8.34 (d, $J$ = 4.8 Hz, 1H), 7.82 (d, $J$ = 29.6 Hz, 1H), 7.23 (d, $J$ = 8.4 Hz, 1H), 7.15 (s, 1H), 6.86 (s, 2H), 6.21 (d, $J$= 15.6 Hz, 1H), 5.76 (dd, $J$= 10.4, 2.2 Hz, 1H), 4.97 (d, $J$ = 29.1 Hz, 1H), 4.41 - 4.29 (m, 2H), 4.14 (s, 1H), 3.76 (dd, $J$ = 40.7, 29.4 Hz, 2H), 3.18 - 3.08 (m, 1H), 2.73 (s, 1H), 1.99 - 1.89 (m, 3H), 1.35 (dd, $J$= 13.8, 6.7 Hz, 3H), 1.06 (dd, $J$ = 6.8, 3.0 Hz, 3H), 0.91 (s, 3H).

**Example 54: Preparation of 4-((S)-4-acryloyl-2-methylpiperazin-1-yl)-6-chloro-7-(6-fluoro-5-methoxybenzofuran-7-yl)-1-(2-isopropyl-4-methylpyridin-3-yl)pyrido[2,3-d]pyrimidin-2(1H)-one (compound 111) (prepared according to Scheme 9)**

Step 54a: Preparation of tert-butyl (3S)-4-(6-chloro-7-(6-fluoro-5-methoxybenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (compound 0904-111): Under the protection of nitrogen, a mixture of tert-butyl (S)-4-(6-chloro-1-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (0906-98) (70 mg, 0.103 mmol, 1.0 eq.), 6-fluoro-7-iodo-5-methoxybenzofuran (0901-111) (60 mg, 0.166 mmol, 2.5 eq.), CuI (2 mg, 0.01 mmol, 0.1 eq.) and tetrakis(triphenylphosphine)palladium (12 mg, 0.01 mmol, 0.1 eq.) in toluene (10 mL) was refluxed overnight. After cooling to room temperature, water and ethyl acetate were added to extract and the organic layer was washed with brine. The solvent was removed in vacuo and the residue was purified by prep_TLC (eluent: petroleum ether/ethyl acetate=1/2)to give tert-butyl (3S)-4-(6-chloro-7-(6-fluoro-5-methoxybenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (35 mg , yield: 50.0%) as a light-yellow solid. MS ($ES^+$): m/z=677 $(M+H)^+$

Step 54b: Preparation of 6-chloro-7-(6-fluoro-5-methoxybenzofuran-7-yl)-1-(2-isopropyl-4-methylpyridin-3-yl)-4-((S)-2-methylpiperazin-1-yl)pyrido[2,3-d]pyrimidin-2(1H)-one (compound 0905-111): To a solution of tert-butyl (3S)-4-(6-chloro-7-(6-fluoro-5-methoxybenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (0904-111) (35 mg, 0.05 mmol, 1.00 eq.) in dichloromethane (10 mL) was added TFA (1mL) The reaction was stirred at room temperature for 30 min and concentrated in vacuo to give 6-chloro-7-(6-fluoro-5-methoxybenzofuran-7-yl)-1-(2-isopropyl-4-methylpyridin-3-yl)-4-((S)-2-methylpiperazin-1-yl)pyrido[2,3-d]pyrimidin-2(1H)-one (20 mg, crude). which was used directly in the next step without purification. MS ($ES^+$): m/z=577 $(M+H)^+$

Step 54c: Preparation of 4-((S)-4-acryloyl-2-methylpiperazin-1-yl)-6-chloro-7-(6-fluoro-5-methoxybenzofuran-7-yl)-1-(2-isopropyl-4-methylpyridin-3-yl)pyrido[2,3-d]pyrimidin-2(1H)-one (compound 111): To a mixture of 6-chloro-7-(6-fluoro-5-methoxybenzofuran-7-yl)-1-(2-isopropyl-4-methylpyridin-3-yl)-4-((S)-2-methylpiperazin-1-yl)pyrido[2,3-d]pyrimidin-2(1H)-one (0905-111) (20 mg, crude) in acetonitrile (5mL) was added DIPEA(0.5mL) and followed by dropwise addition of acryloyl chloride(4.7 mg, 0.052 mmol, 1.50 eq.). The reaction was stirred at room temperature for 30 min. Water and ethyl acetate was added to extract, and the organic layer was washed with brine and concentrated in vacuo. The residue was purified by prep-TLC (silica gel, eluent: dichloromethane/methanol=10/1) to give 4-((S)-4-acryloyl-2-methylpiperazin-1-yl)-6-chloro-7-(6-fluoro-5-methoxybenzofuran-7-yl)-1-(2-isopropyl-4-methylpyridin-3-yl)pyrido[2,3-d] pyrimidin-2(1H)-one (6 mg, yield: 28.5%) as a yellow solid. MS ($ES^+$): m/z=631 $(M+H)^+$. m. p: 101-113°C; $^1H$ NMR (500 MHz, DMSO) δ 8.53 (d, *J* = 13.1 Hz, 1H), 8.34 (d, *J* = 4.8 Hz, 1H), 7.90 (d, *J* = 30.7 Hz, 1H), 7.49 (d, *J* = 8.2 Hz, 1H), 7.15 (s, 1H), 6.94 (d, *J*= 1.8 Hz, 1H), 6.86 (d, *J*= 12.8 Hz, 1H), 6.21 (d, *J*= 16.5 Hz, 1H), 5.77 (dd, *J* = 10.4, 2.3 Hz, 1H), 4.98 (d, *J*= 26.5 Hz, 1H), 4.42 - 4.30 (m, 2H), 4.06 (dd, *J* = 31.5, 27.0 Hz, 1H), 3.91 - 3.55 (m, 5H), 3.14 (d, *J* = 9.6 Hz, 1H), 2.71 (d, *J* = 20.7 Hz, 1H), 1.97 - 1.88 (m, 3H), 1.36 (dd, *J*= 13.3, 6.9 Hz, 3H), 1.06 (dd, *J*= 6.9, 1.7 Hz, 3H), 0.91 (d, *J*= 4.0 Hz, 3H).

**Example 55: Preparation of 4-((S)-4-acryloyl-2-methylpiperazin-1-yl)-7-(6-chlorobenzofuran-7-yl)-6-fluoro-1-(2-isopropyl-4-methylpyridin-3-yl)pyrido[2,3-d]pyrimidin-2(1H)-one (compound 112) (prepared according to Scheme 9)**

Step 55a: Preparation of tert-butyl (3S)-4-(7-(6-chlorobenzofuran-7-yl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (compound 0904-112): Under the protection of nitrogen, 2-(6-chlorobenzofuran-7-yl)-4,4,5,5-tetramethyl-1,3,2-dioxaborolane (0903-105) (261mg, 0.94mmol, 5.0 eq.), tert-butyl (S)-4-(7-chloro-6-fluoro-1-(2-isopropyl-4-methylpyridin-3-yl)-2-oxo-1,2-dihydropyrido[2,3-d]pyrimidin-4-yl)-3-methylpiperazine-1-carboxylate (0112-1) (100 mg, 0.188 mmol, 1.0 eq.), tetrakis(triphenylphosphine)palladium (21.77 mg, 0.019mmol, 0.1 eq.) and sodium bicarbonate (31.58 mg, 0.376 mmol, 2.0 eq.) were added into a mixed solvent of 10 mL acetonitrile and 2 mL water. The mixture was stired at 80°C overnight. The reaction solution was cooled to room temperature, and extracted with ethyl acetate. The organic phase was washed with water and concentrated under vacuum. The residue was purified by column chromatography on silica gel (eluent: dichloromethane/methanol=100/1 to 20/1) to give tert-butyl (3S)-4-(7-(6-chlorobenzofuran-7-yl)-6-fluoro-1-(2-isopropyl-4-methylpyridin-3-yl)-2-oxo-1,2-dihydropyrido[2,3-d]pyrimidin-4-yl)-3-methylpiperazine-1-carboxylate (105 mg, yield: 86.0%) as a yellow solid. MS (ES+): m/z=646 $(M+H)^+$.

Step 55b: Preparation of 7-(6-chlorobenzofuran-7-yl)-6-fluoro-1-(2-isopropyl-4-methylpyridin-3-yl)-4-((S)-2-methylpiperazin-1-yl)pyrido[2,3-d]pyrimidin-2(1H)-one (compound 0905-112): tert-butyl (3S)-4-(6-chloro-7-(6-chlorobenzofuran-7-yl)-1-(2-isopropyl-4-methylpyridin-3-yl)-2-oxo-1,2-dihydropyrido[2,3-d]pyrimidin-4-yl)-3-methylpiperazine-1-carboxylate (0904-112) (105 mg, 0.162 mmol, 1.0 eq.) was dissolved in 6 mL dichloromethane, and TFA (1mL) was then added dropwise. The mixture was stirred at room temperature for 1.0 h. The reaction solution was concentrated under vacuum to dryness to abtain a residue which was used directly in the next step without further purification. MS ($ES^+$): m/z=546$(M+H)^+$.

Step 55c: Preparation of 4-((S)-4-acryloyl-2-methylpiperazin-1-yl)-7-(6-chlorobenzofuran-7-yl)-6-fluoro-1-(2-isopropyl-4-methylpyridin-3-yl)pyrido[2,3-d]pyrimidin-2(1H)-one (compound 112): 7-(6-Chlorobenzofuran-7-yl)-6-fluoro-1-(2-isopropyl-4-methylpyridin-3-yl)-4-((S)-2-methylpiperazin-1-yl)pyrido[2,3-d]pyrimidin-2(1H)-one (0905-112) was dissolved in 10 mL tetrahydrofuran, and 2.0 mL DIPEA was then added. The reaction was cooled in an ice-water bath, and acryloyl chloride (29.3 mg, 0.324 mmol, 2.0 eq.), pre-dissolved in 0.5 mL tetrahydrofuran, was added dropwise into the mixture above. The mixture was stirred for 0.5 h. Water and ethyl acetate was added to extract the product. The organic phase was washed with water and concentrated in vacuo. The residue was purified by prep-TLC (silica gel, eluent: dichloromethane/methanol=12/1) to give 4-((S)-4-acryloyl-2-methylpiperazin-1-yl)-7-(6-chlorobenzofuran-7-yl)-6-fluoro-1-(2-isopropyl-4-methylpyridin-3-yl)pyrido[2,3-d]pyrimidin-2(1H)-one (38mg, yield: 39.1%) as a yellow solid. MS (ES+): m/z=600 (M+H)+. Melting point: 156~160°C; $^1H$ NMR (500 MHz, DMSO) δ 8.46 (dd, *J*= 20.4, 9.4 Hz, 1H), 8.34 (d, *J*= 4.8 Hz, 1H), 7.98 (s, 1H), 7.78 (d, *J*= 8.4 Hz, 1H), 7.43 (d, *J*= 8.4 Hz, 1H), 7.16 (d, *J* = 4.6 Hz, 1H), 7.05 (d, *J* = 2.2 Hz, 1H), 6.95 - 6.74 (m, 1H), 6.21 (d, *J* = 16.0 Hz, 1H), 5.77 (dd, *J* = 10.4, 2.1 Hz, 1H), 4.97 (d, *J* = 40.2 Hz, 1H), 4.35 (dd, *J* = 29.7, 13.7 Hz, 2H), 4.22 - 3.94 (m, 1H), 3.85 - 3.46 (m, 2H), 3.27 - 3.10 (m, 1H), 2.86 - 2.69 (m, 1H), 1.93 (s, 3H), 1.35 (dd, *J* = 20.2, 6.7 Hz, 3H), 1.07 (d, *J* = 6.7 Hz, 3H), 0.90 (s, 3H).

**Example 56: Preparation of (*S*,*E*)-4-(4-(but-2-enoyl)-2-methylpiperazin-1-yl)-6-fluoro-7-(6-fluorobenzofuran-5-yl)-1-(2-isopropyl-4-methylpyridin-3-yl)pyrido[2,3-*d*]pyrimidin-2(1*H*)-one (compound 113)**

(E)-But-2-enoic acid (13 mg, 0.15 mmol, 1.5 eq.), HATU (50 mg, 0.13 mmol, 1.3 eq.), and TEA (21 mg, 0.2 mmol, 2.0 eq.) were added into 10 mL tetrahydrofuran, and the mixture was stirred at room temperature for 15 min. (*S*)-6-Fluoro-7-(6-fluorobenzofuran-5-yl)-1-(2-isopropyl-4-methylpyridin-3-yl)-4-(2-methylpiperazin-1-yl)pyrido[2,3-*d*]pyrimidin-2(1*H*)-one (compound 70) (53 mg, 0.1 mmol, 1.0 eq.) was then added, and the mixture was stirred for 1 h. ethyl acetate was added and the mixture was washed with water. The organic phase was concentrated under vacuum. The residue was purified by prep-TLC (silica gel, eluent:dichloromethane/methanol=30/1) to give (*S*,*E*)-4-(4-(but-2-enoyl)-2-methylpiperazin-1-yl)-6-fluoro-7-(6-fluorobenzofuran-5-yl)-1-(2-isopropyl-4-methylpyridin-3-yl)pyrido[2,3-*d*]pyrimidin-2(1*H*)-one (37 mg, yield: 62%) as a yellow solid. MS ($ES^+$): *m/z*=599 $(M+H)^+$. Melting point: 155°C~157°C. $^1H$ NMR (500 MHz, DMSO) δ 8.35 (d, *J* = 4.7 Hz, 2H), 7.94 (s, 1H), 7.79 (dd, *J*= 8.3, 5.2 Hz, 1H), 7.26 (t, *J* = 9.5 Hz, 1H), 7.18

(d, *J* = 4.6 Hz, 1H), 7.01 (s, 1H), 6.78 (dd, *J*= 14.4, 6.8 Hz, 1H), 6.57 (s, 1H), 4.95 (d, *J* = 39.2 Hz, 1H), 4.33 (dd, *J* = 29.1, 13.1 Hz, 2H), 4.09 (d, *J*= 61.4 Hz, 1H), 3.68 (m, 2H), 3.06 (s, 1H), 2.77 (s, 1H), 1.90 (m, 6H), 1.35 (m, 3H), 1.08 (d, *J*= 6.5 Hz, 3H), 0.93 (d, *J*= 6.5 Hz, 3H).

**Example 57: Preparation of (*S,E*)-4-(4-(4-(dimethylamino)but-2-enoyl)-2-methylpiperazin-1-yl)-6-fluoro-7-(6-fluorobenzofuran-5-yl)-1-(2-isopropyl-4-methylpyridin-3-yl)pyrido[2,3-*d*]pyrimidin-2(1*H*)-one (compound 114)**

(*E*)-4-(Dimethylamino)but-2-enoic acid hydrochloride (25 mg, 0.15 mmol, 1.5 eq.), HATU (50 mg, 0.13 mmol, 1.3 eq.), and TEA (31 mg, 0.3 mmol, 3.0 eq.) were added into 10 mL tetrahydrofuran, and the mixture was stirred at room temperature for 30 min. (*S*)-6-fluoro-7-(6-fluorobenzofuran-5-yl)-1-(2-isopropyl-4-methylpyridin-3-yl)-4-(2-methylpiperazin-1-yl)pyrido[2,3-*d*]pyrimidin-2(1*H*)-one (compound 70) (53 mg, 0.1 mmol, 1.0 eq.) was then added, and the mixture was stirred for 1 h. ethyl acetate was added and the mixture was washed with water. The organic phase was concentrated under vacuum and the residue was purified by prep-TLC (silica gel, eluent:dichloromethane/methanol=30/1) to give (*S*,*E*)-4-(4-(4-(dimethylamino)but-2-enoyl)-2-methylpiperazin-1-yl)-6-fluoro-7-(6-fluorobenzofuran-5-yl)-1-(2-isopropyl-4-methylpyridin-3-yl)pyrido[2,3-*d*]pyrimidin-2(1*H*)-one (26 mg, yield: 40%) as a yellow solid. MS ($ES^+$): *m*/*z*=642 $(M+H)^+$. Melting point: 162°C to 164°C. $^1H$ NMR (500 MHz, DMSO) $\delta$ 8.41 (m, 2H), 7.95 (d, *J*= 2.1 Hz, 1H), 7.80 (dd, *J* = 8.6, 5.2 Hz, 1H), 7.26 (dd, *J* = 10.4, 8.7 Hz, 1H), 7.19 (d, *J* = 4.9 Hz, 1H), 7.01 (t, *J* = 7.9 Hz, 2H), 6.75 (m, 1H), 4.97 (d, *J*= 35.8 Hz, 1H), 4.35 (m, 2H), 4.11 (dd, *J*= 59.8, 12.8 Hz, 1H), 3.85 (d, *J*= 6.7 Hz, 2H), 3.74 (m, 2H), 3.17 (m, 1H), 2.76 (d, *J*= 6.3 Hz, 1H), 2.72 (s, 6H), 1.95 (s, 3H), 1.36 (m, 3H), 1.08 (d, *J* = 6.7 Hz, 3H), 0.94 (d, *J* = 6.7 Hz, 3H).

**Example 58: Preparation of (S,E)-6-fluoro-7-(6-fluorobenzofuran-5-yl)-1-(2-isopropyl-4-methylpyridin-3-yl)-4-(4-(4-methoxybut-2-enoyl)-2-methylpiperazin-1-yl)pyrido[2,3-d]pyrimidin-2(1H)-one (compound 115)**

(E)-4-Methoxybut-2-enoic acid (18 mg, 0.15 mmol, 1.5 eq.), HATU (50 mg, 0.13 mmol, 1.3 eq.), and TEA (31 mg, 0.3 mmol, 3.0 eq.) were added into 10 mL tetrahydrofuran, and the mixture was stirred at room temperature for 30 min. (*S*)-6-fluoro-7-(6-fluorobenzofuran-5-yl)-1-(2-isopropyl-4-methylpyridin-3-yl)-4-(2-methylpiperazin-1-yl)pyrido[2,3-*d*]pyrimidin-2(1*H*)-one (compound 70) (53 mg, 0.1 mmol, 1.0 eq.) was then added, and the mixture was stirred for 1 h. ethyl acetate was added and the mixture was washed with water. The organic phase was concentrated under vacuum. The residue was purified by prep-TLC (silica gel, eluent:dichloromethane/methanol=30/1) to give (*S*,*E*)-6-fluoro-7-(6-fluorobenzofuran-5-yl)-1-(2-isopropyl-4-methylpyridin-3-yl)-4-(4-(4-methoxybut-2-enoyl)-2-methylpiperazin-1-yl)pyrido[2,3-*d*]pyrimidin-2(1*H*)-one (31 mg, yield: 49%) as a yellow solid. MS ($ES^+$): *m*/*z*=629 $(M+H)^+$. Melting point: 136°C to 138°C. $^1H$ NMR (500 MHz, DMSO) $\delta$ 8.35 (s, 2H), 7.93 (s, 1H), 7.78 (s, 1H), 7.25 (m, 1H), 7.17 (s, 1H), 7.00 (s, 1H), 6.74 (s, 1H), 6.65 (s, 1H), 4.95 (d, *J*= 39.8 Hz, 1H), 4.34 (dd, J= 29.2, 12.2 Hz, 2H), 4.04 (d, *J*= 56.7 Hz, 3H), 3.71 (m, 2H), 3.35 (s, 3H), 3.11 (m, 1H), 2.76 (s, 1H), 1.93 (s, 3H), 1.34 (m, 3H), 1.07 (d, *J* = 5.5 Hz, 3H), 0.93 (d, *J*= 5.2 Hz, 3H).

**Example 59: Preparation of 4-((S)-4-acryloyl-2-methylpiperazin-1-yl)-6-fluoro-7-(6-fluorobenzofuran-7-yl)-1-(4-isopropyl-6-methylpyrimidin-5-yl)pyrido [2,3-d] pyrimidin-2(1H)-one (compound 116) (prepared according to Scheme 1 and Scheme 9)**

Step 59a: Preparation of 4-isopropyl-6-methylpyrimidin-5-amine (compound 0103-116): To a mixture of 4,6-dichloropyrimidin-5-amine(1.5 g, 9.15 mmol, 1.0 eq.), 1,1'-bis(diphenylphosphino)ferrocene palladium(II)dichloride(669 mg, 0.915 mmol, 0.1 eq.) and sodium carbonate(2.91 g,27.45 mmol, 3.0 eq.) in dioxane (30 mL) and water (5 mL) was added 4,4,5,5-tetramethyl-2-(prop-1-en-2-yl)-1,3,2-dioxaborolane (1.84 g, 10.98 mmol, 1.2 eq.). The mixture was heated to 95°C under $N_2$ atmosphere for 12h. The solvent was removed under reduced pressure and the residue was purified by column chromatography on silica gel(petroleum ether:ethyl acetate 6:1) to give 4-chloro-6-(prop-1-en-2-yl)pyrimidin-5-amine(1.11 g, yield: 72%) as a yellow solid. LCMS(ESI): *m*/*z* 170$[M+1]^+$; TLC: Rf 0.5(petroleum ether:ethyl acetate = 6:1). To a mixture of 4-chloro-6-(prop-1-en-2-yl)pyrimidin-5-amine(1.11 g, 6.57 mmol, 1.0 eq.) prepared above, 1, 1'-bis(diphenylphosphino)ferrocene palladium(II)dichloride(481 mg, 0.657 mmol, 0.1 eq.) and sodium carbonate(3.48 g,32.85 mmol, 5.0 eq.) in dioxane (22 mL) and water (5.5 mL) was added methylboronic acid(1.97 g,32.85 mmol, 5.0 eq.). The mixture was heated to 102°C under $N_2$ atmosphere for 20h. The solvent was removed under reduced pressure. The residue was purified by column chromatography on silica gel(petroleum ether:ethyl acetate 1:1) to give 4-methyl-6-(prop-1-en-2-yl)pyrimidin-5-amine(0.625 g, yield: 64%) as a brown oil. LCMS(ESI): *m*/*z* 150$[M+1]^+$; TLC: Rf 0.3(petroleum ether:ethyl acetate = 1:1). A mixture of 4-methyl-6-(prop-1-en-2-yl)pyrimidin-5-amine(0.625 g, 4.195 mmol, 1.0 eq.)obtained above and Pd/C (0.1 g) in ethyl acetate(12 mL) was stirred at room temperature under $H_2$ balloon pressure overnight. The mixture was filtered and the filterate was concentrated under reduced pressure to give 4-isopropyl-6-methylpyrimidin-5-amine(406 mg, yield: 64%) as a brown oil. LCMS(ESI): *m*/*z* 152$[M+1]^+$; TLC: Rf 0.4 (petroleum ether:ethyl acetate = 1:1).

Step 59b: Preparation of 2,6-dichloro-5-fluoro-N-((4-isopropyl-6-methylpyrimidin-5-yl)carbamoyl)nicotinamide

(compounds 0109-116): To a mixture of 2,6-dichloro-5-fluoronicotinamide(0.731 g, 3.495 mmol, 1.3 eq.)in tetrahydrofuran(12 mL) was added oxalyl chloride(0.59 mL, 6.99 mmol, 2.6 eq.). The mixture was heated to 75°C under $N_2$ atmosphere for 2h. The solvent was removed under reduced pressure. The residue was dissolved in tetrahydrofuran(10 mL) and cooled to 0°C, and a solution of 4-isopropyl-6-methylpyrimidin-5-amine (0103-116) (0.406 g, 2.689 mmol, 1.0 eq.)in tetrahydrofuran(5 mL) was then added dropwise. The mixture was warmed to room temperature and stirred for 1h. The solvent was removed under reduced pressure. The residue was diluted with dichloromethane/petroleum ether(1:5, 20mL)and then filtered. The solid was collected and dried in vacuo to give 2,6-dichloro-5-fluoro-N-((4-isopropyl-6-methylpyrimidin-5-yl)carbamoyl)nicotinamide(1.04 g, crude) as a pale yellow solid. LCMS(ESI): *m/z* 386[M+1]$^+$; TLC: Rf 0.6(petroleum ether: ethyl acetate=1: 1).

Step 59c: Preparation of 7-chloro-6-fluoro-1-(4-isopropyl-6-methylpyrimidin-5-yl)pyrido[2,3-d]pyrimidine-2,4(1H,3H)-dione (compound 0110-116): To a mixture of sodium hydride(60%, 0.323 g, 8.08 mmol, 3.0 eq.)in NMP(5 mL) was added a solution of 2,6-dichloro-5-fluoro-N-((4-isopropyl-6-methylpyrimidin-5-yl)carbamoyl)nicotinamide (0109-116) (1.04 g, 2.69 mmol, 1.0 eq.)in NMP(5 mL) dropwise at 0°C. The mixture was stirred at 0°C for 40 min. Saturated ammonium chloride solution (50mL) was added to quench the reaction. 1M HCl solution was added to adjust the pH to pH=6. The aqueous layer was extracted with ethyl acetate(20 mL×3). The combined organic layer was washed with brine(20 mL×1),dried over anhydrous sodium sulfate and concentrated to give 7-chloro-6-fluoro-1-(4-isopropyl-6-methylpyrimidin-5-yl)pyrido[2,3-d]pyrimidine-2,4(1H,3H)-dione(0.81 g, crude) as a white solid. LCMS(ESI): *m/z* 350[M+1]$^+$; TLC: Rf 0.5(petroleum ether:ethyl acetate=2:1).

Step 59d: Preparation of tert-butyl (S)-4-(7-chloro-6-fluoro-1-(4-isopropyl-6-methylpyrimidin-5-yl)-2-oxo-1,2-dihydropyrido[2,3-d]pyrimidin-4-yl)-3-methylpiperazine-1-carboxylate (compound 0112-116): To a mixture of 7-chloro-6-fluoro-1-(4-isopropyl-6-methylpyrimidin-5-yl)pyrido[2,3-d]pyrimidine-2,4(1H,3H)-dione (0110-116) (0.81 g, 2.314 mmol, 1.0 eq.)and DIPEA(1.91 mL, 11.57 mmol, 5.0 eq.) in acetonitrile(20 mL) was added phosphorus oxychloride(0.39 mL, 4.17 mmol, 1.8 eq.). The mixture was heated to 85°C under $N_2$ atmosphere for 2h. The solvent was removed under reduced pressure. The residue was dissolved in tetrahydrofuran(15 mL), and DIPEA(1.91 mL, 11.57 mmol, 5.0 eq.)and tert-butyl (S)-3-methylpiperazine-1-carboxylate (0111-1) (0.463 g,2.314 mmol, 1.0 eq.)was then added at 0°C. The mixture was warmed to room temperature and stirred for 30 min. The solvent was removed under reduced pressure, and the residue was purified by column chromatography on silica gel(petroleum ether:ethyl acetate 1:1) to get tert-butyl (S)-4-(7-chloro-6-fluoro-1-(4-isopropyl-6-methylpyrimidin-5-yl)-2-oxo-1,2-dihydropyrido[2,3-d]pyrimidin-4-yl)-3-methylpiperazine-1-carboxylate(0.435 g, yield: 35%) as a brown solid. LCMS(ESI): *m/z* 532[M+1]$^+$; TLC: Rf 0.3 (petroleum ether:ethyl acetate = 1:1).

Step 59e: Preparation of tert-butyl (3S)-4-(6-fluoro-7-(6-fluorobenzofuran-7-yl)-1-(4-isopropyl-6-methylpyrimidin-5-yl)-2-oxo-1,2-dihydropyrido[2,3-d]pyrimidin-4-yl)-3-methylpiperazine-1-carboxylate (compound 0904-116): To a mixture of 7-chloro-6-fluoro-1-(4-isopropyl-6-methylpyrimidin-5-yl)-2-oxo-1,2-dihydropyrido[2,3-d]pyrimidin-4-yl)-3-methylpiperazine-1-carboxylate (0112-116) (130 mg, 0.245 mmol, 1.0 eq.), 1, 1'-bis(diphenylphosphino)ferrocene palladium(II)dichloride(27 mg, 0.037 mmol, 0.15 eq.) and potassium fluoride (57 mg, 0.98 mmol, 4.0 eq.) in dioxane (10 mL) and water (1 mL) was added potassium trifluoro(6-fluorobenzofuran-7-yl)borate(178 mg, 0.734 mmol, 3.0 eq.). The mixture was heated at 100°C under $N_2$ atmosphere overnight. The solvent was removed under reduced pressure. The residue was purified by column chromatography on silica gel (petroleum ether:ethyl acetate 1:2) to get tert-butyl (3S)-4-(6-fluoro-7-(6-fluorobenzofuran-7-yl)-1-(4-isopropyl-6-methylpyrimidin-5-yl)-2-oxo-l,2-dihydropyrido[2,3-d]pyrimidin-4-yl)-3-methylpiperazine-1-carboxylate(138 mg, yield: 89%) as a brown solid. LCMS(ESI): *m/z* 632[M+1]$^+$; TLC: Rf 0.3(petroleum ether:ethyl acetate = 1:2).

Step 59f: Preparation of 6-fluoro-7-(6-fluorobenzofuran-7-yl)-1-(4-isopropyl-6-methylpyrimidin-5-yl)-4-((S)-2-methylpiperazin-1-yl)pyrido[2,3-d]pyrimidin-2(1H)-one (compound 0905-116): To a mixture of tert-butyl (3S)-4-(6-fluoro-7-(6-fluorobenzofuran-7-yl)-1-(4-isopropyl-6-methylpyrimidin-5-yl)-2-oxo-1,2-dihydropyrido[2,3-d]pyrimidin-4-yl)-3-methylpiperazine-1-carboxylate (0904-116) (138 mg, 0.219 mmol, 1.0 eq.)in dichloromethane(6 mL) was added TFA(1.5 mL). The mixture was stirred at room temperature for 0.5 h. The solvent was removed under reduced pressure. The residue was dried in vacuo to give 6-fluoro-7-(6-fluorobenzofuran-7-yl)-1-(4-isopropyl-6-methylpyrimidin-5-yl)-4-((S)-2-methylpiperazin-1-yl)pyrido[2,3-d]pyrimidin-2(1H)-one(140 mg, crude) as a brown oil. LCMS(ESI): *m/z* 532[M+1]$^+$; TLC: Rf 0.3(dichloromethane: methanol=10: 1).

Step 59g: Preparation of 4-((S)-4-acryloyl-2-methylpiperazin-1-yl)-6-fluoro-7-(6-fluorobenzofuran-7-yl)-1-(4-isopropyl-6-methylpyrimidin-5-yl)pyrido[2,3-d]pyrimidin-2(1H)-one (compound 116): To a mixture of 6-fluoro-7-(6-fluorobenzofuran-7-yl)-1-(4-isopropyl-6-methylpyrimidin-5-yl)-4-((S)-2-methylpiperazin-1-yl)pyrido[2,3-d]pyrimidin-2(1H)-one(0905-116) (116 mg, 0.219 mmol, 1.0 eq.)and DIPEA (57 mg, 0.438 mmol, 2.0 eq.) in dichloromethane(5 mL) was added a solution of acryloyl chloride(30 mg, 0.329 mmol, 1.5 eq.)in dichloromethane(1 mL) at 0°C. The mixture was stirred for 10 min. The mixture was diluted with water(15 mL) and extracted with dichloromethane(10 mL×3). The combined organic layer was washed with brine(20 mL×1), dried over anhydrous sodium sulfate and concentrated. The residue was purified by prep-TLC(ethyl acetate:methanol=10:1) to give 4-((S)-4-acryloyl-2-methylpiperazin-1-yl)-6-fluoro-7-(6-fluorobenzofuran-7-yl)-1-(4-isopropyl-6-methylpyrimidin-5-yl)pyrido[2,3-d]pyrimidin-2(1H)-one(89 mg, yield:

70%) as a white solid. LCMS(ESI): *m/z* 586[M+1]$^+$; TLC: Rf 0.6(dichloromethane: methanol=10: 1); Melting point: 143-146°C. $^1$HNMR(DMSO-$d_6$, 500MHz): 88.94(s, 1H), 8.48-8.40(m, 1H), 7.93(d, *J* = 2.0Hz, 1H),7.82-7.79(m, 1H), 7.29-7.25(m, 1H), 7.02(d, *J* = 2.0Hz, 1H),6.90-6.83(m, 1H), 6.23-6.19(m, 1H), 5.78-5.75(m, 1H), 5.02-4.95(m, 1H), 4.41-4.28(m, 2H),4.17-4.02(m, 1H), 3.82-3.71(m, 1H), 3.67-3.48(m, 1H),3.23-3.12(m, 1H),2.83-2.80(m, 1H), 2.15(s, 3H), 1.38-1.34(m, 3H), 1.09(d, *J* = 6.5Hz, 3H), 0.95(d, *J* = 6.5Hz, 3H).

**Example 60: Preparation of 4-((S)-4-acryloyl-2-methylpiperazin-1-yl)-6-fluoro-7-(6-fluorobenzofuran-7-yl)-1-(4-isopropyl-2,6-dimethylpyrimidin-5-yl)pyrido [2,3-d]pyrimidin-2(1H)-one (compound 117) (prepared according to Scheme 1 and Scheme 9)**

Step 60a: Preparation of 4-isopropyl-2,6-dimethylpyrimidin-5-amine (compound 0103-117): To a mixture of 4,6-dichloro-2-methylpyrimidin-5-amine(2.0 g, 11.24 mmol, 1.0 eq.), 1, 1'-bis(diphenylphosphino)ferrocene palladium(II)dichloride(822 mg, 1.124 mmol, 0.1 eq.) and sodium carbonate(3.57 g,33.72 mmol, 3.0 eq.) in dioxane(40 mL)and water(7 mL) was added 4,4,5,5-tetramethyl-2-(prop-1-en-2-yl)-1,3,2-dioxaborolane(2.27 g, 13.48 mmol, 1.2 eq.). The mixture was heated to 95°C under $N_2$ atmosphere for 16h. The solvent was removed under reduced pressure. The residue was purified by column chromatography on silica gel(petroleum ether:ethyl acetate 6:1) to get 4-chloro-2-methyl-6-(prop-1-en-2-yl)pyrimidin-5-amine(1.56 g, yield: 76%) as a brown oil. LCMS(ESI): *m/z* 184[M+1]$^+$; TLC: Rf 0.5(petroleum ether:ethyl acetate = 6:1). To a mixture of 4-chloro-2-methyl-6-(prop-1-en-2-yl)pyrimidin-5-amine(1.1 g, 6.01 mmol, 1.0 eq.)obtained above, 1, 1'-bis(diphenylphosphino)ferrocene palladium(II)dichloride(446 mg, 0.601 mmol, 0.1 eq.) and sodium carbonate(3.19 g,30.05 mmol, 5.0 eq.) in dioxane(22 mL)and water(5.5 mL) was added methylboronic acid(1.8 g,30.05 mmol, 5.0 eq.). The mixture was heated to 102°C under $N_2$ atmosphere for 18h. The solvent was removed under reduced pressure. The residue was purified by column chromatography on silica gel(petroleum ether:ethyl acetate 1:1) to get 2,4-dimethyl-6-(prop-1-en-2-yl)pyrimidin-5-amine(0.668 g, yield: 68%) as a brown oil. LCMS(ESI): *m/z* 164[M+1]$^+$; TLC: Rf 0.3(petroleum ether:ethyl acetate = 1:1). The mixture of 2,4-dimethyl-6-(prop-1-en-2-yl)pyrimidin-5-amine(0.668 g, 4.10 mmol, 1.0 eq.)obtained above and Pd/C (80 mg) in ethyl acetate(15 mL) was stirred at room temperature under $H_2$ balloon pressure overnight. The mixture was filtered. The filterate was concentrated under reduced pressure to get 4-isopropyl-2,6-dimethylpyrimidin-5-amine(439 mg, yield: 65%) as a brown solid. LCMS(ESI): *m/z* 166[M+1]$^+$; TLC: Rf 0.4(petroleum ether:ethyl acetate = 1:1).

Step 60b: Preparation of 2,6-dichloro-5-fluoro-N-((4-isopropyl-2,6-dimethylpyrimidin-5-yl)carbamoyl)nicotinamide (compound 0109-117): To a mixture of 2,6-dichloro-5-fluoronicotinamide(0.723 g, 3.46 mmol, 1.3 eq.)in tetrahydrofuran(15 mL) was added oxalyl chloride(0.59 mL, 6.92 mmol, 2.6 eq.). The mixture was heated to 75°C under $N_2$ atmosphere for 2h. The solvent was removed under reduced pressure. The residue was dissolved in tetrahydrofuran(10 mL) and cooled to 0°C and a solution of 4-isopropyl-2,6-dimethylpyrimidin-5-amine(0103-117) (0.439 g, 2.66 mmol, 1.0 eq.)in tetrahydrofuran(5 mL) was added dropwise. The mixture was warmed to room temperature and stirred for 1h. The solvent was removed under reduced pressure and the residue was diluted with dichloromethane/petroleum ether(1:5, 20mL)and then filtered. The solid was collected and dried in vacuo to give 2,6-dichloro-5-fluoro-N-((4-isopropyl-2,6-dimethylpyrimidin-5-yl)carbamoyl)nicotinamide(1.07 g, crude) as a pale yellow solid. LCMS(ESI): *m/z* 400[M+1]$^+$; TLC: Rf 0.6(petroleum ether: ethyl acetate=1: 1).

Step 60c: Preparation of 7-chloro-6-fluoro- 1 -(4-isopropyl-2,6-dimethylpyrimidin-5-yl)pyrido[2,3-d]pyrimidine-2,4(1H,3H)-dione (compound 0110-117): To a mixture of sodium hydride(60%, 0.319 g, 7.98 mmol, 3.0 eq.)in NMP(5 mL) was added a solution of 2,6-dichloro-5-fluoro- N-((4-isopropyl-2,6-dimethylpyrimidin-5-yl)carbamoyl)nicotinamide(0109-117) (1.06 g, 2.66 mmol, 1.0 eq.)in NMP(5 mL) dropwise at 0°C. The mixture was stirred at 0°C for 40 min. Saturated ammonium chloride solution (40mL)was added to quench the reaction. 1M HCl solution was added to adjust the pH to pH=6. The aqueous layer was extracted with ethyl acetate(20 mL×3). The combined organic layer was washed with brine(20 mL×1),dried over anhydrous sodium sulfate and concentrated to get 7-chloro-6-fluoro-1-(4-isopropyl-2,6-dimethylpyrimidin-5-yl)pyrido[2,3-d]pyrimidine-2,4(1H,3H)-dione(0.8 g, crude) as a pale yellow solid. LCMS(ESI): *m/z* 364[M+1]$^+$; TLC: Rf 0.5(petroleum ether:ethyl acetate=2:1).

Step 60d: Preparation of tert-butyl (S)-4-(7-chloro-6-fluoro-1-(4-isopropyl-2,6-dimethylpyrimidin-5-yl)-2-oxo-1,2-dihydropyrido[2,3-d]pyrimidin-4-yl)-3-methylpiperazine-1-carboxylate (compound 0112-117): To a mixture of 7-chloro-6-fluoro-1-(4-isopropyl-2,6-dimethylpyrimidin-5-yl)pyrido[2,3-d]pyrimidine-2,4(1H,3H)-dione (0110-117) (0.541 g, 1.49 mmol, 1.0 eq.)and DIPEA(1.23 mL,7.45 mmol, 5.0 eq.) in acetonitrile(15 mL) was added phosphorus oxychloride(0.25 mL, 2.68 mmol, 1.8 eq.). The mixture was heated to 85°C under $N_2$ atmosphere for 2h. The solvent was removed under reduced pressure. The residue was dissolved in tetrahydrofuran(15 mL), and DIPEA(1.23 mL,7.45 mmol, 5.0 eq.)and tert-butyl (S)-3-methylpiperazine-1-carboxylate(0.298 g, 1.49 mmol, 1.0 eq.)was then added at 0°C. The mixture was warmed to room temperature and stirred for 1h. The solvent was removed under reduced pressure. The residue was purified by column chromatography on silica gel(petroleum ether:ethyl acetate 1:1) to get tert-butyl (S)-4-(7-chloro-6-fluoro-1-(4-isopropyl-2,6-dimethylpyrimidin-5-yl)-2-oxo-1,2-dihydropyrido[2,3-d]pyrimidin-4-yl)-3-methylpiperazine-1-carboxylate(0.165 g, yield: 20%) as a brown solid. LCMS(ESI): *m/z* 546[M+1]$^+$; TLC: Rf 0.3(petroleum ether:ethyl

acetate = 1:1).

Step 60e: Preparation of tert-butyl (3S)-4-(6-fluoro-7-(6-fluorobenzofuran-7-yl)-1-(4-isopropyl-2,6-dimethylpyrimidin-5-yl)-2-oxo-1,2-dihydropyrido[2,3-d]pyrimidin-4-yl)-3-methylpiperazine-1-carboxylate (compound 0904-117): To a mixture of tert-butyl (S)-4-(7-chloro-6-fluoro-1-(4-isopropyl-2,6-dimethylpyrimidin-5-yl)-2-oxo-1,2-dihydropyrido[2,3-d]pyrimidin-4-yl)-3-methylpiperazine-1-carboxylate(0112-117) (165 mg, 0.302 mmol, 1.0 eq.), 1, 1'-bis(diphenylphosphino)ferrocene palladium(II)dichloride(33 mg, 0.045 mmol, 0.15 eq.) and potassium fluoride(70 mg, 1.208 mmol, 4.0 eq.) in dioxane(15 mL)and water(1.5 mL) was added potassium trifluoro(6-fluorobenzofuran-7-yl)borate(219 mg, 0.906 mmol, 3.0 eq.). The mixture was heated at 100°C under $N_2$ atmosphere overnight. The solvent was removed under reduced pressure. The residue was purified by column chromatography on silica gel(ethyl acetate:methanol 20:1) to get tert-butyl (3S)-4-(6-fluoro-7-(6-fluorobenzofuran-7-yl)-1-(4-isopropyl-2,6-dimethylpyrimidin-5-yl)-2-oxo-1,2-dihydropyrido[2,3-d]pyrimidin-4-yl)-3-methylpiperazine-1-carboxylate(177 mg, yield: 91%) as a brown oil. LCMS(ESI): *m/z* 646$[M+1]^+$; TLC: Rf 0.5(dichloromethane:methanol = 10:1).

Step 60f: Preparation of 6-fluoro-7-(6-fluorobenzofuran-7-yl)-1-(4-isopropyl-2,6-dimethylpyrimidin-5-yl)-4-((S)-2-methylpiperazin-1-yl)pyrido[2,3-d]pyrimidin-2(1H)-one (compound 0905-117): To a mixture of tert-butyl (3S)-4-(6-fluoro-7-(6-fluorobenzofuran-7-yl)-1-(4-isopropyl-2,6-dimethylpyrimidin-5-yl)-2-oxo-1,2-dihydropyrido[2,3-d]pyrimidin-4-yl)-3-methylpiperazine-1-carboxylate (0904-117) (177 mg, 0.274 mmol, 1.0 eq.)in dichloromethane(8 mL) was added TFA(2 mL). The mixture was stirred at room temperature for 0.5h. The solvent was removed under reduced pressure. The residue was dried in vacuo to get 6-fluoro-7-(6-fluorobenzofuran-7-yl)-1-(4-isopropyl-2,6-dimethylpyrimidin-5-yl)-4-((S)-2-methylpiperazin-1-yl)pyrido[2,3-d]pyrimidin-2(1H)-one(160 mg, crude) as a brown oil. LCMS(ESI): *m/z* 546$[M+1]^+$; TLC: Rf 0.3(dichloromethane: methanol=10: 1).

Step 60g: Preparation of 4-((S)-4-acryloyl-2-methylpiperazin-1-yl)-6-fluoro-7-(6-fluorobenzofuran-7-yl)-1-(4-isopropyl-2,6-dimethylpyrimidin-5-yl)pyrido[2,3-d]pyrimidin-2(1H)-one (compound 117): To a mixture of 6-fluoro-7-(6-fluorobenzofuran-7-yl)-1-(4-isopropyl-2,6-dimethylpyrimidin-5-yl)-4-((S)-2-methylpiperazin-1-yl)pyrido[2,3-d]pyrimidin-2(1H)-one(0905-117) (95 mg, 0.174 mmol, 1.0 eq.)and DIPEA(45 mg, 0.348 mmol, 2.0 eq.) in dichloromethane(5 mL) was added a solution of acryloyl chloride(24 mg, 0.261 mmol, 1.5 eq.)in dichloromethane(1 mL) at 0°C. The mixture was stirred for 10 min. The mixture was diluted with water(10 mL) and extracted with dichloromethane(10 mL×3). The combined organic layer was washed with brine(10 mL×1),dried over anhydrous sodium sulfate and concentrated. The residue was purified by prep-TLC(ethyl acetate:dichloromethane:methanol=5:5:1) to get 4-((S)-4-acryloyl-2-methylpiperazin-1-yl)-6-fluoro-7-(6-fluorobenzofuran-7-yl)-1-(4-isopropyl-2,6-dimethylpyrimidin-5-yl)pyrido[2,3-d]pyrimidin-2(1H)-one(55 mg, yield: 53%) as a white solid. LCMS(ESI): *m/z* 600$[M+1]^+$; TLC: Rf 0.6(dichloromethane: methanol=10: 1); Melting point: 133-136°C. $^1$HNMR(DMSO-$d_6$, 500MHz): 88.47-8.39(m, 1H), 7.94(d, *J* = 2.5Hz, 1H), 7.83-7.80(m, 1H), 7.30-7.27(m, 1H), 7.03(d, *J* = 2.0Hz, 1H), 6.90-6.82(m, 1H), 6.23-6.19(m, 1H),5.78-5.75(m, 1H), 5.01-4.94(m, 1H), 4.39-4.28(m, 2H), 4.16-4.02(m, 1H), 3.79-3.73(m, 1H),3.67-3.64(m, 1H), 3.21-3.09(m, 1H), 2.77-2.75(m, 1H),2.55(s, 3H),2.09(s, 3H), 1.38-1.34(m, 3H), 1.07(d, *J* = 7.0Hz, 3H), 0.93(d, *J* = 7.0Hz, 3H).

**Example 61: Preparation of 4-((*S*)-4-acryloyl-2-methylpiperazin-1-yl)-6-chloro-1-(4,6-diisopropylpyrimidin-5-yl)-7-(6-fluorobenzofuran-7-yl)pyrido[2,3-*d*]pyrimidin-2(1*H*)-one (compound 118) (prepared according to Scheme 1 and Scheme 9)**

Step 61a: Preparation of 4,6-diisopropylpyrimidin-5-amine (compound 0103-118): Under the protection of nitrogen, 4,6-dichloropyrimidin-5-amine (984 mg, 6.0 mmol, 1.0 eq.), 4,4,5,5-tetramethyl-2-(prop-1-en-2-yl)-1,3,2-dioxaborolane (2.32 g, 13.8 mmol, 2.3 eq.), sodium carbonate (1.91 g, 18.0 mmol, 3.0 eq.) and [1, 1'-Bis(diphenylphosphino)ferrocene]dichloropalladium(II) (439 mg, 0.6 mmol, 0.1 eq.) were added into a mixed solvent of 36 mL dioxane and 6 mL water, and the mixture was stirred at 95°C for 18 h. After cooling to room temperature, ethyl acetate was added to extract. The organic phase was washed with brine and concentrated in vacuo. The residue was purified by column chromatography on silica gel (eluent: petroleum ether/ethyl acetate=20/1 to 4/1) to give 4,6-di(prop-1-en-2-yl)pyrimidin-5-amine (680 mg, yield: 64%) as a yellow oil. MS ($ES^+$): *m/z*=176 $(M+H)^+$. , 4,6-di(prop-1-en-2-yl)pyrimidin-5-amine (680 mg, 3.89 mmol, 1.0 eq.) obtained above and 10% Pd/C (70 mg) were added into methanol (50 mL), and the mixture was stirred at room temperature under hydrogen atmosphere overnight. The mixture was filtered and the filtrate was concentrated in vacuo to give 4,6-diisopropylpyrimidin-5-amine (662 mg, crude) as a brown oil. MS ($ES^+$): *m/z*=180 $(M+H)^+$.

Step 61b: Preparation of 2,5,6-trichloro-N-((4,6-diisopropylpyrimidin-5-yl)carbamoyl)nicotinamide (compound 0109-118): Under the protection of nitrogen, a mixture of 2,5,6-trichloronicotinamide (775mg, 3.43 mmol, 1.0 eq.) and oxalyl chloride (915 mg, 7.20 mmol, 2.1 eq.) in tetrahydrofuran (25 mL) was stirred at 75°C for 3 h. After cooling to room temperature, the solvent was removed in vacuo. The residue was dissolved in tetrahydrofuran (25 mL), and a solution of 4,6-diisopropylpyrimidin-5-amine (compound 0103-118) (680 mg, 3.78 mmol, 1.1 eq.) in tetrahydrofuran (2 mL) was then added dropwise into the mixture above at 0°C. The mixture was stirred for 1 h. ethyl acetate was added and washed with brine. The organic phase was concentrated in vacuo to give 2,5,6-trichloro-N-((4,6-diisopropylpyrimidin-5-yl)carbamoyl)nicotinamide (1.14 g, yield: 77%) as a yellow oil. MS ($ES^+$): m/z=430 $(M+H)^+$.

Step 61c: Preparation of 6,7-dichloro-1-(4,6-diisopropylpyrimidin-5-yl)-4-hydroxypyrido[2,3-*d*]pyrimidin-2(1*H*)-one (compound 0110-118): Under the protection of nitrogen, to a solution of 2,5,6-trichloro-N-((4,6-diisopropylpyrimidin-5-yl)carbamoyl)nicotinamide (0109-118) (1.0 g, 2.32 mmol, 1.0 eq.) in tetrahydrofuran (50 mL) was added 1M KHMDS tetrahydrofuran solution (5.81 mL, 5.81 mmol, 2.5 eq.) dropwise at - 30°C. The mixture was stirred for 2 h. The reaction solution was quenched with 1M HCl, and the pH of the mixture was adjusted to pH=3.ethyl acetate was added to extract and the organic layer was washed with brine and concentrated in vacuo. The residue was treated with dichloromethane/methanol (1/20) to give 6,7-dichloro-1-(4,6-diisopropylpyrimidin-5-yl)-4-hydroxypyrido[2,3-*d*]pyrimidin-2(1*H*)-one (505 mg, yield: 55%) as a white solid. MS ($ES^+$): m/z=394 $(M+H)^+$.

Step 61d: Preparation of tert-butyl (*S*)-4-(6,7-dichloro-1-(4,6-diisopropylpyrimidin-5-yl)-2-oxo-1,2-dihydropyrido[2,3-*d*]pyrimidin-4-yl)-3-methylpiperazine-1-carboxylate (compound 0112-118): Under the protection of nitrogen, a mixture of 6,7-dichloro-1-(4,6-diisopropylpyrimidin-5-yl)-4-hydroxypyrido[2,3-*d*]pyrimidin-2(1*H*)-one (0110-118) (505 mg, 1.28 mmol, 1.0 eq.), DIPEA (826 mg, 6.40 mmol, 5.0 eq.) and phosphorus oxychloride (589 mg, 3.85 mmol, 3.0 eq.) in acetonitrile (30 mL) was stirred at 85°C for 2 h. After cooling to room temperature, the solvent was removed in vacuo. The residue was dissolved in tetrahydrofuran (30 mL), and cooled in an ice-water bath. DIPEA (991mg, 7.68 mmol, 6.0 eq.) and tert-butyl (*S*)-3-methylpiperazine-1-carboxylate (0111-1) (256 mg, 1.28 mmol, 1.0 eq.) were then added. The mixture was stirred for 0.5 h. ethyl acetate was added and the mixture were washed with water and brine. The organic phase was concentrated in vacuo. The residue was purified by column chromatography on silica gel (eluent: dichloromethane/methanol=200/1 to 50/1) to give tert-butyl (*S*)-4-(6,7-dichloro-1-(4,6-diisopropylpyrimidin-5-yl)-2-oxo-1,2-dihydropyrido[2,3-*d*]pyrimidin-4-yl)-3-methylpiperazine-1-carboxylate (592 mg, yield: 80%) as a yellow solid. MS ($ES^+$): m/z=576 $(M+H)^+$.

Step 61e: Preparation of tert-butyl (3*S*)-4-(6-chloro-1-(4,6-diisopropylpyrimidin-5-yl)-7-(6-fluorobenzofuran-7-yl)-2-oxo-1,2-dihydropyrido[2,3-*d*]pyrimidin-4-yl)-3-methylpiperazine-1-carboxylate (compound 0904-118): Under the protection of nitrogen, 2-(6-fluorobenzofuran-7-yl)-4,4,5,5-tetramethyl-1,3,2-dioxaborolane (0903-73) (97 mg, 0.37 mmol, 1.4 eq.), tert-butyl (*S*)-4-(6,7-dichloro-1-(4,6-diisopropylpyrimidin-5-yl)-2-oxo-1,2-dihydropyrido[2,3-*d*]pyrimidin-4-yl)-3-methylpiperazine-1-carboxylate (0112-118) (150 mg, 0.26 mmol, 1.0 eq.), tetrakis(triphenylphosphine)palladium (30 mg, 0.026 mmol, 0.1 eq.), copper(I) chloride (26 mg, 0.26 mmol, 1.0 eq.) and sodium carbonate (56 mg, 0.52 mmol, 2.0 eq.) were added into a mixed solvent of 15 mL acetonitrile and 1.25 mL water. The mixture was heated at 81°C overnight. The mixture was cooled to room temperature, and extracted with ethyl acetate. The organic phase was washed with water and concentrated under vacuum. The residue was purified by column chromatography on silica gel (eluent: dichloromethane/methanol=150/1 to 30/1) to give tert-butyl (3*S*)-4-(6-chloro-1-(4,6-diisopropylpyrimidin-5-yl)-7-(6-fluorobenzofuran-7-yl)-2-oxo-1,2-dihydropyrido[2,3-*d*]pyrimidin-4-yl)-3-methylpiperazine-1-carboxylate (165 mg, yield: 93%) as a yellow solid. MS ($ES^+$): *m*/*z*=676$(M+H)^+$.

Step 61f: Preparation of 4-((*S*)-4-acryloyl-2-methylpiperazin-1-yl)-6-chloro-1-(4,6-diisopropylpyrimidin-5-yl)-7-(6-fluorobenzofuran-7-yl)pyrido[2,3-*d*]pyrimidin-2(1*H*)-one (compound 118): tert-butyl (3*S*)-4-(6-chloro-1-(4,6-diisopropylpyrimidin-5-yl)-7-(6-fluorobenzofuran-7-yl)-2-oxo-1,2-dihydropyrido[2,3-*d*]pyrimidin-4-yl)-3-methylpiperazine-1-carboxylate (0904-118) (165 mg, 0.24 mmol, 1.0 eq.) was dissolved in 10 mL dichloromethane, and TFA (1.5 mL) was then added dropwise, and the mixture was stirred at room temperature for 2 h. The mixture was concentrated under vacuum to dryness. The residue was dissolved in 10 mL tetrahydrofuran, and 0.5 mL DIPEA was then added. The mixture was cool in an ice-water bath, and acryloyl chloride (27 mg, 0.29 mmol, 1.2 eq.), pre-dissolved in 0.5 mL tetrahydrofuran, was added dropwise into the mixture above. The mixture was stirred for 30 min, and diluted with ethyl acetate. The organic phase was washed with water and concentrated under vacuum. The residue was purified by prep-TLC (silica gel, eluent: dichloromethane/methanol=20/1) to give 4-((*S*)-4-acryloyl-2-methylpiperazin-1-yl)-6-chloro-1-(4,6-diisopropylpyrimidin-5-yl)-7-(6-fluorobenzofuran-7-yl)pyrido[2,3-*d*]pyrimidin-2(1*H*)-one (94 mg, yield: 61%) as a yellow solid. MS ($ES^+$): *m*/*z*=630 $(M+H)^+$. Melting point: 254°C ~256°C. $^1H$ NMR (500 MHz, DMSO) $\delta$ 9.00 (s, 1H), 8.57 (d, *J* = 9.1 Hz, 1H), 7.92 (d, *J* = 2.2 Hz, 1H), 7.77 (dd, *J* = 8.6, 5.3 Hz, 1H), 7.25 (dd, *J* = 10.1, 8.8 Hz, 1H), 7.01 (d, *J* = 2.2 Hz, 1H), 6.95 - 6.79 (m, 1H), 6.28 - 6.15 (m, 1H), 5.77 (dd, *J* = 10.4, 2.3 Hz, 1H), 5.02 (s, 1H), 4.35 (ddd, *J* = 41.2, 20.6, 9.8 Hz, 2H), 4.11 (dd, *J* = 55.7, 13.4 Hz, 1H), 3.83 (s, 1H), 3.69 - 3.49 (m, 1H), 3.21 (d, *J* = 55.6 Hz, 1H), 2.74 (d, *J* = 31.2 Hz, 2H), 1.37 (d, *J* = 5.9 Hz, 3H), 1.08 (d, *J* = 8.3 Hz, 6H), 0.90 (t, *J* = 6.1 Hz, 6H).

**Example 62: Preparation of 4-((S)-4-acryloyl-2-methylpiperazin-1-yl)-6-fluoro-7-(6-fluorobenzofuran-7-yl)-1-(2-isopropylphenyl)pyrido[2,3-d]pyrimidin-2(1H)-one (compound 120) (prepared according to Scheme 1 and Scheme 9)**

Step 62a: Preparation of 2,6-dichloro-5-fluoro-*N*-((2-isopropylphenyl)carbamoyl)nicotinamide (compound 0109-120): Under the protection of nitrogen, a mixture of 2,6-dichloro-5-fluoronicotinamide (400 mg, 1.91 mmol, 1.0 eq.) and oxalyl chloride(485 mg, 3.82 mmol, 2.0 eq.) in tetrahydrofuran (10 mL) was stirred at 75°C for 2 h. After cooling to room temperature, the solvent was removed in vacuo. The residue was dissolved in tetrahydrofuran (10 mL), and a

solution of 2-isopropylaniline (258 mg, 1.91 mmol, 1 eq.) in tetrahydrofuran (1 mL) was then added dropwsie in an ice-water bath. After addition, the mixture was stirred for 30 min. The PH of the mixture was adjust to pH=1 with 2M HCl. The mixture was extracted with ethyl acetate. The organic phase was washed with brine, dried over anhydrous sodium sulfate and concentrated in vacuo to give 2,6-dichloro-5-fluoro-N-((2-isopropylphenyl)carbamoyl)nicotinamide as a yellow oil and which was used directly in the next step without further purification. MS ($ES^+$): m/z=370 $(M+H)^+$.

Step 62b: Preparation of 7-chloro-6-fluoro-1-(2-isopropylphenyl)pyrido[2,3-d]pyrimidine-2,4(1H,3H)-dione (compound 0110-120): Under the protection of nitrogen, to a solution of 2,6-dichloro-5-fluoro-N-((2-isopropylphenyl)carbamoyl)nicotinamide (0109-120) (629 mg, 1.7 mmol, 1.0 eq.) in NMP (10 mL) was added 60%sodium hydride (204 mg, 5.1 mmol, 3.0 eq.) portionwise in an ice-water bath, and the mixture was stirred for 1 h. The reaction solution was quenched with 2M HCl and stirred for 10 min. The mixture was filtered and the residue was washed with water and dried to give 7-chloro-6-fluoro-1-(2-isopropylphenyl)pyrido[2,3-d]pyrimidine-2,4(1H,3H)-dione (450 mg, crude) as a yellow solid. MS ($ES^+$): m/z=334 $(M+H)^+$.

Step 62c: Preparation of tert-butyl (S)-4-(7-chloro-6-fluoro-1-(2-isopropylphenyl)-2-oxo-1,2-dihydropyrido[2,3-d]pyrimidin-4-yl)-3-methylpiperazine-1-carboxylate (compound 0112-120): Under the protection of nitrogen, a mixture of 7-chloro-6-fluoro-1-(2-isopropylphenyl)pyrido[2,3-d]pyrimidine-2,4(1H,3H)-dione (0110-120) (450 mg, 1.35 mmol, 1.0 eq.), DIPEA (870 mg, 6.75 mmol, 5.0 eq.) and phosphorus oxychloride (620 mg, 4.05 mmol, 3 eq.) in acetonitrile (10 mL) was stirred at 85°C for 2 h. After cooling to room temperature, the solvent was removed in vacuo. The residue was dissolved in tetrahydrofuran (10 mL), and DIPEA (522 mg, 4.05 mmol, 3.0 eq.) and tert-butyl (S)-3-methylpiperazine-1-carboxylate (0111-1) (270 mg, 1.35 mmol, 1 eq.) were then added in an ice-water bath, and the mixture was stirred for 1 h. ethyl acetate was added the mixture were washed with water and brine. The organic layer was concentrated in vacuo. The residue was purified by column chromatography on silica gel (eluent: dichloromethane/methanol=200/1 to 80/1) to give tert-butyl (S)-4-(7-chloro-6-fluoro-1-(2-isopropylphenyl)-2-oxo-1,2-dihydropyrido[2,3-d]pyrimidin-4-yl)-3-methylpiperazine-1-carboxylate (380 mg, yield: 54.59%) as a yellow solid. MS ($ES^+$): m/z=516 $(M+H)^+$.

Step 62d: Preparation of tert-butyl (3S)-4-(6-fluoro-7-(6-fluorobenzofuran-7-yl)-1-(2-isopropylphenyl)-2-oxo-1,2-dihydropyrido[2,3-d]pyiimidin-4-yl)-3-methylpiperazine-1-carboxylate (compound 0904-120): Under nitrogen, tert-butyl (S)-4-(7-chloro-6-fluoro-1-(2-isopropylphenyl)-2-oxo-1,2-dihydropyrido[2,3-d]pyrimidin-4-yl)-3-methylpiperazine-1-carboxylate (0112-120) (127 mg, 0.246 mmol, 1 eq.), potassium trifluoro(6-fluorobenzofuran-7-yl)borate (0907-89) (119 mg, 0.492 mmol,2 eq.), 1,1'-bis(diphenylphosphino)ferrocene palladium(II)dichloride (18 mg, 0.0246 mmol, 0.1 eq.) and potassium fluoride (28 mg, 0.492 mmol,2 eq.) were added into the mixed solvent of 1,4-dioxane (10 mL) and water(1 mL). The mixture was stirred at 100°C for 3h and was cooled to room temperature. The mixture was extracted with ethyl acetate. The organic phase was washed with brine, dried over anhydrous sodium sulfate, and concentrated under vacuum. The residue was purified by perp-TLC to give tert-butyl (3 S)-4-(6-fluoro-7-(6-fluorobenzofuran-7-yl)-1 -(2-isopropylphenyl)-2-oxo-1,2-dihydropyrido[2,3-d]pyrimidin-4-yl)-3-methylpiperazine-1-carboxylate as a yellow solid (120 mg, 79.47%). MS (ESI): *m/z* 665$(M+H)^+$.

Step 62e: Preparation of 4-((S)-4-acryloyl-2-methylpiperazin-1-yl)-6-fluoro-7-(6-fluorobenzofuran-7-yl)-1-(2-isopropylphenyl)pyrido[2,3-d]pyrimidin-2(1H)-one (compound 120): tert-butyl (3S)-4-(6-fluoro-7-(6-fluorobenzofuran-7-yl)-1-(2-isopropylphenyl)-2-oxo-1,2-dihydropyrido[2,3-d]pyrimidin-4-yl)-3-methylpiperazine-1-carboxylate (0904-120) (120 mg, 0.18 mmol, 1.0 eq.) was dissolved in 4 mL dichloromethane, and 1 mL TFA was then added into the mixture. The mixture was stirred at room temperature for 2h and concentrated. The residue was dissolved in tetrahydrofuran (5 mL), and DIPEA (69 mg, 0.54 mmol, 3 eq.) was then added into the mixture followed by the additiohn of acryloyl chloride (16 mg, 0.18 mmol, 1.0 eq.). The mixture was stirred at room temperature for 15 min. The reaction was quenched with ammonium hydroxide and the mixture was extracted with dichloromethane. The organic phase was concentrated and purified by prep-TLC (silica gel, eluent: ethyl acetate/methanol=12/1) to give 4-((S)-4-acryloyl-2-methylpiperazin-1-yl)-6-fluoro-7-(6-fluorobenzofuran-7-yl)-1-(2-isopropylphenyl)pyrido[2,3-d]pyrimidin-2(1H)-one (55 mg, 53.5%) as a yellow solid. MS (ESI): *m/z* 570$(M+H)^+$. $^1$H NMR (500 MHz, DMSO) $\delta$ 8.34 (d, *J* = 9.6 Hz, 1H), 7.92 (t, *J* = 2.0 Hz, 1H), 7.77 (dd, *J* = 8.6, 5.2 Hz, 1H), 7.37 (dd, *J* = 7.8, 1.2 Hz, 1H), 7.29 (m, 1H), 7.22 (m, 2H), 7.10 (d, *J* = 7.8 Hz, 1H), 7.00 (d, *J* = 2.2 Hz, 1H), 6.84 (m, 1H), 6.19 (dd, *J* = 18.1, 8.0 Hz, 1H), 5.75 (dd, *J* = 10.4, 2.3 Hz, 1H), 4.90 (d, *J* = 31.7 Hz, 1H), 4.31 (m, 2H), 4.09 (m, 1H), 3.66 (m, 2H), 3.15 (m, 1H), 2.60 (dd, *J* = 15.2, 8.8 Hz, 1H), 1.33 (dd, *J* = 9.2, 6.8 Hz, 3H), 1.08 (d, *J* = 6.8 Hz, 3H), 0.97 (d, *J* = 6.8 Hz, 3H).

**Example 63: Preparation of 4-((S)-4-acryloyl-2-methylpiperazin-1-yl)-1-(2,4-diisopropylpyridin-3-yl)-6-fluoro-7-(6-fluorobenzofuran-7-yl)pyrido[2,3-d]pyrimidin-2(1H)-one (compound 121) (prepared according to Scheme 1 and Scheme 9)**

Step 63a: Preparation of 2,6-dichloro-N-((2,4-diisopropylpyridin-3-yl)carbamoyl)-5-fluoronicotinamide (compound 0109-121): Under the protection of nitrogen, to a solution of 2,6-dichloro-5-fluoronicotinamide (400 mg, 1.91 mmol, 1.0 eq.) in tetrahydrofuran (20 mL) was added oxalyl chloride(486 mg, 3.82 mmol, 2.0 eq.) at 0°C, and the mixture was stirred at 75°C for 2 h. After cooling to room temperature, the solvent was removed in vacuo. The residue was

dissolved in tetrahydrofuran (20 mL), and a solution of 2,4-diisopropylpyridin-3-amine (0103-121) (307 mg, 1.72mmol, 0.9 eq.) in tetrahydrofuran (1 mL) was then added dropwsie in an ice-water bath. After addition, the mixture was stirred for 1 h. ethyl acetate was added and the mixture was filtered. The filtrate was washed with aqueous sodium carbonate solution, water and brine, dried over anhydrous sodium sulfate and concentrated in vacuo to give crude product 2,6-dichloro-N-((2,4-diisopropylpyridin-3-yl)carbamoyl)-5-fluoronicotinamide which was used directly in the next step without further purification. MS ($ES^+$): m/z=413 $(M+H)^+$.

Step 63b: Preparation of 7-chloro-1-(2,4-diisopropylpyridin-3-yl)-6-fluoropyrido[2,3-d]pyrimidine-2,4(1H,3H)-dione (compound 0110-121): Under the protection of nitrogen, to a solution of 2,6-dichloro-N-((2,4-diisopropylpyridin-3-yl)carbamoyl)-5-fluoronicotinamide (0109-121) (360 mg, 0.97 mmol, 1.0 eq.) in NMP (10 mL) was added 60% sodium hydride (155 mg, 3.87 mmol, 4.0 eq.) portionwise in an ice-water bath, and the mixture was stirred for 1 h. The reaction solution was quenched with saturated aqueous ammonium chloride solution and stirred for 10 min. ethyl acetate was added to extract and the organic layer was washed with brine and concentrated in vacuo. The residue was purified by column chromatography on silica gel (eluent: petroleum ether/ethyl acetate=50/1 to 10/1) to give 7-chloro-1-(2,4-diisopropylpyridin-3-yl)-6-fluoropyrido[2,3-d]pyrimidine-2,4(1H,3H)-dione (360 mg, yield: 98.6%) as a white solid. MS (ES+): m/z=377 $(M+H)^+$.

Step 63c: Preparation of tert-butyl (S)-4-(7-chloro-1-(2,4-diisopropylpyridin-3-yl)-6-fluoro-2-oxo-1,2-dihydropyrido[2,3-d]pyrimidin-4-yl)-3-methylpiperazine-1-carboxylate (compound 0112-121)

Under the protection of nitrogen, a mixture of 7-chloro-1-(2,4-diisopropylpyridin-3-yl)-6-fluoropyrido[2,3-d]pyrimidine-2,4(1H,3H)-dione (0110-121) (360 mg, 0.96 mmol, 1.0 eq.), DIPEA(616 mg, 4.77 mmol, 5.0 eq.) and phosphorus oxychloride (440 mg, 2.88 mmol, 3.0 eq.) in acetonitrile (15 mL) was stirred at 85°C for 2 h. After cooling to room temperature, the solvent was removed in vacuo. The residue was dissolved in tetrahydrofuran (10 mL), and DIPEA(371 mg, 2.88 mmol, 3.0 eq.) and tert-butyl (S)-3-methylpiperazine-1-carboxylate (192 mg, 0.96 mmol, 1.0 eq.) were then added in an ice-water bath, and the mixture was stirred for 1 h. ethyl acetate was added, and the mixture was washed with water and brine. The organic layer was concentrated in vacuo. The residue was purified by column chromatography on silica gel (eluent: dichloromethane/methanol=150/1 to 40/1) to give tert-butyl (S)-4-(7-chloro-1-(2,4-diisopropylpyridin-3-yl)-6-fluoro-2-oxo-1,2-dihydropyrido[2,3-d]pyrimidin-4-yl)-3-methylpiperazine-1-carboxylate (350mg, yield: 65.0%) as a yellow solid. MS (ES+): m/z=559 (M+H)+.

Step 63d: Preparation of tert-butyl (3S)-4-(1-(2,4-diisopropylpyridin-3-yl)-6-fluoro-7-(6-fluorobenzofuran-7-yl)-2-oxo-1,2-dihydropyrido[2,3-d]pyrimidin-4-yl)-3-methylpiperazine-1-carboxylate (compound 0904-121): Under the protection of nitrogen, 2-(6-fluorobenzofuran-7-yl)-4,4,5,5-tetramethyl-1,3,2-dioxaborolane (0903-73) (140 mg, 0.536 mmol, 2.0 eq.), tert-butyl (S)-4-(7-chloro-1-(2,4-diisopropylpyridin-3-yl)-6-fluoro-2-oxo-1,2-dihydropyrido[2,3-d]pyrimidin-4-yl)-3-methylpiperazine-1-carboxylate (0112-121) (150mg, 0.26 mmol, 1.0 eq.), tris(dibenzylideneacetone)dipalladium (25 mg, 0.0268 mmol, 0.1 eq.), tricyclohexyl phosphine (15mg, 0.0536 mmol, 0.2 eq.) and sodium bicarbonate (45 mg, 0.536 mmol, 2.0 eq.) were added into a mixed solvent of 10 mL acetonitrile and 2 mL water. The mixture was stirred at 80°C overnight. The reaction solution was cooled to room temperature, and extracted with ethyl acetate. The organic phase was washed with water and concentrated under vacuum. The residue was purified by column chromatography on silica gel (eluent: dichloromethane/methanol=150/1 to 30/1) to give tert-butyl (3S)-4-(1-(2,4-diisopropylpyridin-3-yl)-6-fluoro-7-(6-fluorobenzofuran-7-yl)-2-oxo-1,2-dihydropyrido[2,3-d]pyrimidin-4-yl)-3-methylpiperazine-1-carboxylate (152 mg, yield: 86.3%) as a yellow solid. MS ($ES^+$): m/z=659 $(M+H)^+$.

Step 63e: Preparation of 1-(2,4-diisopropylpyridin-3-yl)-6-fluoro-7-(6-fluorobenzofuran-7-yl)-4-((S)-2-methylpiperazin-1-yl)pyrido[2,3-d]pyrimidin-2(1H)-one (compound 0905-121): tert-butyl (3S)-4-(1-(2,4-diisopropylpyridin-3-yl)-6-fluoro-7-(6-fluorobenzofuran-7-yl)-2-oxo-1,2-dihydropyrido[2,3-d]pyrimidin-4-yl)-3-methylpiperazine-1-carboxylate (0904-121) (152mg, 0.23mmol, 1.0 eq.) was dissolved in 6 mL dichloromethane, and TFA (1mL) was added dropwise, and the mixture was stirred at room temperature for 2 h. The reaction solution was concentrated under vacuum to dryness. The residue was used directly in the next step without further purification. MS ($ES^+$): m/z=569$(M+H)^+$.

Step 63f: Preparation of 4-((S)-4-acryloyl-2-methylpiperazin-1-yl)-1-(2,4-diisopropylpyridin-3-yl)-6-fluoro-7-(6-fluorobenzofuran-7-yl)pyrido[2,3-d]pyrimidin-2(1H)-one (compound 121): 1-(2,4-diisopropylpyridin-3-yl)-6-fluoro-7-(6-fluorobenzofuran-7-yl)-4-((S)-2-methylpiperazin-1-yl)pyrido[2,3-d]pyrimidin-2(1H)-one (0905-121) was dissolved in 10 mL tetrahydrofuran, and 2.0 mL DIPEA was then added. The reaction system was cooled in an ice-water bath and acryloyl chloride (33.9 mg, 0.37 mmol, 1.5 eq.), pre-dissolved in 0.5 mL tetrahydrofuran, was added dropwise into the mixture above. The mixture was stirred for 0.5 h. Water and ethyl acetate was added to extract the product. The organic phase was washed with water and concentrated in vacuo. The residue was purified by prep-TLC (silica gel, eluent: dichloromethane/methanol=12/1) to give 4-((S)-4-acryloyl-2-methylpiperazin-1-yl)-1-(2,4-diisopropylpyridin-3-yl)-6-fluoro-7-(6-fluorobenzofuran-7-yl)pyrido[2,3-d]pyrimidin-2(1H)-one (40 mg, yield: 28.3%) as a yellow solid. MS ($ES^+$): m/z=613 $(M+H)^+$. Melting point: 134~139°C; $^1H$ NMR (500 MHz, DMSO) $\delta$ 8.42 (d, *J*= 4.3 Hz, 2H), 7.89 (s, 1H), 7.78 (m, 1H), 7.25 (dd, *J* = 16.4, 7.0 Hz, 2H), 7.01 (s, 1H), 6.87 (d, *J*= 11.4 Hz, 1H), 6.21 (d, *J*= 14.7 Hz, 1H), 5.77 (d, *J* = 10.1 Hz, 1H), 4.97 (s, 1H), 4.33 (dd, *J* = 36.2, 12.3 Hz, 2H), 4.07 (m, 1H), 3.65 (m, 2H), 3.15 (s, 1H), 2.66 (s, 1H), 1.36 (d, *J*= 5.7 Hz, 3H), 1.07 (d, *J* = 5.6 Hz, 6H), 0.90 (m, 6H).

**Example 64: Preparation of 4-((S)-4-acryloyl-2-methylpiperazin-1-yl)-6-chloro-1-(2,4-diisopropylpyridin-3-yl)-7-(6-fluorobenzofuran-7-yl)pyrido[2,3-d]pyrimidin-2(1H)-one (compound 122) (prepared according to Scheme 1 and Scheme 9)**

Step 64a: Preparation of 2,5,6-trichloro-N-((2,4-diisopropylpyridin-3-yl)carbamoyl)nicotinamide (compound 0109-122): Under the protection of nitrogen, a mixture of 2,5,6-trichloronicotinamide (385 mg, 1.72 mmol, 1.0 eq.) and oxalyl chloride(437 mg, 3.44 mmol, 2.0 eq.) in tetrahydrofuran (15 mL) was stirred at 75°C for 2 h. After cooling to room temperature, the solvent was removed in vacuo. The residue was dissolved in tetrahydrofuran (5 mL), and a solution of 2,4-diisopropylpyridin-3-amine (0103-121) (275 mg, 1.72 mmol, 1.0 eq.) in tetrahydrofuran (5 mL) was then added dropwsie in an ice-water bath. After addition, the mixture was stirred for 30 min. Water and ethyl acetate was added and the mixture was filtered. The filtrate was washed with aqueous sodium carbonate solution, water and brine, dried over anhydrous sodium sulfate and concentrated in vacuo to give crude product 2,5,6-trichloro-N-((2,4-diisopropylpyridin-3-yl)carbamoyl)nicotinamide which was used directly in the next step without further purification. MS ($ES^+$): m/z=429 $(M+H)^+$.

Step 64b: Preparation of 6,7-dichloro-1-(2,4-diisopropylpyridin-3-yl)pyrido[2,3-d]pyrimidine-2,4(1H,3H)-dione (compound 0110-122): Under the protection of nitrogen, to a solution of 2,5,6-trichloro-N-((2,4-diisopropylpyridin-3-yl)carbamoyl)nicotinamide (0109-122) (300 mg, 0.7 mmol, 1.0 eq.) in NMP (15 mL) was added 60%sodium hydride (85 mg, 2.1 mmol, 3.0 eq.) portionwise in an ice-water bath, and the mixture was stirred for 1 h. The reaction solution was quenched with saturated aqueous ammonium chloride solution and stirred for 10 min, and then ethyl acetate was added to extract. The organic layer was washed with brine and concentrated in vacuo. The residue was purified by column chromatography on silica gel (eluent: petroleum ether/ethyl acetate=10/1 to 4/1) to give 6,7-dichloro-1-(2,4-diisopropylpyridin-3-yl)pyrido[2,3-d]pyrimidine-2,4(1H,3H)-dione (235 mg, yield: 85.4%) as a white solid. MS ($ES^+$): m/z=393 $(M+H)^+$.

Step 64c: Preparation of tert-butyl (S)-4-(6,7-dichloro-1-(2,4-diisopropylpyridin-3-yl)-2-oxo-1,2-dihydropyrido[2,3-d]pyrimidin-4-yl)-3-methylpiperazine-1-carboxylate (compound 0112-122): a mixture of 6,7-dichloro-1-(2,4-diisopropylpyridin-3-yl)pyrido[2,3-d]pyrimidine-2,4(1H,3H)-dione (0110-122) (235 mg, 0.599 mmol, 1.0 eq.), DIPEA (386 mg, 2.99 mmol, 5.0 eq.) and phosphorus oxychloride (275 mg, 1.797 mmol, 3.0 eq.) in acetonitrile (10 mL) was stirred at 85°C for 2 h under the protection of nitrogen. After cooling to room temperature, the solvent was removed in vacuo to give 4,6,7-trichloro-1-(2,4-diisopropylpyridin-3-yl)pyrido[2,3-d]pyrimidin-2(1H)-one which was used directly in the next step without further purification. MS ($ES^+$): m/z=411 $(M+H)^+$. 4,6,7-trichloro-1-(2,4-diisopropylpyridin-3-yl)pyrido[2,3-d]pyrimidin-2(1H)-one (245 mg, 0.597 mmol, 1.0 eq.) obtained above was dissolved in tetrahydrofuran (10 mL), DIPEA (231 mg, 1.79 mmol, 3.0 eq.) and tert-butyl (S)-3-methylpiperazine-1-carboxylate (120 mg, 0.597 mmol, 1.0 eq.) were added in an ice-water bath, and the mixture was stirred for 1 h under the protection of nitrogen. Ethyl acetate was added and the mixture was washed with water and brine. The organic layer was concentrated in vacuo. The residue was purified by column chromatography on silica gel (eluent: dichloromethane/methanol=200/1 to 80/1) to give tert-butyl (S)-4-(6,7-dichloro-1-(2,4-diisopropylpyridin-3-yl)-2-oxo-1,2-dihydropyrido[2,3-d]pyrimidin-4-yl)-3-methylpiperazine-1-carboxylate (150 mg, yield: 44.1%) as a yellow solid. MS ($ES^+$): m/z=575 $(M+H)^+$.

Step 64d: Preparation of tert-butyl (3S)-4-(6-chloro-1-(2,4-diisopropylpyridin-3-yl)-7-(6-fluorobenzofuran-7-yl)-2-oxo-1,2-dihydropyrido[2,3-d]pyrimidin-4-yl)-3-methylpiperazine-1-carboxylate (compound 0904-122): A mixture of tert-butyl (S)-4-(6,7-dichloro-1-(2,4-diisopropylpyridin-3-yl)-2-oxo-1,2-dihydropyrido[2,3-d]pyrimidin-4-yl)-3-methylpiperazine-1-carboxylate (0112-122) (130 mg, 0.226 mmol, 1.00 eq.), 2-(6-fluorobenzofuran-7-yl)-4,4,5,5-tetramethyl-1,3,2-dioxaborolane (0903-73) (118 mg, 0.452 mmol, 2.00 eq.), sodium bicarbonate (38 mg, 0.452 mmol, 2.00 eq.), tricyclohexyl phosphine (13 mg, 0.0452 mmol, 0.2 eq.)and $Pd_2(dba)_3$ (21mg, 0.0226 mmol, 0.1 eq.) in acetonitrile (10 mL) and water(1mL) was stirred at 80°C overnight under the protection of nitrogen. After cooling to room temperature, water and ethyl acetate was added to extract and the organic layer was washed with brine. The solvent was removed in vacuo. The residue was purified by prep-TLC (silica gel, eluent: dichloromethane/methanol=16/1)to give tert-butyl (3S)-4-(6-chloro-1-(2,4-diisopropylpyridin-3-yl)-7-(6-fluorobenzofuran-7-yl)-2-oxo-1,2-dihydropyrido[2,3-d]pyrimidin-4-yl)-3-methylpiperazine-1-carboxylate (78 mg , yield: 51.3%) as a light-yellow solid. MS ($ES^+$): m/z=675 $(M+H)^+$.

Step 64e: Preparation of 6-chloro-1-(2,4-diisopropylpyridin-3-yl)-7-(6-fluorobenzofuran-7-yl)-4-((S)-2-methylpiperazin-1-yl)pyrido[2,3-d]pyrimidin-2(1H)-one (compound 0905-122): To a solution of tert-butyl (3S)-4-(6-chloro-1-(2,4-diisopropylpyridin-3-yl)-7-(6-fluorobenzofuran-7-yl)-2-oxo-1,2-dihydropyrido[2,3-d]pyrimidin-4-yl)-3-methylpiperazine-1-carboxylate (0904-122) (56 mg, 0.08 mmol, 1.00 eq.) in dichloromethane (5 mL) was added TFA (0.5mL). The mixture was stirred at room temperature for 30 min and concentrated in vacuo to give 6-chloro-1-(2,4-diisopropylpyiidin-3-yl)-7-(6-fluorobenzofuran-7-yl)-4-((S)-2-methylpiperazin-1-yl)pyrido[2,3-d]pyrimidin-2(1H)-one (50 mg, crude) which was used directly in the next step without purification. MS ($ES^+$): m/z=575 $(M+H)^+$.

Step 64f: Preparation of 4-((S)-4-acryloyl-2-methylpiperazin-1-yl)-6-chloro-1-(2,4-diisopropylpyridin-3-yl)-7-(6-fluorobenzofuran-7-yl)pyrido[2,3-d]pyrimidin-2(1H)-one (compound 122): To a mixture of 6-chloro-1-(2,4-diisopropylpyridin-3-yl)-7-(6-fluorobenzofuran-7-yl)-4-((S)-2-methylpiperazin-1-yl)pyrido[2,3-d]pyrimidin-2(1H)-one (0905-122) (50

mg, crude) and DIPEA(0.5mL) in acetonitrile(5mL) was added dropwise acryloyl chloride(11.8 mg, 0.13 mmol, 1.50 eq.) at 0 °C. The mixture was stirred at room temperature for 30 min, and water and ethyl acetate was added to extract. The organic layer was washed with brine and concentrated in vacuo. The residue was purified by prep-TLC (silica gel, eluent: ethyl acetate/methanol=15/1) to give 4-((S)-4-acryloyl-2-methylpiperazin-1-yl)-6-chloro-1-(2,4-diisopropylpyridin-3-yl)-7-(6-fluorobenzofuran-7-yl)pyrido[2,3-d]pyrimidin-2(1H)-one (42 mg, yield: 77.7%) as a yellow solid. MS ($ES^+$): m/z=629 $(M+H)^+$ , m. p: 162-173°C. $^1H$ NMR (500 MHz, DMSO) δ 8.53 (s, 1H), 8.39 (d, *J* = 5.1 Hz, 1H), 7.89 (d, *J* = 2.2 Hz, 1H), 7.74 (dd, *J* = 8.6, 5.3 Hz, 1H), 7.22 (dd, *J* = 10.1, 8.7 Hz, 2H), 6.99 (d, *J* = 2.2 Hz, 1H), 6.85 (dd, *J*= 25.5, 14.4 Hz, 1H), 6.20 (d, *J*= 16.4 Hz, 1H), 5.76 (dd, *J*= 10.4, 2.3 Hz, 1H), 4.99 (s, 1H), 4.32 (m, 2H), 4.09 (dd, *J* = 59.1, 13.0 Hz, 1H), 3.70 (m, 2H), 3.16 (s, 1H), 2.60 (dd, *J* = 54.8, 23.0 Hz, 2H), 1.35 (d, *J*= 5.4 Hz, 3H), 1.05 (d, *J* = 6.7 Hz, 6H), 0.87 (dd, *J* = 6.6, 4.0 Hz, 6H).

**Example 65: Preparation of 2-(4-((S)-4-acryloyl-2-methylpiperazin-1-yl)-6-fluoro-7-(6-fluorobenzofuran-7-yl)-2-oxopyrido[2,3-d]pyrimidin-1(2H)-yl)-3-isopropylbenzonitrile (compound 123) (prepared according to Scheme 1 and Scheme 9)**

Step 65a: Preparation of 2,6-dichloro-N-((2-cyano-6-isopropylphenyl)carbamoyl)-5-fluoronicotinamide (0109-123): Under the protection of nitrogen, to a solution of 2,6-dichloro-5-fluoronicotinamide (500 mg, 2.39 mmol, 1.0 eq.) in tetrahydrofuran (20 mL) was added oxalyl chloride(608 mg, 4.78 mmol, 2.0 eq.) at 0°C, and the mixture was stirred at 75°C for 2 h. After cooling to room temperature, the solvent was removed in vacuo. The residue was dissolved in tetrahydrofuran (20 mL), and a solution of 2-amino-3-isopropylbenzonitrile (385 mg, 2.39 mmol, 1.0 eq.) in tetrahydrofuran (1 mL) was then added dropwsie in an ice-water bath. After addition, the mixture was stirred for 1 h and ethyl acetate and water were added to extract. The organic phase was dried, concentrated in vacuo and purified by column chromatography on silica gel (petroleum ether:ethyl acetate=5:1 to 1:1) to give product 2,6-dichloro-N-((2-cyano-6-isopropylphenyl)carbamoyl)-5-fluoronicotinamide (0.864 g, yield:91.5%) as a pale yellow solid. MS (ES+): m/z=395 $(M+H)^+$.

Step 65b: Preparation of 2-(7-chloro-6-fluoro-2,4-dioxo-3,4-dihydropyrido[2,3-d]pyrimidin-1(2H)-yl)-3-isopropylbenzonitrile (0110-123) (0110-123): To a mixture of sodium hydride(60%, 0.263 g, 6.56 mmol, 3.0 eq.)in NMP(5 mL) was added a solution of 2,6-dichloro-N-((2-cyano-6-isopropylphenyl)carbamoyl)-5-fluoronicotinamide (0109-123) (0.864 g, 2.187 mmol, 1.0 eq.)in NMP(5 mL) dropwise at 0°C. The mixture was stirred at 0°C for 1h and asaturated ammonium chloride solution (40mL)was added to quench the reaction. The mixture was filtered and the solid was washed with water and dried in vacuo to give 2-(7-chloro-6-fluoro-2,4-dioxo-3,4-dihydropyrido[2,3-d]pyrimidin-1(2H)-yl)-3-isopropylbenzonitrile(0.728 g, Yield:93%) as a gray solid. LCMS(ESI): *m/z* 359$[M+1]^+$; TLC: Rf 0.5(dichloromethane: methanol=30: 1).

Step 65c: Preparation of tert-butyl (S)-4-(7-chloro-1-(2-cyano-6-isopropylphenyl)-6-fluoro-2-oxo-1,2-dihydropyrido[2,3-d]pyrimidin-4-yl)-3-methylpiperazine-1-carboxylate (0112-123): To a mixture of 2-(7-chloro-6-fluoro-2,4-dioxo-3,4-dihydropyrido[2,3-d]pyrimidin-1(2H)-yl)-3-isopropylbenzonitrile (0110-123) (0.35 g, 0.975 mmol, 1.0 eq.)and DIPEA(0.8 mL,4.875 mmol, 5.0 eq.) in acetonitrile(12 mL) was added phosphorus oxychloride(0.18 mL, 1.95 mmol, 2.0 eq.). The mixture was heated to 85°C and reacted 2h under $N_2$ atmosphere. The solvent was removed under reduced pressure. The residue was dissolved in tetrahydrofuran (10 mL), and DIPEA(0.8 mL,4.875 mmol, 5.0 eq.)and tert-butyl (S)-3-methylpiperazine-1-carboxylate(0111-1) (0.195 g, 0.975 mmol, 1.0 eq.)was then added at 0°C. The mixture was warmed to room temperature and stirred for 1h. The solvent was removed under reduced pressure. The residue was purified by column chromatography on silica gel (petroleum ether:ethyl acetate 2:1) to get tert-butyl (S)-4-(7-chloro-1-(2-cyano-6-isopropylphenyl)-6-fluoro-2-oxo-1,2-dihydropyrido[2,3-d]pyrimidin-4-yl)-3-methylpiperazine-1-carboxylate(0.175 g, yield: 33%) as a brown solid. LCMS(ESI): *m/z* 541$[M+1]^+$; TLC: Rf 0.5(petroleum ether:ethyl acetate = 1:1).

Step 65d: Preparation of tert-butyl (3S)-4-(1-(2-cyano-6-isopropylphenyl)-6-fluoro-7-(6-fluorobenzofuran-7-yl)-2-oxo-1,2-dihydropyrido[2,3-d]pyrimidin-4-yl)-3-methylpiperazine-1-carboxylate (0904-123): To a mixture of tert-butyl (S)-4-(7-chloro-1-(2-cyano-6-isopropylphenyl)-6-fluoro-2-oxo-1,2-dihydropyrido[2,3-d]pyrimidin-4-yl)-3-methylpiperazine-1-carboxylate(0112-123) (130 mg, 0.241 mmol, 1.0 eq.), 1, 1'-bis(diphenylphosphino)ferrocene palladium(II)dichloride(17.6 mg, 0.024 mmol, 0.1 eq.) and potassium fluoride(42 mg, 0.723 mmol, 3.0 eq.) in dioxane(10 mL)and water(1 mL) was added potassium trifluoro(6-fluorobenzofuran-7-yl)borate(175 mg, 0.722 mmol, 3.0 eq.). The mixture was heated at 90°C under $N_2$ atmosphere overnight. The solvent was removed under reduced pressure. The residue was purified by column chromatography on silica gel (petroleum ether:ethyl acetate 2:1) to get tert-butyl (3S)-4-(1-(2-cyano-6-isopropylphenyl)-6-fluoro-7-(6-fluorobenzofuran-7-yl)-2-oxo-1,2-dihydropyrido[2,3-d]pyrimidin-4-yl)-3-methylpiperazine-1-carboxylate(70 mg, yield: 45%) as a brown solid. LCMS(ESI): *m/z* 641$[M+1]^+$; TLC: Rf 0.5(petroleum ether:ethyl acetate = 1:1).

Step 65e: Preparation of 2-(6-fluoro-7-(6-fluorobenzofuran-7-yl)-4-((S)-2-methylpiperazin-1-yl)-2-oxopyrido[2,3-d]pyrimidin-1(2H)-yl)-3-isopropylbenzonitrile (compound 0905-123): To a mixture of tert-butyl (3S)-4-(1-(2-cyano-6-isopropylphenyl)-6-fluoro-7-(6-fluorobenzofuran-7-yl)-2-oxo-1,2-dihydropyrido[2,3-d]pyrimidin-4-yl)-3-methylpiperazine-1-carboxylate (0904-123) (70 mg, 0.109 mmol, 1.0 eq.)in dichloromethane(6 mL) was added TFA(1.5 mL). The

mixture was stirred at room temperature for 0.5h. The solvent was removed under reduced pressure. The residue was dried in vacuo to get 2-(6-fluoro-7-(6-fluorobenzofuran-7-yl)-4-((S)-2-methylpiperazin-1-yl)-2-oxopyrido[2,3-d]pyrimidin-1(2H)-yl)-3-isopropylbenzonitrile(70 mg, crude) as a brown oil. LCMS(ESI): *m/z* 541[M+1]$^+$; TLC: Rf 0.3(dichloromethane: methanol=10: 1).

Step 65f: Preparation of 2-(4-((S)-4-acryloyl-2-methylpiperazin-1-yl)-6-fluoro-7-(6-fluorobenzofuran-7-yl)-2-oxopyrido[2,3-d]pyrimidin-1(2H)-yl)-3-isopropylbenzonitrile (compound 123): To a mixture of 2-(6-fluoro-7-(6-fluorobenzofuran-7-yl)-4-((S)-2-methylpiperazin-1-yl)-2-oxopyrido[2,3-d]pyrimidin-1(2H)-yl)-3-isopropylbenzonitrile (0905-123) (59 mg, 0.109 mmol, 1.0 eq.)and DIPEA(28 mg, 0.218 mmol, 2.0 eq.) in dichloromethane(5 mL) was added a solution of acryloyl chloride(15 mg, 0.164 mmol, 1.5 eq.)in dichloromethane(1 mL) at 0°C. The mixture was stirred for 10 min. The mixture was diluted with water(10 mL) and extracted with dichloromethane(10 mL×3). The combined organic layer was washed with brine(20 mL×1), dried over anhydrous sodium sulfate and concentrated. The residue was purified by prep-TLC(ethyl acetate:methanol=80:1) to get 2-(4-((S)-4-acryloyl-2-methylpiperazin-1-yl)-6-fluoro-7-(6-fluorobenzofuran-7-yl)-2-oxopyrido[2,3-d]pyrimidin-1(2H)-yl)-3-isopropylbenzonitrile(36 mg, yield: 56%) as a white solid. LCMS(ESI): *m/z* 595[M+1]$^+$; TLC: Rf 0.6(dichloromethane: methanol=20: 1); Melting point: 146-149°C. $^1$HNMR(DMSO-$d_6$, 500MHz): δ8.52-8.40(m, 1H), 7.94(d, *J* = 2.5Hz, 1H), 7.81-7.77(m, 3H), 7.55-7.52(m, 1H), 7.27-7.23(m, 1H), 7.00(d, *J* = 2.0Hz, 1H),6.89-6.84(m, 1H), 6.21(d, *J* = 16.5Hz, 1H), 5.76(d, *J* = 11.5Hz, 1H), 5.08-4.91(m, 1H), 4.43-4.28(m, 2H), 4.16-4.01(m, 1H), 3.87-3.54(m, 2H),3.22-3.07(m, 1H), 2.80-2.76(m, 1H), 1.38-1.32(m, 3H), 1.11(d, *J*= 3.0Hz, 3H), 0.98(d, *J* = 6.5Hz, 3H).

**Example 66: Preparation of 2-(4-((*S*)-4-acryloyl-2-methylpiperazin-1-yl)-6-chloro-7-(6-fluorobenzofuran-7-yl)-2-oxopyrido[2,3-*d*]pyrimidin-1(2*H*)-yl)-3-isopropylbenzonitrile (compound 124) (prepared according to Scheme 1 and Scheme 9)**

Step 66a: Preparation of 2,5,6-trichloro-*N*-((2-cyano-6-isopropylphenyl)carbamoyl)nicotinamide (compound 0109-124): Under the protection of nitrogen, a mixture of 2,5,6-trichloronicotinamide (339 mg, 1.5 mmol, 1.0 eq.) and oxalyl chloride (401 mg, 3.16 mmol, 2.1 eq.) in tetrahydrofuran (20 mL) was stirred at 75°C for 3 h. After cooling to room temperature, the solvent was removed in vacuo. The residue was dissolved in tetrahydrofuran (20 mL), and a solution of 2-amino-3-isopropylbenzonitrile (0103-123) (258 mg, 1.61 mmol, 1.1 eq.) in tetrahydrofuran (2 mL) was then added dropwise into the mixture above at 0°C. The mixture was stirred for 1 h. ethyl acetate was added, and washed with brine. The organic phase was concentrated in vacuo to give 2,5,6-trichloro-*N*-((2-cyano-6-isopropylphenyl)carbamoyl)nicotinamide (600 mg, yield: 97%) as a yellow solid. MS (ES$^+$): *m/z*=411 (M+H)$^+$.

Step 66b: Preparation of 2-(6,7-dichloro-4-hydroxy-2-oxopyrido[2,3-d]pyrimidin-1(2H)-yl)-3-isopropylbenzonitrile (compound 0110-124): to a solution of 2,5,6-trichloro-*N*-((2-cyano-6-isopropylphenyl)carbamoyl)nicotinamide (0109-124) (550 mg, 1.34 mmol, 1.0 eq.) in tetrahydrofuran (35 mL) was added 1M KHMDS tetrahydrofuran solution (3.34 mL, 3.34 mmol, 2.5 eq.) dropwise at -30°C under the protection of nitrogen. The mixture was stirred for 2 h. The reaction solution was quenched with 1M HCl, and the pH of the mixture was adjusted to pH=1.ethyl acetate was added to extract. The organic layer was washed with brine and concentrated in vacuo. The residue was treated with methanol to give 2-(6,7-dichloro-4-hydroxy-2-oxopyrido[2,3-d]pyrimidin-1(2*H*)-yl)-3-isopropylbenzonitrile (267 mg, yield: 53%) as a yellow solid. MS (ES$^+$): *m/z=375* (M+H)$^+$.

Step 66c: Preparation of tert-butyl (*S*)-4-(6,7-dichloro-1-(2-cyano-6-isopropylphenyl)-2-oxo-1,2-dihydropyrido[2,3-*d*]pyrimidin-4-yl)-3-methylpiperazine-1-carboxylate (compound 0112-124): Under the protection of nitrogen, a mixture of 2-(6,7-dichloro-4-hydroxy-2-oxopyrido[2,3-*d*]pyrimidin-1(2*H*)-yl)-3-isopropylbenzonitrile (0110-124) (230 mg, 0.61 mmol, 1.0 eq.), DIPEA (394 mg, 3.05 mmol, 5.0 eq.) and phosphorus oxychloride (282 mg, 1.84 mmol, 3.0 eq.) in acetonitrile (15 mL) was stirred at 85°C for 2 h. After cooling to room temperature, the solvent was removed in vacuo. The residue was dissolved in tetrahydrofuran (20 mL) and cooled in an ice-water bath. DIPEA (473mg, 3.66 mmol, 6.0 eq.) and tert-butyl (*S*)-3-methylpiperazine-1-carboxylate (0111-1) (122 mg, 0.61 mmol, 1.0 eq.) were added. The mixture was stirred for 0.5 h. ethyl acetate was added, and the mixture were washed with water and brine. The organic phase was concentrated in vacuo. The residue was purified by column chromatography on silica gel (eluent: dichloromethane/methanol=200/1 to 25/1) to give tert-butyl (*S*)-4-(6,7-dichloro-1-(2-cyano-6-isopropylphenyl)-2-oxo-1,2-dihydropyrido[2,3-*d*]pyrimidin-4-yl)-3-methylpiperazine-1-carboxylate (274 mg, yield: 80%) as a yellow solid. MS (ES$^+$): *m/z=557* (M+H)$^+$.

Step 66d: Preparation of tert-butyl (3*S*)-4-(6-chloro-1-(2-cyano-6-isopropylphenyl)-7-(6-fluorobenzofuran-7-yl)-2-oxo-1,2-dihydropyrido[2,3-*d*]pyrimidin-4-yl)-3-methylpiperazine-1-carboxylate (compound 0904-124): Under the protection of nitrogen, 2-(6-fluorobenzofuran-7-yl)-4,4,5,5-tetramethyl-1,3,2-dioxaborolane (0903-73) (85 mg, 0.33 mmol, 1.5 eq.), tert-butyl (*S*)-4-(6,7-dichloro-1-(2-cyano-6-isopropylphenyl)-2-oxo-1,2-dihydropyrido[2,3-*d*]pyrimidin-4-yl)-3-methylpiperazine-1-carboxylate (0112-124) (120 mg, 0.22 mmol, 1.0 eq.), tetrakis(triphenylphosphine)palladium (26 mg, 0.022 mmol, 0.1 eq.), copper(I) chloride (22 mg, 0.22 mmol, 1.0 eq.) and sodium carbonate (47 mg, 0.44 mmol, 2.0 eq.) were added into a mixed solvent of 18 mL acetonitrile and 1.5 mL water. The mixture was heated at 81°C overnight.

The mixture was cooled to room temperature and extracted with ethyl acetate. The organic phase was washed with water, and concentrated under vacuum. The residue was purified by prep-TLC (silica gel, eluent: dichloromethane/methanol= 30/1) to give tert-butyl (3S)-4-(6-chloro-1-(2-cyano-6-isopropylphenyl)-7-(6-fluorobenzofuran-7-yl)-2-oxo-1,2-dihydropyrido[2,3-d]pyrimidin-4-yl)-3-methylpiperazine-1-carboxylate (104 mg, yield: 71%) as a yellow solid. MS ($ES^+$): *m/z=657(M+H)*$^+$.

Step 66e: Preparation of 2-(4-((S)-4-acryloyl-2-methylpiperazin-1-yl)-6-chloro-7-(6-fluorobenzofuran-7-yl)-2-oxopyrido[2,3-d]pyrimidin-1(2H)-yl)-3-isopropylbenzonitrile (compound 124): tert-butyl (3S)-4-(6-chloro-1-(2-cyano-6-isopropylphenyl)-7-(6-fluorobenzofuran-7-yl)-2-oxo-1,2-dihydropyrido[2,3-d]pyrimidin-4-yl)-3-methylpiperazine-1-carboxylate (0904-124) (104 mg, 0.16 mmol, 1.0 eq.) was dissolved in 16 mL dichloromethane, and TFA (2.0 mL) was added dropwise. The mixture was stirred at room temperature for 2 h. The mixture was concentrated under vacuum to dryness. The residue was dissolved in 15 mL tetrahydrofuran, and 1.5 mL DIPEA was then added. The mixture was cool in an ice-water bath and acryloyl chloride (19 mg, 0.21 mmol, 1.3 eq.), pre-dissolved in 0.5 mL tetrahydrofuran, wasd added dropwise into the mixture above. The mixture was stirred for 30 mins and diluted with ethyl acetate. The organic phase was washed with water, and concentrated under vacuum. The residue was purified by prep-TLC (silica gel, eluent: dichloromethane/methanol=20/1) to give 2-(4-((*S*)-4-acryloyl-2-methylpiperazin-1-yl)-6-chloro-7-(6-fluorobenzofuran-7-yl)-2-oxopyrido[2,3-d]pyrimidin-1(2H)-yl)-3-isopropylbenzonitrile (72 mg, yield: 73%) as a yellow solid. MS ($ES^+$): *m/z=611* $(M+H)^+$. Melting point: 164°C ~166°C. $^1H$ NMR (500 MHz, DMSO) δ 8.69 - 8.53 (m, 1H), 7.96 (d, *J*= 31.8 Hz, 1H), 7.77 (dd, *J* = 8.3, 5.2 Hz, 3H), 7.52 (t, *J* = 7.8 Hz, 1H), 7.24 (t, *J* = 9.4 Hz, 1H), 7.01 (d, *J* = 20 Hz, 1H), 6.86 (d, *J* = 10.6 Hz, 1H), 6.22 (d, *J* = 16.5 Hz, 1H), 5.77 (d, *J*= 12.2 Hz, 1H), 5.03 (d, *J*= 74.3 Hz, 1H), 4.48 - 4.24 (m, 2H), 4.25 - 4.02 (m, 1H), 3.95 - 3.50 (m, 2H), 3.15 (d, *J*= 31.2 Hz, 1H), 2.77 (s, 1H), 1.36 *(dd, J*= 15.4, 6.7 Hz, 3H), 1.11 (d, *J*= 6.8 Hz, 3H), 0.97 (d, *J* = 6.8 Hz, 3H).

**Example 67: Preparation of 4-((S)-4-acryloyl-2-methylpiperazin-1-yl)-6-fluoro-7-(6-fluorobenzofuran-7-yl)-1-(2-(hydroxymethyl)-6-isopropylphenyl)pyrido[2,3-d]pyrimidin-2(1H)-one (compound 126) (prepared according to Scheme 1 and Scheme 9)**

Step 67a: Preparation of N-((2-(((tert-butyldiphenylsilyl)oxy)methyl)-6-isopropylphenyl)carbamoyl)-2,6-dichloro-5-fluoronicotinamide (compound 0109-126): To a mixture of 2,6-dichloro-5-fluoronicotinamide(0.543 g, 2.6 mmol, 1.3 eq.)in tetrahydrofuran(10 mL) was added oxalyl chloride(0.44 mL, 5.2 mmol, 2.6 eq.). The mixture was heated to 75°C under $N_2$ atmosphere for 2h. The solvent was removed under reduced pressure. The residue was dissolved in tetrahydrofuran(10 mL) and cooled to 0°C. The solution of 2-(((tert-butyldiphenylsilyl)oxy)methyl)-6-isopropylaniline (0103-126) (0.805 g, 2.0 mmol, 1.0 eq.)in tetrahydrofuran(5 mL) was added dropwise. The mixture was warmed to room temperature and stirred for 2h. The solvent was removed under reduced pressure. The residue was dried in vacuo to get N-((2-(((tert-butyldiphenylsilyl)oxy)methyl)-6-isopropylphenyl)carbamoyl)-2,6-dichloro-5-fluoronicotinamide(1.35 g, crude) as a white solid. LCMS(ESI): *m/z* $638[M+1]^+$; TLC: Rf 0.5 (dichloromethane: petroleum ether=3: 1).

Step 67b: Preparation of 1-(2-(((tert-butyldiphenylsilyl)oxy)methyl)-6-isopropylphenyl)-7-chloro-6-fluoropyrido[2,3-d]pyrimidine-2,4(1H,3H)-dione (compound 0110-126): To a mixture of sodium hydride(60%, 0.24 g, 6.0 mmol, 3.0 eq.)in NMP(7 mL) was added a solution of N-((2-(((tert-butyldiphenylsilyl)oxy)methyl)-6-isopropylphenyl)carbamoyl)-2,6-dichloro-5-fluoronicotinamide (0109-126) (1.28 g, 2.0 mmol, 1.0 eq.)in NMP(6 mL) dropwise at 0°C. The mixture was stirred at 0°C for 2h. water(60mL)was added to quench the reaction. 1M HCl solution was added to adjust the pH to pH=6. The aqueous layer was extracted with ethyl acetate(25 mL×3). The combined organic layer was washed with brine(20 mL×1),dried over anhydrous sodium sulfate and concentrated. The residue was purified by column chromatography on silica gel(dichloromethane:petroleum ether 5:1) to get 1-(2-(((tert-butyldiphenylsilyl)oxy)methyl)-6-isopropylphenyl)-7-chloro-6-fluoropyrido[2,3-d]pyrimidine-2,4(1H,3H)-dione(0.66 g, yield: 55%) as a white solid. LCMS(ESI): *m/z* $603[M+1]^+$; TLC: Rf 0.3(dichloromethane:petroleum ether = 5:1).

Step 67c: Preparation of tert-butyl (S)-4-(1-(2-(((tert-butyldiphenylsilyl)oxy)methyl)-6-isopropylphenyl)-7-chloro-6-fluoro-2-oxo-1,2-dihydropyrido[2,3-d]pyrimidin-4-yl)-3-methylpiperazine-1-carboxylate (compound 0112-126): To a mixture of 1-(2-(((tert-butyldiphenylsilyl)oxy)methyl)-6-isopropylphenyl)-7-chloro-6-fluoropyrido[2,3-d]pyrimidine-2,4(1H,3H)-dione (0110-126) (0.30 g, 0.498 mmol, 1.0 eq.)and DIPEA(0.45 mL,2.739 mmol, 5.5 eq.) in acetonitrile(10 mL) was added phosphorus oxychloride(0.084 mL, 0.897 mmol, 1.8 eq.). The mixture was heated to 85°C under $N_2$ atmosphere for 2h. The solvent was removed under reduced pressure. The residue was dissolved in tetrahydrofuran(15 mL). DIPEA(0.45 mL,2.739 mmol, 5.5 eq.)and tert-butyl (S)-3-methylpiperazine-1-carboxylate (0111-1) (0.10 g, 0.498 mmol, 1.0 eq.)was added at 0°C. The mixture was warmed to room temperature and stirred for 1h. The solvent was removed under reduced pressure. The residue was purified by column chromatography on silica gel (petroleum ether:ethyl acetate 3:1) to get tert-butyl (S)-4-(1-(2-(((tert-butyldiphenylsilyl)oxy)methyl)-6-isopropylphenyl)-7-chloro-6-fluoro-2-oxo-1,2-dihydropyrido[2,3-d]pyrimidin-4-yl)-3-methylpiperazine-1-carboxylate(0.297 g, yield: 76%) as a pale yellow oil. LCMS(ESI): *m/z* $784[M+1]^+$; TLC: Rf 0.3(petroleum ether:ethyl acetate = 3:1).

Step 67d: Preparation of tert-butyl (3S)-4-(1-(2-(((tert-butyldiphenylsilyl)oxy)methyl)-6-isopropylphenyl)-6-fluoro-7-(6-fluorobenzofuran-7-yl)-2-oxo-1,2-dihydropyrido[2,3-d]pyrimidin-4-yl)-3-methylpiperazine-1-carboxylate (compound 0904-126): To a mixture of tert-butyl (S)-4-(1-(2-(((tert-butyldiphenylsilyl)oxy)methyl)-6-isopropylphenyl)-7-chloro-6-fluoro-2-oxo-1,2-dihydropyrido[2,3-d]pyrimidin-4-yl)-3-methylpiperazine-1-carboxylate (0112-126) (170 mg, 0.217 mmol, 1.0 eq.), tetrakis(triphenylphosphine)palladium(38 mg, 0.033 mmol, 0.15 eq.) and sodium bicarbonate(55 mg, 0.651 mmol, 3.0 eq.) in acetonitrile(15 mL)and water(1.5 mL) was added 2-(6-fluorobenzofuran-7-yl)-4,4,5,5-tetramethyl-1,3,2-dioxaborolane (0903-73) (142 mg, 0.543 mmol, 2.5 eq.). The mixture was heated at 90°C under $N_2$ atmosphere overnight. The solvent was removed under reduced pressure. The residue was purified by column chromatography on silica gel(petroleum ether:ethyl acetate 3:1) to get tert-butyl (3 S)-4-(1-(2-(((tert-butyldiphenylsilyl)oxy)methyl)-6-isopropylphenyl)-6-fluoro-7-(6-fluorobenzofuran-7-yl)-2-oxo-1,2-dihydropyrido[2,3-d]pyrimidin-4-yl)-3-methylpiperazine-1-carboxylate(161 mg, yield: 84%) as a pale yellow oil. LCMS(ESI): *m/z* 884[M+1]$^+$; TLC: Rf 0.5(petroleum ether:ethyl acetate = 2:1).

Step 67e: Preparation of 1-(2-(((tert-butyldiphenylsilyl)oxy)methyl)-6-isopropylphenyl)-6-fluoro-7-(6-fluorobenzofuran-7-yl)-4-((S)-2-methylpiperazin-1-yl)pyrido[2,3-d]pyrimidin-2(1H)-one (compound 0905-126): To a mixture of tert-butyl (3S)-4-(1-(2-(((tert-butyldiphenylsilyl)oxy)methyl)-6-isopropylphenyl)-6-fluoro-7-(6-fluorobenzofuran-7-yl)-2-oxo-1,2-dihydropyrido[2,3-d]pyrimidin-4-yl)-3-methylpiperazine-1-carboxylate (0904-126) (161 mg, 0.182 mmol, 1.0 eq.)in dichloromethane(6 mL) was added TFA(1.5 mL). The mixture was stirred at room temperature for 0.5h. The solvent was removed under reduced pressure. The residue was dried in vacuo to get 1-(2-(((tert-butyldiphenylsilyl)oxy)methyl)-6-isopropylphenyl)-6-fluoro-7-(6-fluorobenzofuran-7-yl)-4-((S)-2-methylpiperazin-1-yl)pyrido[2,3-d]pyrimidin-2(1H)-one(160 mg, crude) as a pale yellow oil. LCMS(ESI): *m/z* 784[M+1]$^+$; TLC: Rf 0.3(dichloromethane: methanol=20: 1).

Step 67f: Preparation of 4-((S)-4-acryloyl-2-methylpiperazin-1-yl)-6-fluoro-7-(6-fluorobenzofuran-7-yl)-1-(2-(hydroxymethyl)-6-isopropylphenyl)pyrido[2,3-d]pyrimidin-2(1H)-one (compound 126): To a mixture of 1-(2-(((tert-butyldiphenylsilyl)oxy)methyl)-6-isopropylphenyl)-6-fluoro-7-(6-fluorobenzofuran-7-yl)-4-((S)-2-methylpiperazin-1-yl)pyrido[2,3-d]pyrimidin-2(1H)-one (0905-126) (143 mg, 0.182 mmol, 1.0 eq.)and DIPEA(47 mg, 0.364 mmol, 2.0 eq.) in dichloromethane(5 mL) was added a solution of acryloyl chloride(25 mg, 0.273 mmol, 1.5 eq.)in dichloromethane(1 mL) at 0°C. The mixture was stirred for 10 min and diluted with water(15 mL) and extracted with dichloromethane(10 mL×3). The combined organic layer was washed with brine(10 mL×1), dried over anhydrous sodium sulfate and concentrated to get 4-((S)-4-acryloyl-2-methylpiperazin-1-yl)-1-(2-(((tert-butyldiphenylsilyl)oxy)methyl)-6-isopropylphenyl)-6-fluoro-7-(6-fluorobenzofuran-7-yl)pyrido[2,3-d]pyrimidin-2(1H)-one(155 mg, crude) as a pale yellow oil. LCMS(ESI): *m/z* 838[M+1]$^+$; TLC: Rf 0.5(dichloromethane: methanol=20: 1). To a mixture of 4-((S)-4-acryloyl-2-methylpiperazin-1-yl)-1-(2-(((tert-butyldiphenylsilyl)oxy)methyl)-6-isopropylphenyl)-6-fluoro-7-(6-fluorobenzofuran-7-yl)pyrido[2,3-d]pyrimidin-2(1H)-one(150 mg, 0.179 mmol, 1.0 eq.)obtained above in tetrahydrofuran(8 mL) was added TBAF(1M solution in tetrahydrofuran, 0.27 mL, 0.27 mmol, 1.5 eq.). The mixture was stirred at room temperature for 40h. The solvent was removed under reduced pressure. The residue was purified by prep-TLC(ethyl acetate:methanol=10:1) to get 4-((S)-4-acryloyl-2-methylpiperazin-1-yl)-6-fluoro-7-(6-fluorobenzofuran-7-yl)-1-(2-(hydroxymethyl)-6-isopropylphenyl)pyrido[2,3-d]pyrimidin-2(1H)-one(40 mg, yield: 37%) as a white solid. LCMS(ESI): *m/z* 600[M+1]$^+$; TLC: Rf 0.5(dichloromethane: methanol=20: 1); Melting point: 146-149°C. $^1$HNMR(DMSO-$d_6$, 500MHz): δ8.41-8.37(m, 1H), 7.92(d, *J* = 2.0Hz, 1H),7.80-7.77(m, 1H), 7.35-7.30(m, 2H), 7.27-7.23(m, 2H), 7.01(d, *J* = 2.5Hz, 1H),6.91-6.83(m, 1H), 6.21(d, *J* = 15.5Hz, 1H), 5.78-5.76(m, 1H), 4.96-4.93(m, 2H), 4.43-4.29(m, 2H), 4.24-4.19(m, 1H),4.14-4.02(m, 2H), 3.72-3.48(m, 2H), 3.18-3.12(m, 1H),2.63-2.55(m, 1H), 1.34(d, *J*= 6.5Hz, 3H), 1.08-1.06(m, 3H), 0.98-0.96(m, 3H).

**Example 68: Resolution of Atropisomers**

There are stereoisomers of the compounds of the present invention, wherein the substitution of ortho substituents R5, R6 may produce separable atropisomers. The mixture of atropisomers can be separated by chiral SFC or chiral HPLC to obtain two single compounds (method 1) or the intermediate 0110 can be separated by chiral SFC (method 2) and then the final product can be prepared by subsequent multi-step reactions, or the intermediate 0110 can be prepared as a chiral salt which can be resolved (method 3) and then the final product can be prepared by subsequent multi-step reactions.

**Method 1: Chiral SFC or chiral HPLC separation of the final product**

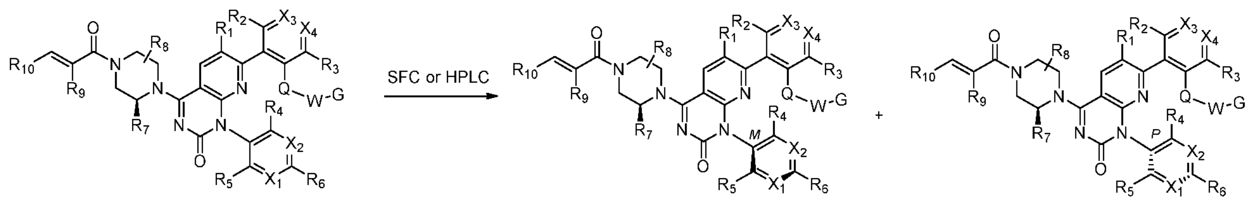


Chiral SFC separation conditions : Column: IG column or IC column. Mobile phase: $CO_2$/methanol
Chiral HPLC separation conditions : Column: AD-H column or IC column, Mobile phase: (methanol/isopropyl alcohol): cyclohexane system.

**Method 2: Chiral SFC separation of intermediate 0110**

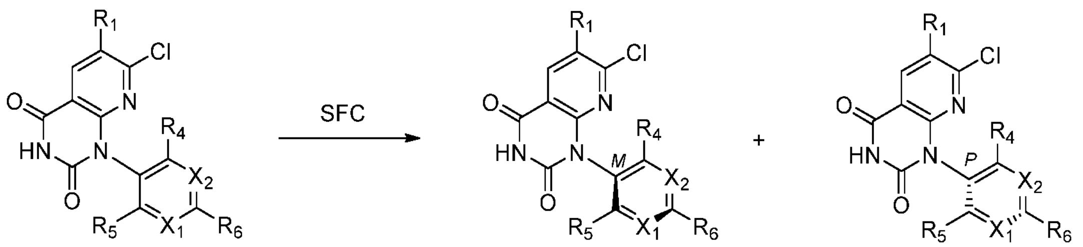


0110 1002 1003

**Example 68-1 : Preparation of 7-Chloro-6-fluoro-1(*M*)-(2-isopropyl-4-methylpyridin-3-yl)pyrido[2,3-d]pyrimidine-2,4(1H,3H)-dione (compound 1002-1) and 7-chloro-6-fluoro-1(*P*)-(2-isopropyl-4-methylpyridin-3-yl)pyrido[2,3-d]pyrimidine-2,4(1H,3H)-dione (compound 1003-1):**

7-Chloro-6-fluoro-1-(2-isopropyl-4-methylpyridin-3-yl)pyrido[2,3-d]pyrimidine-2,4(1H,3H)-dione (0110-1) (30 g) was dissolved in methanol (1800 mL) and separated by chiral SFC. Separation conditions: Instrument: SFC-150 (Thar, Waters), column: IG 20*250mm, 10um (Daicel), column temperature: 35°C, mobile phase: $CO_2$/methanol = 50/50, flow rate: 100 g/min. Obtained after separation:

Compound 7-chloro-6-fluoro-1(P)-(2-isopropyl-4-methylpyridin-3-yl)pyrido[2,3-d]pyrimidine-2,4(1H,3H)-dione (compound 1003-1) (14.5 g) was the first peak. LCMS(ESI): *m/z* 349[M+1]$^+$; $^1$H NMR (500 MHz, DMSO) δ 12.24 (s, 1H), 8.50 (dd, *J*= 16.7, 6.1 Hz, 2H), 7.27 (d, *J*= 4.9 Hz, 1H), 2.97 - 2.66 (m, 1H), 2.03 (s, 3H), 1.08 (d, *J*= 6.7 Hz, 3H), 1.00 (d, *J* = 6.7 Hz, 3H).

Compound 7-chloro-6-fluoro-1(*M*)-(2-isopropyl-4-methylpyridin-3-yl)pyrido[2,3-d]pyrimidine-2,4(1H,3H)-dione (compound 1002-1) (14.5 g) was the second peak. LCMS(ESI): *m/z* 349[M+1]$^+$; $^1$H NMR (500 MHz, DMSO) δ 12.24 (s, 1H), 8.49 (dd, *J* = 16.7, 6.1 Hz, 2H), 7.27 (d, *J* = 4.9 Hz, 1H), 2.98 - 2.72 (m, 1H), 2.03 (s, 3H), 1.07 (d, *J* = 6.7 Hz, 3H), 1.00 (d, *J* = 6.7 Hz, 3H).

**Example 68-2 : Preparation of 6,7-Chloro-1(*M*)-(2-isopropyl-4-methylpyridin-3-yl)pyrido[2,3-d]pyrimidine-2,4(1H,3H)-dione (compound 1002-73) and 6,7-chloro-1(*P*)-(2-isopropyl-4-methylpyridin-3-yl)pyrido[2,3-d]pyrimidine-2,4(1H,3H)-dione (compound 1003-73):**

6,7-Dichloro-1-(2-isopropyl-4-methylpyridin-3-yl)pyrido[3,2-e]pyrimidine-2,4(1H)-dione (0110-73) (40 g) was dissolved in methanol (1000 mL) and separated by chiral SFC. Separation conditions: Instrument: SFC-80 (Thar, Waters), column: IG 20*250mm, 10um (Daicel), column temperature: 35°C, mobile phase: $CO_2$/methanol = 45/55, flow rate: 80 g/min. Obtained after separation:

Compound 6,7-chloro-1(*P*)-(2-isopropyl-4-methylpyridin-3-yl)pyrido[2,3-d]pyrimidine-2,4(1H,3H)-dione (compound 1003-73) (14.5 g) was the first peak. LCMS(ESI): *m/z* 365[M+1]$^+$; $^1$H NMR (500 MHz, DMSO) δ 12.26 (s, 1H), 8.64 - 8.47 (m, 2H), 7.27 (d, *J* = 4.9 Hz, 1H), 2.87 (dt, *J* = 13.3, 6.6 Hz, 1H), 2.03 (d, *J* = 5.8 Hz, 3H), 1.08 (d, *J* = 6.7 Hz, 3H), 1.02 (t, *J* = 5.8 Hz, 3H).

Compound 6,7-chloro-1(*M*)-(2-isopropyl-4-methylpyridin-3-yl)pyrido[2,3-d]pyrimidine-2,4(1H,3H)-dione (compound

1002-73) (14.5 g) was the second peak. LCMS(ESI): *m/z* 365[M+1]$^+$; $^1$H NMR (500 MHz, DMSO) δ 12.26 (s, 1H), 8.71 - 8.40 (m, 2H), 7.27 (d, *J* = 4.9 Hz, 1H), 2.89 - 2.67 (m, 1H), 2.04 (s, 3H), 1.08 (d, *J* = 6.7 Hz, 3H), 1.01 (d, *J* = 6.7 Hz, 3H).

**Method 3: Chiral salt preparation of Intermediate 0110 and their chiral resolution**

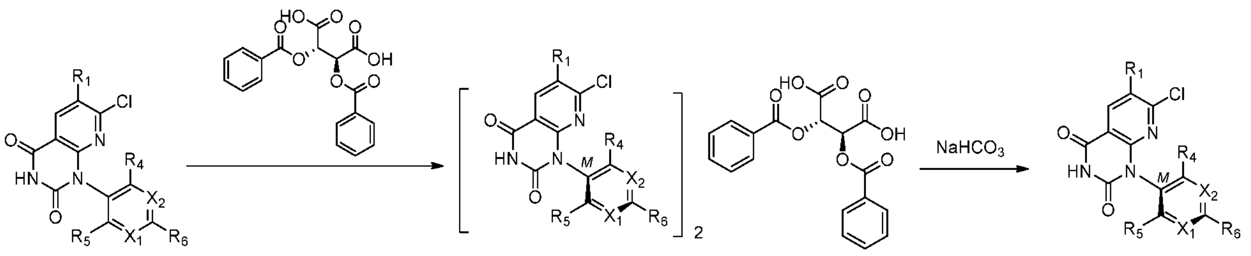


0110 1001 1002

**Example 68-3: Preparation of 6,7-dichloro-1(*M*)-(2-isopropyl-4-methylpyridin-3-yl)pyrido[3,2-e]pyrimidine-2,4(1H)-dione (compound 1002-73):**

Step 68-3a: Preparation of 6,7-dichloro-1(*M*)-(2-isopropyl-4-methylpyridin-3-yl)pyrido[2, 3-d]pyrimidine-2,4(1H,3H)-dione-dibenzoyltartrate (compound 1001-73) : 6,7-dichloro-1-(2-isopropyl-4-methylpyridin-3-yl)pyridine [3,2-e]pyrimidine-2,4 (1H)-dione (0110-73) (2.0 g, 5.5 mmol, 1.0 eq.) and D-dibenzoyltartaric acid (3.9 g, 11.0 mmol , 2.0 eq.) was dissolved in 2-methyltetrahydrofuran (30 mL). The reaction was warmed to 60°C and stirred for 30 min. After cooling to room temperature, the solvent was removed. 2-Methyltetrahydrofuran (24 mL) was added to the reaction solution and the reaction was warmed to 60°C. After solid in the reaction was completely dissolved, n-hexane (24 mL) was added slowly. The heating device was removed and a small amount of seed crystals were added to the reaction solution while it was still hot (the seed crystals were prepared by pure single compound of 6,7-dichloro-1(M)-(2-isopropyl-4-methylpyridin-3-yl)pyridine [3,2-e]pyrimidine-2,4 (1H)-dione and D-dibenzoyltartaric acid). The mixture was stirred at room temperature for 1 h, and then stand overnight. The mixture was filtered and the filter cake was washed twice with 2-methyltetrahydrofuran/n-hexane=1/2 (10 ml each time), and dried in vacuo to get 6,7-dichloro-1(M)-(2-isopropyl-4-methylpyridin-3-yl)pyrido[2,3-d]pyrimidine-2,4(1H,3H)-dione-dibenzoyltartrate (containing 2 molecules of methyltetrahydrofuran) (1.34 g, yield: 38.8%, ee: 99.31%) as a white powder solid . MS (ESI): *m/z* =365(M+H)$^+$. 1H NMR (500 MHz, DMSO) δ 13.86 (s, 1H), 12.26 (s, 1H), 8.58 (s, 1H), 8.51 (d, *J*= 4.8 Hz, 1H), 8.02 (d, *J*= 7.3 Hz, 2H), 7.73 (t, *J* = 7.4 Hz, 1H), 7.60 (t, *J* = 7.7 Hz, 2H), 7.27 (d, *J* = 4.9 Hz, 1H), 5.87 (s, 1H), 3.82 (dq, *J*= 12.4, 6.1 Hz, 1H), 3.74 (td, *J* = 7.8, 6.1 Hz, 1H), 3.55 (dd, *J* = 14.5, 7.9 Hz, 1H), 2.87 (dt, *J*= 13.3, 6.6 Hz, 1H), 2.04 (s, 3H), 1.97 - 1.88 (m, 1H), 1.87 - 1.73 (m, 2H), 1.31 (ddd, *J* = 16.4, 11.9, 7.6 Hz, 1H), 1.12 (d, *J* = 6.1 Hz, 3H), 1.07 (d, *J* = 6.7 Hz, 3H), 1.01 (d, *J* = 6.6 Hz, 3H).

Step 68-3b: Preparation of 6,7-dichloro-1(*M*)-(2-isopropyl-4-methylpyridin-3-yl)pyrido[2,3-d]pyrimidine-2,4(1H,3H)-dione (compound 1002-73) : 6,7-dichloro-1(*M*)-(2-isopropyl-4-methylpyridin-3-yl)pyrido[2,3-d]pyrimidine-2,4(1H,3H)-dione-dibenzoyltartrate (1001-73) (1.34 g, 2.1 mmol, 1.0 eq.) was dissolved in ethyl acetate (20 mL), and water (20 mL) was then added followed by dropwise addition of an aqueous solution of sodium bicarbonate to adjust the pH to about pH 7 to 8. After phase separation, the organic phase was washed with brine, dried, and evaporated to obtain 6,7-dichloro-1(*M*)-(2-isopropyl-4-methylpyridin-3-yl)pyrido[2,3-d]pyrimidine-2,4(1H,3H)-dione (770 mg, yield: 99.2%, ee: 99.51%) as a white powder solid. MS (ESI): *m/z* =365(M+H)$^+$; 1H NMR (500 MHz, DMSO) δ 12.26 (s, 1H), 8.71-8.40 (m, 2H), 7.27 (d, *J* = 4.9 Hz , 1H), 2.89-2.67 (m, 1H), 2.04 (s, 3H), 1.08 (d, *J* = 6.7 Hz, 3H), 1.01 (d, *J* = 6.7 Hz, 3H).

The following compounds can be prepared according to the methods of Examples 69 to 87.

| | | | |
|---|---|---|---|
| Example 69 | 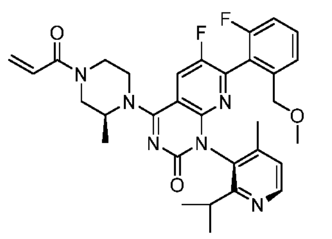 Compound 48-1 | Example 69 | 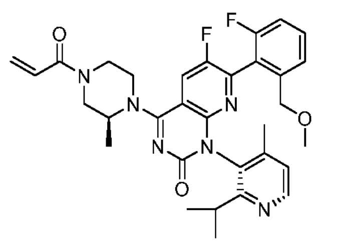 Compound 48-2 |

(continued)

| | | | |
|---|---|---|---|
| Example 70 | 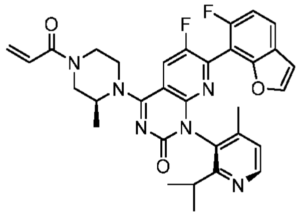 Compound 70-1 | Example 71 | 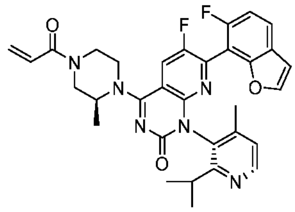 Compound 70-2 |
| Example 72 | 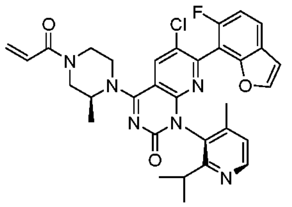 Compound 73-1 | Example 73 | 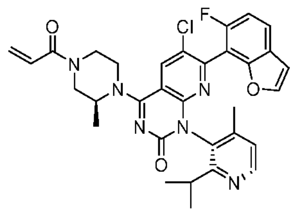 Compound 73-2 |
| Example 74 | 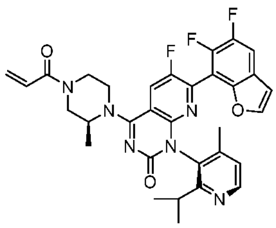 Compound 74-1 | Example 75 | 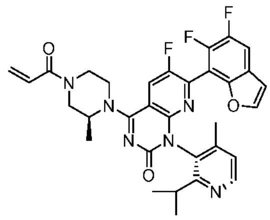 Compound 74-2 |
| Example 76 | 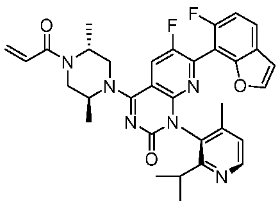 Compound 89-1 | Example 77 | 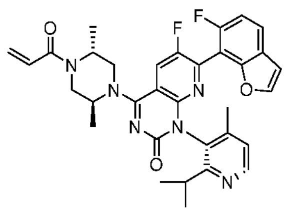 Compound 89-2 |
| Example 78 | 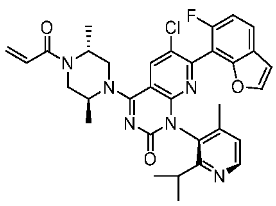 Compound 90-1 | Example 79 | 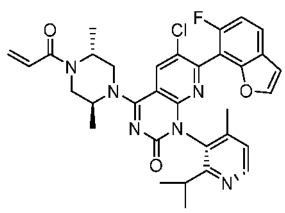 Compound 90-2 |
| Example 80 | 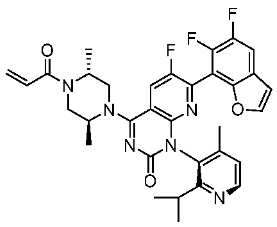 Compound 91-1 | Example 81 | 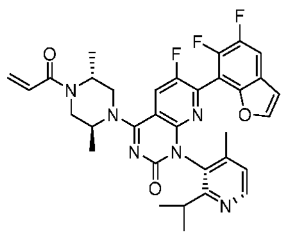 Compound 91-2 |

(continued)

| | | | |
|---|---|---|---|
| Example 82 | 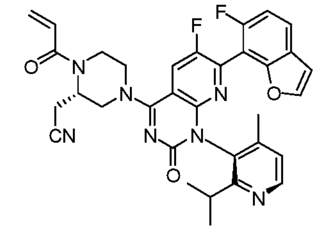 Compound 100-1 | Example 83 | 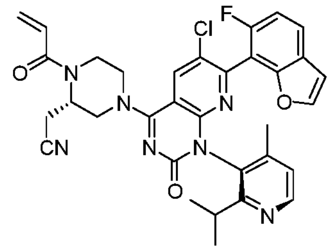 Compound 101-1 |
| Example 84 | 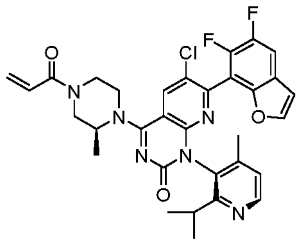 Compound 103-1 | Example 85 | 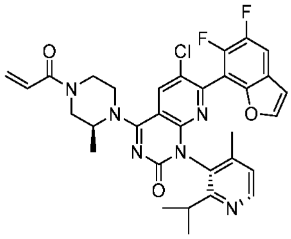 Compound 103-2 |
| Example 86 | 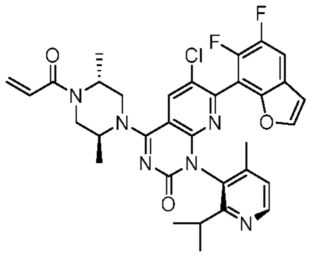 Compound 104-1 | Example 87 | 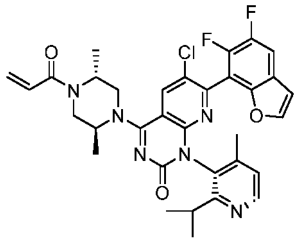 Compound 104-2 |

**Example 69: Separation of Compound 48**

Compound 48 (4-((S)-4-acryloyl-2-methylpiperazin-1-yl)-6-fluoro-7-(2-fluoro-6-(methoxymethyl)phenyl) -1-(2-isopropyl-4-methylpyridin-3-yl)pyrido[2,3-d]pyrimidin-2(1H)-one) (220 mg) was separated by a chiral preparation column (column type : AD-H, 10*5mm, 5um,; Mobile phase: (methanol/isopropyl alcohol=2/3): cyclohexane= 7/93 to 15/85, 50 min) to get two fractions: 1st fraction (compound 48-2, 100 mg) and 2nd fraction (compound 48-1, 90 mg).

The first fraction (compound 48-2): $^1$H NMR (500 MHz, DMSO) δ 8.40 (s, 2H), 7.50 (s, 1H), 7.27 (d, *J* = 13.6 Hz, 3H), 6.86 (s, 1H), 6.22 (s, 1H), 5.77 (s, 1H), 4.99 (s, 1H), 4.34 (d, *J* = 63.5 Hz, 2H), 4.07 (d, *J* = 23.6 Hz, 3H), 3.74 (d, *J*= 59.8 Hz, 2H), 3.03 (s, 4H), 2.73 (s, 1H), 2.50 (s, 3H), 1.31 (s, 3H), 1.06 (s, 3H), 0.85 (s, 3H).

The second fraction (compound 48-1): $^1$H NMR (500 MHz, DMSO) δ 8.36 (d, *J* = 32.4 Hz, 2H), 7.50 (s, 1H), 7.27 (d, *J* = 11.5 Hz, 3H), 6.85 (s, 1H), 6.20 (d, *J* = 13.2 Hz, 1H), 5.77 (s, 1H), 4.88 (s, 1H), 4.33 (m, 2H), 4.05 (d, *J* = 41.0 Hz, 3H), 3.61 (d, *J* = 62.7 Hz, 2H), 3.15 (m, 1H), 3.03 (s, 3H), 2.74 (s, 1H), 1.94 (s, 3H), 1.36 (s, 3H), 1.06 (s, 3H), 0.85 (s, 3H).

**Example 70: Preparation of 4-((S)-4-acryloyl-2-methylpiperazin-1-yl)-6-fluoro-7-(6-fluorobenzofuran-7-yl)-1(*M*)-(2-isopropyl-4-methylpyridin-3-yl)pyridin[2,3-d]pyrimidin-2(1H)-one (compound 70-1) (prepared according to Scheme 10)**

Step 70a: Preparation of tert-butyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate (compound 1004-70-1): 7-Chloro-6-fluoro-1(*M*)-(2-isopropyl-4-methylpyridin-3-yl)pyrido[2,3-d]pyrimidine-2,4(1H,3H)-dione (compound 1002 -1) (3 g, 8.62 mmol, 1.0 eq.), DIPEA (5.56 g, 43.1 mmol, 5.0 eq.) and phosphorus oxychloride (3.95 g, 25.86 mmol, 3.0 eq.) in acetonitrile (30 mL) was stirred at 85°C for 2 h under the protection of nitrogen. After cooling to room temperature, the solvent was removed under reduced pressure. The residue was dissolved in tetrahydrofuran (30 mL), and DIPEA (3.34 g, 25.86

mmol, 3.0 eq.) and tert-butyl (S)-3-methylpiperazine-1-carboxylate (1.724 g, 8.62 mmol, 1 eq.) were then added in an ice-water bath. The mixture was stirred for 1 h and ethyl acetate was added. The organic layer was washed with water and brine, and concentrated under reduced pressure. The residue was purified by column chromatography on silica gel (eluent: dichloromethane/methanol=200/1 to 80/1) to get tert-butyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate (3.57 g, yield: 78.29%) as a yellow solid. MS (ES+): m/z=531 $(M+H)^+$.

Step 70b: Preparation of tert-butyl (3S)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (compound 1006-70-1): a mixture of tert-butyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate (1004-70-1) (100 mg, 0.19 mmol, 1.0 eq.), 2-(6-fluorobenzofuran-7-yl)-4,4,5,5-tetramethyl-1,3,2-dioxaborolane (0902-70) (74 mg, 0.28 mmol, 1.5 eq.), sodium carbonate(40 mg, 0.38 mmol, 2.0 eq.), copper(I) chloride (19 mg, 0.19 mmol, 1.0 eq.) and tetrakis(triphenylphosphine)palladium (22 mg, 0.019 mmol, 0.1 eq.) in acetonitrile/water=10/1 (11 mL) was stirred at 82°C overnight under the protection of nitrogen. After cooling to room temperature, the mixture was poured into water, and ethyl acetate was added to extract. The organic layer was washed with brine and concentrated in vacuo. The residue was purified by prep-TLC (silica gel, eluent: petroleum ether/ethyl acetate=1/2) to give tert-butyl (3S)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (110 mg, yield: 92.4%) as a yellow solid. MS $(ES^+)$: m/z=631 $(M+H)^+$.

Step 70c: Preparation of 4-((S)-4-acryloyl-2-methylpiperazin-1-yl)-6-fluoro-7-(6-fluorobenzofuran-7-yl)-1(*M*)-(2-isopropyl-4-methylpyridin-3-yl)pyrido[2,3-d]pyrimidin-2(1H)-one (compound 70-1): To a solution of tert-butyl (3S)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (110 mg, 0.7 mmol, 1.0 eq.) in dichloromethane (4 mL) was added TFA (2 mL), and the mixture was stirred at room temperature for 1 h. The solvent was removed in vacuo. The residue was dissolved in tetrahydrofuran (6 mL), and DIPEA (65 mg, 0.51 mmol, 3.0 eq.) was then added. A solution of acryloyl chloride (18.5 mg, 0.2 mmol, 1.2 eq.) in tetrahydrofuran (0.5 mL) was added dropwise to the mixture in an ice-water bath. The mixture was stirred for 30 min. Water and ethyl acetate were added to extract. The organic layer was concentrated in vacuo. The residue was purified by prep-TLC (silica gel, eluent: ethyl acetate/methanol=12/1) to give 4-((S)-4-acryloyl-2-methylpiperazin-1-yl)-6-fluoro-7-(6-fluorobenzofuran-7-yl)-1(M)-(2-isopropyl-4-methylpyridin-3-yl)pyrido[2,3-d]pyrimidin-2(1H)-one (93 mg, yield: 91.2%) as a yellow solid. MS $(ES^+)$: m/z=585 $(M+H)^+$. $^1H$ NMR (500 MHz, DMSO) δ 8.38 (dd, *J* = 20.9, 7.0 Hz, 2H), 7.94 (d, *J* = 2.2 Hz, 1H), 7.79 (dd, *J* = 8.6, 5.2 Hz, 1H), 7.25 (dd, *J* = 10.4, 8.7 Hz, 1H), 7.19 (d, *J* = 4.7 Hz, 1H), 7.01 (d, *J* = 2.2 Hz, 1H), 6.84 (m, 1H), 6.20 (d, *J* = 16.7 Hz, 1H), 5.76 (dd, *J* = 10.4, 2.3 Hz, 1H), 4.91 (s, 1H), 4.34 (m, 2H), 4.08 (dd, *J*= 62.4, 13.2 Hz, 1H), 3.63 (dd, *J* = 49.3, 35.1 Hz, 2H), 3.18 (d, *J* = 12.2 Hz, 1H), 2.77 (m, 1H), 1.94 (s, 3H), 1.36 (d, *J* = 6.7 Hz, 3H), 1.07 (d, *J*= 6.7 Hz, 3H), 0.93 (d, *J*= 6.7 Hz, 3H).

**Example 71: Preparation of 4-((S)-4-acryloyl-2-methylpiperazin-1-yl)-6-fluoro-7-(6-fluorobenzofuran-7-yl)-1(*P*)-(2-isopropyl-4-methylpyridin-3-yl)pyridin[2,3-d]pyrimidin-2(1H)-one (compound 70-2) (prepared according to Scheme 10)**

Step 71a: Preparation of tert-butyl (S)-4-(7-chloro-6-fluoro-1(*P*)-(2-isopropyl-4-methylpyridin-3-yl)-2-oxo-1,2-dihydropyrido[2,3-d]pyrimidin-4-yl)-3-methylpiperazine-1-carboxylate (compound 1008-70-2) : The synthetic method was similar to Step 70a of Example 70, except that 7-chloro-6-fluoro-1(*M*)-(2-isopropyl-4-methylpyridin-3-yl)pyrido[2,3-d]pyrimidine-2,4(1H,3H)-dione (compound 1002-1) was replaced by 7-chloro-6-fluoro-1(*P*)-(2-isopropyl-4-methylpyridin-3-yl)pyrido[2,3-d]pyrimidine-2,4(1H,3H)-dione (1003-1) (3.0 g, 8.6 mmol, 1.0 eq.), tert-butyl (S)-4-(7-chloro-6-fluoro-1(*P*)-(2-isopropyl-4-methylpyridin-3-yl)-2-oxo-1,2-dihydropyrido[2,3-d]pyrimidin-4-yl)-3-methylpiperazine-1-carboxylate (4.1g, yield: 89.1%) was obtained as a white solid. MS $(ES^+)$: m/z=531 $(M+H)^+$.

Step 71b: Preparation of tert-butyl (3S)-4-(6-fluoro-7-(6-fluorobenzofuran-7-yl)-1(*P*)-(2-isopropyl-4-methylpyridin-3-yl)-2-oxo-1,2-dihydropyrido[2,3-d]pyrimidin-4-yl)-3-methylpiperazine-1-carboxylate (compound 1010-70-2) : The synthetic method was similar to Step 70b of Example 70, except that tert-butyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate (1004-70-1) was replaced by tert-butyl (S)-4-(7-chloro-6-fluoro-1(*P*)-(2-isopropyl-4-methylpyridin-3-yl)-2-oxo-1,2-dihydropyrido[2,3-d]pyrimidin-4-yl)-3-methylpiperazine-1-carboxylate (100 mg, 0.19 mmol, 1.0 eq.), tert-butyl (3S)-4-(6-fluoro-7-(6-fluorobenzofuran-7-yl)-1(P)-(2-isopropyl-4-methylpyridin-3-yl)-2-oxo-1,2-dihydropyrido[2,3-d]pyrimidin-4-yl)-3-methylpiperazine-1-carboxylate (110 mg, yield: 92.4%) was obtained as a yellow solid. MS $(ES^+)$: m/z=631 $(M+H)^+$.

Step 71c: Preparation of 4-((S)-4-acryloyl-2-methylpiperazin-1-yl)-6-fluoro-7-(6-fluorobenzofuran-7-yl)-1(P)-(2-isopropyl-4-methylpyridin-3-yl)pyridin[2,3-d]pyrimidin-2(1H)-one (compound 1008-70-2) : The synthetic method was similar to Step 70c of Example 70, except that tert-butyl (3S)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (1006-70-1) was replaced by tert-butyl (3S)-4-(6-fluoro-7-(6-fluorobenzofuran-7-yl)-1(*P*)-(2-isopropyl-4-methylpyridin-3-yl)-2-oxo-1,2-dihydropyrido[2,3-d]pyrimidin-4-yl)-3-methylpiperazine-1-carboxylate (1010-70-2) (100 mg, 0.19 mmol, 1.0 eq.), 4-((S)-4-acryloyl-2-methylpiperazin-1-yl)-6-fluoro-7-(6-fluorobenzofuran-7-yl)-1(*P*)-(2-isopropyl-4-methylpyridin-3-yl)pyridin[2,3-d]pyrimidin-2(1H)-one (85 mg, yield: 83.3%) was obtained as a yellow solid. MS ($ES^+$): m/z=585 $(M+H)^+$. $^1H$ NMR (500 MHz, DMSO) δ 8.44 (d, *J* = 9.6 Hz, 1H), 8.35 (d, *J* = 4.9 Hz, 1H), 7.94 (d, *J* = 2.2 Hz, 1H), 7.79 (dd, *J* = 8.6, 5.2 Hz, 1H), 7.25 (dd, *J* = 10.5, 8.7 Hz, 1H), 7.17 (d, *J* = 5.3 Hz, 1H), 7.01 (d, *J* = 2.2 Hz, 1H), 6.85 (m, 1H), 6.20 (d, *J* = 16.8 Hz, 1H), 5.76 (dd, *J* = 10.4, 2.3 Hz, 1H), 4.99 (m, 1H), 4.36 (dd, *J* = 50.2, 12.5 Hz, 2H), 4.09 (dd, *J* = 57.6, 13.5 Hz, 1H), 3.66 (m, 2H), 3.09 (t, *J* = 11.4 Hz, 1H), 2.76 (d, *J* = 5.7 Hz, 1H), 1.94 (s, 3H), 1.32 (d, *J* = 6.7 Hz, 3H), 1.07 (d, *J* = 6.7 Hz, 3H), 0.93 (d, *J* = 6.7 Hz, 3H).

**Example 72: Preparation of 4-((S)-4-acryloyl-2-methylpiperazin-1-yl)-6-chloro-7-(6-fluorobenzofuran-7-yl)-1(*M*)-(2-isopropyl-4-methylpyridin-3-yl)pyridin[2,3-d]pyrimidin-2(1H)-one (compound 73-1) (prepared according to Scheme 10)**

Step 72a: Preparation of tert-butyl (S)-4-(6,7-dichloro-1(*M*)-(2-isopropyl-4-methylpyridin-3-yl)-2-oxo-1,2-dihydropyrido[2,3-d]pyrimidin-4-yl)-3-methylpiperazine-1-carboxylate (compound 1004-73-1): A mixture of 6,7-dichloro-1(M)-(2-isopropyl-4-methylpyridin-3-yl)pyrido[2,3-d]pyrimidine-2,4(1H,3H)-dione (1002-73) (1.0 g, 2.7 mmol, 1.0 eq.), DIPEA(1.7 g, 13.5 mmol, 5.0 eq.) and phosphorus oxychloride (1.3 g, 8.1 mmol, 3.0 eq.) in acetonitrile (30 mL) was stirred at 85°C for 2 h under the protection of nitrogen. After cooling to room temperature, the solvent was removed in vacuo. The residue was dissolved in tetrahydrofuran (20 mL), and then DIPEA (1.0 g, 8.1 mmol, 3.0 eq.) and tert-butyl (S)-3-methylpiperazine-1-carboxylate (540 mg, 2.7 mmol, 1.0 eq.) were added in an ice-water bath. The mixture was stirred for 1 h, and then ethyl acetate was added. The mixture was washed with water and brine. The organic layer was concentrated in vacuo. The residue was purified by column chromatography on silica gel (eluent: dichloromethane/methanol=150/1 to 50/1) to give tert-butyl (S)-4-(6,7-dichloro-1(*M*)-(2-isopropyl-4-methylpyridin-3-yl)-2-oxo-1,2-dihydropyrido[2,3-d]pyrimidin-4-yl)-3-methylpiperazine-1-carboxylate (1.48 g, yield: 98.7%) as a yellow solid. MS ($ES^+$): m/z=547 $(M+H)^+$.

Step 72b: Preparation of tert-butyl (3S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (compound 1006-73-1): A mixture of tert-butyl (S)-4-(6,7-dichloro-1(*M*)-(2-isopropyl-4-methylpyridin-3-yl)-2-oxo-1,2-dihydropyrido[2,3-d]pyrimidin-4-yl)-3-methylpiperazine-1-carboxylate (1.0 g, 1.8 mmol, 1.0 eq.), 2-(6-fluorobenzofuran-7-yl)-4,4,5,5-tetramethyl-1,3,2-dioxaborolane (720 mg, 2.7 mmol, 1.5 eq.), sodium carbonate(382 mg, 3.6 mmol, 2.0 eq.), copper(I) chloride (178 mg, 1.8 mmol, 1.0 eq.) and tetrakis(triphenylphosphine)palladium (208 mg, 0.18 mmol, 0.1 eq.) in acetonitrile/water=10/1 (22 mL) was stirred at 82°C overnight under the protection of nitrogen. After cooling to room temperature, the mixture was poured into water, and then extracted with ethyl acetate. The organic layer was washed with brine and concentrated in vacuo. The residue was purified by column chromatography on silica gel (eluent: dichloromethane/methanol=150/1 to 30/1) to give tert-butyl (3S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (1.2g, yield: 100%) as a yellow solid. MS ($ES^+$): m/z=647 $(M+H)^+$.

Step 72c: Preparation of 4-((S)-4-acryloyl-2-methylpiperazin-1-yl)-6-chloro-7-(6-fluorobenzofuran-7-yl)-1(*M*)-(2-isopropyl-4-methylpyridin-3-yl)pyrido[2,3-d]pyrimidin-2(1H)-one (compound 73-1): To a solution of tert-butyl (3S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (1006-73-1) (1.2 g, 1.86 mmol, 1.0 eq.) in dichloromethane (10 mL) was added TFA (4 mL), and the mixture was stirred at room temperature for 1 h. The solvent was removed in vacuo. The residue was dissolved in tetrahydrofuran (20 mL), and DIPEA (720 mg, 5.58 mmol, 3.0 eq.) was added. A solution of acryloyl chloride (202 mg, 2.23 mmol, 1.2 eq.) in tetrahydrofuran (2 mL) was added dropwise to the mixture in an ice-water bath. The mixture was stirred for 30 min. Water was added, then ethyl acetate was added to extract. The organic layer was concentrated in vacuo. The residue was purified by column chromatography on silica gel (eluent: dichloromethane/methanol=150/1 to 30/1) to give 4-((S)-4-acryloyl-2-methylpiperazin-1-yl)-6-chloro-7-(6-fluorobenzofuran-7-yl)-1(*M*)-(2-isopropyl-4-methylpyridin-3-yl)pyrido[2,3-d]pyrimidin-2(1H)-one as a yellow solid (850 mg, yield: 77.3%). MS ($ES^+$): m/z=601 $(M+H)^+$. $^1H$ NMR (500 MHz, DMSO) δ 8.53 (s, 1H), 8.34 (d, *J* = 4.9 Hz, 1H), 7.95 (d, *J* = 29.9 Hz, 1H), 7.77 (dd, *J* = 8.6, 5.3 Hz, 1H), 7.24 (dd, *J* = 10.2, 8.7 Hz, 1H), 7.16 (d, *J* = 4.9 Hz, 1H), 7.02 (d, *J* = 2.1 Hz, 1H), 6.87 (dd, *J* = 25.7, 14.0 Hz, 1H), 6.22 (d, *J* = 16.3 Hz, 1H), 5.77 (dd, *J* = 10.4, 2.3 Hz, 1H), 4.96 (s, 1H), 4.35 (m, 2H), 4.09 (m, 1H), 3.68 (m, 2H), 3.18 (d, *J*= 5.3 Hz, 1H), 2.75 (s, 1H), 1.94 (d, *J* = 21.7 Hz, 3H), 1.38 (d, *J* = 6.7 Hz, 3H), 1.08 (d, *J* = 6.7 Hz, 3H), 0.92 (d, *J* = 6.7 Hz, 3H).

**Example 73: Preparation of 4-((S)-4-acryloyl-2-methylpiperazin-1-yl)-6-chloro-7-(6-fluorobenzofuran-7-yl)-1(*P*)-(2-isopropyl-4-methylpyridin-3-yl)pyrido[2,3-d]pyrimidin-2(1H)-one (compound 73-2) (Preparation according to Scheme 10)**

Step 73a: Preparation oftert-butyl (S)-4-(6,7-dichloro-1(*P*)-(2-isopropyl-4-methylpyridin-3-yl)-2-oxo-1,2-dihydropyrido[2,3-d]pyrimidin-4-yl)-3-methylpiperazine-1-carboxylate (compound 1008-73-2) : The synthetic method was similar to Step 72a of Example 72, except that 6,7-dichloro-1(*M*)-(2-isopropyl-4-methylpyridin-3-yl)pyrido[2,3-d]pyrimidine-2,4(1H,3H)-dione (1002-73) was replaced by 6,7-dichloro-1(*P*)-(2-isopropyl-4-methylpyridin-3-yl)pyrido[2,3-d]pyrimidine-2,4(1H,3H)-dione (1003-73) (5.0 g, 13.7 mmol, 1.0 eq.), tert-butyl (S)-4-(6,7-dichloro-1(*P*)-(2-isopropyl-4-methylpyridin-3-yl)-2-oxo-1,2-dihydropyrido[2,3-d]pyrimidin-4-yl)-3-methylpiperazine-1-carboxylate (5.9 g, yield: 78.7%) was obtained as a white solid. MS ($ES^+$): m/z=547 $(M+H)^+$.

Step 73b: Preparation of tert-butyl (3S)-4-(6-chloro-7-(6-fluorobenzofuran-7-yl)-1(*P*)-(2-isopropyl-4-methylpyridin-3-yl)-2-oxo-1,2-dihydropyrido[2,3-d]pyrimidin-4-yl)-3-methylpiperazine-1-carboxylate (compound 1008-73-2) : The synthetic method was similar to Step 72b of Example 72, except that tert-butyl (S)-4-(6,7-dichloro-1(*M*)-(2-isopropyl-4-methylpyridin-3-yl)-2-oxo-1,2-dihydropyrido[2,3-d]pyrimidin-4-yl)-3-methylpiperazine-1-carboxylate (1004-73-1) was replaced by tert-butyl (S)-4-(6,7-dichloro-1(*P*)-(2-isopropyl-4-methylpyridin-3-yl)-2-oxo-1,2-dihydropyrido[2,3-d]pyrimidin-4-yl)-3-methylpiperazine-1-carboxylate (compound 1008-73-2) (5.7 g, 10.4 mmol, 1.0 eq.), tert-butyl (3S)-4-(6-chloro-7-(6-fluorobenzofuran-7-yl)-1(*P*)-(2-isopropyl-4-methylpyridin-3-yl)-2-oxo-1,2-dihydropyrido[2,3-d]pyrimidin-4-yl)-3-methylpiperazine-1-carboxylate (7.0 g, yield: 104.5%) was obtained as a yellow solid. MS ($ES^+$): m/z=647 $(M+H)^+$.

Step 73c: Preparation of 4-((S)-4-acryloyl-2-methylpiperazin-1-yl)-6-chloro-7-(6-fluorobenzofuran-7-yl)-1(*P*)-(2-isopropyl-4-methylpyridin-3-yl)pyrido[2,3-d]pyrimidin-2(1H)-onee (compound 73-2) : The synthetic method was similar to Step 72c of Example 72, except that tert-butyl (3S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (1006-73-1) was replaced by tert-butyl (3S)-4-(6-chloro-7-(6-fluorobenzofuran-7-yl)-1(P)-(2-isopropyl-4-methylpyridin-3-yl)-2-oxo-1,2-dihydropyrido[2,3-d]pyrimidin-4-yl)-3-methylpiperazine-1-carboxylate (1010-73-2) (5.7 g, 10.4 mmol, 1.0 eq.), 4-((S)-4-acryloyl-2-methylpiperazin-1-yl)-6-chloro-7-(6-fluorobenzofuran-7-yl)-1(P)-(2-isopropyl-4-methylpyridin-3-yl)pyrido[2,3-d]pyrimidin-2(1H)-one (5.0 g, yield: 76.9%) was obtained as a yellow solid. MS ($ES^+$): m/z=601 $(M+H)^+$. $^1H$ NMR (500 MHz, DMSO) $\delta$ 8.55 (s, 1H), 8.34 (d, *J* = 4.8 Hz, 1H), 7.95 (d, *J* = 30.0 Hz, 1H), 7.76 (dd, *J* = 8.6, 5.3 Hz, 1H), 7.24 (dd, *J* = 10.2, 8.7 Hz, 1H), 7.15 (d, *J* = 4.8 Hz, 1H), 7.01 (d, *J* = 2.0 Hz, 1H), 6.94 - 6.74 (m, 1H), 6.21 (d, *J* = 16.0 Hz, 1H), 5.77 (dd, *J* = 10.4, 2.3 Hz, 1H), 5.02 (s, 1H), 4.44 - 4.25 (m, 2H), 4.10 (dd, *J* = 57.1, 12.4 Hz, 1H), 3.91 - 3.43 (m, 2H), 3.11 (s, 1H), 2.76 (s, 1H), 1.93 (d, *J*= 25.5 Hz, 3H), 1.35 (d, *J* = 6.7 Hz, 3H), 1.14 - 1.01 (m, 3H), 0.92 (d, *J* = 6.7 Hz, 3H).

**Example 74: Preparation of 4-((S)-4-acryloyl-2-methylpiperazin-1-yl)-7-(5,6-difluorobenzofuran-7-yl)-6-fluoro-1(*M*)-(2-isopropyl-4-methylpyridin-3-yl)pyrido[2,3-d]pyrimidin-2(1H)-one (compound 74-1) (Preparation according to Scheme 10)**

Step 74a: Preparation of tert-butyl (S)-4-(6-fluoro-1(*M*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (compound 1005-74-1): Tert-butyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate (1004-70-1) (474 mg, 0.894 mmol, 1 eq.), hexamethyldistannane (586 mg, 1.788 mmol, 2 eq.) and tetrakis(triphenylphosphine)palladium (103 mg, 0.09 mmol, 0.1 eq.) were added into toluene (15 mL). The mixture was stirred at 110°C for 2h under the protection of nitrogen. The reaction was cooled to room temperature. The mixture was concentrated and purified by column chromatography on silica gel ( eluent: petroleum ether/ethyl acetate=5/1-1/2) to give tert-butyl (S)-4-(6-fluoro-1(*M*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (400 mg, 67.79% as a yellow solid). MS (ESI): *m/z* 661$(M+H)^+$.

Step 74b: Preparation of tert-butyl (3S)-4-(7-(5,6-difluorobenzofuran-7-yl)-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate (compound 1006-74-1): Tert-butyl (S)-4-(6-fluoro-1(*M*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (1005-74-1) (300 mg, 0.454 mmol, 1 eq.), 5,6-difluoro-7-iodobenzofuran (0901-74) (190 mg, 0.681 mmol, 1.5 eq.), CuI (8.6 mg, 0.045 mmol, 0.1 eq.) and tetrakis(triphenylphosphine)palladium (52 mg, 0.045 mmol, 0.1 eq.) were added into toluene (15 mL). The mixture was stirred at 125°C for 16h under the protection of nitrogen. The reaction was cooled to room temperature. The mixture was concentrated and purified by column chromatography on silica gel ( eluent: petroleum ether/ethyl acetate=10/1-1/3) to give tert-butyl (3S)-4-(7-(5,6-difluorobenzofuran-7-yl)-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate(200 mg, 67.91%) as a yellow solid . MS (ESI): *m/z* 649$(M+H)^+$.

Step 74c: Preparation of 4-((S)-4-acryloyl-2-methylpiperazin-1-yl)-7-(5,6-difluorobenzofuran-7-yl)-6-fluoro-1(M)-(2-isopropyl-4-methylpyridin-3-yl)pyrido[2,3-**d]pyrimidin-2(1H)-one** (compound 74-1): tert-butyl (3S)-4-(7-(5,6-difluorobenzofuran-7-yl)-6-fluoro-1(M)-(2-isopropyl-4-methylpyridin-3-yl)-2-oxo-1,2-dihydropyrido[2,3-d]pyrimidin-4-yl)-3-methylpiperazine-1-carboxylate (1006-74-1) (200 mg, 0.308 mmol, 1.0 eq.) was dissolved in 4 mL dichloromethane, and 1 mL TFA was added into the mixture. The mixture was stirred at room temperature for 2h. Then, the mixture was concentrated. The residue was dissolved in tetrahydrofuran (5 mL). DIPEA (119 mg, 0.924 mmol, 3 eq.) was added into the mixture. Acryloyl chloride (28 mg, 0.308 mmol, 1.0 eq.) was added into the mixture. The mixture was stirred at room temperature for 15 min. Then, the reaction was quenched with ammonium hydroxide. The mixture was extracted with dichloromethane. The organic phase was concentrated and purified by column chromatography on silica gel ( eluent: dichloromethane/methanol=200/1-20/1) to give 4-((S)-4-acryloyl-2-methylpiperazin-1-yl)-7-(5,6-difluorobenzofuran-7-yl)-6-fluoro-1(*M*)-(2-isopropyl-4-methylpyridin-3-yl)pyrido[2,3-d]pyrimidin-2(1H)-one as a yellow solid (118 mg, 63.5%). MS (ESI): *m*/*z* 603(M+H) $^+$. $^1$H NMR (500 MHz, DMSO) δ 8.41 (m, 1H), 8.35 (d, *J* = 4.9 Hz, 1H), 8.01 (d, *J* = 2.1 Hz, 1H), 7.86 (dd, *J* = 10.0, 7.9 Hz, 1H), 7.17 (d, *J* = 4.9 Hz, 1H), 7.01 (d, *J* = 2.2 Hz, 1H), 6.84 (dd, *J* = 25.7, 14.5 Hz, 1H), 6.19 (d, *J* = 16.4 Hz, 1H), 5.75 (dd, *J* = 10.4, 2.2 Hz, 1H), 4.90 (s, 1H), 4.31 (dd, *J* = 48.3, 13.4 Hz, 2H), 4.07 (dd, *J* = 61.9, 12.8 Hz, 1H), 3.59 (m, 2H), 3.20 (m, 1H), 2.76 (dt, *J* = 12.9, 6.3 Hz, 1H), 1.92 (s, 3H), 1.35 (d, *J* = 6.7 Hz, 3H), 1.06 (d, *J* = 6.7 Hz, 3H), 0.92 (d, *J* = 6.7 Hz, 3H).

**Example 75: Preparation of 4-((S)-4-acryloyl-2-methylpiperazin-1-yl)-7-(5,6-difluorobenzofuran-7-yl)-6-fluoro-1(*P*)-(2-isopropyl-4-methylpyridin-3-yl)pyrido[2,3-d]pyrimidin-2(1H)-one (compound 74-2) (Preparation according to Scheme 10)**

Step 75a: Preparation of tert-butyl (S)-4-(6-fluoro-1(*P*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylatee (compound 1009-74-2) : The synthetic method was similar to Step 74a of Example 74, except thattert-butyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate (1004-70-1) was replaced by tert-butyl (S)-4-(7-chloro-6-fluoro-1(*P*)-(2-isopropyl-4-methylpyridin-3-yl)-2-oxo-1,2-dihydropyrido[2,3-d]pyrimidin-4-yl)-3-methylpiperazine-1-carboxylate (300 mg, 0.56 mmol, 1.0 eq.), tert-butyl (S)-4-(6-fluoro-1(*P*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (200 mg, yield: 53.6%) was obtained as a yellow solid. MS (ESI): *m*/*z* 661(M+H)$^+$.

Step 75b: Preparation of tert-butyl (3S)-4-(7-(5,6-difluorobenzofuran-7-yl)-6-fluoro-1(*P*)-(2-isopropyl-4-methylpyridin-3-yl)-2-oxo-1,2-dihydropyrido[2,3-d]pyrimidin-4-yl)-3-methylpiperazine-1-carboxylate (compound 1010-74-2) : The synthetic method was similar to Step 74b of Example 74, except tert-butyl (S)-4-(6-fluoro-1(*M*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate(1005-74-1) was replaced by tert-butyl (S)-4-(6-fluoro-1(*P*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (1009-74-2) (200 mg, 0.3 mmol, 1 eq.), tert-butyl (3 S)-4-(7-(5,6-difluorobenzofuran-7-yl)-6-fluoro-1(*P*)-(2-isopropyl-4-methylpyridin-3-yl)-2-oxo-1,2-dihydropyrido[2,3-d]pyrimidin-4-yl)-3-methylpiperazine-1-carboxylate (150 mg, yield: 76.5%)was obtained as a yellow solid. MS (ESI): *m*/*z* 649(M+H) $^+$.

Step 75c: Preparation of 4-((S)-4-acryloyl-2-methylpiperazin-1-yl)-7-(5,6-difluorobenzofuran-7-yl)-6-fluoro-1(*P*)-(2-isopropyl-4-methylpyridin-3-yl)pyrido[2,3-d]pyrimidin-2(1H)-one (compound 74-2) : The synthetic method was similar to Step 74c of Example 74, except that tert-butyl (3S)-4-(7-(5,6-difluorobenzofuran-7-yl)-6-fluoro-1(*M*)-(2-isopropyl-4-methylpyridin-3-yl)-2-oxo-1,2-dihydropyrido[2,3-d]pyrimidin-4-yl)-3-methylpiperazine-1-carboxylate (1006-74-1) was replaced by tert-butyl (3S)-4-(7-(5,6-difluorobenzofuran-7-yl)-6-fluoro-1(*P*)-(2-isopropyl-4-methylpyridin-3-yl)-2-oxo-1,2-dihydropyrido[2,3-d]pyrimidin-4-yl)-3-methylpiperazine-1-carboxylate (1010-74-2), 4-((S)-4-acryloyl-2-methylpiperazin-1-yl)-7-(5,6-difluorobenzofuran-7-yl)-6-fluoro-1(P)-(2-isopropyl-4-methylpyridin-3-yl)pyrido[2,3-d]pyrimidin-2(1H)-one (100 mg, yield: 71.9%) was obtained as a yellow solid. MS (ESI): *m*/*z* 603(M+H) $^+$. $^1$H NMR (500 MHz, DMSO) δ 8.47 (d, *J* = 9.0 Hz, 1H), 8.35 (d, *J* = 4.9 Hz, 1H), 8.01 (d, *J* = 2.1 Hz, 1H), 7.86 (dd, *J* = 10.0, 7.9 Hz, 1H), 7.17 (d, *J* = 4.9 Hz, 1H), 7.01 (d, *J* = 2.2 Hz, 1H), 6.83 (m, 1H), 6.19 (d, *J* = 16.6 Hz, 1H), 5.75 (dd, *J*= 10.4, 2.3 Hz, 1H), 4.99 (m, 1H), 4.35 (dd, *J*= 48.9, 12.1 Hz, 2H), 4.08 (dd, *J* = 56.4, 12.9 Hz, 1H), 3.63 (m, 2H), 3.12 (dd, J= 49.4, 37.7 Hz, 1H), 2.75 (d, *J*= 5.6 Hz, 1H), 1.93 (s, 3H), 1.31 (d, *J*= 6.7 Hz, 3H), 1.06 (d, *J* = 6.7 Hz, 3H), 0.92 (d, *J* = 6.7 Hz, 3H).

**Example 76: Preparation of 4-((2S,5R)-4-acryloyl-2,5-dimethylpiperazin-1-yl)-6-fluoro-7-(6-fluorobenzofuran-7-yl)-1(M)-(2-isopropyl-4-methylpyridin-3-yl)pyrido[2,3-d]pyrimidin-2(1H)-one (compound 89-1) (Preparation according to Scheme 10)**

Step 73a: Preparation of tert-butyl (2R,5S)-4-(7-chloro-6-fluoro-1(*M*)-(2-isopropyl-4-methylpyridin-3-yl)-2-oxo-1,2-dihydropyrido[2,3-d]pyrimidin-4-yl)-2,5-dimethylpiperazine-1-carboxylate (compound 1004-89-1): A mixture of 7-chloro-6-fluoro-1(*M*)-(2-isopropyl-4-methylpyridin-3-yl)pyrido[2,3-d]pyrimidine-2,4(1*H*,3*H*)-dione (1002-1) (1.0 g, 2.87 mmol, 1.0 eq.), DIPEA (1.85 g, 14.35 mmol, 5.0 eq.) and phosphorus oxychloride (1.32 g, 8.61 mmol, 3.0 eq.) in acetonitrile (50 mL) was stirred at 85°C for 2 h under the protection of nitrogen. After cooling to room temperature, the solvent was removed in vacuo. The residue was dissolved in tetrahydrofuran (50 mL), and cooled in an ice-water bath. DIPEA (1.11

g, 8.61 mmol, 3.0 eq.) and tert-butyl (2R,5S)-2,5-dimethylpiperazine-1-carboxylate (615 mg, 2.87 mmol, 1.0 eq.) were added. The mixture was stirred for 1 h. ethyl acetate was added, and the mixture were washed with water and brine. The organic phase was concentrated in vacuo. The residue was purified by column chromatography on silica gel (eluent: dichloromethane/methanol=200/1 to 50/1) to give tert-butyl (2R,5S)-4-(7-chloro-6-fluoro-1(M)-(2-isopropyl-4-methylpyridin-3-yl)-2-oxo-1,2-dihydropyrido[2,3-d]pyrimidin-4-yl)-2,5-dimethylpiperazine-1-carboxylate (1.22 g, yield: 78%) as a yellow solid. MS ($ES^+$): *m/z=545* $(M+H)^+$.

Step 76b: Preparation of tert-butyl (2*R*,5*S*)-4-(6-fluoro-7-(6-fluorobenzofuran-7-yl)-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1006-89-1): 2-(6-Fluorobenzofuran-7-yl)-4,4,5,5-tetramethyl-1,3,2-dioxaborolane (0903-73) (511 mg, 1.95 mmol, 1.3 eq.), tert-butyl (2R,5S)-4-(7-chloro-6-fluoro-1(*M*)-(2-isopropyl-4-methylpyridin-3-yl)-2-oxo-1,2-dihydropyrido[2,3-d]pyrimidin-4-yl)-2,5-dimethylpiperazine-1-carboxylate (1004-89-1) (816 mg, 1.50 mmol, 1.0 eq.), tetrakis(triphenylphosphine)palladium (174 mg, 0.15 mmol, 0.1 eq.), copper(I) chloride (149 mg, 1.5 mmol, 1.0 eq.) and sodium carbonate (318 mg, 3.0 mmol, 2.0 eq.) were added into a mixed solvent of 45 mL acetonitrile and 3.8 mL water. The mixture was heated at 80°C overnight under the protection of nitrogen.. The mixture was cooled to room temperature, and extracted with ethyl acetate. The organic phase was washed with water, and concentrated under vacuum. The residue was purified by column chromatography on silica gel (eluent: dichloromethane/methanol=150/1 to 30/1) to give tert-butyl (2R,5S)-4-(6-fluoro-7-(6-fluorobenzofuran-7-yl)-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (962 mg, yield: 99%) as a yellow solid. MS ($ES^+$): *m/z*=645$(M+H)^+$.

Step 76c: Preparation of 4-((2*S*,5*R*)-4-acryloyl-2,5-dimethylpiperazin-1-yl)-6-fluoro-7-(6-fluorobenzofuran-7-yl)-1(*M*)-(2-isopropyl-4-methylpyridin-3-yl)pyrido[2,3-d]pyrimidin-2(1H)-one (compound 89-1): tert-butyl (2*R*,5*S*)-4-(6-fluoro-7-(6-fluorobenzofuran-7-yl)-1(M)-(2-isopropyl-4-methylpyridin-3-yl)-2-oxo-1,2-dihydropyrido[2,3-*d*]pyrimidin-4-yl)-2,5-dimethylpiperazine-1-carboxylate (1006-89-1) (962 mg, 1.49 mmol, 1.0 eq.) was dissolved in 80 mL dichloromethane. TFA (12 mL) was added dropwise, and the mixture was stirred at room temperature for 2.0 h. The mixture was concentrated under vacuum to dryness. The residue was dissolved in 50 mL tetrahydrofuran, and 2 mL DIPEA was added. The mixture was cool in an ice-water bath. Acryloyl chloride (149 mg, 1.64 mmol, 1.1 eq.) was dissolved in 0.5 mL tetrahydrofuran, and added dropwise into the mixture above. The mixture was stirred for 1 h, and diluted with ethyl acetate. The organic phase was washed with water, and concentrated under vacuum. The residue was purified by column chromatography on silica gel (eluent: dichloromethane/methanol=150/1 to 30/1) to give 4-((2*S*,5*R*)-4-acryloyl-2,5-dimethylpiperazin-1-yl)-6-fluoro-7-(6-fluorobenzofuran-7-yl)-1(*M*)-(2-isopropyl-4-methylpyridin-3-yl)pyrido[2,3-*d*]pyrimidin-2(1H)-one (300 mg, yield: 33%) as a yellow solid. MS ($ES^+$): *m/z=599* $(M+H)^+$. Melting point: 275°C ~277°C. $^1H$ NMR (500 MHz, DMSO) δ 8.38 (dd, *J* = 14.4, 6.8 Hz, 2H), 7.95 (d, *J*= 2.1 Hz, 1H), 7.80 (dd, *J* = 8.6, 5.2 Hz, 1H), 7.26 (dd, *J* = 10.4, 8.7 Hz, 1H), 7.18 (d, *J* = 4.9 Hz, 1H), 7.02 (d, *J* = 2.2 Hz, 1H), 6.84 (ddd, *J* = 29.8, 16.6, 10.5 Hz, 1H), 6.19 (d, *J* = 16.7 Hz, 1H), 5.75 (ddd, *J* = 10.4, 5.4, 2.1 Hz, 1H), 4.80 (m, 2H), 3.90 (m, 4H), 2.75 (dq, *J*= 13.2, 6.6 Hz, 1H), 1.95 (s, 3H), 1.36 (m, 3H), 1.25 (dd, *J* = 23.4, 9.8 Hz, 3H), 1.07 (d, *J*= 6.7 Hz, 3H), 0.95 (dd, *J*= 6.5, 2.7 Hz, 3H).

**Example 77: Preparation of 4-((2*S*,5*R*)-4-acryloyl-2,5-dimethylpiperazin-1-yl)-6-fluoro-7-(6-fluorobenzofuran-7-yl)-1(*P*)-(2-isopropyl-4-methylpyridin-3-yl)pyrido[2,3-*d*]pyrimidin-2(1H)-one (compound 89-2) (Preparation according to Scheme 10)**

Step 77a: Preparation of tert-butyl (2*R*,5*S*)-4-(7-chloro-6-fluoro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1008-89-2) : The synthetic method was similar to Step 76a of Example 76, except that 7-chloro-6-fluoro-1(*M*)-(2-isopropyl-4-methylpyridin-3-yl)pyrido[2,3-*d*]pyrimidine-2,4(1*H*,3*H*)-dione (1002-1) was replaced by 7-chloro-6-fluoro-1(*P*)-(2-isopropyl-4-methylpyridin-3-yl)pyrido[2,3-*d*]pyrimidine-2,4(1*H*,3*H*)-dione (1003-1) (300 mg, 0.86 mmol, 1.0 eq.), tert-butyl (2*R*,5*S*)-4-(7-chloro-6-fluoro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (330 mg, yield: 69.4%) was obtained as a yellow solid.

Step 77b: Preparation of tert-butyl (2*R*,5*S*)-4-(6-fluoro-7-(6-fluorobenzofuran-7-yl)-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1011-89-2) : The synthetic method was similar to Step 76b of Example 76, except that tert-butyl (2*R*,5*S*)-4-(7-chloro-6-fluoro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1004-89-1) was replaced by tert-butyl (2*R*,5*S*)-4-(7-chloro-6-fluoro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (1008-89-2) (150 mg, 0.275 mmol, 1.00 eq.), tert-butyl (2*R*,5*S*)-4-(6-fluoro-7-(6-fluorobenzofuran-7-yl)-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (120 mg, yield: 70.1%) was obtained as a pale yellow solid. MS ($ES^+$): m/z=645 $(M+H)^+$.

Step 77c: Preparation of 4-((2*S*,5*R*)-4-acryloyl-2,5-dimethylpiperazin-1-yl)-6-fluoro-7-(6-fluorobenzofuran-7-yl)-1(*P*)-(2-isopropyl-4-methylpyridin-3-yl)pyrido[2,3-*d*]pyrimidin-2(1*H*)-one (compound 89-2) : The synthetic method was similar to Step 76c of Example 76, except that tert-butyl (2*R*,5*S*)-4-(6-fluoro-7-(6-fluorobenzofuran-7-yl)-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1006-89-1) was replaced by tert-butyl (2*R*,5*S*)-4-(6-fluoro-7-(6-fluorobenzofuran-7-yl)-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (1011-89-2) (120 mg, 0.186 mmol, 1.00 eq.), 4-((2*S*,5*R*)-4-acryloyl-2,5-dimethylpiperazin-1-yl)-6-fluoro-7-(6-fluorobenzofuran-7-yl)-1(*P*)-(2-isopropyl-4-methylpyridin-3-yl)pyrido[2,3-*d*]pyrimidin-2(1*H*)-one (92 mg, yield: 71.8%) was obtained as a yellow solid. MS ($ES^+$): m/z=599 $(M+H)^+$. $^1H$ NMR (500 MHz, DMSO) δ 8.45 (d, *J* = 9.2 Hz, 1H), 8.36 (d, *J* = 4.9 Hz, 1H), 7.96 (d, *J* = 1.2 Hz, 1H), 7.80 (dd, *J* = 8.4, 5.3 Hz, 1H), 7.26 (t, *J* = 9.5 Hz, 1H), 7.19 (d, *J* = 4.8 Hz, 1H), 7.02 (s, 1H), 6.83 (ddd, *J*= 20.2, 16.8, 10.5 Hz, 1H), 6.19 (dd, *J* = 16.7, 2.0 Hz, 1H), 5.75 (m, 1H), 4.72 (m, 2H), 3.81 (m, 4H), 2.74 (dt, *J*= 6.4, 5.5 Hz, 1H), 1.98 (s, 3H), 1.32 (dd, *J*= 10.9, 6.7 Hz, 3H), 1.21 (dd, *J*= 40.9, 6.7 Hz, 3H), 1.07 (d, *J*= 6.7 Hz, 3H), 0.94 (d, *J*= 6.7 Hz, 3H).

**Example 78: Preparation of 4-((2*S*,5*R*)-4-acryloyl-2,5-dimethylpiperazin-1-yl)-6-chloro-7-(6-fluorobenzofuran-7-yl)-1(*M*)-(2-isopropyl-4-methylpyridin-3-yl)pyrido[2,3-*d*]pyrimidin-2(1*H*)-one (compound 90-1) (Preparation according to Scheme 10)**

Step 78a: Preparation of tert-butyl (2*R*,5*S*)-4-(6,7-dichloro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1004-90-1): A mixture of 6,7-dichloro-1(*M*)-(2-isopropyl-4-methylpyridin-3-yl)pyrido[2,3-*d*]pyrimidin-2(1*H*)-one (1002-73) (1.0 g, 2.74 mmol, 1.0 eq.), DIPEA (1.77 g, 13.70 mmol, 5.0 eq.) and phosphorus oxychloride (1.26 g, 8.22 mmol, 3.0 eq.) in acetonitrile (50 mL) was stirred at 85°C for 2 h under the protection of nitrogen. After cooling to room temperature, the solvent was removed in vacuo. The residue was dissolved in tetrahydrofuran (50 mL), and cooled in an ice-water bath. DIPEA (1.06 g, 8.22 mmol, 3.0 eq.) and tert-butyl (2*R*,5*S*)-2,5-dimethylpiperazine-1-carboxylate (0111-89) (587 mg, 2.74 mmol, 1.0 eq.) were added. The mixture was stirred for 1 h. ethyl acetate was added, and the mixture was washed with water and brine. The organic phase was concentrated in vacuo. The residue was purified by column chromatography on silica gel (eluent: dichloromethane/methanol=200/1 to 50/1) to give tert-butyl (2*R*,5*S*)-4-(6,7-dichloro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1.37 g, yield: 88%) as a yellow solid. MS ($ES^+$): *m*/*z*=561 $(M+H)^+$.

Step 78b: Preparation of tert-butyl (2*R*,5*S*)-4-(6-chloro-7-(6-fluorobenzofuran-7-yl)-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1006-90-1): 2-(6-Fluorobenzofuran-7-yl)-4,4,5,5-tetramethyl-1,3,2-dioxaborolane (0903-73) (590 mg, 2.25 mmol, 1.5 eq.), tert-butyl (2*R*,5*S*)-4-(6,7-dichloro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1004-90-1) (842 mg, 1.5 mmol, 1.0 eq.), tetrakis(triphenylphosphine)palladium (174 mg, 0.15 mmol, 0.1 eq.), copper(I) chloride (149 mg, 1.5 mmol, 1.0 eq.) and sodium carbonate (318 mg, 3.0 mmol, 2.0 eq.) were added into a mixed solvent of 45 mL acetonitrile and 3.8 mL water. The mixture was heated at 80°C overnight under the protection of nitrogen. The mixture was cooled to room temperature, and extracted with ethyl acetate. The organic phase was washed with water, and concentrated under vacuum. The residue was purified by column chromatography on silica gel (eluent: dichloromethane/methanol=150/1 to 30/1) to give tert-butyl (2*R*,5*S*)-4-(6-chloro-7-(6-fluorobenzofuran-7-yl)-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate as a yellow solid (944 mg, yield: 95%). MS ($ES^+$): *m*/*z*=661$(M+H)^+$.

Step 78c: Preparation of 4-((2*S*,5*R*)-4-acryloyl-2,5-dimethylpiperazin-1-yl)-6-chloro-7-(6-fluorobenzofuran-7-yl)-1(*M*)-(2-isopropyl-4-methylpyridin-3-yl)pyrido[2,3-*d*]pyrimidin-2(1*H*)-one (compound 90-1): tert-butyl (2*R*,5*S*)-4-(6-chloro-7-(6-fluorobenzofuran-7-yl)-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1006-90-1) (944 mg, 1.43 mmol, 1.0 eq.) was dissolved in 66 mL dichloromethane.TFA (10 mL) was added dropwise, and the mixture was stirred at room temperature for 2.0 h. The mixture was concentrated under vacuum to dryness. The residue was dissolved in 40 mL tetrahydrofuran, and 2 mL DIPEA was added. The mixture was cool in an ice-water bath. Acryloyl chloride (143 mg, 1.58 mmol, 1.1 eq.) was dissolved in 0.5 mL tetrahydrofuran, and added dropwise into the mixture above. The mixture was stirred for 30 min, and diluted with ethyl acetate. The organic phase was washed with water, and concentrated under vacuum. The residue was purified by column chromatography on silica gel (eluent: dichloromethane/methanol=150/1 to 30/1) to give 4-((2*S*,5*R*)-4-acryloyl-2,5-dimethylpiperazin-1-yl)-6-chloro-7-(6-fluorobenzofuran-7-yl)-1(*M*)-(2-isopropyl-4-methylpyridin-3-yl)pyrido[2,3-*d*]pyrimidin-2(1*H*)-one (500 mg, yield: 56%) as a yellow solid. MS ($ES^+$): *m*/*z*=615 $(M+H)^+$. Melting point: 175°C~177°C. $^1H$ NMR (500 MHz, DMSO) δ 8.51 (d, *J* = 7.7 Hz, 1H), 8.34 (d, *J* = 4.8 Hz, 1H), 7.95 (d, *J* = 25.0 Hz, 1H), 7.76 (dd, *J* = 8.2, 5.4 Hz, 1H), 7.24 (t, *J* = 9.4 Hz, 1H), 7.15 (d, *J* = 4.7 Hz, 1H), 7.01 (s, 1H), 6.83 (m, 1H), 6.19 (d, *J*= 16.7 Hz, 1H), 5.75 (m, 1H), 4.76 (dd, *J* = 117.1, 84.1 Hz, 2H), 3.91 (m, 4H), 2.74 (s, 1H), 1.93 (d, *J*= 17.6 Hz, 3H), 1.36 (t, *J*= 5.5 Hz, 3H), 1.25 (dd, *J* = 32.9, 6.7 Hz, 3H), 1.06 (d, *J* = 6.7 Hz, 3H), 0.93 (d, *J*= 5.0 Hz, 3H).

Example 79: **Preparation of 4-((2*S*,5*R*)-4-acryloyl-2,5-dimethylpiperazin-1-yl)-6-chloro-7-(6-fluorobenzofuran-7-yl)-1(*P*)-(2-isopropyl-4-methylpyridin-3-yl)pyrido[2,3-*d*]pyrimidin-2(1*H*)-one (compound 90-2) (Preparation according to Scheme 10)**

Step 79a: Preparation of tert-butyl (2*R*,5*S*)-4-(6,7-dichloro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1008-90-2) : The synthetic method was similar to Step 78c of Example 78, except that 6,7-dichloro-1(*M*)-(2-isopropyl-4-methylpyridin-3-yl)pyrido[2,3-*d*]pyrimidin-2(1*H*)-one (1002-73) was replaced by 6,7-dichloro-1(*P*)-(2-isopropyl-4-methylpyridin-3-yl)pyrido[2,3-*d*]pyrimidin-2(1*H*)-one (1003-73) (400 mg, 1.09 mmol, 1.0 eq.), tert-butyl (2*R*,5*S*)-4-(6,7-dichloro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (220 mg, yield: 36.0%) was obtained as a yellow solid. MS ($ES^+$): m/z=561 $(M+H)^+$.

Step 79b: Preparation of tert-butyl (2*R*,5*S*)-4-(6-chloro-7-(6-fluorobenzofuran-7-yl)-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1010-90-2) : The synthetic method was similar to Step 78c of Example 78, except that tert-butyl (2*R*,5*S*)-4-(6,7-dichloro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate(1004-90-1) was replaced by tert-butyl (2*R*,5*S*)-4-(6,7-dichloro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate(1008-90-2) (150 mg, 0.267 mmol, 1.00 eq.), tert-butyl (2*R*,5*S*)-4-(6-chloro-7-(6-fluorobenzofuran-7-yl)-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylatee (160 mg, yield: 90.9%) was obtained as a pale yellow solid. MS ($ES^+$): m/z=661 $(M+H)^+$.

Step 79c: Preparation of 4-((2*S*,5*R*)-4-acryloyl-2,5-dimethylpiperazin-1-yl)-6-chloro-7-(6-fluorobenzofuran-7-yl)-1(*P*)-(2-isopropyl-4-methylpyridin-3-yl)pyrido[2,3-*d*]pyrimidin-2(1*H*)-one (compound 90-2) : The synthetic method was similar to Step 78c of Example 78, except that tert-butyl (2*R*,5*S*)-4-(6-chloro-7-(6-fluorobenzofuran-7-yl)-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate(1006-90-1) was replaced by tert-butyl (2*R*,5*S*)-4-(6-chloro-7-(6-fluorobenzofuran-7-yl)-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate(1010-90-2), 4-((2*S*,5*R*)-4-acryloyl-2,5-dimethylpiperazin-1-yl)-6-chloro-7-(6-fluorobenzofuran-7-yl)-1(*P*)-(2-isopropyl-4-methylpyridin-3-yl)pyrido[2,3-*d*]pyrimidin-2(1*H*)-one (102 mg, yield: 70.8%) was obtained as a yellow solid. MS ($ES^+$): m/z=615 $(M+H)^+$. Melting point: 152-161°C. $^1H$ NMR (500 MHz, DMSO) $\delta$ 8.55 (s, 1H), 8.33 (d, *J* = 4.9 Hz, 1H), 7.95 (d, *J* = 29.6 Hz, 1H), 7.76 (dd, *J* = 8.6, 5.2 Hz, 1H), 7.23 (m, 1H), 7.15 (d, *J* = 4.7 Hz, 1H), 7.00 (d, *J* = 1.8 Hz, 1H), 6.82 (td, *J* = 17.3, 10.5 Hz, 1H), 6.18 (dd, *J* = 16.7, 2.2 Hz, 1H), 5.74 (m, 1H), 4.71 (m, 2H), 3.94 (m, 4H), 2.71 (s, 1H), 1.95 (d, *J* = 22.7 Hz, 3H), 1.34 (t, *J* = 6.9 Hz, 3H), 1.21 (m, 3H), 1.05 (d, *J* = 6.7 Hz, 3H), 0.91 (d, *J* = 6.7 Hz, 3H).

**Example 80: Preparation of 4-((2*S*,5*R*)-4-acryloyl-2,5-dimethylpiperazin-1-yl)-7-(5,6-difluorobenzofuran-7-yl)-6-fluoro-1(*M*)-(2-isopropyl-4-methylpyridin-3-yl)pyrido[2,3-*d*]pyrimidin-2(1*H*)-one (compound 91-1) (Preparation according to Scheme 10)**

Step 80a: Preparation of tert-butyl (2*R*,5*S*)-4-(6-fluoro-1(*M*)-(2-isopropyl-4-methylpyridin-3-yl)-2-oxo-7-(trimethylstannyl)-1,2-dihydropyrido[2,3-*d*]pyrimidin-4-yl)-2,5-dimethylpiperazine-1-carboxylate (compound 1005-91-1): Tert-butyl (2*R*,5*S*)-4-(7-chloro-6-fluoro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1004-89-1) (300 mg, 0.55 mmol, 1.0 eq.), hexamethyldistannane (380 mg, 1.16 mmol, 2.1 eq.) and tetrakis(triphenylphosphine)palladium (64 mg, 0.055 mmol, 0.1 eq.) were added into dry toluene (50 mL), and the mixture was stirred at 110°C for 3 h under the protection of nitrogen. The mixture was diluted with ethyl acetate, and filtered through a celite pad. The filtrate was concentrated under vacuum. The residue was purified by column chromatography on silica gel (eluent: Petroleum ether/ethyl acetate=1/2 to 1/3) to give tert-butyl (2*R*,5*S*)-4-(6-fluoro-1(*M*)-(2-isopropyl-4-methylpyridin-3-yl)-2-oxo-7-(trimethylstannyl)-1,2-dihydropyrido[2,3-*d*]pyrimidin-4-yl)-2,5-dimethylpiperazine-1-carboxylate (332 mg, yield: 89%) as a yellow solid. MS ($ES^+$): *m*/*z*=675 $(M+H)^+$.

Step 80b: Preparation of tert-butyl (2*R*,5*S*)-4-(7-(5,6-difluorobenzofuran-7-yl)-6-fluoro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1006-91-1): Tert-butyl (2*R*,5*S*)-4-(6-fluoro-1(*M*)-(2-isopropyl-4-methylpyridin-3-yl)-2-oxo-7-(trimethylstannyl)-1,2-dihydropyrido[2,3-*d*]pyrimidin-4-yl)-2,5-dimethylpiperazine-1-carboxylate (1005-91-1) (332 mg, 0.49 mmol, 1.0 eq.), 5,6-difluoro-7-iodobenzofuran (0901-74) (206 mg, 0.74 mmol, 1.5 eq.), tetrakis(triphenylphosphine)palladium (58 mg, 0.05 mmol, 0.1 eq.) and CuI (9.5 mg, 0.05 mmol, 0.1 eq.) were added into dry toluene (40 mL). The mixture was stirred at 125°C overnight under the protection of nitrogen. The mixture was diluted with ethyl acetate. The organic phase was washed with water, and concentrated under vacuum. The residue was purified by column chromatography on silica gel (eluent: dichloromethane/methanol=100/1 to 30/1) to give tert-butyl (2*R*,5*S*)-4-(7-(5,6-difluorobenzofuran-7-yl)-6-fluoro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (210 mg, yield: 64%) as a yellow solid. MS ($ES^+$): *m*/*z*=663 $(M+H)^+$.

Step 80c: Preparation of 4-((2*S*,5*R*)-4-acryloyl-2,5-dimethylpiperazin-1-yl)-7-(5,6-difluorobenzofuran-7-yl)-6-fluoro-1(*M*)-(2-isopropyl-4-methylpyridin-3-yl)pyrido[2,3-*d*]pyrimidin-2(1*H*)-one (compound 91-1): tert-butyl (2*R*,5*S*)-4-(7-(5,6-difluorobenzofuran-7-yl)-6-fluoro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1006-91-1) (210 mg, 0.32 mmol, 1.0 eq.) was dissolved in 15 mL dichloromethane. TFA (1.5 mL) was added dropwise, and the mixture was stirred at room temperature for 2.0 h. The mixture was concentrated under vacuum to dryness. The residue was dissolved in 20 mL tetrahydrofuran, and 1 mL DIPEA was added. The mixture was cool in an ice-water bath. Acryloyl chloride (35 mg, 0.38 mmol, 1.2 eq.) was dissolved in 0.5 mL tetrahydrofuran, and added dropwise into the mixture above. The mixture was stirred for 30 min, and diluted with ethyl acetate. The organic phase was washed with water, and concentrated under vacuum. The residue was purified by prep-TLC (silica gel, eluent: dichloromethane/methanol=20/1) to give 4-((2*S*,5*R*)-4-acryloyl-2,5-dimethylpiperazin-1-yl)-7-(5,6-difluorobenzofuran-7-yl)-6-fluoro-1(*M*)-(2-isopropyl-4-methylpyridin-3-yl)pyrido[2,3-*d*]pyrimidin-2(1*H*)-one (35 mg, yield: 62%) as a yellow solid. MS (ES$^+$): *m/z*=617 (M+H)$^+$. Melting point: 156°C ~158°C. $^1$H NMR (500 MHz, DMSO) δ 8.43 (m, 2H), 8.03 (d, *J*= 2.1 Hz, 1H), 7.89 (dd, *J*= 9.8, 8.0 Hz, 1H), 7.32 (s, 1H), 7.04 (d, *J*= 2.1 Hz, 1H), 6.84 (ddd, *J*= 30.0, 16.6, 10.5 Hz, 1H), 6.19 (d, *J* = 16.7 Hz, 1H), 5.75 (m, 1H), 4.46 (m, 3H), 3.85 (m, 2H), 3.49 (d, *J*= 3.6 Hz, 1H), 2.83 (dd, *J* = 13.3, 6.7 Hz, 1H), 2.00 (s, 3H), 1.37 (dd, *J*= 6.3, 3.2 Hz, 3H), 1.26 (m, 3H), 1.10 (d, *J*= 6.7 Hz, 3H), 0.98 (dd, *J*= 6.5, 2.9 Hz, 3H).

**Example 81: Preparation of 4-((2*S*,5*R*)-4-acryloyl-2,5-dimethylpiperazin-1-yl)-7-(5,6-difluorobenzofuran-7-yl)-6-fluoro-1(*P*)-(2-isopropyl-4-methylpyridin-3-yl)pyrido[2,3-*d*]pyrimidin-2(1*H*)-one (compound 91-2) (Preparation according to Scheme 10)**

Step 81a: Preparation of tert-butyl (2*R*,5*S*)-4-(6-fluoro-1(*P*)-(2-isopropyl-4-methylpyridin-3-yl)-2-oxo-7-(trimethylstannyl)-1,2-dihydropyrido[2,3-*d*]pyrimidin-4-yl)-2,5-dimethylpiperazine-1-carboxylate (compound 1009-91-2) : The synthetic method was similar to Step 80a of Example 80, except that tert-butyl (2*R*,5*S*)-4-(7-chloro-6-fluoro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1004-89-1) was replaced by tert-butyl (2*R*,5*S*)-4-(7-chloro-6-fluoro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (1008-89-1) (165 mg, 0.3 mmol, 1.0 eq.), tert-butyl (2*R*,5*S*)-4-(6-fluoro-1(*P*)-(2-isopropyl-4-methylpyridin-3-yl)-2-oxo-7-(trimethylstannyl)-1,2-dihydropyrido[2,3-*d*]pyrimidin-4-yl)-2,5-dimethylpiperazine-1-carboxylate (160 mg, yield: 79.2%) was obtained as a yellow solid. MS (ES$^+$): m/z=675 (M+H)$^+$.

Step 81b: Preparation of tert-butyl (2*R*,5*S*)-4-(7-(5,6-difluorobenzofuran-7-yl)-6-fluoro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (compound 1010-91-2) : The synthetic method was similar to Step 80b of Example 80, except that tert-butyl (2*R*,5*S*)-4-(6-fluoro-1(*M*)-(2-isopropyl-4-methylpyridin-3-yl)-2-oxo-7-(trimethylstannyl)-1,2-dihydropyrido[2,3-*d*]pyrimidin-4-yl)-2,5-dimethylpiperazine-1-carboxylate (1005-91-1) was replaced by tert-butyl (2*R*,5*S*)-4-(6-fluoro-1(*P*)-(2-isopropyl-4-methylpyridin-3-yl)-2-oxo-7-(trimethylstannyl)-1,2-dihydropyrido[2,3-*d*]pyrimidin-4-yl)-2,5-dimethylpiperazine-1-carboxylate (1009-91-2) (160 mg, 0.237 mmol, 1.00 eq.), tert-butyl (2*R*,5*S*)-4-(7-(5,6-difluorobenzofuran-7-yl)-6-fluoro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (90 mg, yield: 57.6%) was obtained as a pale yellow solid. MS (ES$^+$): m/z=663 (M+H)$^+$.

Step 81c: Preparation of 4-((2*S*,5*R*)-4-acryloyl-2,5-dimethylpiperazin-1-yl)-7-(5,6-difluorobenzofuran-7-yl)-6-fluoro-1(*P*)-(2-isopropyl-4-methylpyridin-3-yl)pyrido[2,3-*d*]pyrimidin-2(1*H*)-one (compound 91-2) : The synthetic method was similar to Step 80c of Example 80, except that tert-butyl (2*R*,5*S*)-4-(7-(5,6-difluorobenzofuran-7-yl)-6-fluoro-1(*M*)-(2-isopropyl-4-methylpyridin-3-yl)-2-oxo-1,2-dihydropyrido[2,3-*d*]pyrimidin-4-yl)-2,5-dimethylpiperazine-1-carboxylate (1006-91-1) was replaced by tert-butyl (2*R*,5*S*)-4-(7-(5,6-difluorobenzofuran-7-yl)-6-fluoro-1(*P*)-(2-isopropyl-4-methylpyridin-3-yl)-2-oxo-1,2-dihydropyrido[2,3-*d*]pyrimidin-4-yl)-2,5-dimethylpiperazine-1-carboxylate (1010-91-2) (90 mg, 0.136 mmol, 1.00 eq.), 4-((2*S*,5*R*)-4-acryloyl-2,5-dimethylpiperazin-1-yl)-7-(5,6-difluorobenzofuran-7-yl)-6-fluoro-1(*P*)-(2-isopropyl-4-methylpyridin-3-yl)pyrido[2,3-*d*]pyrimidin-2(1*H*)-one (42 mg, yield: 57.5%) was obtained as a pale yellow solid. MS (ES$^+$): m/z=617 (M+H)$^+$. Melting point: 161-173°C. $^1$H NMR (500 MHz, DMSO) δ 8.49 (d, *J* = 9.2 Hz, 1H), 8.36 (d, *J* = 4.9 Hz, 1H), 8.03 (d, *J* = 2.0 Hz, 1H), 7.88 (dd, *J* = 9.9, 7.9 Hz, 1H), 7.20 (d, *J*= 4.9 Hz, 1H), 7.02 (d, *J*= 2.1 Hz, 1H), 6.83 (ddd, *J*= 20.4, 16.7, 10.5 Hz, 1H), 6.18 (dd, *J* = 16.7, 2.2 Hz, 1H), 5.74 (m, 1H), 4.71 (m, 2H), 3.81 (m, 4H), 2.74 (dt, *J* = 6.4, 5.4 Hz, 1H), 1.97 (s, 3H), 1.31 (dd, *J* = 10.7, 6.6 Hz, 3H), 1.19 (dd, *J* = 35.1, 5.7 Hz, 3H), 1.06 (d, *J* = 6.7 Hz, 3H), 0.93 (d, *J*= 6.7 Hz, 3H).

**Example 82: Preparation of 2-((2S)-1-acryloyl-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (compound 100-1) (Preparation according to Scheme 10)**

Step 82a: Preparation of benzyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-2-(cyanomethyl)piperazine-1-carboxylate (compound 1004-100-1): A mixture of 7-chloro-6-fluoro-1(*M*)-(2-isopropyl-4-methylpyridin-3-yl)pyrido[2,3-d]pyrimidine-2,4(1H,3H)-dione (1002-1) (200 mg, 0.573 mmol, 1.0 eq.), DIPEA (369 mg, 2.865 mmol, 5.0 eq.) and phosphorus oxychloride (438 mg, 2.865 mmol, 5 eq.) in acetonitrile (10 mL) was stirred at 85°C for 2 h under the protection of nitrogen. After cooling to room temperature, the solvent was removed in vacuo. The residue was dissolved in tetrahydrofuran (10 mL), DIPEA (222 mg, 1.719 mmol, 3.0 eq.) and benzyl (S)-2-(cyanomethyl)piperazine-1-carboxylate (148 mg, 0.573 mmol, 1 eq.) were added in an ice-water bath, and the mixture was stirred for 1 h. ethyl acetate was added, the mixture were washed with water and brine. The organic layer was concentrated in vacuo. The residue was purified by column chromatography on silica gel (eluent: dichloromethane/methanol=200/1 to 80/1) to give benzyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-2-(cyanomethyl)piperazine-1-carboxylate (300 mg, yield: 88.88%) as a yellow solid. MS ($ES^+$): m/z=590 $(M+H)^+$.

Step 82b: Preparation of benzyl (2S)-2-(cyanomethyl)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazine-1-carboxylate (compound 1006-100-1): Benzyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-2-(cyanomethyl)piperazine-1-carboxylate (1004-100-1) (300 mg, 0.508 mmol, 1 eq.), 2-(6-fluorobenzofuran-7-yl)-4,4,5,5-tetramethyl-1,3,2-dioxaborolane (0903-73) (176 mg, 0.66 mmol, 1.3 eq.), tetrakis(triphenylphosphine)palladium (59 mg, 0.0508 mmol, 0.1 eq.), copper(I) chloride (50 mg, 0.508 mmol, 1 eq.) and sodium carbonate (108 mg, 1.016 mmol,2 eq.) were added into the mixture of acetonitrile (10 mL) and water(1 mL). The mixture was stirred at 82°C for 16h under the protection of nitrogen. Then, the mixture was cooled to room temperature. The mixture was extracted with ethyl acetate. The organic phase was washed with brine and dried over anhydrous sodium sulfate, and concentrated under vacuum. The residue was purified by column chromatography on silica gel (eluent: dichloromethane/methanol=200/1 to 80/1) to give benzyl (2S)-2-(cyanomethyl)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazine-1-carboxylate (343 mg, 97.77%) as a yellow solid. MS (ESI): *m/z* 690$(M+H)^+$.

Step 82c: Preparation of 2-((2S)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (compound 1007-100-1): To a solution of triethylsilane (144 mg, 1.24 mmol, 2.5 eq.) in dichloromethane (5 mL) was added $Et_3N$ (10 mg, 0.2 mmol, 0.2 eq.) and $Pd(OAc)_2$ (11 mg, 0.05 mmol, 0.1 eq.) at room temperature under the protection of nitrogen. The mixture was stirred for 0.5 h at room temperature. To the mixture was added a solution of benzyl (2S)-2-(cyanomethyl)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazine-1-carboxylate (343 mg, 0.497 mmol, 1 eq.) in dichloromethane (1 mL). The mixture was stirred for 6h at room temperature. The reaction was quenched with a solution of aqueous saturated ammonium chloride. The aqueous phase was extracted with dichloromethane. The organic phase was washed with water and brine, dried over sodium sulfate and concentrated in vacuo. The residue was purified by column chromatography on silica gel (eluent: dichloromethane/methanol/ammonium hydroxide=200/1/1 to 80/1/1) to give 2-((2S)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (220 mg, 79.71%) as a yellow solid. MS (ESI): *m/z* 556$(M+H)^+$.

Step 82d: Preparation of 2-((2S)-1-acryloyl-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (compound 100-1): 2-((2S)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (220 mg, 0.396 mmol, 1.0 eq.) was dissolved in 10 mL tetrahydrofuran. DIPEA (153 mg, 1.188 mmol, 3 eq.) was added into the mixture. acryloyl chloride (43 mg, 0.475 mmol, 1.2 eq.) was added into the mixture. The mixture was stirred at room temperature for 15 min. Then, the reaction was quenched with ammonium hydroxide. The mixture was extracted with dichloromethane. The organic phase was concentrated and purified by prep-TLC (silica gel, eluent: ethyl acetate/methanol=12/1) to give 2-((2S)-1-acryloyl-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (139 mg, 57.67%) as a yellow solid. MS (ESI): *m/z* 610$(M+H)^+$. $^1H$ NMR (500 MHz, DMSO) δ 8.57 (d, *J*= 9.4 Hz, 1H), 8.36 (d, *J* = 4.9 Hz, 1H), 7.95 (d, *J* = 2.1 Hz, 1H), 7.80 (dd, *J* = 8.6, 5.2 Hz, 1H), 7.26 (dd, *J* = 10.4, 8.7 Hz, 1H), 7.19 (d, *J* = 4.9 Hz, 1H), 7.02 (d, *J* = 2.1 Hz, 1H), 6.85 (s, 1H), 6.23 (dd, *J* = 16.6, 2.0 Hz, 1H), 5.87 - 5.75 (m, 1H), 4.97 (s, 1H), 4.44 (s, 1H), 4.38 - 4.04 (m, 2H), 4.01 - 3.52 (m, 3H), 3.22 - 2.99 (m, 2H), 2.82 (dt, *J* = 13.1, 6.5 Hz, 1H), 1.93 (s, 3H), 1.07 (d, *J*= 6.7 Hz, 3H), 0.93 (d, *J*= 6.7 Hz, 3H).

**Example 83: Preparation of 2-((2S)-1-acryloyl-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (compound 101-1) (Preparation according to Scheme 10)**

Step 83a: Preparation of benzyl (S)-2-(cyanomethyl)-4-(6,7-dichloro-1(*M*)-(2-isopropyl-4-methylpyridin-3-yl)-2-oxo-1,2-dihydropyrido[2,3-d]pyrimidin-4-yl)piperazine-1-carboxylate (compound 1004-101-1) : The synthetic method

was similar to Step 82a of Example 82, except that 7-chloro-6-fluoro-1(*M*)-(2-isopropyl-4-methylpyridin-3-yl)pyrido[2,3-d]pyrimidine-2,4(1H,3H)-dione (1002-1) was replaced by 6,7-dichloro-1(*M*)-(2-isopropyl-4-methylpyridin-3-yl)pyrido[2,3-d]pyrimidine-2,4(1H,3H)-dione (1002-73) (200 mg, 0.548 mmol, 1.0 eq.), benzyl (S)-2-(cyanomethyl)-4-(6,7-dichloro-1(*M*)-(2-isopropyl-4-methylpyridin-3-yl)-2-oxo-1,2-dihydropyrido[2,3-d]pyrimidin-4-yl)piperazine-1-carboxylate (300 mg, yield: 90.36%) was obtained as a yellow solid. MS ($ES^+$): m/z=606 $(M+H)^+$.

Step 83b: Preparation of benzyl (2S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-2-(cyanomethyl)piperazine-1-carboxylate (compound 1006-101-1) : The synthetic method was similar to Step 82b of Example 82, except that benzyl (S)-4-(7-chloro-6-fluoro-1(*M*)-(2-isopropyl-4-methylpyridin-3-yl)-2-oxo-1,2-dihydropyrido[2,3-d]pyrimidin-4-yl)-2-(cyanomethyl)piperazine-1-carboxylate (1004-100-1) was replaced by benzyl (S)-2-(cyanomethyl)-4-(6,7-dichloro-1(*M*)-(2-isopropyl-4-methylpyridin-3-yl)-2-oxo-1,2-dihydropyrido[2,3-d]pyrimidin-4-yl)piperazine-1-carboxylate(1004-101-1) (300 mg, 0.496 mmol, 1 eq.), benzyl (2S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)-2-(cyanomethyl)piperazine-1-carboxylate (342.5 mg, yield: 97.99%) was obtained as a yellow solid. MS ($ES^+$): m/z=706 $(M+H)^+$.

Step 83c: Preparation of 2-((2S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (compound 1007-101-1) : The synthetic method was similar to Step 82c of Example 82, 2-((2S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (234 mg, yield: 84.47%) was obtained as a yellow solid. MS ($ES^+$): m/z=572 $(M+H)^+$.

Step 83d: Preparation of 2-((2S)-1-acryloyl-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (compound 101-1) : The synthetic method was similar to Step 82d of Example 82, except that 2-((2S)-4-(6-fluoro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (1007-100-1) was replaced by 2-((2S)-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (234 mg, 0.41 mmol, 1.0 eq.), 2-((2S)-1-acryloyl-4-(6-chloro-7-(6-fluorobenzofuran-7-yl)-1(*M*)-(2-isopropyl-4-methylpyridin-3-yl)-2-oxo-1,2-dihydropyrido[2,3-d]pyrimidin-4-yl)piperazin-2-yl)acetonitrile (178 mg, yield: 69.53%) was obtained as a yellow solid. MS ($ES^+$): m/z=626 $(M+H)^+$. $^1H$ NMR (500 MHz, DMSO) $\delta$ 8.70 (s, 1H), 8.34 (d, *J* = 4.7 Hz, 1H), 7.95 (d, *J* = 31.5 Hz, 1H), 7.76 (dd, *J* = 8.6, 5.3 Hz, 1H), 7.24 (t, *J* = 9.4 Hz, 1H), 7.16 (d, *J* = 4.7 Hz, 1H), 7.01 (d, *J* = 1.7 Hz, 1H), 6.85 (s, 1H), 6.23 (dd, *J* = 16.7, 1.6 Hz, 1H), 5.81 (d, *J* = 10.8 Hz, 1H), 4.95 (s, 1H), 4.44 (s, 1H), 4.39 - 4.01 (m, 2H), 3.78 (t, *J* = 89.8 Hz, 3H), 3.23 - 2.99 (m, 2H), 2.81 (s, 1H), 1.92 (d, *J* = 31.6 Hz, 3H), 1.07 (d, *J* = 6.7 Hz, 3H), 0.91 (d, *J* = 6.7 Hz, 3H).

**Example 84: Preparation of 4-((S)-4-acryloyl-2-methylpiperazin-1-yl)-6-chloro-7-(5,6-difluorobenzofuran-7-yl)-1(*M*)-(2-isopropyl-4-methylpyridin-3-yl)pyrido[2,3-d]pyrimidin-2(1H)-one (compound 103-1) (Preparation according to Scheme 10)**

Step 84a: Preparation of tert-butyl (S)-4-(6-chloro-1(*M*)-(2-isopropyl-4-methylpyridin-3-yl)-2-oxo-7-(trimethylstannyl)-l,2-dihydropyrido[2,3-d]pyrimidin-4-yl)-3-methylpiperazine-1-carboxylate (compound 1005-103-1): tert-butyl (S)-4-(6,7-dichloro-1(*M*)-(2-isopropyl-4-methylpyridin-3-yl)-2-oxo-1,2-dihydropyrido[2,3-d]pyrimidin-4-yl)-3-methylpiperazine-1-carboxylate (1004-73-1) (340 mg, 0.62 mmol, 1 eq.), hexamethyldistannane (612 mg, 1.86 mmol, 3 eq.)and tetrakis(triphenylphosphine)palladium (69 mg, 0.06 mmol, 0.1 eq.) were added into toluene (15 mL). The mixture was stirred at 110°C for 2h under the protection of nitrogen. The reaction was cooled to room temperature. The mixture was concentrated and purified by column chromatography on silica gel ( eluent: petroleum ether/ethyl acetate=5/1-1/2) to give tert-butyl (S)-4-(6-chloro-1(*M*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (313 mg, 74.36%) as a yellow solid. MS (ESI): *m/z* 677(M+H) $^+$.

Step 84b: Preparation of tert-butyl (3S)-4-(6-chloro-7-(5,6-difluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (compound 1006-103-1): tert-butyl (S)-4-(6-chloro-1(*M*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (1005-103-1) (150 mg, 0.221 mmol, 1 eq.), 5,6-difluoro-7-iodobenzofuran (0901-74) (123 mg, 0.442 mmol, 2 eq.), CuI (4 mg, 0.02 mmol, 0.1 eq.) and tetrakis(triphenylphosphine)palladium (25 mg, 0.02 mmol, 0.1 eq.) were added into toluene (10 mL). The mixture was stirred at 125°C for 16h under the protection of nitrogen. The reaction was cooled to room temperature. The mixture was concentrated and purified by prep-TLC (silica gel, eluent: petroleum ether/ethyl acetate=1/3) to give tert-butyl (3S)-4-(6-chloro-7-(5,6-difluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (67 mg, 45.57%) as a yellow solid. MS (ESI): *m/z* 665$(M+H)^+$.

Step 84c: Preparation of 4-((S)-4-acryloyl-2-methylpiperazin-1-yl)-6-chloro-7-(5,6-difluorobenzofuran-7-yl)-1(*M*)-(2-isopropyl-4-methylpyridin-3-yl)pyrido[2,3-d]pyrimidin-2(1H)-one (compound 103-1): tert-butyl (3S)-4-(6-chloro-7-(5,6-difluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (1006-103-1) (67 mg, 0.101 mmol, 1.0 eq.) was dissolved in 4 mL dichloromethane, and 1 mL TFA was added into the mixture. The mixture was stirred at room temperature for 2h. Then, the mixture was concentrated. The residue was dissolved in tetrahydrofuran (5 mL). DIPEA (39 mg, 0.303 mmol, 3 eq.) was added into the mixture. Acryloyl chloride (9 mg, 0.101 mmol, 1.0 eq.) was added into the mixture. The mixture was stirred at room temperature for 15 min. Then, the reaction was quenched with ammonium hydroxide. The mixture was extracted with dichloromethane. The organic phase was concentrated and purified by prep-TLC (silica gel, eluent: ethyl acetate/methanol=12/1) to give 4-((S)-4-acryloyl-2-methylpiperazin-1-yl)-6-chloro-7-(5,6-difluorobenzofuran-7-yl)-1(*M*)-(2-isopropyl-4-methylpyridin-3-yl)pyrido[2,3-d]pyrimidin-2(1H)-one (54 mg, 86.67%) as a yellow solid. MS (ESI): *m/z* 619(M+H)$^+$. $^1$H NMR (500 MHz, DMSO) δ 8.56 (s, 1H), 8.38 (d, *J*= 5.0 Hz, 1H), 8.06 (s, 1H), 7.86 (dd, *J* = 10.0, 7.9 Hz, 1H), 7.25 (s, 1H), 7.03 (d, *J* = 2.1 Hz, 1H), 6.84 (m, 1H), 6.21 (d, *J* = 16.1 Hz, 1H), 5.76 (dd, *J* = 10.4,2.3 Hz, 1H), 4.96 (s, 1H), 4.33 (dd, *J* = 41.5, 12.8 Hz, 2H), 4.12 (m, 1H), 3.69 (m, 2H), 3.16 (s, 1H), 2.79 (m, 1H), 1.98 (s, 3H), 1.37 (d, *J* = 6.7 Hz, 3H), 1.09 (d, *J* = 6.7 Hz, 3H), 0.93 (d, *J* = 6.4 Hz, 3H).

**Example 85: Preparation of 4-((S)-4-acryloyl-2-methylpiperazin-1-yl)-6-chloro-7-(5,6-difluorobenzofuran-7-yl)-1(*P*)-(2-isopropyl-4-methylpyridin-3-yl)pyrido[2,3-d]pyrimidin-2(1H)-one (compound 103-1) (Preparation according to Scheme 10)**

Step 85a: Preparation of tert-butyl (S)-4-(6-chloro-1(*P*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (compound 1005-103-2) : The synthetic method was similar to Step 84a of Example 84, except that tert-butyl (S)-4-(6,7-dichloro-1(*M*)-(2-isopropyl-4-methylpyridin-3-yl)-2-oxo-1,2-dihydropyrido[2,3-d]pyrimidin-4-yl)-3-methylpiperazine-1-carboxylate (1004-73-1) was replaced by tert-butyl (S)-4-(6,7-dichloro-1(*P*)-(2-isopropyl-4-methylpyridin-3-yl)-2-oxo-1,2-dihydropyrido[2,3-d]pyrimidin-4-yl)-3-methylpiperazine-1-carboxylate (1004-73-2), tert-butyl (S)-4-(6-chloro-1(*P*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (217 mg, yield: 58.49%) was obtained as a yellow solid. MS (ES$^+$): m/z=677 (M+H)$^+$.

Step 85b: Preparation of tert-butyl (3S)-4-(6-chloro-7-(5,6-difluorobenzofuran-7-yl)-1(*P*)-(2-isopropyl-4-methylpyridin-3-yl)-2-oxo-1,2-dihydropyrido[2,3-d]pyrimidin-4-yl)-3-methylpiperazine-1-carboxylate (compound 1006-103-2) : The synthetic method was similar to Step 84b of Example 84, except that tert-butyl (S)-4-(6-chloro-1(*M*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate (1005-73-1) was replaced by tert-butyl (S)-4-(6-chloro-1(*P*)-(2-isopropyl-4-methylpyridin-3-yl)-2-oxo-7-(trimethylstannyl)-1,2-dihydropyrido[2,3-d]pyrimidin-4-yl)-3-methylpiperazine-1-carboxylate, tert-butyl (3S)-4-(6-chloro-7-(5,6-difluorobenzofuran-7-yl)-1(*P*)-(2-isopropyl-4-methylpyridin-3-yl)-2-oxo-1,2-dihydropyrido[2,3-d]pyrimidin-4-yl)-3-methylpiperazine-1-carboxylate (145 mg, yield: 68.07%) was obtained as a yellow solid. MS (ES$^+$): m/z=665 (M+H)$^+$.

Step 85c: Preparation of 4-((S)-4-acryloyl-2-methylpiperazin-1-yl)-6-chloro-7-(5,6-difluorobenzofuran-7-yl)-1(*P*)-(2-isopropyl-4-methylpyridin-3-yl)pyrido[2,3-d]pyrimidin-2(1H)-one (compound 103-2) : The synthetic method was similar to Step 84c of Example 84, except that tert-butyl (3S)-4-(6-chloro-7-(5,6-difluorobenzofuran-7-yl)-1(*M*)-(2-isopropyl-4-methylpyridin-3-yl)-2-oxo-1,2-dihydropyrido[2,3-d]pyrimidin-4-yl)-3-methylpiperazine-1-carboxylate (1006-73-1) was replaced by tert-butyl (3S)-4-(6-chloro-7-(5,6-difluorobenzofuran-7-yl)-1(*P*)-(2-isopropyl-4-methylpyridin-3-yl)-2-oxo-1,2-dihydropyrido[2,3-d]pyrimidin-4-yl)-3-methylpiperazine-1-carboxylate (140 mg, 0.21 mmol, 1.0 eq.), 4-((S)-4-acryloyl-2-methylpiperazin-1-yl)-6-chloro-7-(5,6-difluorobenzofuran-7-yl)-1(*P*)-(2-isopropyl-4-methylpyridin-3-yl)pyrido[2,3-d]pyrimidin-2(1H)-one (45 mg, yield: 34.61%) was obtained as a yellow solid. MS (ES$^+$): m/z=619 (M+H)$^+$. $^1$H NMR (500 MHz, DMSO) δ 8.56 (s, 1H), 8.32 (d, *J* = 4.8 Hz, 1H), 8.01 (d, *J* = 23.2 Hz, 1H), 7.83 (dd, *J* = 9.9, 8.0 Hz, 1H), 7.14 (d, *J* = 4.6 Hz, 1H), 7.00 (d, *J* = 2.1 Hz, 1H), 6.83 (m, 1H), 6.19 (d, *J* = 16.1 Hz, 1H), 5.75 (dd, *J* = 10.4, 2.2 Hz, 1H), 5.00 (s, 1H), 4.34 (m, 2H), 4.08 (dd, *J* = 56.1, 12.5 Hz, 1H), 3.68 (m, 2H), 3.10 (d, *J*= 11.2 Hz, 1H), 2.75 (s, 1H), 1.93 (s, 3H), 1.33 (d, *J* = 6.7 Hz, 3H), 1.05 (d, *J*= 6.7 Hz, 3H), 0.90 (d, *J*= 6.6 Hz, 3H).

**Example 86: Preparation of 4-((2S,5R)-4-acryloyl-2,5-dimethylpiperazin-1-yl)-6-chloro-7-(5,6-difluorobenzofuran-7-yl)-1(*M*)-(2-isopropyl-4-methylpyridin-3-yl)pyrido[2,3-d]pyrimidin-2(1H)-one (compound 104-1) (Preparation according to Scheme 10)**

Step 86a; tert-butyl (2R,5S)-4-(6-chloro-1(*M*)-(2-isopropyl-4-methylpyridin-3-yl)-2-oxo-7-(tributylstannyl)-1,2-dihydropyrido[2,3-d]pyrimidin-4-yl)-2,5-dimethylpiperazine-1-carboxylate (compound 1005-104-1): Tert-butyl (2R,5S)-4-(6,7-dichloro-1(*M*)-(2-isopropyl-4-methylpyridin-3-yl)-2-oxo-1,2-dihydropyrido[2,3-d]pyrimidin-4-yl)-2,5-dimethylpiperazine-1-carboxylate (1004-90-1) (300 mg, 0.535 mmol, 1 eq.), hexamethyldistannane (526 mg, 1.607 mmol, 3 eq.)and tetrakis(triphenylphosphine)palladium (62 mg, 0.05 mmol, 0.1 eq.) were added into toluene (15 mL). The mixture was stirred at 110°C for 2h under the protection of nitrogen. The reaction was cooled to room temperature. The mixture was concentrated and purified by column chromatography on silica gel ( eluent: petroleum ether/ethyl acetate=5/1-1/2) to give tert-butyl (2R,5S)-4-(6-chloro-1(*M*)-(2-isopropyl-4-methylpyridin-3-yl)-2-oxo-7-(tributylstannyl)-1,2-dihydropyrido[2,3-d]pyrimidin-4-yl)-2,5-dimethylpiperazine-1-carboxylate (300 mg, 81.16%) as a yellow solid. MS (ESI): *m/z* 691(M+H)$^+$.

Step 86b: tert-butyl (2R,5S)-4-(6-chloro-7-(5,6-difluorobenzofuran-7-yl)-1(*M*)-(2-isopropyl-4-methylpyridin-3-yl)-2-oxo-1,2-dihydropyrido[2,3-d]pyrimidin-4-yl)-2,5-dimethylpiperazine-1-carboxylate (compound 1006-104-1): Tert-butyl (2R,5S)-4-(6-chloro-1(M)-(2-isopropyl-4-methylpyridin-3-yl)-2-oxo-7-(trimethylstannyl)-1,2-dihydropyrido[2,3-d]pyrimidin-4-yl)-2,5-dimethylpiperazine-1-carboxylate (1005-404-1) (300 mg, 0.434 mmol, 1 eq.), 5,6-difluoro-7-iodobenzofuran (0901-74) (182 mg, 0.652 mmol, 1.5 eq.), CuI (8 mg, 0.04 mmol, 0.1 eq.) and tetrakis(triphenylphosphine)palladium (50 mg, 0.04 mmol, 0.1 eq.) were added into toluene (10 mL). The mixture was stirred at 125°C for 16h under the protection of nitrogen. The reaction was cooled to room temperature. The mixture was concentrated and purified by prep-TLC (silica gel, eluent: petroleum ether/ethyl acetate=1/3) to give tert-butyl (2R,5S)-4-(6-chloro-7-(5,6-difluorobenzofuran-7-yl)-1(*M*)-(2-isopropyl-4-methylpyridin-3-yl)-2-oxo-1,2-dihydropyrido[2,3-d]pyrimidin-4-yl)-2,5-dimethylpiperazine-1-carboxylate (115 mg, 39.02%) as a yellow solid. MS (ESI): *m/z* 679(M+H)$^+$.

Step 86c. 4-((2S,5R)-4-acryloyl-2,5-dimethylpiperazin-1-yl)-6-chloro-7-(5,6-difluorobenzofuran-7-yl)-1(*M*)-(2-isopropyl-4-methylpyridin-3-yl)pyrido[2,3-d]pyrimidin-2(1H)-one (compound 104-1): tert-butyl (2R,5S)-4-(6-chloro-7-(5,6-difluorobenzofuran-7-yl)-1(*M*)-(2-isopropyl-4-methylpyridin-3-yl)-2-oxo-1,2-dihydropyrido[2,3-d]pyrimidin-4-yl)-2,5-dimethylpiperazine-1-carboxylate (1006-104-1) (115 mg, 0.169 mmol, 1.0 eq.) was dissolved in 4 mL dichloromethane, and 1 mL TFA was added into the mixture. The mixture was stirred at room temperature for 2h. Then, the mixture was concentrated. The residue was dissolved in tetrahydrofuran (5 mL). DIPEA (65 mg, 0.507 mmol, 3 eq.) was added into the mixture. Acryloyl chloride (15 mg, 0.169 mmol, 1.0 eq.) was added into the mixture. The mixture was stirred at room temperature for 15 min. Then, the reaction was quenched with ammonium hydroxide. The mixture was extracted with dichloromethane. The organic phase was concentrated and purified by prep-TLC (silica gel, eluent: ethyl acetate/methanol=12/1) to give 4-((2S,5R)-4-acryloyl-2,5-dimethylpiperazin-1-yl)-6-chloro-7-(5,6-difluorobenzofuran-7-yl)-1(*M*)-(2-isopropyl-4-methylpyridin-3-yl)pyrido[2,3-d]pyrimidin-2(1H)-one (79 mg, 73.83%) as a yellow solid. MS (ESI): *m/z* 633(M+H)$^+$. $^1$H NMR (500 MHz, DMSO) δ 8.52 (d, *J* = 8.3 Hz, 1H), 8.33 (d, *J*= 4.9 Hz, 1H), 8.03 (s, 1H), 7.83 (dd, *J*= 10.0, 7.9 Hz, 1H), 7.14 (d, *J*= 4.6 Hz, 1H), 7.00 (d, *J*= 2.2 Hz, 1H), 6.82 (m, 1H), 6.17 (d, *J*= 16.8 Hz, 1H), 5.74 (ddd, *J*= 10.4, 5.7, 2.2 Hz, 1H), 4.80 (m, 2H), 3.86 (m, 4H), 2.74 (dq, *J* = 13.0, 6.3 Hz, 1H), 1.92 (s, 3H), 1.35 (m, 3H), 1.23 (dd, *J*= 32.7, 6.7 Hz, 3H), 1.05 (d, *J*= 6.7 Hz, 3H), 0.91 (s, 3H).

**Example 87: Preparation of 4-((2S,5R)-4-acryloyl-2,5-dimethylpiperazin-1-yl)-6-chloro-7-(5,6-difluorobenzofuran-7-yl)-1(*P*)-(2-isopropyl-4-methylpyridin-3-yl)pyrido[2,3-d]pyrimidin-2(1H)-one (compound 104-2) (Preparation according to Scheme 10)**

Step 87a: Preparation of tert-butyl (2R,5S)-4-(6-chloro-1(*P*)-(2-isopropyl-4-methylpyridin-3-yl)-2-oxo-7-(trimethylstannyl)-1,2-dihydropyrido[2,3-d]pyrimidin-4-yl)-2,5-dimethylpiperazine-1-carboxylate (compound 1009-104-2) : The synthetic method was similar to Step 86a of Example 86, except that tert-butyl (2R,5S)-4-(6,7-dichloro-1(*M*)-(2-isopropyl-4-methylpyridin-3-yl)-2-oxo-1,2-dihydropyrido[2,3-d]pyrimidin-4-yl)-2,5-dimethylpiperazine-1-carboxylate (1004-90-1) was replaced by tert-butyl (2R,5S)-4-(6,7-dichloro-1(*P*)-(2-isopropyl-4-methylpyridin-3-yl)-2-oxo-1,2-dihydropyrido[2,3-d]pyrimidin-4-yl)-2,5-dimethylpiperazine-1-carboxylate (1008-90-2) (160 mg, 0.285 mmol, 1.0 eq.), tert-butyl (2R,5S)-4-(6-chloro-1(*P*)-(2-isopropyl-4-methylpyidin-3-yl)-2-oxo-7-(trimethylstannyl)-1,2-dihydropyrido[2,3-*d*]pyrimidin-4-yl)-2,5-dimethylpiperazine-1-carboxylate (91 mg, yield: 46.8%) was obtained as a yellow solid. MS (ES$^+$): m/z=691(M+H)$^+$.

Step 87b: Preparation of tert-butyl (2R,5S)-4-(6-chloro-7-(5,6-difluorobenzofuran-7-yl)-1(*P*)-(2-isopropyl-4-methylpyridin-3-yl)-2-oxo-1,2-dihydropyrido[2,3-d]pyrimidin-4-yl)-2,5-dimethylpiperazine-1-carboxylate (compound 1010-104-2) : The synthetic method was similar to Step 86b of Example 86, except that tert-butyl (2R,5S)-4-(6-chloro-1(*M*)-(2-isopropyl-4-methylpyridin-3-yl)-2-oxo-7-(trimethylstannyl)-1,2-dihydropyrido[2,3-d]pyrimidin-4-yl)-2,5-dimethylpiperazine-1-carboxylate (1005-104-1) was replaced by tert-butyl (2R,5S)-4-(6-chloro-1(*P*)-(2-isopropyl-4-methylpyidin-3-yl)-2-oxo-7-(trimethylstannyl)-1,2-dihydropyrido[2,3-*d*]pyrimidin-4-yl)-2,5-dimethylpiperazine-1-carboxylate (1009-104-2) (91 mg, 0.134 mmol, 1.00 eq.), tert-butyl (2R,5S)-4-(6-chloro-7-(5,6-difluorobenzofuran-7-yl)-1(*P*)-(2-isopropyl-4-methylpyridin-3-yl)-2-oxo-1,2-dihydropyrido[2,3-d]pyrimidin-4-yl)-2,5-dimethylpiperazine-1-carboxylate (90 mg, yield: 99.6%) was obtained as a pale yellow solid. MS (ES$^+$): m/z=679(M+H)$^+$.

Step 87c: Preparation of 4-((2S,5R)-4-acryloyl-2,5-dimethylpiperazin-1-yl)-6-chloro-7-(5,6-difluorobenzofuran-7-yl)-1(*P*)-(2-isopropyl-4-methylpyridin-3-yl)pyrido[2,3-d]pyrimidin-2(1H)-one (compound 104-2) : The synthetic method was similar to Step 86c of Example 86, except that tert-butyl (2R,5S)-4-(6-chloro-7-(5,6-difluorobenzofuran-7-yl)-1(*M*)-(2-isopropyl-4-methylpyridin-3-yl)-2-oxo-1,2-dihydropyrido[2,3-d]pyrimidin-4-yl)-2,5-dimethylpiperazine-1-carboxylat (1006-104-1) was replaced by tert-butyl (2R,5S)-4-(6-chloro-7-(5,6-difluorobenzofuran-7-yl)-1(*P*)-(2-isopropyl-4-methylpyridin-3-yl)-2-oxo-1,2-dihydropyrido[2,3-d]pyrimidin-4-yl)-2,5-dimethylpiperazine-1-carboxylat (1010-104-2) (90 mg, 0.132 mmol, 1.00 eq.), 4-((2S,5R)-4-acryloyl-2,5-dimethylpiperazin-1-yl)-6-chloro-7-(5,6-difluorobenzofuran-7-yl)-1(*P*)-(2-isopropyl-4-methylpyridin-3-yl)pyrido[2,3-d]pyrimidin-2(1H)-one (30 mg, yield: 55.5%) was obtained as a yellow

solid. MS ($ES^+$): m/z=633$(M+H)^+$. Melting point: 153-162°C. $^1H$ NMR (500 MHz, DMSO) δ 8.60 (s, 1H), 8.36 (d, *J* = 4.8 Hz, 1H), 8.05 (d, *J* = 20.5 Hz, 1H), 7.87 (m, 1H), 7.18 (d, *J* = 3.7 Hz, 1H), 7.04 (d, *J*= 1.3 Hz, 1H), 6.84 (td, *J*= 17.4, 10.5 Hz, 1H), 6.20 (dd, *J*= 16.7, 1.6 Hz, 1H), 5.76 (m, 1H), 4.73 (m, 2H), 3.96 (m, 4H), 2.73 (s, 1H), 1.98 (s, 3H), 1.36 (t, *J* = 6.7 Hz, 3H), 1.23 (m, 3H), 1.07 (d, *J*= 6.7 Hz, 3H), 0.93 (d, *J*= 6.6 Hz, 3H).

EXAMPLE: Biological activity test

The positive control compound used in the present disclosure is AMG 510 (US2018334454A1), with the structure as following:

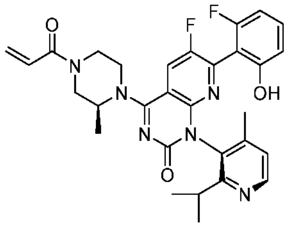

I. KRAS-G12C/SOS1 binding experiment

1. Experimental Materials

| | |
|---|---|
| KRAS-G12C/SOS1 Binding Assay Kit | Cisbio, #63ADK000CB16PEG |
| HTRF 96-well microplate | Cisbio, #C11995500BT |
| TECAN Microplate reader | Infinite 200 PRO |

2. Experimental principle

The HTRF KRAS-G12C/SOS1 binding assay was used to study the interaction between KRAS-G12C and SOS1 protein. The use of HTRF (Homogeneous Time-Resolved Fluorescence) technology enables simple and rapid determination of the properties of compounds in a high-throughput format. When the HTRF donor antibody anti-Tag1-$Tb^{3+}$ is closed to the acceptor antibody anti-Tag2-XL665, due to the binding of SOS1 and KRAS-G12C, excitation of the donor antibody triggers fluorescence resonance energy transfer (FRET) to the receptor antibody. The receptor antibody in turn emits specifically at 665 nm. This specific signal is proportional to the degree of KRAS-G12C/SOS1 interaction. Therefore, when compounds blocked the KRAS-G12C/SOS1 interaction, it results in reduced HTRF signaling.

KRAS-G12C/SOS1 Binding Kit contains: Donor antibody anti-Tag1-$Tb^{3+}$, receptor antibody anti-Tag2-XL665, Tag1-SOS1 protein, Tag2-KRAS-G12C protein, diluent, detection buffer.

3. Experimental method

1) Diluted Tag2-KRAS-G12C protein and Tag1-SOS1 protein with diluent at the ratio of 1:100, and anti-Tag1-$Tb^{3+}$ antibody and anti-Tag2-XL665 with detection buffer at the ratio of 1:100 or 1:25, respectively.
2) Diluted the compound to be tested with diluent into 10x stock solution, starting from a concentration of 10000nM, diluting in 4-fold gradient and a total of 6 concentration gradients.
3) In a 96-well plate, added 4uL of Tag2-KRAS-G12C protein, 4uL of Tag1-SOS1 protein, 2uL of dilution solution (positive control) or test compound (different concentrations of 10 x stock solution) to each well in sequence, with a total of 10uL. After incubated at room temperature for 15 minutes, added pre-mixed 5uL of anti-Tag1-$Tb^{3+}$ and 5uL of anti-Tag2-XL665. Sealed the plate and incubated at room temperature for 2 hours. The HTRF signal was read with a TECAN INFINITE F NANO+ microplate reader.

4. Experimental results

Calculated the ratio of the acceptor and donor excitation signals for each single well using the formula ratio = 665 nm signal value / 620 nm signal value * $10^4$. Data were processed by using Graphpad prism software. $IC_{50}$'s values were calculated by sigmoidal dose-response curve fitting and the results are shown in Table 1 below.

Table 1. Inhibitory activity $IC_{50}$ (nM) of compounds on KRAS-G12C/SOS1 binding.

| Compound | IC50(nM) on KRAS-G12C/SOS1 binding | | Compound | IC50(nM) on KRAS-G12C/SOS1 binding |
|---|---|---|---|---|
| 70 | III | | 70-1 | IV |
| 70-2 | I | | 73 | V |
| 73-1 | V | | 73-2 | III |
| 74-1 | IV | | 79 | III |
| 89-1 | IV | | 90 | IV |
| 90-1 | V | | 91-1 | III |
| 97 | IV | | 103-1 | V |
| 104-1 | V | | 122 | V |
| AMG 510 | IV | | | |

Wherein: I > 1000 nM, 1000 nM ≥ II > 500 nM, 500 nM ≥ III > 100 nM, 100 nM ≥ IV > 50 nM, V ≤ 50 nM.
It can be seen from the data in the table that the Pyridopyrimidinone compounds of the present disclosure can effectively block the binding of KRAS-G12C/SOS.

II. Tumor cell proliferation inhibition assay

1. Experimental Materials

| | |
|---|---|
| RPMI1640 medium | GIBCO, #C11875500BT |
| DMEM medium | GIBCO, #C11995500BT |
| Fetal bovine serum (FBS) | Biological Industries, #040011ACS |
| CellTiter-Glo Luminescent Cell Viability Assay Kit | Promega, #G7573 |
| Scepter Automated Cell Counter | Millipore, #PHCC00000 |

2. Cell lines (all source cells have been identified by STR data)

| Name of the cell line | Mutation site | species | source |
|---|---|---|---|
| H358 | KRAS G12C | Human | BeBetter Cell Bank |
| H23 | KRAS G12C | Human | BeBetter Cell Bank |
| MIA-Paca2 | KRAS G12C | Human | BeBetter Cell Bank |

3. Experimental method

The H358, H23 or MIA-Paca2 cells were digested, centrifuged and resuspended, and the cell density was measured with a Scepter automatic cell counter. Cells were diluted to a solution containing 44,000 cells per milliliter,

then added to a 96-well culture plate at 90 μL per well. The 96-well plate was placed in a 5% $CO_2$ incubator at 37°C, and after the cells were cultured for 24 hours, different concentrations of the compounds to be tested were added. Cells were incubated with compounds in the presence of 10% fetal bovine serum for 72 hours. The inhibition of cell growth was assessed by measuring ATP levels by using the CellTiter-Glo Luminescent Cell Viability Assay Kit (details in manufacturer's instructions). In brief, 30 μL of CellTiter-Glo reagent was added to each well and the plate was shaken for 10 minutes to induce cell lysis, then the fluorescent signal was detected and recorded with a Fluoroskan Ascent FL (Thermo). Maximum signal values were obtained from cells treated with DMSO for 72 hours. Minimum signal values were obtained with medium only (zero cell number). Inhibition rate %= (maximum signal value-compound signal value)/(maximum signal value-minimum signal value) ×100%. Data were processed by using Graphpad prism5 software. $IC_{50}$ values were calculated using a sigmoidal dose-response curve.

4. Experimental Results

The $IC_{50}$ of each compound in the above experiment was calculated, and the results are shown in Table 2 below.

Table 2. Inhibitory activity $IC_{50}$(nM) of compounds on tumor cell proliferation.

| Compound | H358 (KRAS G12C) | MIA-Paca2 (KRAS G12C) | H23 (KRAS G12C) |
|---|---|---|---|
| | $IC_{50}$ (nM) | | |
| 1 | IV | | V |
| 3 | III | | IV |
| 6 | II | | IV |
| 7 | III | | IV |
| 8 | II | | IV |
| 13 | I | | II |
| 14 | IV | | V |
| 15 | IV | | V |
| 16 | III | | IV |
| 22 | IV | IV | |
| 31 | II | | IV |
| 33 | I | | I |
| 34 | IV | | V |
| 35 | IV | | V |
| 37 | IV | | V |
| 38 | IV | IV | |
| 39 | IV | IV | |
| 43 | II | | IV |
| 44 | IV | IV | IV |
| 45 | III | | IV |
| 46 | IV | | IV |
| 48 | III | | V |
| 48-1 | IV | IV | |
| 48-2 | I | I | |
| 49 | III | II | |
| 50 | II | II | |
| 52 | III | III | |

(continued)

| Compound | H358 (KRAS G12C) | MIA-Paca2 (KRAS G12C) | H23 (KRAS G12C) |
|---|---|---|---|
| | $IC_{50}$ (nM) | | |
| 58 | IV | IV | |
| 63 | III | | IV |
| 65 | II | | II |
| 67 | II | | II |
| 69 | IV | III | |
| 70 | IV | IV | |
| 70-1 | IV | IV | V |
| 70-2 | II | I | III |
| 71 | IV | III | |
| 72 | IV | IV | |
| 73 | V | V | V |
| 73-1 | V | V | V |
| 73-2 | IV | II | V |
| 74 | IV | III | V |
| 74-1 | IV | IV | V |
| 74-2 | I | I | II |
| 78 | II | I | |
| 79 | IV | IV | |
| 82 | I | I | |
| 89 | IV | IV | |
| 89-1 | IV | IV | |
| 89-2 | II | I | |
| 90 | V | IV | V |
| 90-1 | V | V | V |
| 90-2 | III | III | V |
| 91 | IV | III | V |
| 91-1 | IV | IV | V |
| 91-2 | I | I | III |
| 97 | V | IV | |
| 98 | V | IV | |
| 99 | III | II | V |
| 100-1 | II | | |
| 101-1 | IV | | |
| 102 | V | IV | |
| 103 | V | IV | V |
| 103-1 | V | V | V |
| 103-2 | II | II | IV |

(continued)

| Compound | H358 (KRAS G12C) | MIA-Paca2 (KRAS G12C) | H23 (KRAS G12C) |
|---|---|---|---|
| | $IC_{50}$ (nM) | | |
| 104 | V | IV | V |
| 104-1 | V | IV | V |
| 104-2 | II | II | IV |
| 105 | V | IV | |
| 106 | II | II | |
| 107 | II | I | |
| 108 | IV | II | |
| 109 | III | II | |
| 110 | IV | | |
| 111 | III | | |
| 112 | IV | III | |
| 113 | I | I | |
| 114 | I | I | |
| 115 | I | I | |
| 116 | III | II | |
| 117 | III | III | |
| 118 | V | | |
| 120 | II | II | |
| 121 | IV | IV | |
| 122 | V | V | |
| 123 | IV | III | |
| 124 | V | IV | |
| 125 | II | II | |
| 126 | III | II | |
| AMG 510 | IV | V | IV |

Where: I > 500 nM, 500 nM ≥ II > 100 nM, 100 nM ≥ III > 50 nM, 50 nM ≥ IV > 10 nM, V ≤ 10 nM.
It can be seen from the data in the table that the Pyridopyrimidinone compounds of the present disclosure have strong anti-cell proliferation activity against KRAS G12C mutant tumors.

III. Pharmacokinetic (PK) Experiment

1. Experimental method

The compounds to be tested were dissolved in a mixed solvent of 0.5% hypromellose (HPMC) and 0.1% Tween 80, or 30% sulfobutyl-β-cyclodextrin (SBE-β-CD). Male SD rats, weighing 180-250 g, were fasted overnight before the test and stomach administrated with the test compound at 20 mg/kg. 15 minutes, 30 minutes, and 1, 2, 4, 6, 8, and 24 hours after administration, blood was taken from the end of the tail of the fracture, about 0.3 mL per time point, then placed in a centrifuge tube containing K2-EDTA, and centrifuged (2000 g, 10 min, at 4 °C). Plasma was collected and stored in an ultra-low temperature freezer at -80 °C. 50 μL of plasma samples were vortexed with 135 μL of acetonitrile (containing 1 μg/mL internal standard) for protein precipitation and centrifuged, and the supernatant was collected for LC-MS/MS analysis.

2. Experimental results

After the rats oral administrated with the Pyridopyrimidinone compounds provided by the present disclosure, the absorption is good, and the blood exposure is relatively high. The results are shown in Figures 1, 2 and Table 3. The $T_{max}$ of the Pyridopyrimidinone compounds of the present disclosure is 0.67-2.0 hours, the $C_{max}$ is 1626.7-5606.7 ng/mL, and the $AUC_{0\text{-}24h}$ is 4304.5-20075.1 ng/mL*h. $C_{max}$ refers to the maximum plasma concentration, $T_{1/2}$ is the half-life, $AUC_{0\text{-}24}$ refers to the area under the 0-24 hour time-concentration curve, and $AUC_{0\text{-}inf}$ refers to the area under the 0-Inf time-concentration curve.

Table 3. Pharmacokinetic parameters of rats administered by gavage (20 mg/kg)

| Compound | PK parameter | | | | |
|---|---|---|---|---|---|
| | $T_{max}$ (hr) | $C_{max}$ (ng/ml) | $T_{1/2}$ (hr) | $AUC_{0\text{-}24}$ (hr*ng/mL) | $AUC_{0\text{-}inf}$ (hr*ng/mL) |
| 69 | 0.83 | 4390.0 | 5.38 | 9920.2 | 9926.1 |
| 70-1 | 1.00 | 3530.0 | 2.77 | 7889.6 | 7911.1 |
| 73 | 1.00 | 3356.7 | 3.97 | 8629.3 | 8637.9 |
| 73-1 | 0.67 | 1750.0 | 3.23 | 4304.5 | 4318.6 |
| 74-1 | 0.83 | 3183.3 | 5.30 | 6199.3 | 6217.1 |
| 89 | 1.00 | 4296.7 | 2.11 | 14045.9 | 14055.2 |
| 89-1 | 1.00 | 3200.0 | 3.10 | 9006.7 | 9023.3 |
| 90 | 1.00 | 5606.7 | 5.54 | 20075.1 | 20434.7 |
| 90-1 | 1.33 | 3716.7 | 3.26 | 13809.4 | 13841.2 |
| 91-1 | 1.17 | 2970.0 | 2.72 | 9681.8 | 9708.2 |
| 97 | 1.00 | 4663.3 | 5.98 | 10360.7 | 10384.0 |
| 102 | 1.00 | 3720.0 | 2.85 | 14208.8 | 14227.6 |
| 103 | 1.00 | 3313.3 | 3.77 | 9402.8 | 9423.9 |
| 103-1 | 1.00 | 2700.0 | 6.03 | 6189.6 | 6239.4 |
| 104 | 2.00 | 2106.0 | 2.46 | 13014.5 | 13025.0 |
| 104-1 | 1.00 | 2586.7 | 4.35 | 10331.7 | 10536.6 |
| 122 | 1.67 | 1626.7 | 3.64 | 5989.4 | 6002.1 |
| AMG 510 | 0.33 | 3253.33 | 6.76 | 3369.3 | 3430.6 |

IV. Caco-2 cell transmembrane transport experiments of drugs

1. Experiment content

| | |
|---|---|
| Test compounds | AMG 510<br>70-1<br>73-1<br>90-1<br>104-1<br>103-1 |
| Reference compounds | Erythromycin<br>Metoprolol Tartrate<br>Atenolol |

(continued)

| | |
|---|---|
| Test Concentration | Erythromycin, Metoprolol Tartrate and Atenolol: 10 μM<br>AMG 510, 70-1, 73-1, 90-1, 104-1, 103-1: 10 μM |
| Test system | Caco-2 cell / HBSS buffer |
| Test Conditions | pH 7.4, 37°C; 0, 90 min |
| Replicate sample | 2 |
| Analytical method | LC-MS/MS |

2. Experimental method

The Caco-2 cell model is a human colon adenocarcinoma cell used for experiments simulating intestinal transit in vivo (J Mass Spectrom 35: 71-76, 2000). In this experiment, a monolayer of Caco-2 cells was used to assess drug penetration. 10 μM of metoprolol tartrate, atenolol and erythromycin were used as positive control markers for high passive permeability, low passive permeability and high efflux ratio, respectively. The compounds to be tested was respectively added on the apical (Apical, referred to as A in the results) or basolateral (Basolateral, referred to as B in the results) of the monolayer of Caco-2 cells which were formed on the ester microporous membrane and incubated at 37 °C for 90 min, then the concentration on the other side was measured by LC-MS/MS. The apparent permeability coefficient from apical to basolateral (Papp A-B) and the basolateral to apical permeability coefficient (Papp B-A) of compounds represent the ability of the compound to be transported in both directions, respectively. The efflux rate (Efflux Rate=Papp B-A/ Papp A-B) reflects the strength of active efflux of the compounds.

Penetration of the test compounds:

Apparent permeability coefficient (Papp) = (the volume of the receiver chamber / the filter surface area × culture time)) × (the drug concentration of receiver chamber at the end of culture / (the drug concentration of donor chamber at the initial culture × dilution factor)

In this experiment, Millipore cell culture plates (PSHT 010 R5): membrane area = 0.7 $cm^2$, recipient side volume = 0.8 mL (A-to-B) or 0.4 mL (B-to-A), incubation time = 90 minutes.23.

3.Experimental Results

Table 4. Apparent permeability coefficient (Papp) and efflux rate (Papp B-A/ Papp A-B)

| compound | | $P_{app}$ ($10^{-6}$ $cm \cdot s^{-1}$) | | | | $P_{app}$(B-A)/ $P_{app}$(A-B) |
|---|---|---|---|---|---|---|
| | | Sample - 01 | Sample - 02 | mean value | RSD | |
| Metoprolol Tartrate | A-B | 33.32 | 36.36 | 34.84 | 0.06 | 0.93 |
| | B-A | 29.38 | 35.43 | 32.41 | 0.13 | |
| Atenolol | A-B | 0.52 | 0.57 | 0.55 | 0.06 | 0.95 |
| | B-A | 0.50 | 0.54 | 0.52 | 0.07 | |
| Erythromycin, | A-B | 0.46 | 0.50 | 0.48 | 0.05 | 30.60 |
| | B-A | 13.82 | 15.50 | 14.66 | 0.08 | |
| AMG 510 | A-B | 5.83 | 4.94 | 5.39 | 0.12 | 6.22 |
| | B-A | 27.90 | 39.15 | 33.52 | 0.24 | |
| 70-1 | A-B | 19.04 | 18.75 | 18.89 | 0.01 | 1.19 |
| | B-A | 21.13 | 23.66 | 22.39 | 0.08 | |

(continued)

| compound | | $P_{app}$ ($10^{-6}$ cm·s$^{-1}$) | | | | $P_{app}$(B-A)/ $P_{app}$(A-B) |
|---|---|---|---|---|---|---|
| | | Sample - 01 | Sample - 02 | mean value | RSD | |
| 73-1 | A-B | 16.04 | 20.79 | 18.42 | 0.18 | 1.12 |
| | B-A | 17.19 | 24.02 | 20.60 | 0.23 | |
| 90-1 | A-B | 20.08 | 20.38 | 20.23 | 0.01 | 1.08 |
| | B-A | 18.65 | 25.25 | 21.95 | 0.21 | |
| 104-1 | A-B | 11.20 | 10.44 | 10.82 | 0.05 | 1.92 |
| | B-A | 22.05 | 19.49 | 20.77 | 0.09 | |
| 103-1 | A-B | 12.13 | 10.71 | 11.42 | 0.09 | 2.08 |
| | B-A | 19.66 | 27.83 | 23.74 | 0.24 | |

Under the test conditions, if the apparent permeability coefficient of the drug Papp A-B > 10 × $10^6$cm/s, it indicates that the drug has high permeability. The apparent permeability coefficient Papp A-B values of compounds 70-1, 73-1, 90-1, 103-1 and 104-1 were all greater than 10 × $10^6$cm/s, suggesting that the absorption rate of compounds 70-1, 73-1, 90- 1, 103-1 and 104-1 through the intestinal tract is very high, which is better than that of AMG 510. The efflux rates (Papp B-A/ Papp A-B) of compounds 70-1, 73-1, 90-1, 103-1 and 104-1 were between 1-2, indicating that compounds 70-1, 73-1, 90- 1, 103-1 and 104-1 have weak efflux, while AMG 510 has a higher efflux rate of 6.22.

V. Pharmacodynamic Experiment on Tumor Model

1. Experimental method

(1) H358 human non-small cell lung cancer subcutaneous mouse xenograft model:

In this experiment, the anticancer activities of compounds 73-1 and 90-1 in the subcutaneous tumor model of H358 human non-small cell lung cancer xenograft mice, and the dose-response relationship of compound 73-1 in inhibiting the growth of H358 xenograft tumors were investigated. BALB/c nude mice were purchased from Gempharmatech Co., Ltd., and the adaptation period was at least one week. When H358 cells were cultured to a sufficient number, cells were collected. 2 million cells suspended in 0.1 mL of serum-free medium and Matrigel (1:1, v/v) were injected subcutaneously into the right flank area of each mouse, carefully avoiding blood vessels. Vernier calipers were used to measure the size of the tumor. The formula for calculating the tumor volume is: tumor volume = (length × width$^2$)/2, and the formula for calculating the relative tumor proliferation rate T/C value is: T/C (%) =100×ΔT/ ΔC (ΔT>0) or T/T (%) =100×ΔT/T0 (ΔT<0). ΔT is the difference of tumor volume in the treatment group, ΔC is the difference of tumor volume in the vehicle control group, and T0 is the tumor volume in the treatment group before administration.

In the study of anticancer activity of compounds 73-1 and 90-1 in H358 human non-small cell lung cancer xenograft mouse subcutaneous tumor model, when the H358 tumor volume reached an average of about 160 mm$^3$, the animals were randomly divided into 4 oral administration groups, namely vehicle control group (30% sulfobutyl-β-cyclodextrin), AMG510 positive control group, and compounds 73-1, 90-1 administration groups (n=3/group). The dosage of the all groups, except the vehicle control group, was 100 mg/kg, 10 mL/kg orally administered by gavage, once a day. Compounds were respectively dissolved in 30% sulfobutyl-β-cyclodextrin (SBE-β-CD) and 1.5 equivalent of 1 M HCl (about pH 4) at a final concentration of 10 mg/mL. All the groups were administered continuously for 26 days.

In the dose-response study of compound 73-1 inhibiting the growth of H358 xenograft tumors, when the H358 tumor volume reached an average of about 183 mm$^3$, the animals were respectively divided into different treatment groups, including vehicle (30% sulfobutyl-β - Cyclodextrin) control group, compound 73-1 orally administered group with doses of 12.5 mg/kg, 25 mg/kg, 50 mg/kg, and 100 mg/kg (n=8/group), administered once every day. Compound 73-1 was dissolved in 30% sulfobutyl-β-cyclodextrin (SBE-β-CD) and 1.5 equivalent of 1 M HCl (about pH 4) at the corresponding concentrations. All the groups were administered continuously for 28 days with 10 mL/kg oral administration.

(2) MIA Paca2 T2 human pancreatic cancer xenograft mouse subcutaneous model:

In this experiment, the anticancer activity of compound 73-1 in the MIA Paca2 human pancreatic cancer xenograft

mouse subcutaneous tumor model was investigated. BALB/c nude mice were purchased from Gempharmatech Co., Ltd., and acclimatize environment at least one week. MIAPaca2 cells were cultured to sufficient numbers in DMEM containing 10% fetal bovine serum and 2.5% horse serum, then harvested. 5 million cells suspended in 0.1 mL of serum-free medium and Matrigel (1:1, v/v) were injected subcutaneously into the right flank area of each mouse, carefully avoiding blood vessels and serially passaged 2 times in animals. The size of the tumor was measured using a vernier caliper, and the method for calculating the tumor volume and T/C value was the same as above. When the MIA Paca2 T2 tumor volume reached an average of about 163 $mm^3$, the animals were randomly divided into 3 oral administration groups, namely the vehicle control group (30% sulfobutyl-β-cyclodextrin), the AMG510 positive control group (administered100 mg /kg once a day) and Compound 73-1 group (administered 50 mg/kg twice a day) (n=8/group). The compounds were respectively dissolved in 30% sulfobutyl-β-cyclodextrin (SBE-β-CD) and 1.5 equivalent of 1 M HCl (pH 4-5) at the corresponding concentrations. 10mL/kg was orally administered continuously by gavage for 28 days.

2. Experimental results

(1) Anticancer activity of compounds 73-1 and 90-1 in H358 human non-small cell lung cancer xenograft mouse subcutaneous tumor model:

The dose of compound 73-1 and 90-1 were both 100 mg/kg. After continuous oral administration once a day for 26 days, the T/C value of compound 73-1 administration group was -100.0% (p<0.001); the T/C value of the compound 90-1 administration group was -99.5% (p<0.001); the dose of the positive compound AMG510 was 100 mg/kg. After continuous oral administration once a day for 26 days, the T/C value was -93.8% (p<0.001). Compared with the vehicle control group in the same period, the animals in the treatment group had no obvious clinical symptoms and no obvious changes in body weight (results are shown in Table 5 and Figure 3).

The results showed that in the subcutaneous tumor model of KRAS-mutated H358 human non-small cell lung cancer xenograft mice, compound 73-1 could cause the tumor to disappear completely, and AMG510 at the MTD dose could shrink the tumor, which is consistent with the literature report (Nature 575:217, 2019).

(2) The dose-response relationship of compound 73-1 inhibiting the growth of H358 xenograft tumors:

After continuous oral administration with Compound 73-1 once a day for 28 days, the T/C value of the 12.5 mg/kg group was -68.5% (p<0.001); the T/C value of the 25 mg/kg group was -74.0% (p<0.001); the T/C value of the 50 mg/kg group was -93.5% (p<0.001); the T/C value of the 100 mg/kg group was -96.6% (p<0.001). After continuous oral administration with Compound 73-1 at doses of 12.5 mg/kg, 25 mg/kg, 50 mg/kg and 100 mg/kg once a day for 28 days, respectively, there was no significant clinical symptoms and no significant changes in body weight between the animals and the vehicle control group during the same period (results are shown in Table 6 and Figure 4).

The results showed that compound 73-1 inhibited the growth of H358 tumor in a dose-dependent manner and could cause partial disappearance of tumor, which was well tolerated by animals.

(3) Anticancer activity of compound 73-1 in MIA Paca2 human pancreatic cancer xenograft mouse subcutaneous tumor model:

After 28 days of continuous oral administration, the T/C value of the compound 73-1 at a dose of 50 mg/kg (administered twice a day) was -100% (p<0.001); The T/C value of the positive compound AMG510 group at a dose of 100 mg/kg (administered once a day) was -96% (p<0.001). Compared with the vehicle control group in the same period, the animals in the treatment group had no obvious clinical symptoms and no obvious changes in body weight (results are shown in Table 7 and Figure 5).

The results showed that in the KRAS G12C-mutated MIA Paca2 human pancreatic cancer xenograft mouse subcutaneous tumor model, compound 73-1 could cause the tumor to disappear completely, and AMG510 could shrink the tumor at the MTD dose, which is consistent with the literature report (Nature 575:217, 2019).

Table 5. Antitumor activity of compounds 73-1, 90-1 and effects on mouse body weight in H358 xenograft tumor model.

| Treatment group | N | tumor suppressor | | Changes of body weight (%) |
|---|---|---|---|---|
| | | T/C value (%) | P value | |
| Vehicle control group, po, qd | 3 | / | / | 17.3 |
| AMG510,100 mg/kg, po, qd | 3 | -93.8 | <0.001 | 16.4 |

(continued)

| Treatment group | N | tumor suppressor | | Changes of body weight (%) |
|---|---|---|---|---|
| | | T/C value (%) | P value | |
| Compound 73-1,100 mg/kg, po, qd | 3 | -100.0 | <0.001 | 10.1 |
| Compound 90-1, 100 mg/kg, po, qd | 3 | -99.5 | <0.001 | 23.3 |

Table 6. Antitumor dose-response activity of compound 73-1 and effect on mouse body weight in H358 xenograft tumor model.

| Treatment group | N | Antitumor activity | | Changes of body weight (%) |
|---|---|---|---|---|
| | | T/C value (%) | P value | |
| Vehicle control group, po, qd | 8 | / | / | 5.2 |
| Compound 73-1, 12.5mg/kg,po, qd | 8 | -68.5 | <0.001 | -0.2 |
| Compound 73-1, 25 mg/kg, po, qd | 8 | -74.0 | <0.001 | -0.1 |
| Compound 73-1, 50 mg/kg, po, qd | 8 | -93.5 | <0.001 | 2.2 |
| Compound 73-1, 100 mg/kg, po, qd | 8 | -96.6 | <0.001 | 1.9 |

Table 7 Antitumor activity of compound 73-1 and its effect on mouse body weight in MIA Paca2 T2 tumor model.

| Treatment group | N | Antitumor activity | | Changes of body weight (%) |
|---|---|---|---|---|
| | | T/C value (%) | P value | |
| Vehicle control group, po, qd | 8 | / | / | 17.6 |
| AMG510,100 mg/kg, po, qd | 8 | -95.7 | <0.001 | 13.8 |
| Compound 73-1, 50 mg/kg, po, bid | 8 | -100.0 | <0.001 | 10.2 |

The technical features of the above embodiments can be combined arbitrarily. To simplify description, all possible combinations of the technical features of the above embodiments are not described. However, as long as there is no contradiction in the combination of these technical features, they should be considered to be the scope recorded in the description.

The above embodiments express several implementations of the present disclosure only. The description of the embodiments is relatively specific and detailed, but may not therefore be construed as the limitation on the patent scope of the present disclosure. It should be noted that a person of ordinary skill in the art may further make several variations and improvements without departing from the concept of the present disclosure. these variations and improvements all fall within the protection scope of the present disclosure. Therefore, the patent protection scope of the present disclosure shall be defined by the appended claims.

## Claims

1. Pyridopyrimidinone compounds with the structure shown in Formula (II) or their pharmaceutically acceptable salts, or stereoisomer or prodrug molecules:

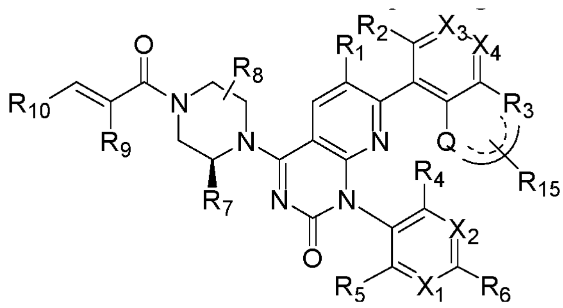

(II)

wherein,

the dotted line between Q and $R_3$ indicates that the chemical bond between Q and $R_3$ can be a single bond or a double bond;
Q is selected from: O, S;
$R_3$ is selected from: N, $CR_{15}$, O, S, $NR_{15}$, $CHR_{15}$, $C(R_{15})_2$;
Q, $R_3$ and the attached C atoms together form a 5-7 membered heterocyclic group or a heteroaryl group;
$X_1$ and $X_2$ are independently selected from: N or CH;
$X_3$ and $X_4$ are independently selected from: N or $CR_{11}$;
$R_1$ is selected from: halogen;
$R_2$ is selected from: H, halogen, C1-C6 alkyl, C1-C6 alkoxy;
$R_4$ and $R_5$ are independently selected from: H, C1-C6 alkyl group, C2-C6 alkenyl group, C2-C6 alkynyl group, C3-C8 cycloalkyl group, cyano group, hydroxy substituted C1-C6 alkyl group;
$R_6$ is selected from: H, halogen, -OR, nitro, cyano, $-N(R)_2$, C1-C6 alkyl, $-C(O)N(R)_2$, - C(O)R;
$R_7$, $R_8$ are independently selected from: H, C1-C6 alkyl, cyano-substituted C1-C6 alkyl;
$R_9$ is selected from: H, halogen;
$R_{10}$ is selected from: H, halogen, C1-C4 alkyl, C1-C4 alkyl substituted by C1-C3 alkoxy, amino-substituted C1-C4 alky, C1-C3 alkylamino-substituted C1-C4 alkyl, C1-C4 alkyl substituted by 3-8 membered heterocyclyl; or, $R_9$ and $R_{10}$ are connected to form a carbon-carbon bond;
each $R_{11}$ is independently selected from: H, halogen, -OR, -SR, $-N(R)_2$, nitro, cyano, C1-C6 alkyl, C2-C6 alkenyl, C2-C6 alkynyl, C3- C8 cycloalkyl, C1-C6 alkyl substituted by C3-C8 cycloalkyl, halogen-substituted C1-C6 alkyl, hydroxy-substituted C1-C6 alkyl, C1-C6 alkyl substituted by C1-C6 alkoxy, C1-C6 alkyl substituted by amino group, C1-C6 alkyl substituted by C1-C6 alkylamino group;
each $R_{15}$ is independently selected from: H, halogen, OR, -SR, $-N(R)_2$, C1-C6 alkyl, C3-C8 cycloalkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino-substituted C1-C6 alkyl;
each R is independently selected from: H, C1-C6 alkyl, C3-C8 cycloalkyl, C3-C8 cycloalkyl-substituted C1-C6 alkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino-substituted C1-C6 alkyl.

**2.** The Pyridopyrimidinone compounds or their pharmaceutically acceptable salts or stereoisomers or prodrug molecules according to claim 1, wherein the Pyridopyrimidinone compounds have the structure shown in Formula (III):

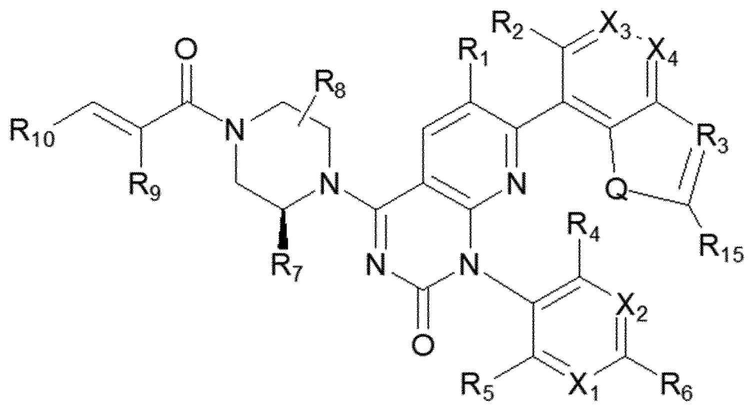

(III)

wherein,

Q is selected from: O, S;
$R_3$ is selected from: N, $CR_{15}$.

**3.** The Pyridopyrimidinone compounds or their pharmaceutically acceptable salts or stereoisomers or prodrug molecules according to claim 2, wherein $R_3$ is selected from: CH.

**4.** The Pyridopyrimidinone compounds or their pharmaceutically acceptable salts or stereoisomers or prodrug molecules according to claim 1, wherein the Pyridopyrimidinone compounds have the structure shown in Formula (IV):

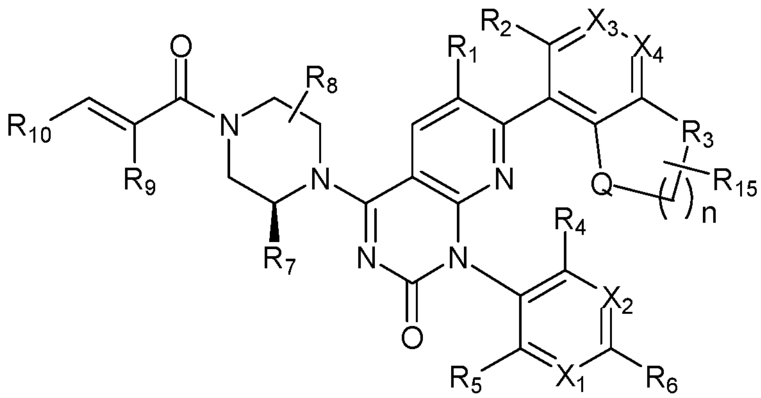

(IV)

wherein, Q is selected from: O, S;

$R_3$ is selected from: O, S, $NR_{15}$, $CHR_{15}$, $C(R_{15})_2$;
n is selected from: 1 or 2.

**5.** The Pyridopyrimidinone compounds or their pharmaceutically acceptable salts or stereoisomers or prodrug molecules according to claim 4, wherein $R_3$ is selected from: O, S, $CH_2$.

**6.** The Pyridopyrimidinone compounds or their pharmaceutically acceptable salts or stereoisomers or prodrug molecules according to claim 1, wherein Q, $R_3$ and the attached C atoms together form a 5-6 membered heterocyclic group or a heteroaryl group; the 5-6 membered heterocyclic group or the heteroaryl group combines with the six-membered aryl group to form the following structures together:

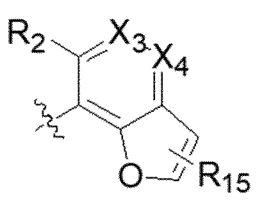
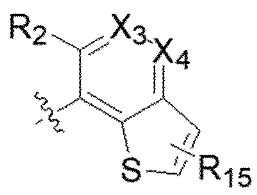
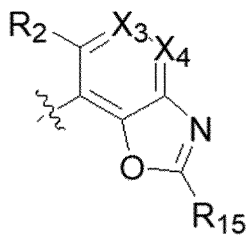
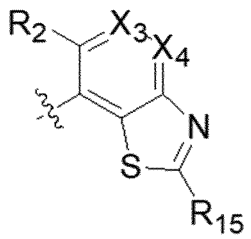
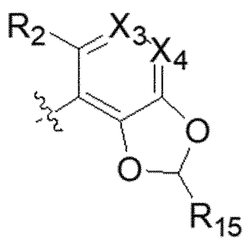
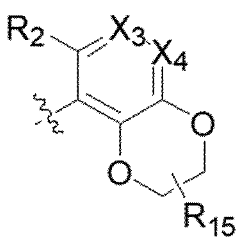
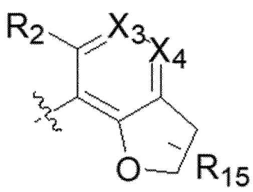
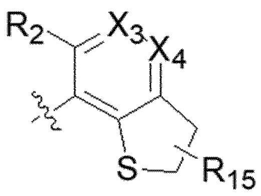

.

7. The Pyridopyrimidinone compounds or their pharmaceutically acceptable salts or stereoisomers or prodrug molecules according to claim 6, wherein the 5-6 membered heterocyclic group or the heteroaryl group combines with the six-membered aryl group to form the following structures:

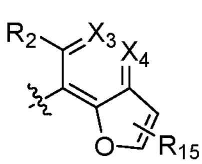
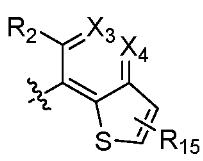
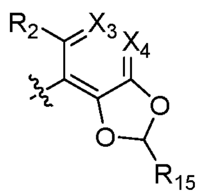
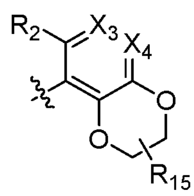
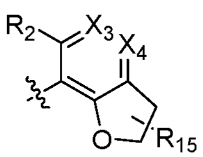
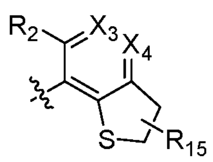

8. The Pyridopyrimidinone compounds or their pharmaceutically acceptable salts or stereoisomers or prodrug molecules according to any one of claims 1-7, wherein $X_1$ is N, $X_2$ is CH.

9. The Pyridopyrimidinone compounds or their pharmaceutically acceptable salts or stereoisomers or prodrug molecules according to any one of claims 1-7, wherein $X_1$ is CH, $X_2$ is CH, $R_4$ is cyano, and $R_5$ is C1-C6 alkyl.

10. The Pyridopyrimidinone compounds or their pharmaceutically acceptable salts or stereoisomers or prodrug molecules according to any one of claims 1-7, wherein $X_1$ is N, $X_2$ is N, $R_4$ is isopropyl, and $R_5$ is C1-C6 alkyl.

11. The Pyridopyrimidinone compounds or their pharmaceutically acceptable salts or stereoisomers or prodrug molecules according to any one of claims 1-7, wherein both $X_3$ and $X_4$ are $CR_{11}$; wherein, $R_{11}$ is selected from: H, halogen, OR, and R is selected from: H, C1-C3 alkyl.

12. The Pyridopyrimidinone compounds or their pharmaceutically acceptable salts or stereoisomers or prodrug molecules according to claim 11, wherein $X_3$ is selected from: CH, COH, CF; $X_4$ is selected from: CH, CF.

13. The Pyridopyrimidinone compounds or their pharmaceutically acceptable salts or stereoisomers or prodrug molecules according to any one of claims 1-7, wherein $R_1$ is selected from: F, Cl.

14. The Pyridopyrimidinone compounds or their pharmaceutically acceptable salts or stereoisomers or prodrug molecules according to any one of claims 1-7, wherein $R_2$ is selected from: F, Cl.

**15.** The Pyridopyrimidinone compounds or their pharmaceutically acceptable salts or stereoisomers or prodrug molecules according to any one of claims 1-7, wherein $R_4$ and $R_5$ are independently selected from: H, C1-C3 alkyl, C3-C6 cycloalkyl, cyano, and hydroxy-substituted C1-C3 alkyl.

**16.** The Pyridopyrimidinone compounds or their pharmaceutically acceptable salts or stereoisomers or prodrug molecules according to claim 15, wherein $R_4$ is selected from: methyl, isopropyl, cyano; $R_5$ is selected from: isopropyl.

**17.** The Pyridopyrimidinone compounds or their pharmaceutically acceptable salts or stereoisomers or prodrug molecules according to any one of claims 1-7, wherein $R_6$ is selected from: H, C1-C3 alkyl.

**18.** The Pyridopyrimidinone compounds or their pharmaceutically acceptable salts or stereoisomers or prodrug molecules according to any one of claims 1-7, wherein $R_7$ and $R_8$ are independently selected from: H, C1-C3 alkyl, cyano-substituted C1-C3 alkyl.

**19.** The Pyridopyrimidinone compounds or their pharmaceutically acceptable salts or stereoisomers or prodrug molecules according to claim 18, wherein $R_7$ is selected from: methyl; $R_8$ is selected from: H, methyl, cyanomethyl.

**20.** The Pyridopyrimidinone compounds or their pharmaceutically acceptable salts or stereoisomers or prodrug molecules according to any one of claims 1-7, wherein $R_9$ and $R_{10}$ are independently selected from: H, halogen.

**21.** The Pyridopyrimidinone compounds or their pharmaceutically acceptable salts or stereoisomers or prodrug molecules according to any one of claims 1-7, wherein $R_{15}$ is selected from: H.

**22.** The Pyridopyrimidinone compounds or their pharmaceutically acceptable salts or stereoisomers or prodrug molecules according to any one of claims 1-7, wherein

$X_1$ and $X_2$ are independently selected from: N or CH;
$X_3$ and $X_4$ are independently selected from: N or CR11;
$R_1$, $R_2$ are independently selected from: F, Cl;
$R_4$, $R_5$ are independently selected from: H, C1-C6 alkyl, C2-C6 alkenyl, C3-C8 cycloalkyl, cyano;
$R_6$ is selected from: H, halogen, OR, C1-C6 alkyl;
$R_7$ is selected from: C1-C6 alkyl;
$R_8$ is selected from: H, C1-C6 alkyl, cyano-substituted C1-C6 alkyl;
$R_9$ is selected from: H;
$R_{10}$ is selected from: H;
each $R_{11}$ is independently selected from: H, halogen, OR, -SR, -N(R)$_2$, C1-C6 alkyl, C3-C8 cycloalkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkylamino-substituted C1-C6 alkyl;
each R is independently selected from: H, C1-C6 alkyl, halogen-substituted C1-C6 alkyl, hydroxy-substituted C1-C6 alkyl, C1-C6 alkoxy-substituted C1-C6 alkyl, amino-substituted C1-C6 alkyl, C1-C6 alkyl substituted by C1-C6 alkylamino.

**23.** The Pyridopyrimidinone compounds or their pharmaceutically acceptable salts or stereoisomers or prodrug molecules according to claim 1, **characterized in that** they are selected from the following compounds:

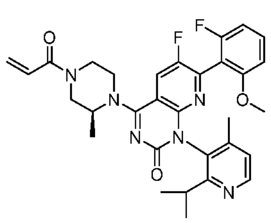
compound 1

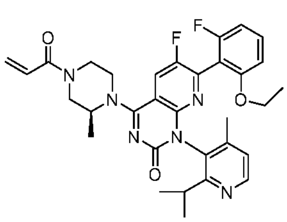
compound 2

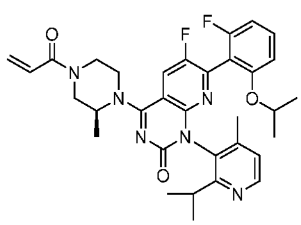
compound 3

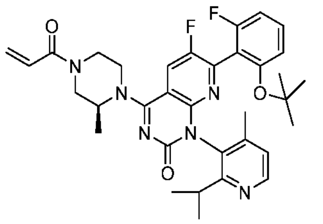

compound 4

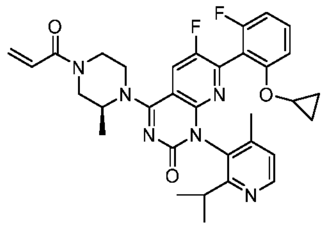

compound 5

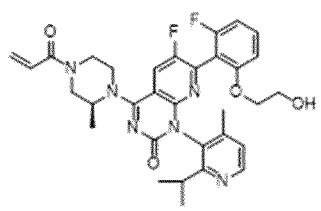

compound 6

compound 7

compound 8

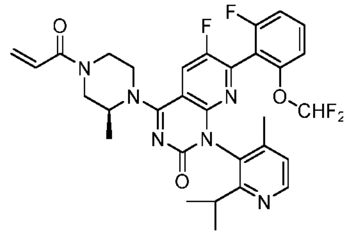

compound 9

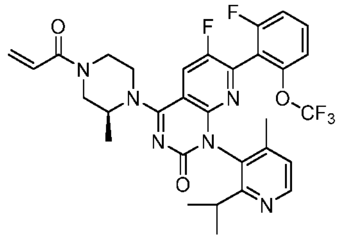

compound 10

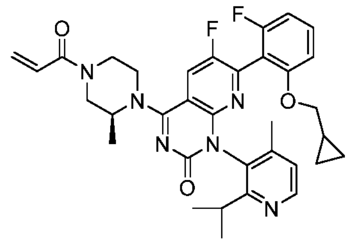

compound 11

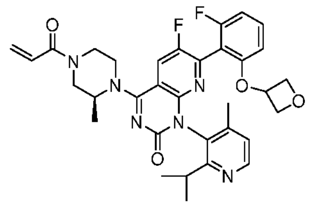

compound 12

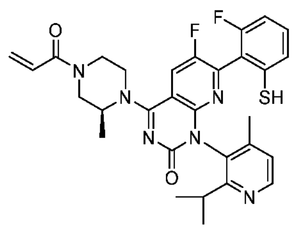

compound 13

compound 14

compound 15

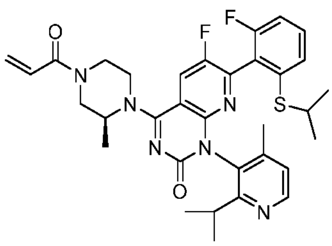

compound 16

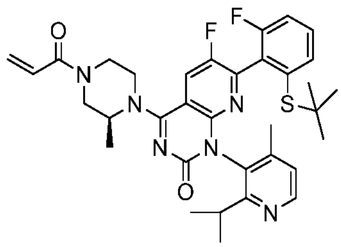

compound 17

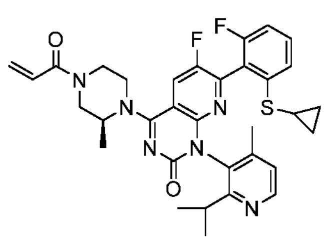

compound 18

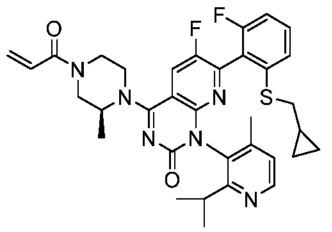
compound 19

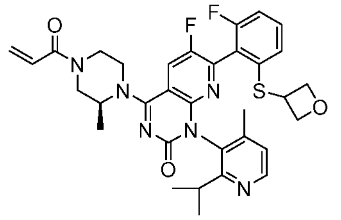
compound 20

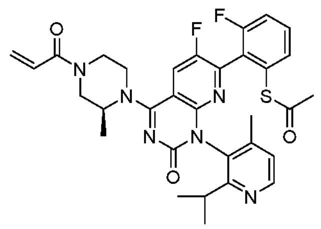
compound 21

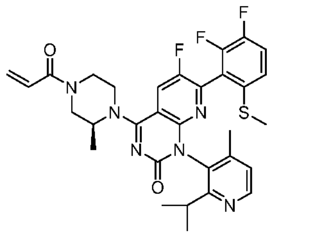
compound 22

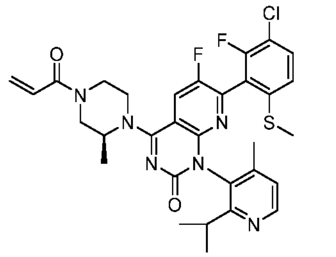
compound 23

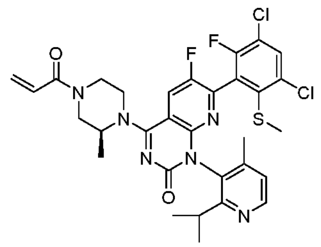
compound 24

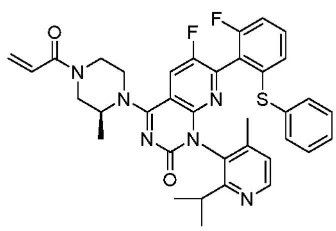
compound 25

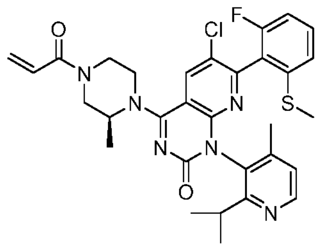
compound 26

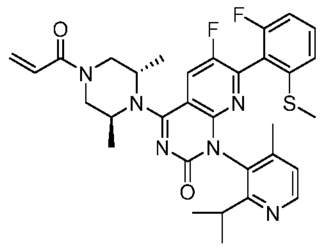
compound 27

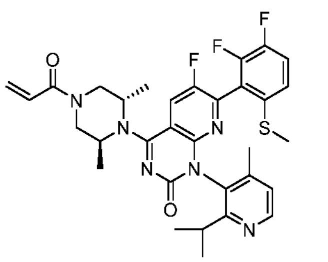
compound 28

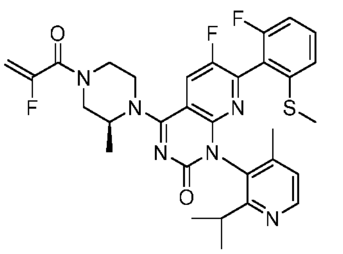
compound 29

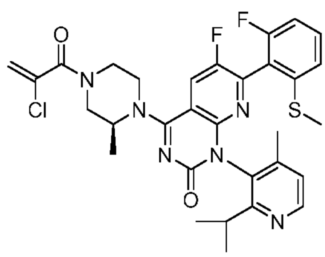
compound 30

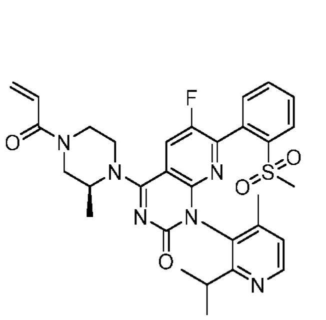

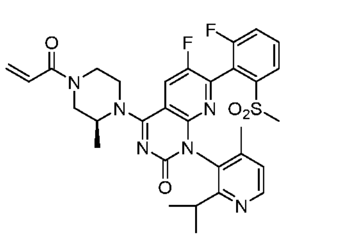
compound 32

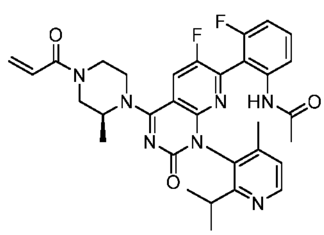
compound 33

compound 31

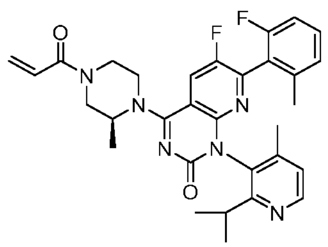

compound 34

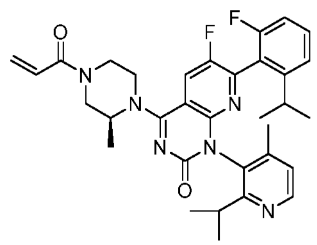

compound 35

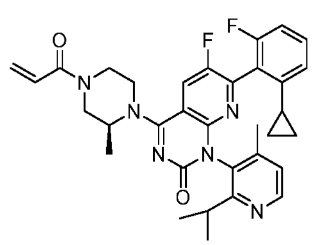

compound 36

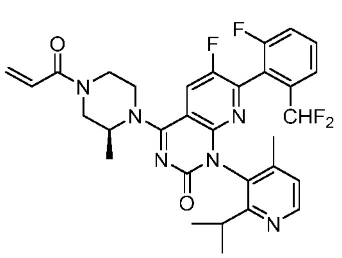

compound 37

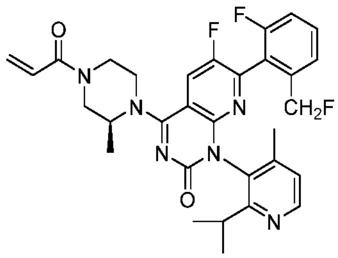

compound 38

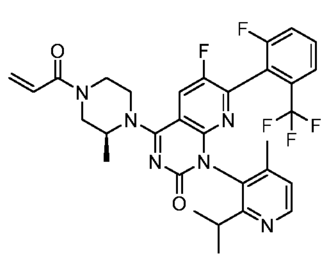

compound 39

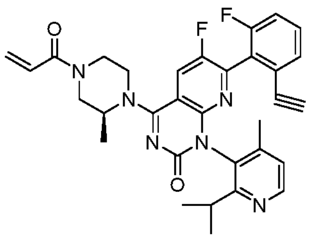

compound 40

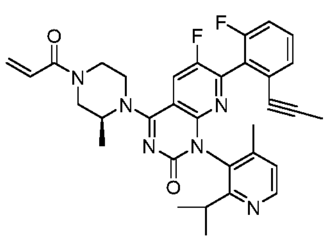

compound 41

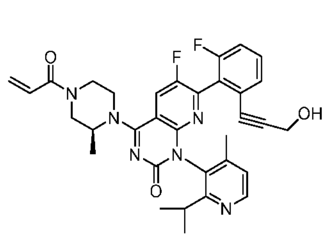

compound 42

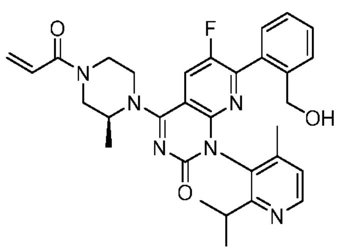

compound 43

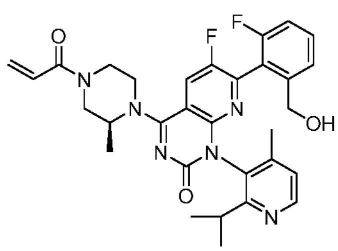

compound 44

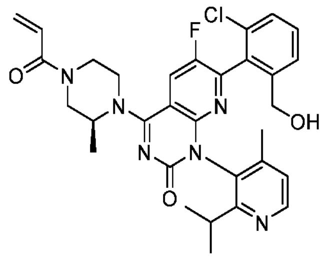

compound 45

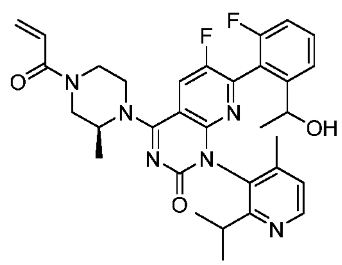

compound 46

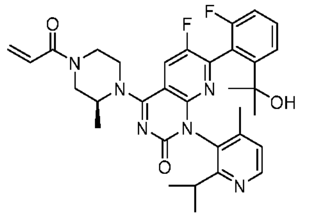

compound 47

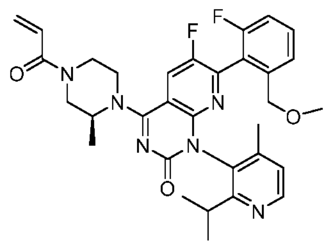

compound 48

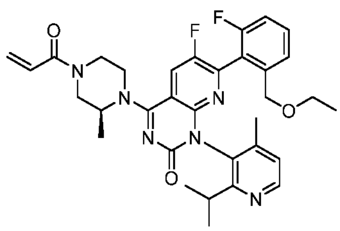

compound 49

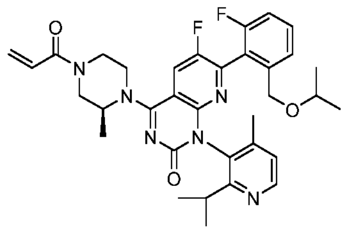

compound 50

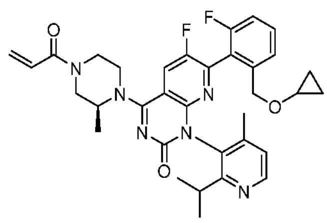

compound 51

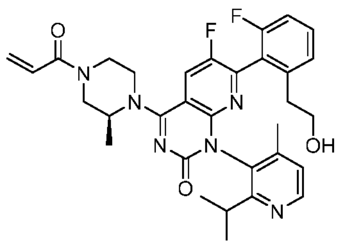

compound 52

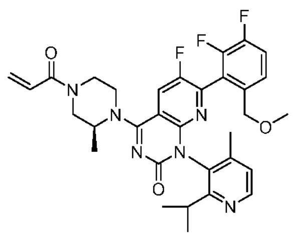

compound 53

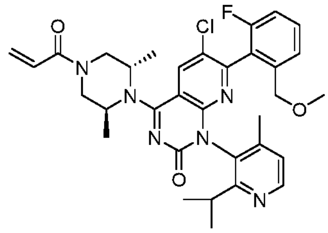

compound 54

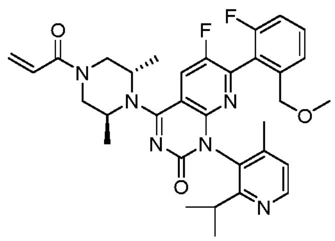

compound 55

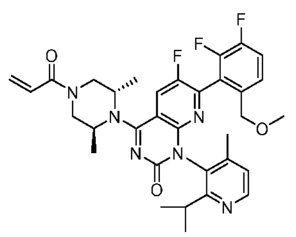

compound 56

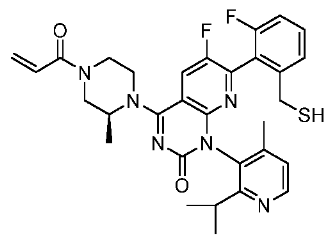

compound 57

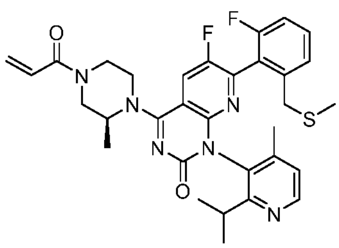

compound 58

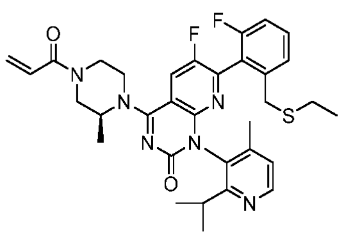

compound 59

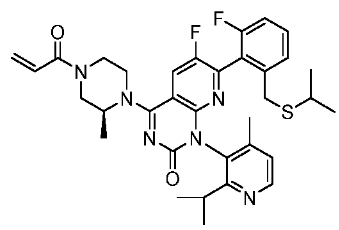

compound 60

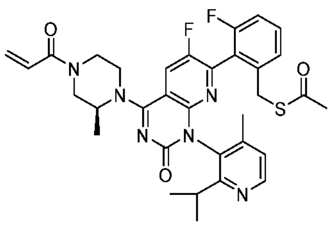

compound 61

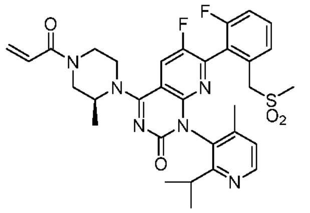

compound 62

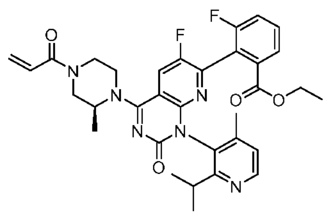

compound 63

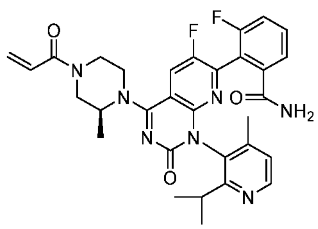

compound 64

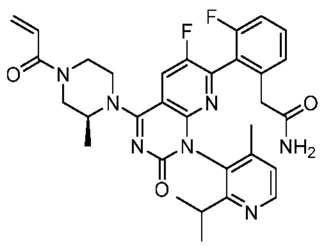

compound 65

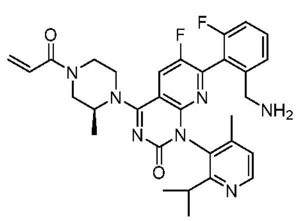

compound 66

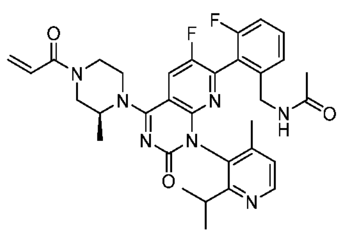

compound 67

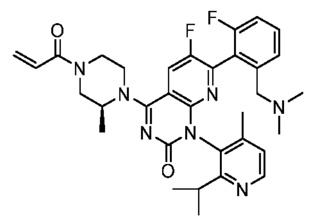

compound 68

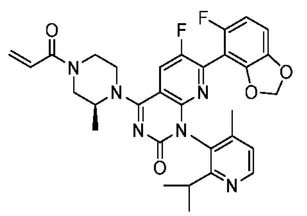

compound 69

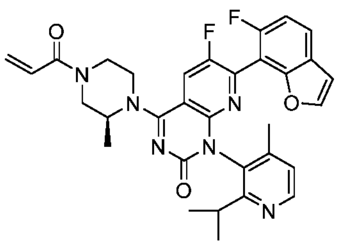

compound 70

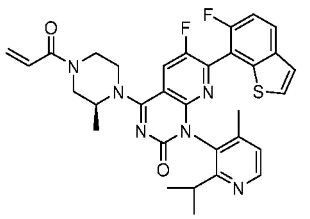

compound 71

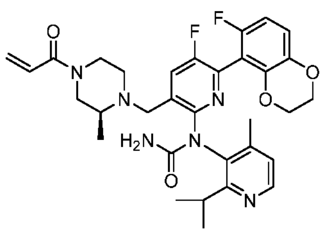

compound 72

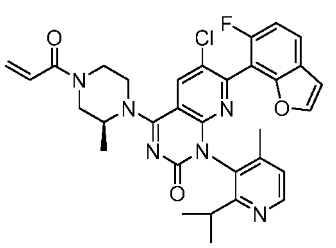

compound 73

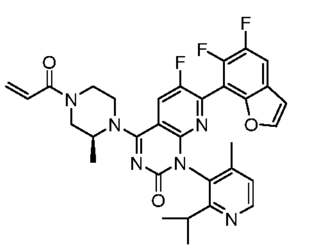

compound 74

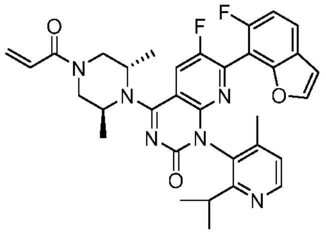

compound 75

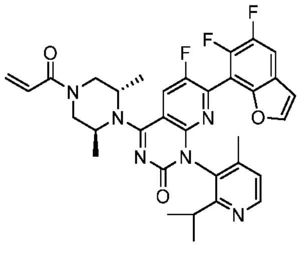
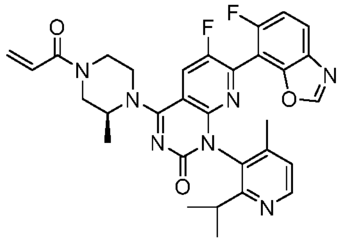
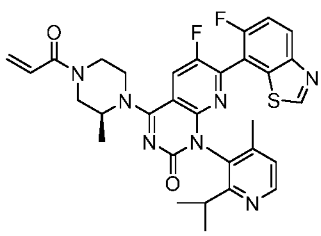

compound 76 compound 77 compound 78

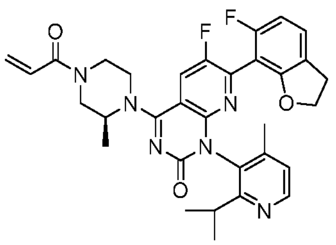
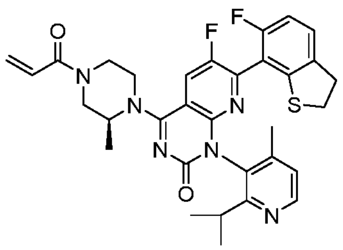
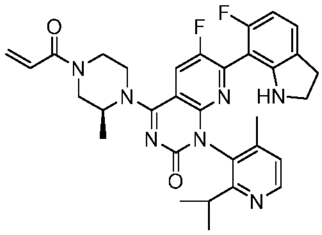

compound 81

compound 79 compound 80

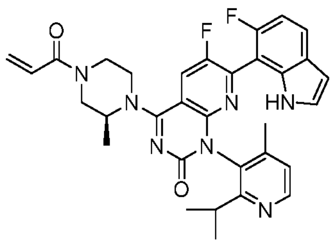
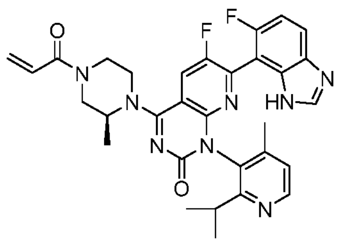
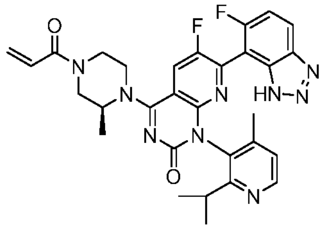

compound 84

compound 82 compound 83

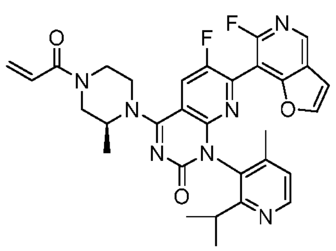
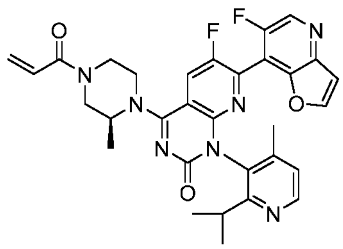
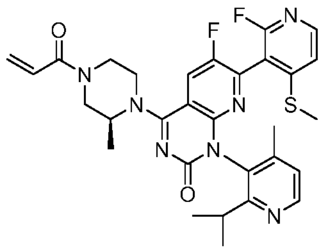

compound 85 compound 86 compound 87

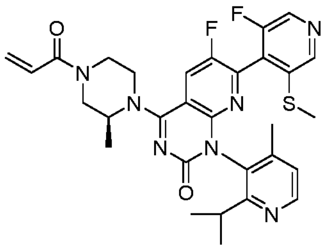

compound 88

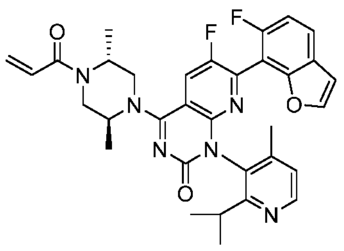

compound 89

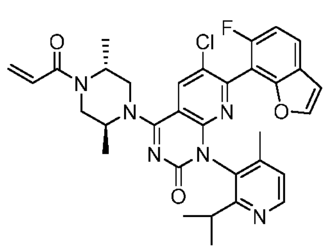

compound 90

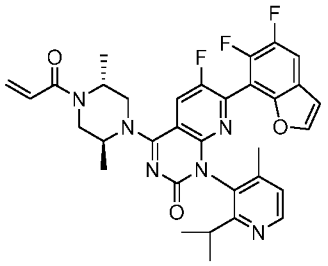

compound 91

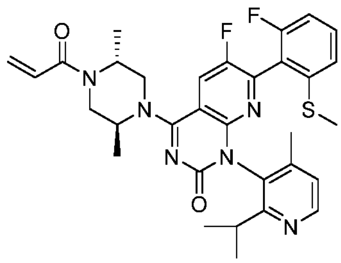

compound 92

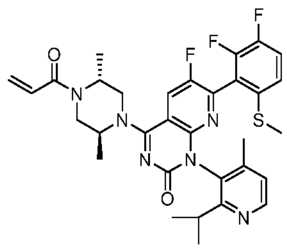

compound 93

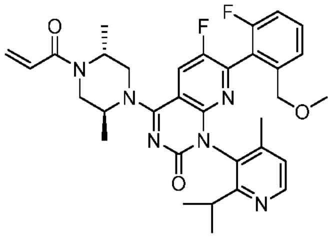

compound 94

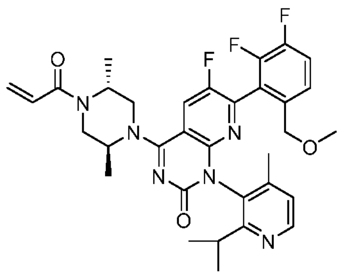

compound 95

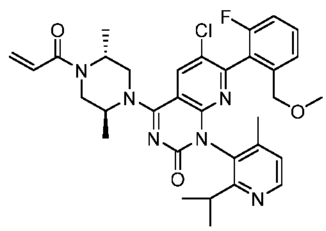

compound 96

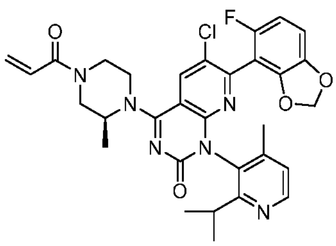

compound 97

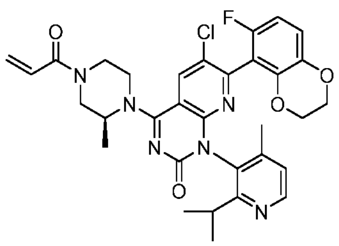

compound 98

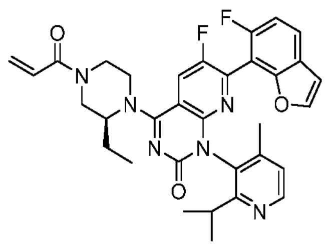

compound 99

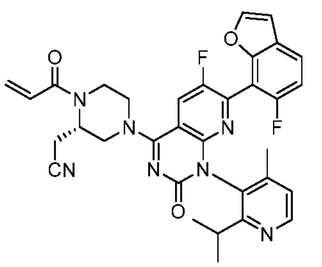

compound 100

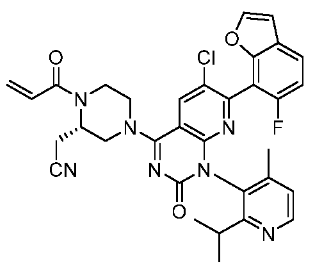

compound 101

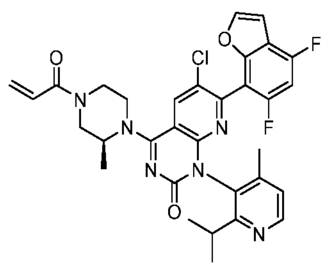

compound 102

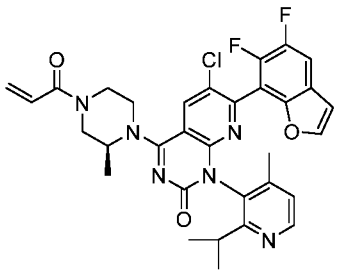

compound 103

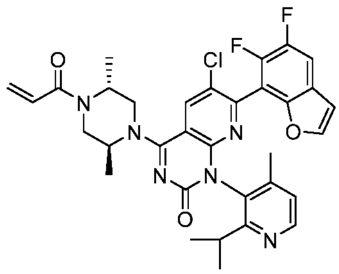

compound 104

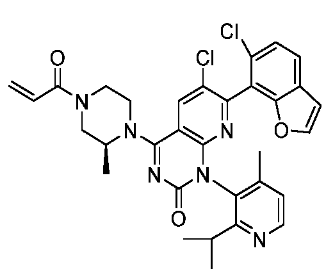

compound 105

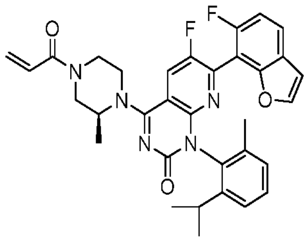

compound 106

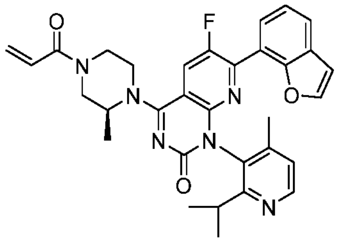

compound 107

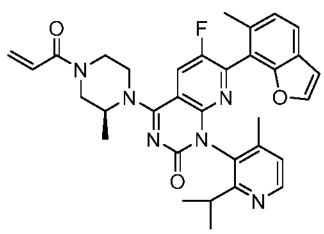

compound 108

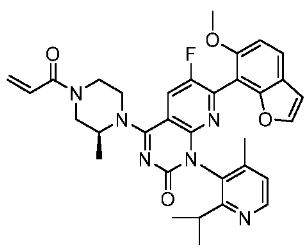

compound 109

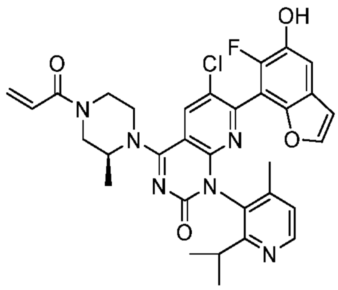

compound 110

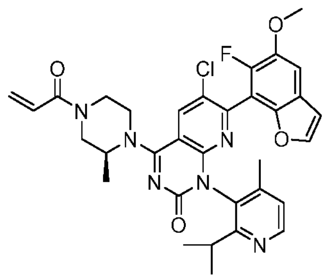

compound 111

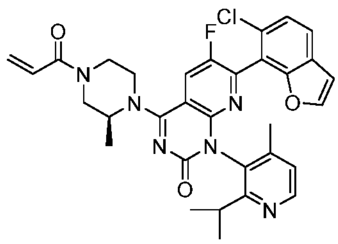

compound 112

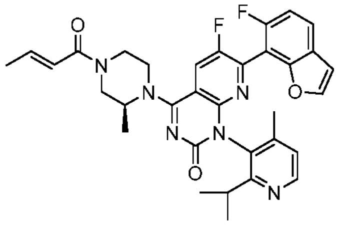

compound 113

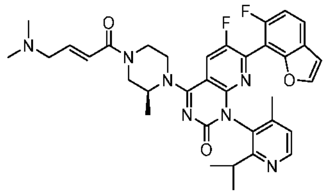

compound 114

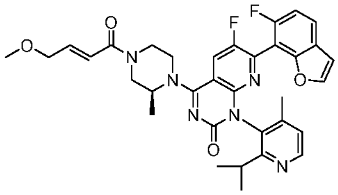

compound 115

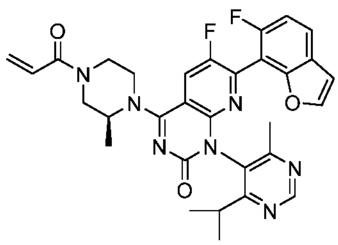

compound 116

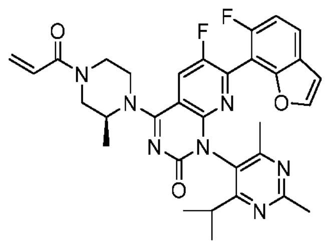

compound 117

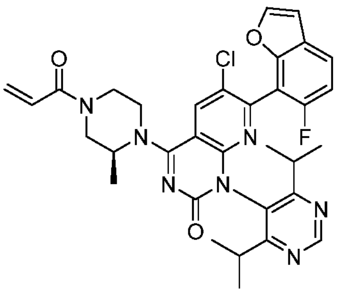
compound 118

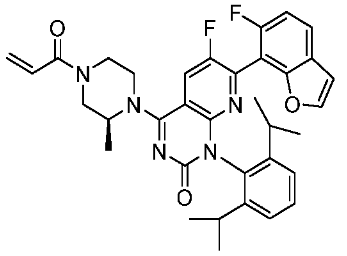
compound 119

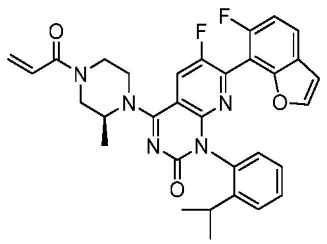
compound 120

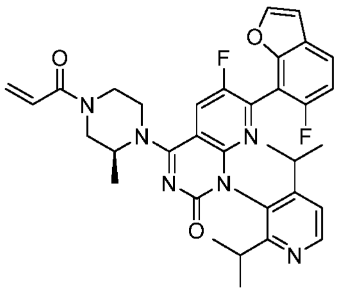
compound 121

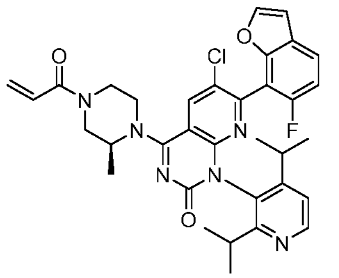
compound 122

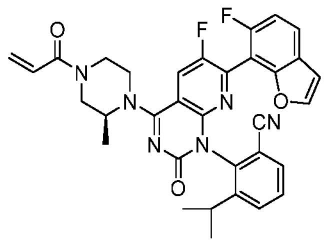
compound 123

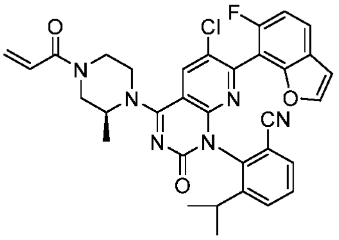
compound 124

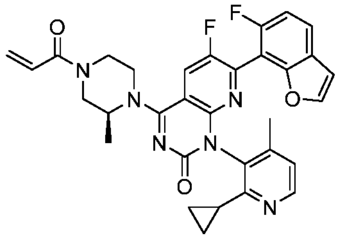
compound 125

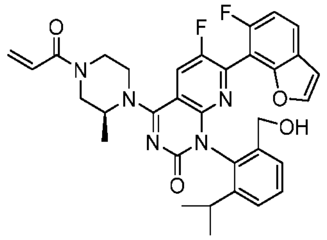
compound 126

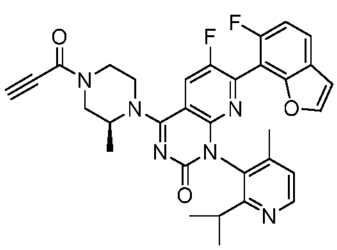
compound 127

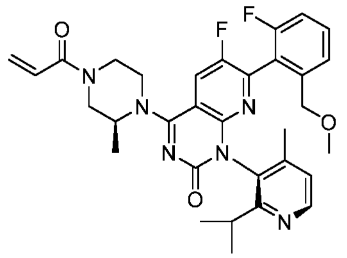
compound 48-1

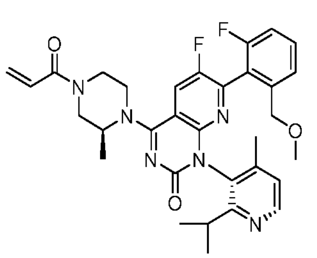
compound 48-2

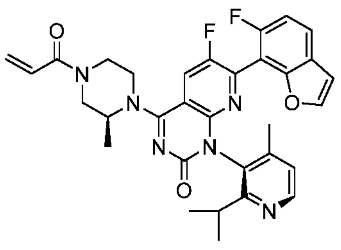

compound 70-1

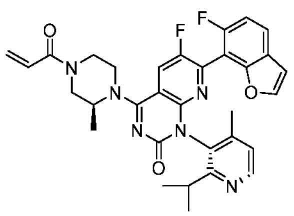

compound 70-2

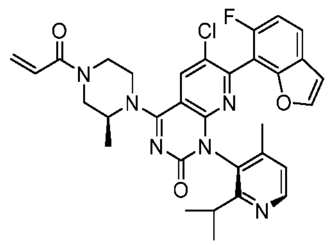

compound 73-1

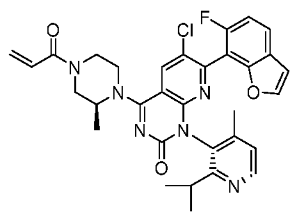

compound 73-2

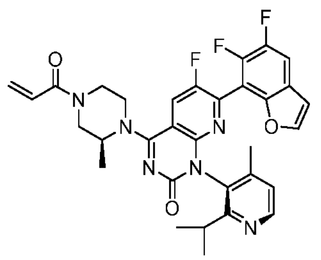

compound 74-1

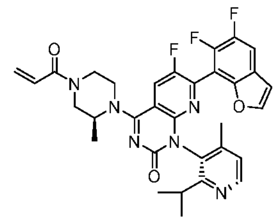

compound 74-2

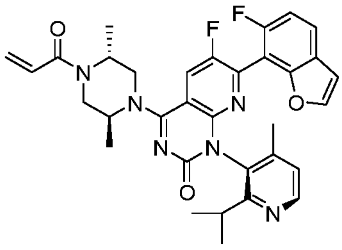

compound 89-1

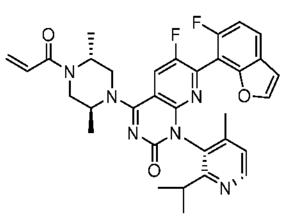

compound 89-2

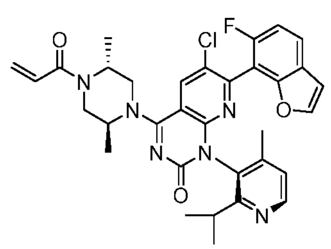

compound 90-1

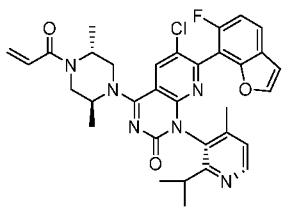

compound 90-2

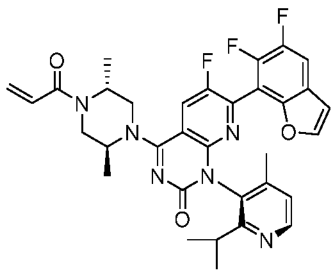

compound 91-1

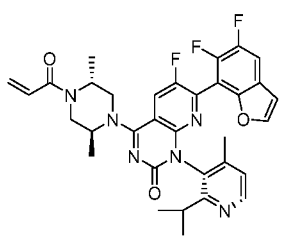

compound 91-2

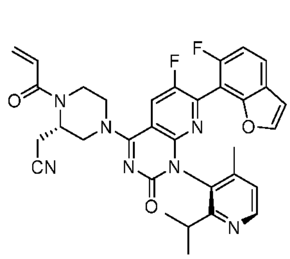

compound 100-1

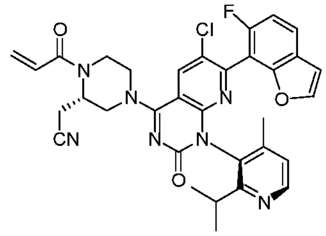

compound 101-1

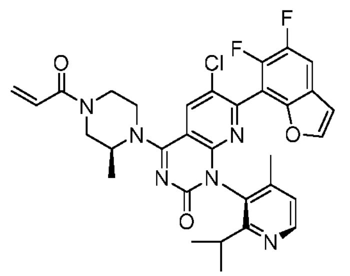

compound 103-1

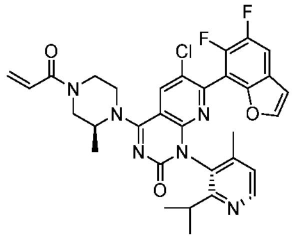

compound 103-2

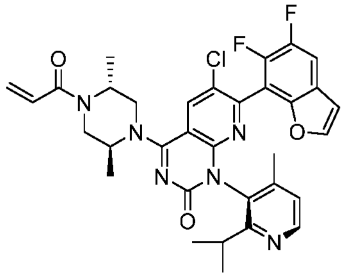

compound 104-1

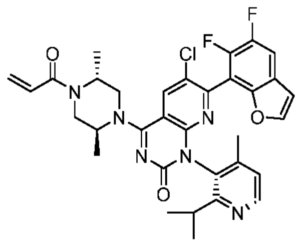

compound 104-2.

**24.** An application of the Pyridopyrimidinone compounds or their pharmaceutically acceptable salts or stereoisomers or prodrug molecules according to any one of claims 1-23 in the preparation of mutant KRAS inhibitors.

**25.** An application of the Pyridopyrimidinone compounds or their pharmaceutically acceptable salts or stereoisomers or prodrug molecules according to any one of claims 1-23 in the preparation of KRAS G12C inhibitors.

**26.** An application of the Pyridopyrimidinone compounds or their pharmaceutically acceptable salts or stereoisomers or prodrug molecules according to any one of claims 1-23 in the preparation of medicaments for preventing and/or treating tumors.

**27.** The application according to claim 26, wherein the tumor is a hematological tumor or a solid tumor haboring KRAS G12C gene mutation.

**28.** The application according to claim 27, wherein the tumor is: non-small cell lung cancer, small cell lung cancer, lymphoma, esophageal cancer, ovarian cancer, melanoma, cervical cancer, urothelial cancer, pancreatic cancer, breast cancer, liver cancer, stomach cancer, bile duct cancer, leukemia, melanoma, colon cancer, rectal cancer, endometrial cancer or glioma.

**29.** An inhibitor against KRAS mutant, wherein its active ingredient comprises the Pyridopyrimidinone compounds or their pharmaceutically acceptable salts or stereoisomers or prodrug molecules according to any one of claims 1-23.

**30.** A pharmaceutical composition for preventing and/or treating tumors, comprising an active ingredient and a pharmaceutically acceptable excipient and/or carrier, wherein the active ingredient comprises the Pyridopyrimidinone compounds or their pharmaceutically acceptable salts or stereoisomers or prodrug molecules according to any one of claims 1-23.

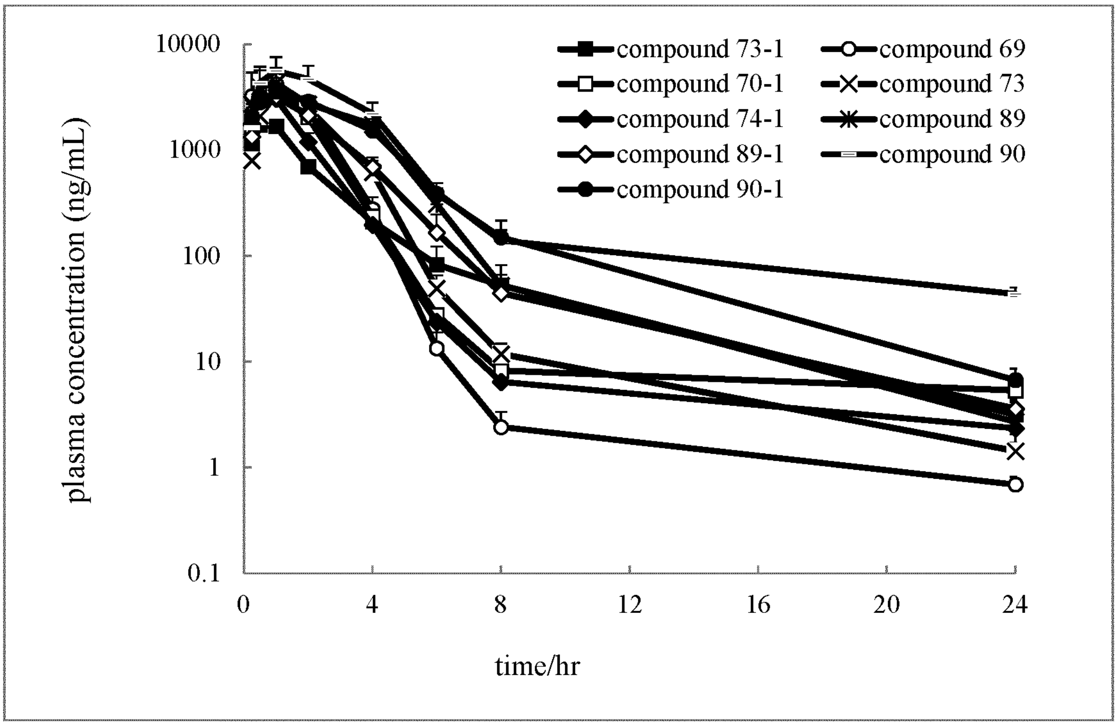
10000
1000
100
10
1
0.1
plasma concentration (ng/mL)
0
4
8
12
16
20
24
time/hr
compound 73-1
compound 69
compound 70-1
compound 73
compound 74-1
compound 89
compound 89-1
compound 90
compound 90-1

Fig. 1

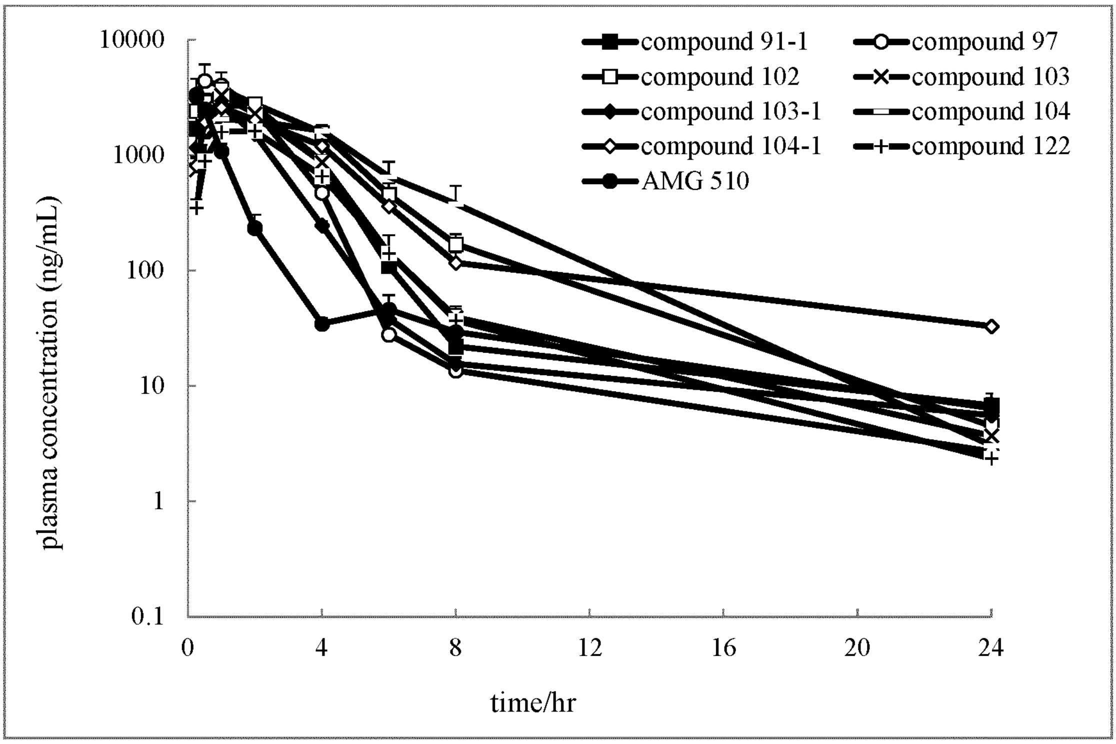
10000
1000
100
10
1
0.1
plasma concentration (ng/mL)
0
4
8
12
16
20
24
time/hr
compound 91-1
compound 97
compound 102
compound 103
compound 103-1
compound 104
compound 104-1
compound 122
AMG 510

Fig. 2

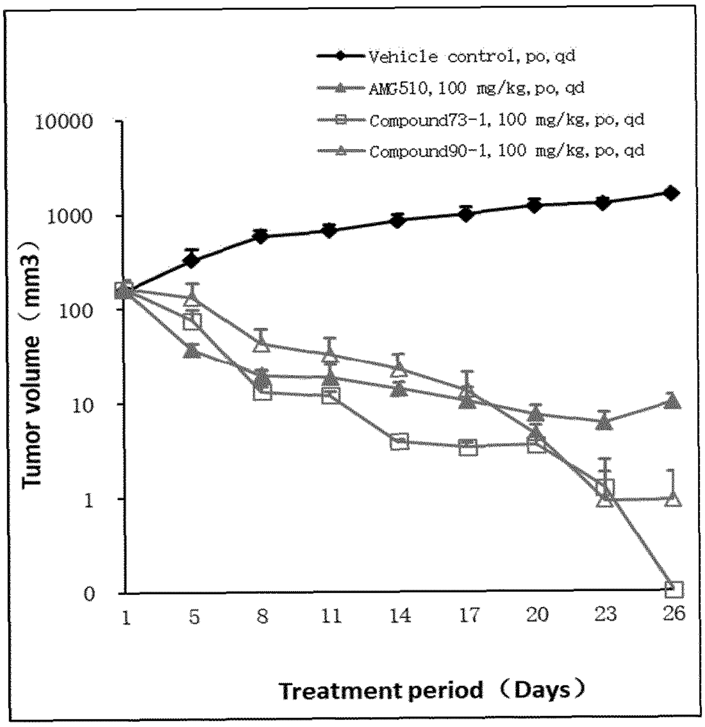
Vehicle control, po, qd
AMG510, 100 mg/kg, po, qd
Compound73-1, 100 mg/kg, po, qd
Compound90-1, 100 mg/kg, po, qd
10000
1000
100
10
1
0
Tumor volume (mm3)
1
5
8
11
14
17
20
23
26
Treatment period (Days)

Fig. 3

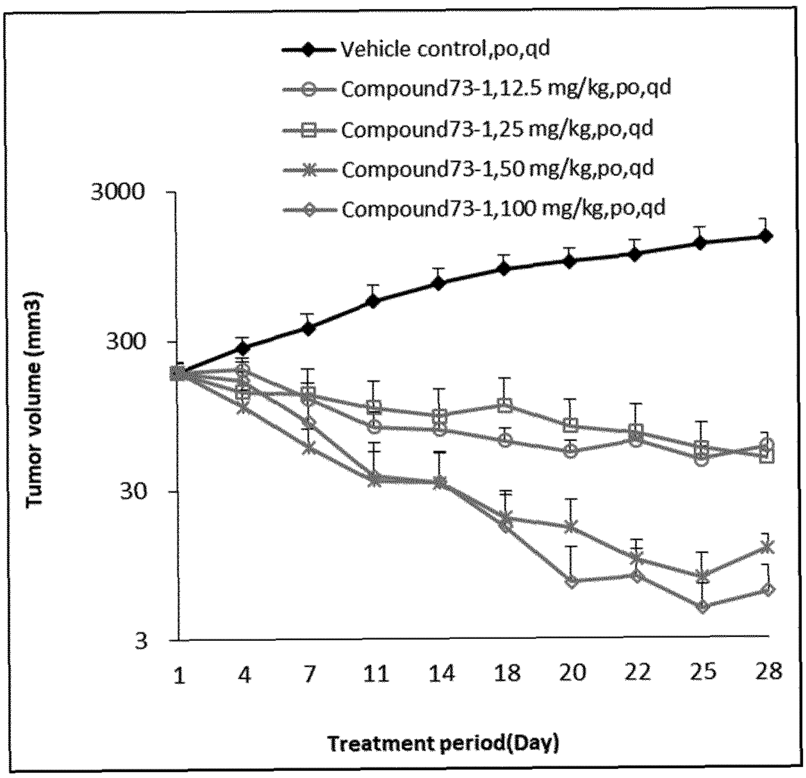
Vehicle control,po,qd
Compound73-1,12.5 mg/kg,po,qd
Compound73-1,25 mg/kg,po,qd
Compound73-1,50 mg/kg,po,qd
Compound73-1,100 mg/kg,po,qd
3000
300
30
3
Tumor volume (mm3)
1
4
7
11
14
18
20
22
25
28
Treatment period(Day)

Fig. 4

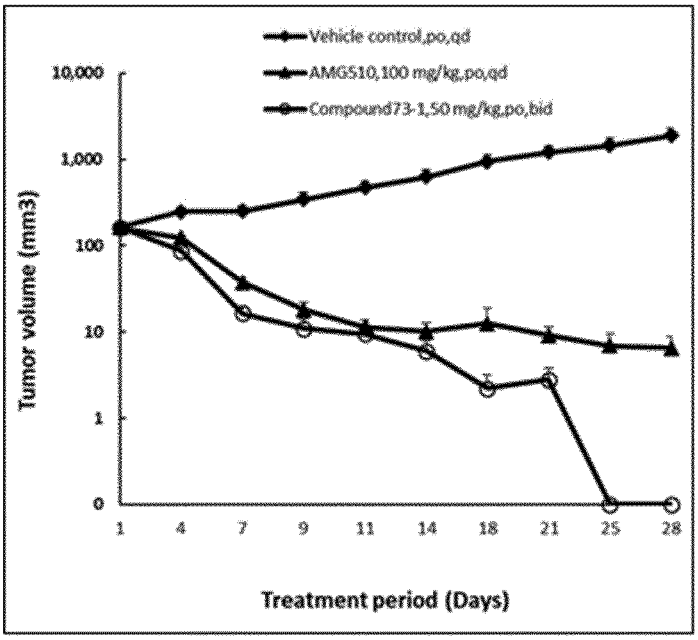
Vehicle control,po,qd
AMG510,100 mg/kg,po,qd
Compound73-1,50 mg/kg,po,bid
10,000
1,000
100
10
1
0
Tumor volume (mm3)
1
4
7
9
11
14
18
21
25
28
Treatment period (Days)

Fig. 5

**INTERNATIONAL SEARCH REPORT**

International application No. **PCT/CN2020/136848**

**A. CLASSIFICATION OF SUBJECT MATTER**

C07D 471/04(2006.01)i; A61K 31/519(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D471/-, A61K31/-, A61P35/-

Documentation searched other than minimum documentation to the extent that such documents are included in the fields searched

Electronic data base consulted during the international search (name of data base and, where practicable, search terms used)

CNPAT, CNKI, WPI, EPODOC, ISI Web of Knowledge, Registry, Caplus: 广州必贝特医, 吡啶, 嘧啶酮, 癌症, 肿瘤, 突变, 抑制剂, KRAS G12C, pyridine, pyrimidinone, cancer, tumor, mutation, inhibitor

**C. DOCUMENTS CONSIDERED TO BE RELEVANT**

| Category* | Citation of document, with indication, where appropriate, of the relevant passages | Relevant to claim No. |
|---|---|---|
| X | CN 110366550 A (AMGEN INC.) 22 October 2019 (2019-10-22) the abstract, claims 3, 4, 51 and 86-90, and description, paragraph [0377], and page 62, compounds 9-12 | 1-30 |
| X | WO 2018217651 A1 (AMGEN INC.) 29 November 2018 (2018-11-29) description, paragraphs [0005]-[0008], and table 1, compounds 9-12 | 1-30 |
| X | US 2019374542 A1 (AMGEN INC.) 12 December 2019 (2019-12-12) the abstract, and page 17, compound 6, page 21, compound 2, page 27, compound 3, page 79, compound 4, page 83, compound 5, page 89, compound 1, table 92, compounds 92-2, 92-11, 92-15 and 92-30, and table 94 compounds 94-14; D4, page 700, compound 8, page 709, compound 4, and page 722, compound 10 | 1-30 |
| X | WO 2019213516 A1 (AMGEN INC.) 07 November 2019 (2019-11-07) the abstract, and page 700, compound 8, page 709, compound 4, and page 722, compound 10 | 1-30 |
| PX | US 2020055845 A1 (AMGEN INC.) 20 February 2020 (2020-02-20) the abstract, and description, paragraphs [0025]-[0053] | 1-30 |
| PX | US 2020069657 A1 (AMGEN INC.) 05 March 2020 (2020-03-05) the abstract, and description, paragraphs [0024]-[0053] | 1-30 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **23 February 2021** | **17 March 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/136848**

**C. DOCUMENTS CONSIDERED TO BE RELEVANT**

| Category* | Citation of document, with indication, where appropriate, of the relevant passages | Relevant to claim No. |
|---|---|---|
| PX | WO 2020233592 A1 (INVENTISBIO SHANGHAI LTD.) 26 November 2020 (2020-11-26) the abstract, claims 1-41, and page 34, compounds 37 and 49, and page 36, compound 114 | 1-30 |
| PX | WO 2020239077 A1 (SHANGHAI HANSOH BIOMEDICAL CO., LTD. et al.) 03 December 2020 (2020-12-03) the abstract, and claims 1-42 | 1-30 |

Form PCT/ISA/210 (second sheet) (January 2015)

**INTERNATIONAL SEARCH REPORT**
**Information on patent family members**

International application No.
**PCT/CN2020/136848**

| Patent document cited in search report | | | Publication date (day/month/year) | Patent family member(s) | | | Publication date (day/month/year) |
|---|---|---|---|---|---|---|---|
| CN | 110366550 | A | 22 October 2019 | AU | 2017379074 | A1 | 18 July 2019 |
| | | | | SG | 11201906223T | A | 27 August 2019 |
| | | | | CL | 2020000784 | A1 | 28 August 2020 |
| | | | | IL | 267495 | D0 | 29 August 2019 |
| | | | | EP | 3558955 | A2 | 30 October 2019 |
| | | | | SG | 10201913491P | A | 30 March 2020 |
| | | | | PE | 20191207 | A1 | 10 September 2019 |
| | | | | CL | 2019001762 | A1 | 18 October 2019 |
| | | | | US | 2020069657 | A1 | 05 March 2020 |
| | | | | JP | 2020504738 | A | 13 February 2020 |
| | | | | CR | 20190338 | A | 09 September 2019 |
| | | | | US | 2018177767 | A1 | 28 June 2018 |
| | | | | KR | 20190097201 | A | 20 August 2019 |
| | | | | PH | 12019501670 | A1 | 04 November 2019 |
| | | | | US | 10532042 | B2 | 14 January 2020 |
| | | | | CA | 3048217 | A1 | 28 June 2018 |
| | | | | WO | 2018119183 | A2 | 28 June 2018 |
| | | | | MX | 2019007643 | A | 09 September 2019 |
| | | | | BR | 112019012976 | A2 | 31 December 2019 |
| | | | | WO | 2018119183 | A3 | 23 August 2018 |
| | | | | ZA | 201904548 | B | 25 March 2020 |
| | | | | CO | 2019006598 | A2 | 28 June 2019 |
| | | | | EA | 201991528 | A1 | 16 January 2020 |
| WO | 2018217651 | A1 | 29 November 2018 | KR | 20200010384 | A | 30 January 2020 |
| | | | | JP | 6785819 | B2 | 18 November 2020 |
| | | | | CA | 3063469 | A1 | 29 November 2018 |
| | | | | BR | 112019024525 | A2 | 09 June 2020 |
| | | | | CR | 20190534 | A | 20 March 2020 |
| | | | | US | 2020055845 | A1 | 20 February 2020 |
| | | | | EA | 201992781 | A1 | 01 April 2020 |
| | | | | MX | 2019013858 | A | 20 January 2020 |
| | | | | AU | 2018273356 | A1 | 21 November 2019 |
| | | | | PE | 20200733 | A1 | 23 July 2020 |
| | | | | CO | 2019013010 | A2 | 01 April 2020 |
| | | | | EP | 3630761 | A1 | 08 April 2020 |
| | | | | AR | 111882 | A1 | 28 August 2019 |
| | | | | CL | 2019003394 | A1 | 13 April 2020 |
| | | | | US | 2018334454 | A1 | 22 November 2018 |
| | | | | CN | 110997668 | A | 10 April 2020 |
| | | | | JP | 2019031476 | A | 28 February 2019 |
| | | | | UY | 37744 | A | 02 January 2019 |
| | | | | PH | 12019502579 | A1 | 13 July 2020 |
| | | | | TW | 201906821 | A | 16 February 2019 |
| | | | | SG | 10201913195R | A | 27 February 2020 |
| | | | | US | 10519146 | B2 | 31 December 2019 |
| US | 2019374542 | A1 | 12 December 2019 | WO | 2020050890 | A2 | 12 March 2020 |
| | | | | WO | 2020050890 | A3 | 25 June 2020 |
| WO | 2019213516 | A1 | 07 November 2019 | CA | 3098574 | A1 | 07 November 2019 |
| | | | | AU | 2019262589 | A1 | 29 October 2020 |
| | | | | US | 2019343838 | A1 | 14 November 2019 |

Form PCT/ISA/210 (patent family annex) (January 2015)

**INTERNATIONAL SEARCH REPORT**
**Information on patent family members**

International application No.
**PCT/CN2020/136848**

| Patent document cited in search report | Publication date (day/month/year) | Patent family member(s) | Publication date (day/month/year) |
|---|---|---|---|
| US 2020055845 A1 | 20 February 2020 | KR 20200010384 A | 30 January 2020 |
| | | JP 6785819 B2 | 18 November 2020 |
| | | CA 3063469 A1 | 29 November 2018 |
| | | BR 112019024525 A2 | 09 June 2020 |
| | | CR 20190534 A | 20 March 2020 |
| | | EA 201992781 A1 | 01 April 2020 |
| | | MX 2019013858 A | 20 January 2020 |
| | | AU 2018273356 A1 | 21 November 2019 |
| | | PE 20200733 A1 | 23 July 2020 |
| | | CO 2019013010 A2 | 01 April 2020 |
| | | EP 3630761 A1 | 08 April 2020 |
| | | AR 111882 A1 | 28 August 2019 |
| | | CL 2019003394 A1 | 13 April 2020 |
| | | US 2018334454 A1 | 22 November 2018 |
| | | CN 110997668 A | 10 April 2020 |
| | | JP 2019031476 A | 28 February 2019 |
| | | WO 2018217651 A1 | 29 November 2018 |
| | | UY 37744 A | 02 January 2019 |
| | | PH 12019502579 A1 | 13 July 2020 |
| | | TW 201906821 A | 16 February 2019 |
| | | SG 10201913195R A | 27 February 2020 |
| | | US 10519146 B2 | 31 December 2019 |
| US 2020069657 A1 | 05 March 2020 | AU 2017379074 A1 | 18 July 2019 |
| | | SG 11201906223T A | 27 August 2019 |
| | | CL 2020000784 A1 | 28 August 2020 |
| | | IL 267495 D0 | 29 August 2019 |
| | | EP 3558955 A2 | 30 October 2019 |
| | | SG 10201913491P A | 30 March 2020 |
| | | PE 20191207 A1 | 10 September 2019 |
| | | CL 2019001762 A1 | 18 October 2019 |
| | | JP 2020504738 A | 13 February 2020 |
| | | CR 20190338 A | 09 September 2019 |
| | | US 2018177767 A1 | 28 June 2018 |
| | | KR 20190097201 A | 20 August 2019 |
| | | PH 12019501670 A1 | 04 November 2019 |
| | | US 10532042 B2 | 14 January 2020 |
| | | CA 3048217 A1 | 28 June 2018 |
| | | WO 2018119183 A2 | 28 June 2018 |
| | | CN 110366550 A | 22 October 2019 |
| | | MX 2019007643 A | 09 September 2019 |
| | | BR 112019012976 A2 | 31 December 2019 |
| | | WO 2018119183 A3 | 23 August 2018 |
| | | ZA 201904548 B | 25 March 2020 |
| | | CO 2019006598 A2 | 28 June 2019 |
| | | EA 201991528 A1 | 16 January 2020 |
| WO 2020233592 A1 | 26 November 2020 | None | |
| WO 2020239077 A1 | 03 December 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2018334454 A1


### Non-patent literature cited in the description


- *Cell,* 2017, vol. 170, 17-33
- *Cell,* 1993, vol. 74, 205-214
- *Nature,* 1994, vol. 370, 527-532
- *EMBO J,* 1996, vol. 15, 5256-5267
- *Nature,* 2019, vol. 575, 217-223
- *C&EN,* 2016, vol. 94 (23), 28-33
- *Nat. Rev. Drug Discov,* 2014, vol. 13, 828-851
- *Nat Rev Drug Discov,* 2016, vol. 15, 771-785
- **BERG et al.** Pharmaceutical Salts. *J. Pharm. Sci.,* 1977, vol. 66, 1-19